(19) Europäisches Patentamt
European Patent Office
Office européen des brevets

(11) EP 1 752 143 A1

(12) EUROPEAN PATENT APPLICATION

(43) Date of publication:
**14.02.2007 Bulletin 2007/07**

(21) Application number: **05291692.1**

(22) Date of filing: **08.08.2005**

(51) Int Cl.:
***A61K 31/00*** *(2006.01)*
***A61K 31/198*** *(2006.01)*
***A61P 7/00*** *(2006.01)*
***A61K 31/663*** *(2006.01)*
***A61K 45/00*** *(2006.01)*
***G01N 33/00*** *(2006.01)*
***A61K 31/554*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **NewThera**
**68127 Niederentzen (FR)**

(72) Inventors:
- **Roegel, Jean-Christophe**
  **68127 Niederentzen (FR)**
- **Eftekhari, Pierre**
  **67200 Strasbourg (FR)**

(74) Representative: **Gallois, Valérie et al**
**Cabinet BECKER & ASSOCIES**
**25, rue Louis Le Grand**
**75002 Paris (FR)**

# (54) Novel uses for drugs targeting glutamine synthetase

(57) The present invention relates to novel therapeutic uses of tianeptine, salts, isomers, prodrugs, metabolites and structural analogs thereof. Furthermore, the present invention relates to the use of tianeptine, salts, isomers, pro-drugs, metabolites and structural analogs thereof, in obtaining methods of screening and of developing drugs. Finally, the present invention relates to the novel therapeutic use of other glutamine synthetase (GS) ligands and to the use of these ligands in obtaining methods for screening and developing drugs.



Printed by Jouve, 75001 PARIS (FR)

## Description

### FIELD OF THE INVENTION

The present invention first relates to novel therapeutic uses of tianeptine, salts, isomers, pro-drugs, metabolites and structural analogs thereof. Furthermore, the present invention relates to the use of tianeptine, salts, isomers, pro-drugs, metabolites and structural analogs thereof, in obtaining methods of screening and of developing drugs. Finally, the present invention relates to novel therapeutic uses of other glutamine synthetase (GS) ligands and to the use of these ligands in obtaining methods for screening and developing drugs.

### BACKGROUND OF THE INVENTION

***Tianeptine*,** which has the systematic name 7-[(3-chloro-6,11-dihydro-6-methyldibenzo[c,f][1,2] thiazepin-11-yl) amino] heptanoic acid S,S-dioxide ($C_{21}H_{24}ClN_2NaO_4S$; F.W. 458.9), is a tricyclic anti-depressant of the dibenzothiazepine type.

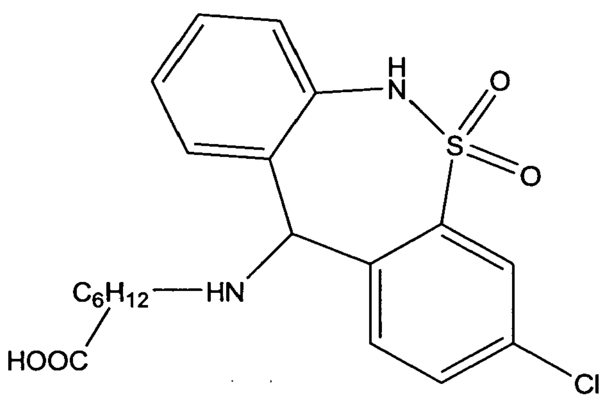

Tianeptine has been patented as a drug for use in the treatment of psychoneurotic disorders, pain and cough (FR 2 104 728), stress (FR 2 635 461), mnemo-cognitive disorders (FR 2 716 623), neurodegenerative diseases (US 6,599,896), and irritable bowel syndrome and nonulcer dyspepsia (US 6,683,072). It is used to treat depression (major depression with or without melancholia or dysthymic disorders, without psychotic features, depressed bipolar disorders, ...), neurotic or reactive states of depression, anxiodepressive states with somatic complaints such as digestive problems, anxiodepressive states observed in alcoholic detoxification, and asthma (for asthma: see Lechin et al., 1998). Despite of these therapeutic uses, its target remains unclear. The emerging pharmacological profile suggests that tianeptine serves to balance glutamatergic neurotransmission, in particular in conditions of high glucocorticoid exposure and stress (Plaisant et al., 2003; Reagan et al., 2004). Tianeptine might provide an original way of action in treating functional as well as structural changes that are associated with stress. For instance, tianeptine can both counteract behavioural and biochemical consequences of stress (Luine, 1992; Conrad et al., 1996; Delbende et al., 1991), and the dendritic atrophy of CA3 pyramidal neurons induced by stress (Watanabe et al., 1992). Atrophy of hippocampal dendrites in animal models of stress may be of the same nature than atrophy of the human hippocampus associated with recurrent depressive illness (Sheline et al., 1996), post-traumatic stress disorder (Bremner et al., 1995; Gurvits et al., 1996), mild cognitive impairment in aging (Convit et al., 1997), etc..

***L-methionine sulfoximine* (L-MSO or MS),** which has the systematic name 2-amino-4-(S-methylsulfonimidosyl)butanoic acid or L-s-[3-amino-3-carboxypropyl]-s-methylsulfoximine ($C_5H_{12}N_2O_3S$; F.W. 180.22), is a rare amino acid with a structural resemblance to glutamate. It occurs in nature and as a by-product of some forms of food processing. A notable example of the latter is a former method for bleaching wheat flour, using nitrogen trichloride, the "agene process", in use for most of the first 50 years of the previous century. "Agenized flour" was found to be responsible for various neurological disorders in animals, and MS was identified as the toxic agent. The agene process was subsequently discontinued.

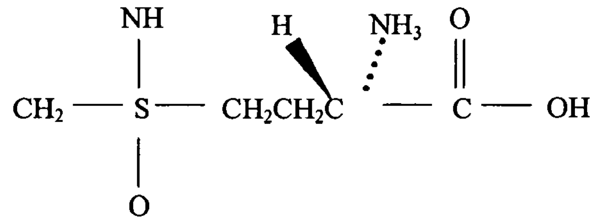

MS is believed to act as an inhibitor of the synthesis of glutathione (GSH) by blocking γ-glutamylcysteine synthetase (Meister and Tate, 1976) and as an inhibitor of the conversion of glutamate to glutamine by blocking glutamine synthetase (GS; EC 6.3.1.2) (Meister and Tate, 1976; Griffith et al., 1979; Meister, 1985). It is probable that the known actions of MS on the nervous system arise mostly from alterations in the activity, amount and distribution of these target proteins. The consequence of the former would be a decrease in GSH status (Kosower and Kosower, 1978) and a subsequent increase in oxidative stress (Richie et al., 1987; Reiter, 1995), a factor to which neurons may be particularly vulnerable (Evans, 1993; Bains and Shaw, 1997). Consequences of the latter could include a decrease in the control of ammonia, glutamate, glutamine, ... (Finch and Cohen, 1997). Ammonia has been shown to be toxic to neurons and has been implicated as one causal factor in neurological disorders (Seiler, 1993; Hoyer, 1994). Any increase in extracellular glutamate can (potentially) be (excito)toxic, directly or indirectly, upon its binding on glutamatergic ionotropic (GluR) and/or glutamatergic metabotropic (mGluR) receptors. Furthermore, MS has a direct effect on glutamatergic neurotransmission. In particular, it has been reported to increase glutamate release (Shaw et al., 1999), and to increase the neurotoxic effects of kainate and NMDA (Kollegger et al., 1991). Finally, glutamine serves as a fuel source, and as the most important carrier of nitrogen and one of the major build stones in protein synthesis. It is involved in crucial homeostatic functions (see below).

Administration of MS was observed to produce neurological disorders, in particular seizures, in a number of animal species (Lodin and Kolousek, 1956; Lodin, 1958; Hrebicek and Kolousek, 1968) at doses as low as 2 mg/kg body weight (bw) (Campbell et al., 1950; Wada et al., 1967). On the contrary, MS was also observed to protect both the cerebral cortex from ischaemia after middle cerebral artery occlusion (Swanson et al., 1990) and from hepatic encephalopathy (see below).

***Glufosinate ammonium (GF),*** which has the systematic name ammonium 4-[hydroxy (methyl)phosphinoyl]-DL-homoalaninate or 2-amino-4-(hydroxymethylphosphinyl) butyric acid ammonium salt ($C_5H_{15}N_2O_4P$; F.W. 198.16), is a phosphinic acid analog of glutamic acid. It is also denominated phosphinothrycin (PPT). It is produced in nature as the tripeptide L-phosphinothricyl-L-alanyl-L-alanine (bialaphos/bialophos) by the bacteria *Streptomyces hygroscopicus* and *Streptomyces viridochromogenes.*

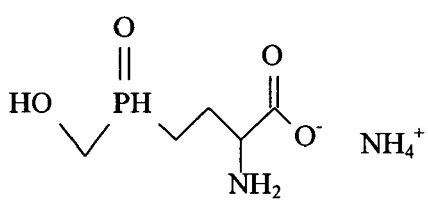

GF is used as an active ingredient in non-selective herbicides (Basta®; Liberty®). Its action is related to inhibition of plant's glutamine synthetase, which is involved in critical homeostatic functions (see below). After application on foliage, GF provides effective weed control in three to seven days. The symptoms appear as chlorotic lesions followed by necrosis. There is limited translocation of GF after application. GF has no soil activity.

GF is non-selective except to crops that carry the gene coding for phosphinothricin tolerance (*bar*), which encodes the enzyme phosphinothricin acetyltransferase (PAT) (PAT was also derived from *Streptomyces hygroscopicus* and *Streptomyces viridochromogenes;* EP 275957 filed in 1987 by Aventis CropScience (former AgrEvo)).

GF can inhibit glutamine synthetase in animals. It has also been observed to behave as an agonist on glutamate receptors (Nakaki et al., 2000; Matsumura et al., 2001; Lapouble et al., 2002). However, only at high (sublethal) doses, glutamate, ammonia and glutamine levels in brain, liver and kidney are affected (Hack et al., 1994; Ohtake et al., 2001).

The toxicokinetics of GF has been investigated in rats and dogs as well as in livestock. The substance is rapidly excreted in all these species regardless of the route of administration. About 80-90% of an oral dose of GF remains unabsorbed and is eliminated unchanged in the faeces over 48 hours, while about 10-15% is eliminated in the urine.

The main metabolite of GF found in urine and in faeces is 3-[hydroxy(methyl)phosphinoyl] propionic acid.

GF shows slight to moderate acute oral toxicity in mice, rats and dogs. In mice, the no observed adverse effect level (NOAEL) is 80 ppm (parts per million), equal to 17 and 19 mg/kg bw/day (bw: body weight) in males and females, respectively, based upon increased plasma potassium levels at the next highest tested dose (67 mg/kg bw/day). In rats, effects on kidney weight are seen at dose levels as low as 0.52 mg/kg bw/day. These effects on kidney weight are not found in long-term bioassays. In dogs, the NOAEL is 4.5 mg/kg bw/day, based on decreased body weight at higher doses.

GF is not teratogenic in rabbits and rats. The NOAELs for maternal and embryo/fetal toxicity is 6.3 mg/kg bw/day in rabbits and 2.2 mg/kg bw/day in rats.

GF is not carcinogenic in long-term/carcinogenicity studies in mice and rats. In mice fed 0, 20, 80 or 160 ppm (males) and 0, 20, 80 or 320 ppm (females), the NOAEL is 80 ppm (equal to 11 mg/kg bw/day) with increased mortality at higher dietary concentrations. In rats fed 0, 40, 140 or 500 ppm, the NOAEL is 40 ppm (equal to 2.1 mg/kg bw/day) with increased kidney weight at 140 ppm. At higher doses, a reduction in glutathione level is noted in liver and blood.

Thus, estimate of oral acceptable daily intake (ADI) of this non-selective herbicide has been fixed for man to 0-0.02 mg/kg bw (based upon the NOAEL determined from the long-term study in rats using a 100-fold safety factor).

For review of toxicological evaluation of glufosinate ammonium see:

Website address: http://www.inchem.org/documents/jmpr/jmpmono/v91pr12.htm
Website address: http://www.inchem.org/documents/jmpr/jmpmono/v99pr06.htm

GF and MS exhibit the kinetic properties of a slow tight-binding inhibitor and undergo phosphorylation during the course of inactivation (ATP-dependent) (Logusch et al., 1990; Abell and Villafranca, 1991; Abell et al., 1995; Eisenberg et al., 2000). The rate-limiting step in the inhibition reaction is the binding of the inhibitor and/or the conformational change associated with its binding. During the course of the inactivation, progressively slower rates for binding and phosphoryl transfer are observed, indicating communication between the different GS enzyme active sites (see below).

Finally, while GF and MS, and analogs thereof, exhibit herbicidal activity at "high concentrations" i.e. concentrations which completely inhibit glutamine synthetase (excess ammonium and glutamine deficiency act in concert to cause plant death), "low concentrations" of MS and GF, and derivatives thereof, have been observed to have an opposite effect: glutamine synthetase was activated with concomitant stimulation of plant growth and productivity (Evstigneeva et al., 2003).

## SUMMARY OF THE INVENTION

The Inventors have first discovered that tianeptine, an atypical antidepressant with neuroprotective properties, is a selective ligand of the enzyme glutamine synthetase (GS; EC 6.3.1.2). Depending on the concentration/dose and experimental or (patho)physiological condition, tianeptine can activate, inhibit or modulate/regulate GS activity in animals and bacteria, like observed previously, only in plants, with glufosinate (GF), L-methionine sulfoximine (MS) and derivatives thereof (Evstigneeva et al., 2003). In short, i) at "low concentrations", tianeptine behaves as a GS activator; ii) at "high concentrations", tianeptine behaves as a GS inhibitor, while iii) at "intermediate concentrations", tianeptine is tolerated, or can activate or inhibit GS activity, depending on the experimental or (patho)physiological condition. In addition to the above mentioned effects, tianeptine was observed to protect GS from oxidation. It could also influence GS protein expression. The dose/effect relationships differed according to the subcellular compartment, cell, tissue, organ, species, ..., and changed over time. Thus, GS and its regulation have to be approached by considering the multidimensional nature of the system. These "modulatory/regulatory" effects of tianeptine on GS may likely underlie its already proposed clinical applications contrary to its unclear effects on 5-HT uptake that are usually proposed. They can be reconciled with the emerging pharmacological profile of tianeptine which suggest that tianeptine may balance glutamatergic neurotransmission.

Accordingly, the present invention relates to enlarge the use of tianeptine, salts, isomers, pro-drugs, metabolites and structural analogs thereof, in obtaining activators, inhibitors and/or modulators/regulators of GS activity intended for the activation, inhibition and/or modulation/regulation of all functions which involve GS activity or can be influenced by GS activity, directly or indirectly, and the prevention and/or treatment of all types of conditions and disorders which involve or can be influenced by GS activity, directly or indirectly. In particular, the invention concerns the prevention and/or treatment of conditions and disorders related to absolute or relative excess of ammonia, absolute or relative deficiency or excess of glutamate, and/or absolute or relative deficiency or excess of glutamine, in vertebrate animals including humans.

Therefore, the present invention concerns the use of tianeptine, salts thereof, isomers thereof, pro-drugs thereof, metabolites thereof and structural analogs thereof, in the manufacture of medicaments intended for prevention and/or treatment of all types of conditions and disorders which involve or can be influenced by GS activity, directly or indirectly, in particular those related to absolute or relative excess of ammonia, those related to absolute or relative deficiency or

excess of glutamate, and those related to absolute or relative deficiency or excess of glutamine, with the exception of the already claimed or proposed clinical applications of tianeptine. In particular, said conditions and disorders which involve or can be influenced by GS activity are selected from those disclosed in Tables 1-4; potential factors and mechanisms which can underlie these conditions and disorders are disclosed in Table 4, without to be limited thereto. The already claimed or proposed clinical applications of tianeptine are disclosed in Table 5. In particular, these already claimed or proposed clinical applications of tianeptine include: asthma, cough, depression (major depression with or without melancholia, dysthymic disorders, depressed bipolar disorder, ...), irritable bowel syndrome, mnemo-cognitive disorders, neurodegenerative diseases (such as cerebral hypoxia, cerebral ischaemia, cerebral traumatism, cerebral ageing, Alzheimer's disease, multiple sclerosis, amyotrophic lateral sclerosis, demyelinating pathologies, encephalopathies, myalgic encephalomyelitis, chronic fatigue syndrome, post-viral fatigue syndrome, the state of fatigue and depression following a bacterial or viral infection, the dementia syndrome of AIDS, ...), nonulcer dyspepsia, pain, psychoneurotic disorders (neurotic or reactive states of depression, anxiodepressive states with somatic complaints such as digestive problems, anxiodepressive states observed in alcoholic detoxification, ...), stress and stroke. The present invention also concerns a method for prevention and/or treatment of any condition or disorder selected from those disclosed in Tables 1-4, with the exception of the conditions and disorders disclosed in Table 5, comprising administering an efficient amount of tianeptine or a salt thereof, an isomer thereof, a pro-drug thereof, a metabolite thereof or a structural analog thereof, thereby preventing and/or treating said condition or disorder. In a preferred embodiment, said conditions and disorders are selected from those disclosed in Tables 2 and 3, with the exception of the conditions and disorders disclosed in Table 5. In a preferred embodiment, said conditions and disorders are related to or result in a local excess of ammonia or a systemic excess of ammonia i.e. hyperammon(em)ia (see the conditions and disorders in Table 1 and Tables 2-3 (the "double-underlined" conditions and disorders)). In a particular embodiment, said metabolites or structural analogs of tianeptine are selected from the group consisting of the compounds in Table 8. Optionally, tianeptine or a salt, an isomer, a pro-drug, a metabolite or a structural analog thereof, can be used in combination with an other drug intended to prevent/treat hyperammon(emi)a or resulting in hyperammon(em)ia, an other drug intended to prevent/treat conditions and disorders related to deficiency or excess of glutamate or resulting in deficiency or excess of glutamate, or an other drug intended to prevent/treat conditions and disorders related to deficiency or excess of glutamine or resulting in deficiency or excess of glutamine. An other object of the present invention is a pharmaceutical composition comprising tianeptine or a salt, an isomer, a pro-drug, a metabolite or a structural analog thereof, in combination with a drug selected from the group consisting of drugs intended to prevent/treat hyperammon(emi)a or resulting in hyperammon (em)ia, or an other drug intended to prevent/treat conditions and disorders related to deficiency or excess of glutamate or resulting in deficiency or excess of glutamate, or an other drug intended to prevent/treat conditions and disorders related to deficiency or excess of glutamine or resulting in deficiency or excess of glutamine, in a pharmaceutically acceptable carrier. In particular, these drugs include: i) drugs intended to prevent/treat hyperammon(em)ia and/or hyperammon(em)ia symptoms e.g. flumazenil, zinc, sodium benzoate, sodium phenylacetate and arginine hydrochloride, ii) drugs resulting in hyperammon(em)ia e.g. anti-cancer therapies (5-fluorouracil, asparaginase, ...) and antiepileptics (sodium valproate, primidone, ...), iii) drugs altering glutamate receptor(s) function(s) e.g. ampakines, glutamate release inhibitors (lamotrigine, riluzole, ...), glutamate receptor antagonists (D-AP5, ketamine, memantine, MK-801, ...) and glycine, iv) drugs intended to prevent/treat disorders where GS protein/activity is altered or relatively ineffective e.g. anti-epileptic treatments (benzodiazepines, carbamazepine, ethosuximide, hydantoins, gabapentin, lamotrigin, phenobarbital, primidone, valproate, ...) and antibiotics (aminoglycosides, cephalosporins, chloramphenicol, macrolides, penicillins, quinolones, rifampicine, sulfonamides, tetracyclines, trimethoprim-sulfamethoxazole, ...) in certain forms of epilepsy and critical sepsis, respectively, v) drugs intended to combat neoplasms and/or inflammatory diseases e.g. antiproliferative (5-fluorouracil, asparaginase, ...) and vi) anti-inflammatory drugs or drugs preventing from the occurrence of inflammation, in particular glucocorticoids.

In a first preferred embodiment, said conditions and disorders are related to or result in a local excess of ammonia or a systemic excess of ammonia i.e. hyperammon(em)ia. Preferably said hyperammon(em)ia is related to a severe liver disease (resulting from cirrhosis, hepatitis, intoxication, ...), an inborn error of metabolism (urea cycle disorder, ...) and/or a porto-systemic shunt, but various other conditions and disorders, alone or combined, (can) result in a localized ammonia increase or a systemic ammonia increase (see below, and Tables 1, 2-3 (the double-underlined conditions and disorders) and 4). In particular, said conditions and disorders are selected from the group consisting of severe hepatic disorders (resulting from cirrhosis, hepatitis, intoxication, ...), errors of metabolism (urea cycle disorder, ...), porto-systemic shunts, gastrointestinal haemorrhages, transplant rejections and catabolic states.

In a second preferred embodiment, said conditions and disorders are related to or result in (absolute or relative) deficiency or excess of glutamate. Thus, said conditions and disorders are preferably selected from those disclosed in Tables 2 (in particular those marked in *"italic")* and 3, with the exception of those disclosed in Table 5. In a preferred embodiment, without to be limited thereto, said conditions and disorders are selected from the group consisting of Platelet disorders, Cardiac arrhythmias, Reiter's syndrome, Lupus erythematosus and associated conditions, Rheumatoid arthritis and associated conditions, Bone and joint injuries, Osteoporosis, Schizophrenia and other psychotic disorders,

Penile and scrotal disorders, Sexual function and fertility disorders, Blood brain barrier defect, Nervous system disorders and Psychiatric disorders, with the exception of the conditions and disorders disclosed in Table 5.

In a third preferred embodiment, said conditions and disorders are related to or result in (absolute or relative) deficiency or excess of glutamine, and/or sensitive to glutamine deprivation or glutamine supplementation. A myriad of conditions and disorders are prone to be associated with a localized deficiency or excess of glutamine (see below). De novo glutamine synthesis is more or less crucial depending on the cell type and (patho)physiological condition. Preferably, said deficiency or excess of glutamine is related to conditions and disorders mentioned in Tables 2 and 3, with the exception of those disclosed in Table 5; but the conditions and disorders mentioned in the Table 1 can also be considered.

In vitro and in vivo studies have already demonstrated that inhibition of GS is a feasible therapeutic strategy against pathogenic mycobacteria (GS is believed to play a crucial role in the synthesis of a poly-L-glutamate-glutamine cell wall component which is found exclusively in pathogenic mycobacteria). So, in a fourth preferred embodiment, tianeptine or a salt thereof, an isomer thereof, a pro-drug thereof, a metabolite thereof or a structural analog thereof, is used against pathogenic mycobacterium growth preferably combined with other antituberculous agents e.g. isoniazid, ethambutol, pyrazinamide, rifampicin or streptomycin, or salts, isomers, pro-drugs, metabolites or structural analogs thereof Preferably said pathogenic mycobacterium is selected from the group consisting of *M. tuberculosis, M. bovis,* and *M. avium*. More preferably, said pathogenic mycobacterium is *M. tuberculosis*.

The present invention also relates to the use of tianeptine, salts thereof, isomers thereof, pro-drugs thereof, metabolites thereof or structural analogs thereof, in the preparation of compositions to be used to activate, inhibit and/or modulate/regulate GS activity intended for the activation, inhibition and/or modulation/regulation of functions which involve GS activity or can be influenced by GS activity, directly or indirectly, in others organisms with eukaryotic cells such as invertebrate animals (arthropods, protozoa, shellfish, worms, ...), algae, fungi (yeasts, molds, ...) or plants, but also prokaryotes e.g. algae and bacteria, aiming to facilitate their growth or protect them from stresses and disorders or, inversely, aiming to combat their growth. Thus, the present invention concerns methods to facilitate the growth of others non-vertebrate organisms with eukaryotic cells such as invertebrate animals (arthropods, protozoa, shellfish, worms, ...), algae, fungi (yeasts, molds, ...) or plants, but also of prokaryotes e.g. algae or bacteria, either to protect them from stresses and disorders, or to combat them, comprising administering an efficient amount of tianeptine or a salt thereof, an isomer thereof, a pro-drug thereof, a metabolite thereof or a structural analog thereof. In a particular embodiment, said metabolites or structural analogs of tianeptine are selected from the group consisting of the compounds disclosed in Table 8. Therefore, the invention concerns the use of tianeptine or a salt, an isomer, a pro-drug, a metabolite or a structural analog thereof, for the preparation of a composition aiming to facilitate the growth of non-vertebrate organisms with eukaryotic cells such as invertebrate animals (arthropods, protozoa, shellfish, worms, ...), algae, fungi (yeasts, molds, ...) or plants, and prokaryotes e.g. algae or bacteria, or to protect them from stresses and disorders or, inversely, aiming to combat their growth.

Furthermore, the present invention relates to novel uses of other GS ligands such as glufosinate (GF; phosphinothrycin (PPT) and L-methionine sulfoximine (L-MSO or MS), but also hydrazines and bisphosphonates, and salts thereof, isomers thereof, pro-drugs thereof, metabolites thereof and structural analogs thereof. These "other GS ligands" are claimed to be used for treating all the conditions and disorders already proposed to be sensitive to tianeptine or isomers thereof, pro-drugs thereof, metabolites thereof or structural analogs thereof, and/or for screening and developing drugs to be used in all the conditions and disorders already proposed to be sensitive to tianeptine or isomers thereof, pro-drugs thereof, metabolites thereof or structural analogs thereof, with the exception of: if said GS ligand is MS, the conditions and disorders related to cerebral ischemia; and, if said GS ligand is a hydrazine, the conditions and disorders disclosed in Table 6. More particularly, the conditions and disorders already proposed to be sensitive to tianeptine or salts thereof, isomers thereof, pro-drugs thereof, metabolites thereof or structural analogs thereof, are selected from the group consisting of the conditions and disorders disclosed in Table 5. In particular, these conditions and disorders include: asthma, cough, depression (major depression with or without melancholia, dysthymic disorders, depressed bipolar disorder, ...), irritable bowel syndrome, mnemo-cognitive disorders, neurodegenerative diseases (such as cerebral hypoxia, cerebral ischaemia, cerebral traumatism, cerebral ageing, Alzheimer's disease, multiple sclerosis, amyotrophic lateral sclerosis, demyelinating pathologies, encephalopathies, myalgic encephalomyelitis, chronic fatigue syndrome, post-viral fatigue syndrome, the state of fatigue and depression following a bacterial or viral infection, the dementia syndrome of AIDS, ...), nonulcer dyspepsia, pain, psychoneurotic disorders (neurotic or reactive states of depression, anxiodepressive states with somatic complaints such as digestive problems, anxiodepressive states observed in alcoholic detoxification, ...), stress and stroke, with the exception of: if said GS ligand is MS, the conditions and disorders related to cerebral ischemia; and, if said GS ligand is a hydrazine, the conditions and disorders disclosed in Table 6. Then, the present invention concerns the use of a GS ligand, with the exception of tianeptine, for the preparation of a medicament for the prevention and/or treatment of a condition or a disorder selected from the group consisting of asthma, cough, depression (major depression with or without melancholia, dysthymic disorders, depressed bipolar disorder, ...), irritable bowel syndrome, mnemo-cognitive disorders, neurodegenerative diseases (such as cerebral hypoxia, cerebral ischaemia, cerebral traumatism, cerebral ageing, Alzheimer's disease, multiple sclerosis, amyotrophic lateral sclerosis, demyelinating pathologies, encephalopathies, myalgic encephalomyelitis, chronic fatigue syndrome, post-viral fatigue syndrome, the state of fatigue and depression following a bacterial or viral infection, the dementia syndrome of AIDS, ...), nonulcer dyspepsia, pain, psychoneurotic disorders (neurotic or reactive states of depression, anxiodepressive states with somatic complaints such as digestive problems, anxiodepressive states observed in alcoholic detoxification, ...), stress and stroke, with the exception of: if said GS ligand is MS, the conditions and disorders related to cerebral ischemia; and, if said GS ligand is a hydrazine, the conditions and disorders disclosed in Table 6. Preferably, said "other GS ligand" is selected from the group consisting of GF, MS, hydrazines and bisphophonates, and salts, isomers, pro-drugs, metabolites and structural analogs thereof. In one preferred embodiment, said GS ligand is GF or a salt, an isomer, a pro-drug, a metabolite or a structural analog thereof. In another preferred embodiment, said GS ligand is MS or a salt, an isomer, a pro-drug, a metabolite or a structural analog thereof. In a further preferred embodiment, said GS ligand is a hydrazine or a salt, an isomer, a pro-drug, a metabolite or a structural analog thereof. In an additional preferred embodiment, said GS ligand is a bisphophonate or a salt, an isomer, a pro-drug, a metabolite or a structural analog thereof.

These "other GS ligands" can be used for obtaining activators and/or modulators/regulators of GS activity intended for activating and/or modulating/regulating all types of functions which involve GS activity or can be influenced by GS activity, directly or indirectly, and/or for preventing and/or treating all types of conditions and disorders which involve or can be influenced by GS activity, directly or indirectly, in particular those related to absolute or relative excess of ammonia, those related to absolute or relative deficiency or excess of glutamate, and those related to absolute or relative deficiency or excess of glutamine, either alone or combined.

In fact, the Inventors have discovered that these "other GS ligands", which were unanimously believed to be GS inhibitors in animals, can present, as in plants, an activating and/or modulating/regulating activity when they are used at concentrations/doses lower as compared to those which inhibit GS activity. In short, i) at low concentrations, these GS ligands behave as GS activators; ii) at "intermediate concentrations", they are tolerated, or can activate or inhibit GS activity depending on the experimental or (patho)physiological condition; and iii) at high "saturating" concentrations, they behave as GS inhibitors.

Therefore, the present invention concerns the use of these "other GS ligands" for the preparation of medicaments having an activating and/or modulating/regulating effect on GS, wherein said GS ligands are used at "low or intermediate doses" i.e. doses resulting in concentrations which activate or modulate/regulate the activity of GS in the target cells/tissues/organs. In a most preferred embodiment, the present invention concerns the use of these "other GS ligands" for the preparation of medicaments having an activating effect on GS, wherein said GS ligands are used at "low doses" i.e. doses resulting in concentrations which increase the activity of GS in the target cells/tissues/organs.

Therefore, the present invention concerns the use of a GS ligand, with the exception of tianeptine, for the preparation of a medicament to prevent and/or treat all types of conditions and disorders which involve or can be influenced by GS activity, directly or indirectly, in particular those related to absolute or relative excess of ammonia, those related to absolute or relative deficiency or excess of glutamate, and those related to absolute or relative deficiency or excess of glutamine. In particular, said conditions and disorders are selected from those disclosed in Tables 1-4, with the exception of the already known indications of these ligands i.e. if said GS ligand is MS, the conditions and disorders related to cerebral ischemia, hyperammonemia and mycobacterial infectious disorders; if said GS ligand is a hydrazine, the conditions and disorders disclosed in Table 6; and, if said GS ligand is a bisphosphonate, the conditions and disorders disclosed in Table 7. The present invention also concerns a method for prevention and/or treatment of any condition or disorder selected from those disclosed in Tables 1-4, comprising administering an efficient amount of a GS ligand or a salt thereof, an isomer thereof, a pro-drug thereof, a metabolite thereof or a structural analog thereof, thereby preventing or treating said condition or disorder, with the exception of: if said GS ligand is MS, the conditions and disorders related to cerebral ischemia, hyperammonemia and mycobacterial infectious disorders; if said GS ligand is a hydrazine, the conditions and disorders disclosed in Table 6; and, if said GS ligand is a bisphosphonate, the conditions and disorders disclosed in Table 7. Potential factors and mechanisms which can underlie these conditions and disorders are disclosed in Table 4, without to be limited thereto. In a preferred embodiment, said conditions and disorders are selected from those disclosed in Tables 2 and 3. In an other preferred embodiment, said conditions and disorders are related to hyperammon(em)ia (see Tables 1 and 2-3 (the double-underlined conditions and disorders)). In a particular embodiment, said GS ligand is selected from the group consisting of GF, MS, hydrazines and bisphophonates, and salts, isomers, pro-drugs, metabolites and structural analogs thereof. In one preferred embodiment, said GS ligand is GF or a salt, an isomer, a pro-drug, a metabolite or a structural analog thereof. In another preferred embodiment, said GS ligand is MS or a salt, an isomer, a pro-drug, a metabolite or a structural analog thereof. In a further preferred embodiment, said GS ligand is a hydrazine or a salt, an isomer, a pro-drug, a metabolite or a structural analog thereof. In an additional preferred embodiment, said GS ligand is a bisphophonate or a salt, an isomer, a pro-drug, a metabolite or a structural analog thereof. Optionally, said GS ligand or salt, isomer, prodrug, metabolite or structural analog thereof, can be used in combination with an other drug intended to prevent/treat hyperammon(emi)a or resulting in hyperammon(em)ia, or an other drug intended to prevent/treat conditions and disorders related to deficiency or excess of glutamate or resulting in deficiency or excess of glutamate, or an other drug intended to prevent/treat conditions and disorders related to

deficiency or excess of glutamine or resulting in deficiency or excess of glutamine. An other object of the present invention is a pharmaceutical composition comprising a GS ligand, a salt, an isomer, a pro-drug, a metabolite or a structural analog thereof in combination with a drug selected from the group consisting of drugs intended to prevent/treat hyperammon (emi)a or resulting in hyperammon(em)ia, drugs intended to prevent/treat conditions and disorders related to deficiency or excess of glutamate or resulting in deficiency or excess of glutamate, and drugs intended to prevent/treat conditions and disorders related to deficiency or excess of glutamine or resulting in deficiency or excess of glutamine, in a pharmaceutically acceptable carrier. In particular, these drugs include: i) drugs intended to prevent/treat hyperammon(em) ia and/or hyperammon(em)ia symptoms e.g. flumazenil, zinc, sodium benzoate, sodium phenylacetate and arginine hydrochloride, ii) drugs resulting in hyperammon(em)ia e.g. anti-cancer therapies (5-fluorouracil, asparaginase, ...) and antiepileptics (sodium valproate, primidone, ...), iii) drugs altering glutamate receptor(s) function(s) e.g. ampakines, glutamate release inhibitors (lamotrigine, riluzole, ...), glutamate receptor antagonists (D-AP5, ketamine, memantine, MK-801, ...) and glycine, iv) drugs intended to prevent/treat disorders where GS protein/activity is altered or relatively ineffective e.g. anti-epileptic treatments (benzodiazepines, carbamazepine, ethosuximide, hydantoins, gabapentin, lamotrigin, phenobarbital, primidone, valproate, ...) and antibiotics (aminoglycosides, cephalosporins, chloramphenicol, macrolides, penicillins, quinolones, rifampicine, sulfonamides, tetracyclines, trimethoprim-sulfamethoxazole, ...) in certain forms of epilepsy and critical sepsis, respectively, v) drugs intended to combat neoplasms and/or inflammatory diseases, e.g. anti-proliferative (5-fluorouracil, asparaginase, ...), and vi) anti-inflammatory drugs or drugs preventing from the occurrence of inflammation, in particular glucocorticoids.

In a first preferred embodiment, said conditions and disorders are related to or result in hyperammon(em)ia. Preferably said hyperammon(em)ia is related to a severe liver disease (resulting from cirrhosis, hepatitis, intoxication, ...), an inborn error of metabolism (urea cycle disorder, ...) and/or a porto-systemic shunt but various other conditions and disorders, alone or combined, (can) result in a localized ammonia increase or a systemic ammonia increase (see below, and Tables 1, 2 (the "double-underlined" conditions and disorders) and 4). In particular, said conditions and disorders are selected from the group consisting of severe hepatic disorders (resulting from cirrhosis, hepatitis, intoxication, ...), errors of metabolism (urea cycle disorder, ...), porto-systemic shunts, gastrointestinal haemorrhages, transplant rejections and catabolic states.

In a second preferred embodiment, said conditions and disorders are related to or result in (absolute or relative) deficiency or excess of glutamate. Thus, said conditions and disorders are preferably selected from those disclosed in Tables 2 (in particular those marked in *"italic")* and 3. In a preferred embodiment, without to be limited thereto, said conditions and disorders are selected from the group consisting of Platelet disorders, Cardiac arrhythmias, Reiter's syndrome, Lupus erythematosus and associated conditions, Rheumatoid arthritis and associated conditions, Bone and joint injuries, Osteoporosis, Schizophrenia and other psychotic disorders, Penile and scrotal disorders, Sexual function and fertility disorders, Blood brain barrier defect and Nervous system disorders.

In a third preferred embodiment, said conditions or disorders are associated or related to (absolute or relative) deficiency or excess of glutamine, and/or sensitive to glutamine deprivation or glutamine supplementation. Preferably, said deficiency or excess of glutamine is related to conditions and disorders mentioned in the Tables 2 and 3; but the conditions and disorders mentioned in the Table 1 can be also considered.

Preferably, said "other GS ligands" are used at "low/intermediate doses" i.e. doses resulting in concentrations which activate or modulate/regulate the activity of GS in the target cells/tissues/organs. In a most preferred embodiment, said "other GS ligands" are used at "low doses" i.e. doses resulting in concentrations which increase the activity of GS in the target cells/tissues/organs.

In particular, the present invention concerns GF, salts thereof, isomers thereof, pro-drugs thereof, metabolites thereof and structural analogs thereof, as a medicament, preferably at "low or intermediate doses" i.e. doses resulting in concentrations which can increase or modulate/regulate GS activity (directly or indirectly) in the target cells/tissues/organs. Therefore, the present invention concerns a pharmaceutical composition comprising GF or a salt thereof, an isomer thereof, a pro-drug thereof, a metabolite thereof or a structural analog thereof, in a pharmaceutically acceptable carrier. In a preferred embodiment, said metabolite or analog of GF is selected from the group consisting of the compounds disclosed in Tables 9 and 13, preferably Table 9. Preferably, the compound is selected from the group consisting of GF, 4-methylphosphinico-2-oxo-butanoic acid, 3-methylphosphinicopropionic acid, 4-methylphosphinico-2-hydroxybutanoic acid, 4-methylphosphinicobutanoic acid, 2-methylphosphinicoacetic acid, 2-acetamido-4-methylbutanoic acid, gamma-hydroxy phosphinothricin, gamma-methyl phosphinothricin, gamma-acetoxy phosphinothricin, alpha-methyl phosphinothricin, alpha-ethyl phosphinothricin, cyclohexane phosphinothricin, cyclopentane phosphinothricin, tetrahydrofuran phosphinothricin, s-phosphonomethyl homocysteine sulfoxide, s-phosphonomethyl homocysteine sulfone, 2-amino-4-[(phosphonomethyl)hydroxyphosphinyl-1]butanoic acid, 2-amino-4-phosphono butanoic acid, 4-amino-4-phosphono butanoic acid, 2-amino-2-methyl-4-phosphono butanoic acid, 4-amino-4-(hydroxymethylphosphinyl)-4-methyl butanoic acid, 2-methoxycarbonyl-4-phosphono butamoic acid, methyl 4-amino-4-phosphono butanate, 4-amino-4-(hydroxymethylphosphinyl)butanoic acid, 2-amino-4-(hydroxymethylphosphinyl)butanoic acid, phosphinothricin, s-phosphonomethyl homocysteine, 4-(phosphonoacetyl)-L-alpha-aminobutyrate, threo-4-hydroxy-D-glutamic acid, erythro-4-fluoro-D,L-glutamic acid, 4-amino-4-(hydroxy-methylphosphinoyl)-butyric acid, 2-methoxycarbonyl-4-phosphono butanoic acid, methyl 4-amino-4-phosphono butanoate and 2-amido-4-phosphono butanoic acid, and salts and isomers thereof. Optionally, the pharmaceutical composition further comprises an other etiologic and/or symptomatic treatment i.e. a drug selected from the group consisting of drugs intended to prevent and/or treat conditions and disorders which involve or can be influenced by GS activity, in particular the conditions and disorders related to absolute or relative excess of ammonia, those related to absolute or relative deficiency or excess of glutamate, and those related to absolute or relative deficiency or excess of glutamine. In particular, these include: i) drugs intended to prevent/treat hyperammon(em)ia and/or hyperammon(em)ia symptoms e.g. flumazenil, zinc, sodium benzoate, sodium phenylacetate and arginine hydrochloride, ii) drugs resulting in hyperammon(em)ia e.g. anti-cancer therapies (5-fluorouracil, asparaginase, ...) and antiepileptics (sodium valproate, primidone, ...), iii) drugs altering glutamate receptor(s) function(s) e.g. ampakines, glutamate release inhibitors (lamotrigine, riluzole, ...), glutamate receptor antagonists (D-AP5, ketamine, memantine, MK-801, ...) and glycine, iv) drugs intended to prevent/treat disorders where GS protein/activity is altered or relatively ineffective e.g. anti-epileptic treatments (benzodiazepines, carbamazepine, ethosuximide, hydantoins, gabapentin, lamotrigin, phenobarbital, primidone, valproate, ...) and antibiotics (aminoglycosides, cephalosporins, chloramphenicol, macrolides, penicillins, quinolones, rifampicine, sulfonamides, tetracyclines, trimethoprim-sulfamethoxazole, ...) in certain forms of epilepsy and critical sepsis, respectively, v) drugs intended to combat neoplasms and/or inflammatory diseases, e.g. anti-proliferative (5-fluorouracil, asparaginase, ...), and vi) anti-inflammatory drugs or drugs preventing from the occurrence of inflammation, in particular glucocorticoids.

Furthermore, the present invention concerns the use of GF, salts thereof, isomers thereof, pro-drugs thereof, metabolites thereof and structural analogs thereof, in the manufacture of medicaments for preventing and/or treating all types of conditions and disorders which involve or can be influenced by GS activity, directly or indirectly, in particular those related to absolute or relative excess of ammonia, those related to absolute or relative deficiency or excess of glutamate, and those related to absolute or relative deficiency or excess of glutamine. The present invention also concerns a method for the prevention and/or the treatment of any condition or disorder selected from those disclosed in Tables 1-4, comprising administering an efficient amount of GF or a salt thereof, an isomer thereof, a pro-drug thereof, a metabolite thereof or a structural analog thereof, thereby preventing and/or treating said condition or disorder. Preferably, said conditions and disorders are selected from those disclosed in Tables 1-4, preferably Tables 2-3. In a particular embodiment, said conditions and disorders are selected from the group consisting of asthma, cough, depression (major depression with or without melancholia, dysthymic disorders, depressed bipolar disorder, ...), irritable bowel syndrome, mnemo-cognitive disorders, neurodegenerative diseases (such as cerebral hypoxia, cerebral ischaemia, cerebral traumatism, cerebral ageing, Alzheimer's disease, multiple sclerosis, amyotrophic lateral sclerosis, demyelinating pathologies, encephalopathies, myalgic encephalomyelitis, chronic fatigue syndrome, post-viral fatigue syndrome, the state of fatigue and depression following a bacterial or viral infection, the dementia syndrome of AIDS, ...), nonulcer dyspepsia, pain, psychoneurotic disorders (neurotic or reactive states of depression, anxiodepressive states with somatic complaints such as digestive problems, anxiodepressive states observed in alcoholic detoxification, ...), stress and stroke. In a preferred embodiment, the conditions and disorders are related to or result in hyperammon(em)ia or result in hyperammon (em)ia (see Tables 1, 2-3 (the "double-underlined" conditions and disorders) and 4). In an other preferred embodiment, said conditions and disorders are related to or result in (absolute or relative) deficiency or excess of glutamate, and are preferably selected from those disclosed in Tables 2 (in particular those in *"italic")* and 3, in particular from the group consisting of Platelet disorders, Cardiac arrhythmias, Reiter's syndrome, Lupus erythematosus and associated conditions, Rheumatoid arthritis and associated conditions, Bone and joint injuries, Osteoporosis, Penile and scrotal disorders, Sexual function and fertility disorders, Blood brain barrier defect, Nervous system disorders and Psychiatric disorders. In a further preferred embodiment, said condition or disorder is related to or result in (absolute or relative) deficiency or excess of glutamine, and/or sensitive to glutamine deprivation or glutamine supplementation, preferably selected from those disclosed in the Table 2 and 3; but the conditions and disorders mentioned in the Table 1 and 4 can be also considered.

In particular, the present invention concerns the use of MS, salts thereof, isomers thereof, pro-drugs thereof, metabolites thereof and structural analogs thereof, preferably at "low or intermediate doses" i.e. doses resulting in concentrations which can increase or modulate/regulate GS activity (directly or indirectly) in the target cells/tissues/organs, in the manufacture of medicaments for preventing and/or treating all types of conditions and disorders which involve or can be influenced by GS activity, directly or indirectly, in particular those related to absolute or relative deficiency or excess of glutamate, and those related to absolute or relative deficiency or excess of glutamine, with the exception of the conditions and disorders related to cerebral ischemia, hyperammonemia and mycobacterial infectious disorders. The present invention also concerns a method for the prevention and/or the treatment of any condition or disorder selected from those disclosed in Tables 2-4, with the exception of the conditions and disorders related to cerebral ischemia, hyperammonemia (marked in "double-underlined" in Table 2) and mycobacterial infectious disorders, comprising administering an efficient amount of MS or a salt thereof, an isomer thereof, a pro-drug thereof, a metabolite thereof or a structural analog thereof, thereby preventing or treating said condition or disorder. Preferably, said conditions

and disorders are selected from those disclosed in Tables 2-4, preferably Tables 2-3, with the exception of the conditions and disorders related cerebral ischemia, hyperammonemia (marked in "double-underlined" in Table 2) and mycobacterial infectious disorders. In a particular embodiment, said conditions or disorders are selected from the group consisting of asthma, cough, depression (major depression with or without melancholia, dysthymic disorders, depressed bipolar disorder, ...), irritable bowel syndrome, mnemo-cognitive disorders, neurodegenerative diseases (such as cerebral traumatism, cerebral ageing, Alzheimer's disease, multiple sclerosis, amyotrophic lateral sclerosis, demyelinating pathologies, encephalopathies myalgic encephalomyelitis, chronic fatigue syndrome, post-viral fatigue syndrome, the state of fatigue and depression following a bacterial or viral infection, the dementia syndrome of AIDS, ..., with the exception of those related cerebral ischemia,), nonulcer dyspepsia, pain, psychoneurotic disorders (neurotic or reactive states of depression, anxiodepressive states with somatic complaints such as digestive problems, anxiodepressive states observed in alcoholic detoxification, ...) and stress. In a preferred embodiment, said metabolites and analogs of MS are selected from the group consisting of the compounds disclosed in Tables 10 and 13, preferably Table 10. In a preferred embodiment, said conditions and disorders are related to or result in (absolute or relative) deficiency or excess of glutamate, and preferably selected from those disclosed in Tables 2 (in particular the conditions and disorders in *"italic")* and 3, in particular from the group consisting of Platelet disorders, Cardiac arrhythmias, Reiter's syndrome, Lupus erythematosus and associated conditions, Rheumatoid arthritis and associated conditions, Bone and joint injuries, Osteoporosis, Penile and scrotal disorders, Sexual function and fertility disorders, Blood brain barrier defect, Nervous system disorders and Psychiatric disorders, with the exception of those conditions and disorders related to cerebral ischemia, hyperammonemia and mycobacterial infectious disorders. In a further preferred embodiment, said condition or disorder is related to or result in (absolute or relative) deficiency or excess of glutamine, and/or sensitive to glutamine deprivation or glutamine supplementation, preferably selected from those disclosed in the Tables 2-3, with the exception of those conditions and disorders related to cerebral ischemia, hyperammonemia (marked in "double-underlined" in Table 2) and mycobacterial infectious disorders.

In particular, the present invention concerns the use of hydrazines, salts thereof, isomers thereof, pro-drugs thereof, metabolites thereof and structural analogs thereof, preferably at "low or intermediate doses" i.e. doses resulting in concentrations which can increase or modulate/regulate GS activity (directly or indirectly) in the target cells/tissues/organs, in the manufacture of medicaments for preventing and/or treating all types of conditions and disorders which involve or can be influenced by GS activity, directly or indirectly, in particular those related to absolute or relative excess of ammonia, those related to absolute or relative deficiency or excess of glutamate, and those related to absolute or relative deficiency or excess of glutamine, with the exception of the conditions and disorders disclosed in Table 6. The present invention also concerns a method for the prevention and/or the treatment of any condition or disorder selected from those disclosed in Tables 1-4, with the exception of the conditions and disorders disclosed in Table 6, comprising administering an efficient amount of a hydrazine or a salt thereof, an isomer thereof, a pro-drug thereof, a metabolite thereof or a structural analog thereof, thereby preventing or treating said condition or disorder. Preferably, said conditions and disorders are selected from those disclosed in Tables 1-4, preferably Tables 2-3, with the exception of the conditions and disorders disclosed in Table 6. In a particular embodiment, said conditions or disorders are selected from the group consisting of asthma, cough, irritable bowel syndrome, mnemo-cognitive disorders, neurodegenerative diseases (such as cerebral hypoxia, cerebral ischaemia, cerebral traumatism, cerebral ageing, Alzheimer's disease, multiple sclerosis, amyotrophic lateral sclerosis, demyelinating pathologies, encephalopathies, chronic fatigue syndrome, myalgic encephalomyelitis, post-viral fatigue syndrome, the state of fatigue and depression following a bacterial or viral infection, the dementia syndrome of AIDS, ...), nonulcer dyspepsia, stress and strokes. In a preferred embodiment, said metabolites and analogs of hydrazine are selected from the group consisting of the compounds disclosed in Table 11. In a preferred embodiment, said conditions and disorders are related to or result in hyperammon(em)ia or result in hyperammon(em)ia. In an other preferred embodiment, said conditions and disorders are related to or result in (absolute or relative) deficiency or excess of glutamate, and preferably selected from those disclosed in Tables 2 (in particular the conditions and disorders in *"italic")* and 3, in particular from the group consisting of Platelet disorders, Cardiac arrhythmias, Reiter's syndrome, Lupus erythematosus and associated conditions, Rheumatoid arthritis and associated conditions, Bone and joint injuries, Osteoporosis, Schizophrenia and other psychotic disorders, Penile and scrotal disorders, Sexual function and fertility disorders, Blood brain barrier defect and Nervous sytem disorders, with the exception of the conditions and disorders disclosed in Table 6. In a further preferred embodiment, said conditions and disorders are related to or result in (absolute or relative) deficiency or excess of glutamine, and/or sensitive to glutamine deprivation or glutamine supplementation, preferably selected from those disclosed in the Tables 2 and 3, with the exception of the conditions and disorders disclosed in Table 6.

In particular, the present invention concerns the use of bisphosphonates, salts thereof, isomers thereof, pro-drugs thereof, metabolites thereof and structural analogs thereof, preferably at "low or intermediate doses" i.e. doses resulting in concentrations which can increase or modulate/regulate GS activity (directly or indirectly) in the target cells/tissues/organs, in the manufacture of medicaments for preventing and/or treating all types of conditions and disorders which involve or can be influenced by GS activity, directly or indirectly, in particular those related to absolute or relative

excess of ammonia, those related to absolute or relative deficiency or excess of glutamate, and those related to absolute or relative deficiency or excess of glutamine, with the exception of the conditions and disorders disclosed in Table 7. The present invention also concerns a method for the prevention and/or the treatment of any condition or disorder selected from those disclosed in Tables 1-4, with the exception of the conditions and disorders disclosed in Table 7, comprising administering an efficient amount of a bisphosphonate or a salt thereof, an isomer thereof, a pro-drug thereof, a metabolite thereof or a structural analog thereof, thereby preventing or treating said condition or disorder. Preferably, said conditions and disorders are selected from those disclosed in Tables 1-4, preferably Tables 2-3, with the exception of those disclosed in Table 7. In a particular embodiment, said conditions and disorders are selected from the group consisting of asthma, cough, depression (major depression with or without melancholia, dysthymic disorders, depressed bipolar disorder, ...), irritable bowel syndrome, mnemo-cognitive disorders, neurodegenerative diseases (such as cerebral hypoxia, cerebral ischaemia, cerebral traumatism, cerebral ageing, Alzheimer's disease, multiple sclerosis, amyotrophic lateral sclerosis, demyelinating pathologies, encephalopathies, myalgic encephalomyelitis, chronic fatigue syndrome, post-viral fatigue syndrome, the state of fatigue and depression following a bacterial or viral infection, the dementia syndrome of AIDS, ...), nonulcer dyspepsia, pain, psychoneurotic disorders (neurotic or reactive states of depression, anxiodepressive states with somatic complaints such as digestive problems, anxiodepressive states observed in alcoholic detoxification, ...), stress and stroke. In a preferred embodiment, said conditions and disorders are related to or result in hyperammon(em)ia or result in hyperammon(em)ia. In an other preferred embodiment, said conditions and disorders are related to or result in (absolute or relative) deficiency or excess of glutamate, and preferably selected from those disclosed in Table 2 (in particular the conditions and disorders in *"italic")* and Table 3, in particular from the group consisting of Platelet disorders, Cardiac arrhythmias, Reiter's syndrome, Lupus erythematosus and associated conditions, Rheumatoid arthritis and associated conditions, Schizophrenia and other psychotic disorders, Penile and scrotal disorders, Sexual function and fertility disorders, Blood brain barrier defect, Nervous system disorders and Psychiatric disorders, with the exception of the conditions and disorders disclosed in Table 7. In a further preferred embodiment, said conditions and disorders are associated or related to or result in (absolute or relative) deficiency or excess of glutamine, and/or sensitive to glutamine deprivation or glutamine supplementation, preferably selected from those disclosed in Tables 2 and 3, with the exception of those disclosed in Table 7.

In an additional embodiment, a GS ligand selected from the group consisting of GF, MS, hydrazines and bisphosphonates, and salts, isomers, pro-drugs, metabolites and structural analogs thereof, is used to activate and/or modulate/regulate GS activity, intended for the activation and/or modulation/regulation of all types of functions which involve GS activity or can be influenced by GS activity, directly or indirectly, in others organisms with eukaryotic cells such as invertebrate animals (arthropods, protozoa, shellfish, worms, ...), algae, fungi (yeasts, molds, ...) or plants, but also prokaryotes e.g. algae or bacteria, aiming to facilitate their growth or protect them from stresses and disorders. In a most preferred embodiment, GS ligand is used at "low doses" i.e. doses resulting in compound concentrations which increase GS activity in the target cells/tissues/organs. Therefore, the invention concerns the use of a GS ligand selected from the group consisting of glufosinate (GF), L-methionine sulfoximine (MS), hydrazines and bisphosphonates, and salts, isomers, pro-drugs, metabolites and structural analogs thereof, for the preparation of a composition aiming to facilitate the growth of non-vertebrate organisms with eukaryotic cells such as invertebrate animals (arthropods, protozoa, shellfish, worms, ...), algae, fungi (yeasts, molds, ...) or plants, or prokaryotes e.g. algae or bacteria, or to protect them from stresses and disorders, wherein said GS ligand is used at "low or intermediate dose" i.e. a dose resulting in compound concentrations which activate GS activity, provided that if said GS ligand is GF, MS, or a bisphosphonate, plants are excluded.

Furthermore, the present invention concerns methods for screening, identifying and/or developing a medicament for prevention and/or treatment of all conditions and disorders demonstrated or proposed to be sensitive to tianeptine, comprising essentially the screening and the identification of compounds activating, modulating/regulating and/or inhibiting the GS activity.

The present invention also concerns methods for screening, identifying and/or developing a new biological active compound for the treatment of all types conditions and disorders which involve or can be influenced by GS activity, directly or indirectly, comprising an assay based on tianeptine.

Finally, the present invention concerns methods for screening, identifying and/or developing treatments activating and/or modulating/regulating the GS activity in organisms with eukaryotic cells other than plants e.g. algae, (vertebrate and invertebrate) animals including humans, and fungi (yeasts, molds, ...), and prokaryotes e.g. algae or bacteria, comprising an assay with GF, MS, an hydrazine e.g. isoniazid or iproniazid, or a bisphosphonate e.g. pamidronate or risedronate, or salts thereof, isomers thereof, pro-drugs thereof, metabolites thereof or structural analogs thereof.

## LEGEND TO THE FIGURES

**Figure 1:** Analytic HPLC (FIG.1A) and mass spectrometry (FIG.1B) profiles of biotinylated tianeptine.

**Figure 2:** Western blotting on BALB/c mouse hippocampus membrane proteins. Membrane proteins with peroxidase conjugated streptavidin in absence (lane 1) or presence (lane 2) of n-octyl glucoside. Membrane proteins with biotinylated tianeptine and peroxidase conjugated streptavidin in absence (lane 3) or presence of n-octyl glucoside (lane 4).

**Figure 3:** 2D gel electrophoresis and western blotting on BALB/c mouse hippocampus membrane proteins. The gel in FIG.3A is stained with colloidal blue (Coomassie R 250). The arrows point the two consecutive spots at 45 kDa, which are recognised by biotinylated tianeptine in western blot as it is shown in FIG.3B.

**Figure 4:** In silico study of the interaction between tianeptine and bovine brain GS. The model shows the tianeptine molecule (ball and stick) in its binding site (amino acids at a distance equal or less than 5Å of the center of the tianeptine molecule are shown as sticks). Glu A327 linked by a hydrogen bond to the NH of tianeptine is shown in red. The arginines A344 and A355 linked by a salt bridge to the carboxyl group of tianeptine are shown in dark blue. The two electropositive arginines A339 and A359 counterbalancing the electronegative $SO_2$ group of tianeptine are shown in blue. The other amino acids, forming the combining site (Glu A129, Glu A131, Tyr A179, His A210, Val A213, Asn A264, His A269, His A271, Pro A346 and Val A347) are coloured in yellow. Comparing the active site residues in the glufosinate (phosphinotricin) - glutamine synthetase complex (Gill and Eisenberg, 2001) with those in the tianeptine - glutamine synthetase complex, 10 out of the 12 amino acids are similar. The charge asymmetry of the binding site is conserved with the positive charges neutralizing the carboxyl group and the $SO_2$ group of tianeptine, and Glu A327 is blocked with a hydrogen bond of the NH group in the tianeptine cycle. Tianeptine thus recognizes the glutamine synthetase binding site in its closed form. This blocks the glutamate entrance to the catalytic site.

**Figure 5:** Effects of glufosinate (FIG.5A), iproniazid (FIG.5B), L-methionine sulfoximine (FIG.5C) and tianeptine (FIG.5D) on sheep brain GS subunit (monomer) activity. GS activity was measured as the amount gamma-glutamyl hydroxamate produced (absorbance at 535nm) in presence of 50 mM L-glutamine.

**Figure 6:** Inhibition of sheep brain GS activity (absorbance related to gamma-glutamylhydroxamate formation) by MS (5mM) and tianeptine (28 μM) in presence of various concentrations of L-glutamine (0.75-12.5 mM).

**Figure 7:** Effects of glufosinate (FIG.7A), iproniazid (FIG.7B), L-methionine sulfoximine (FIG.7C) and tianeptine (FIG.7D) on sheep brain GS activity. Activity of GS was measured as the amount of gamma-glutamyl hydroxamate produced (absorbance at 535nm) in presence of 50 mM L-glutamine.

**Figure 8:** Thin layer chromatography with different concentrations of tianeptine in presence of GS before (A) and after (B) 24 hours dialysis against reaction buffer (see material and methods). Lane 3: reaction buffer with 20 μM ADP; Lane 4: reaction buffer with 20 μM ADP, 5 nM tianeptine and 0.06 units GS; Lane 5: reaction buffer with 20 μM ADP, 1 nM tianeptine and 0.06 units GS; Lane 6: reaction buffer with 20 μM ADP, 0.1 nM tianeptine and 0.06 units GS. Lanes 1, 2 and 7 both in A and B are controls and have not been dialysed. Lane 1: 20 μM ADP in distilled water; Lane 2: 5 nM tianeptine in presence (A) or absence (B) of reaction buffer; Lane 7: 0.06 units GS in distilled water.

**Figure 9:** Inhibition of sheep brain GS activity by tianeptine (1-5 nM) and its revovery after a 24 h dialysis against the reaction buffer supplemented with 20 μM ADP.

**Figure 10:** Effects of L-glutamate (0.0016-10 mM) on the inhibition of sheep brain GS activity by 5 μM tianeptine in presence of 12.5 mM L-glutamine. The effect of tianeptine on GS activity is dependent on the concentration of L-glutamate.

**Figure 11:** Effects of tianeptine (100 nM) on extracellular free amine induced by glutamate (1(1), 10(2), 100(3) and 500(4) μM) after 1 h incubation of C6 cells in serum free HBSS. The effects of tianeptine alone (0.1(1), 1(2), 10(3), 100(4) nM), NMDA alone (1(1), 10(2), 100(3) and 500(4) μM), and NMDA (1(1), 10(2), 100(3) and 500(4) μM) plus tianeptine (100 nM) are shown for comparison. The effect of tianeptine on production of free amine is dependent on the dose of L-glutamate.

**Figure 12:** Effects of pamidronate on Wistar rat brain GS activity in absence (FIG.12A) or presence (FIG.12B) of 2β-mercaptoethanol (25 mM). Activity of GS was measured as the amount of gamma-glutamyl hydroxamate produced (absorbance at 535nm) in presence of 50 mM L-glutamine.

**Figure 13:** Effects of risedronate on Wistar rat brain GS activity in absence (FIG.13A) or presence (FIG.13B) of 2β-mercaptoethanol (25 mM). Activity of GS was measured as the amount of gamma-glutamyl hydroxamate produced (absorbance at 535nm) in presence of 50 mM L-glutamine.

**Figure 14:** Brain GS activity (FIG. 14A) and expression (FIG.14B) 24 h after a single intraperitoneal administration of different doses of MS or tianeptine (Tia) (0.078, 0.312, 1.25, 5, 20 mg/kg) in BALB/c mice. GS activity was measured as the amount of gamma-glutamyl hydroxamate produced (absorbance at 535nm) in presence of 50 mM L-glutamine. GS expression was assessed by western blotting and estimated using densitometry ($AU/px^2$).

**Figure 15:** Brain GS activity (A) and expression (B) after a 7 days repeated administration of glufosinate (GF) (0.1, 3, 15 mg/kg), L-methionine sulfoximine (MS) (0.5, 15, 45 mg/kg) or tianeptine (Tia) (0.5, 15, 45 mg/kg) in BALB/c mice. All the treatments were administered once a day intraperitoneally. The animals were decapitated 1 h after the

last administration on day 7. GS expression was determined using ELISA (absorbance at 405 nm).

**Figure 16:** GS activity in the cortex (FIG.16A), hippocampus (FIG.16B) and thalamus/hypothalamus (FIG.16C) of ICR mice 24 h after a single intraperitoneal administration of glufosinate (GF) or tianeptine (Tia). Activity of GS was measured as the amount of gamma-glutamyl hydroxamate produced (absorbance at 535nm) in presence of 50 mM L-glutamine.

**Figure 17:** Effects of tianeptine on Wistar rat brain GS activity in presence of ammonium dioxy persulfate (APS) (1.5%). Activity of GS was measured as the amount of gamma-glutamyl hydroxamate produced (absorbance at 535nm) in presence of 50 mM L-glutamine.

**Figure 18:** Effects of L-methionine sulfoximine (MS) and tianeptine (Tia) on extracellular $NH_4^+$ induced by $NH_4Cl$ 10 mM ($NH_4$) after 1h in C6 cells in serum free HBSS. The amount of $NH_4^+$ was determined according to Nessler's method.

**Figure 19:** Effects of L-methionine sulfoximine (MS) and tianeptine (Tia) on extracellular $NH4^+$ induced by glutamate 50 mM (Glu) and NH4Cl 10 mM ($NH_4$) after 1h incubation of C6 cells in serum free HBSS. The amount of $NH_4^+$ was determined according to Nessler's method.

**Figure 20:** Effects of L-methionine sulfoximine (MS) and tianeptine (Tia) on extracellular free amine induced by glutamate 50 mM (Glu) after 1h incubation of C6 cells in serum free HBSS. Quantitative Kaiser's method was used to determine the free amine concentration.

**Figure 21:** Effects of L-methionine sulfoximine (MS) and tianeptine (Tia) on extracellular free amine induced by NH4Cl 10 mM (NH4) after 1h incubation of C6 cells in serum free HBSS. Quantitative Kaiser's method was used to determine the free amine concentration.

**Figure 22:** Effects of L-methionine sulfoximine (MS) and tianeptine (Tia) on extracellular free amine induced by glutamate 50 mM (Glu) and NH4Cl 10 mM (NH4) after 1h incubation of C6 cells in serum free HBSS. Quantitative Kaiser's method was used to determine the free amine concentration.

**Figure 23:** Effects of L-methionine sulfoximine (MS) and tianeptine (Tia) on extracellular free amine (absorbance at 570 nm) after 1h and 24h incubation in C6 cells in serum free HBSS. MS alone interacts with the Kaiser's method (from 6.9. $10^{-7}$ M ).

**Figure 24:** Effects of glutamine (2 mM) on extracellular ammonium ion (A) and free amine (B) concentrations induced by NH4C1 10 mM (NH4) in presence L-methionine sulfoximine 5 mM (MS) or tianeptine 100 nM (Tia) after 1 h incubation in C6 cells in serum free HBSS. The blunting effects of MS and tianeptine on the NH4Cl-induced increase of ammonium were not reversed by glutamine.

**Figure 25:** Effects of L-methionine sulfoximine (MS) and tianeptine (Tia) on total apoptosis (early apoptosis and necrosis) induced by 10 mM NH4Cl (NH4) after 72 h in C6 cells cultured in DMEM.

**Figure 26:** Effects of L-methionine sulfoximine (MS) and tianeptine (Tia) on total apoptosis (early apoptosis and necrosis) induced by 50 mM glutamate (Glu) after 24 h in C6 cells cultured in DMEM.

**Figure 27:** Effects of L-methionine sulfoximine (MS) and tianeptine (Tia) on total apoptosis (early apoptosis and necrosis) induced by 50 mM glutamate (Glu) plus NH4C1 10 mM (NH4) after 24 h in C6 cells cultured in DMEM.

**Figure 28:** Effect of a repeated administration of glufosinate (GF) (0.1, 3 and 15 mg/kg), L-methionine sulfoximine (MS) (0.5, 15 and 45 mg/kg) or tianeptine (Tia) (0.5, 15 and 45 mg/kg) compared to vehicle (PCB) on resting time (%) in an open field task (10 min) in Wistar rats administered with 35 mg/kg corticosterone. All the treatments were administered once a day during 15 days. GF, MS and Tia were administered intraperitoneally. Corticosterone was administrered subcutaneously. Rats administered with both corticosterone's vehicle and treatment's vehicle were tested during the same experiment for comparison (Control).

**Figure 29:** Effect of a repeated administration of glufosinate (GF) (0.1, 3 and 15 mg/kg), L-methionine sulfoximine (MS) (0.5, 15 and 45 mg/kg) or tianeptine (Tia) (0.5, 15 and 45 mg/kg) compared to vehicle (PCB) on immobility (sec) in a forced swimming test (5 min) in Wistar rats administered with 35 mg/kg corticosterone. All the treatments were administered once a day during 19 days. GF, MS and Tia were administered intraperitoneally. Corticosterone was administrered subcutaneously. Rats administered with both corticosterone's vehicle and treatment's vehicle were tested during the same experiment for comparison (Control).

**Figure 30:** Effect of a 21 days repeated administration of glufosinate (GF) (0.1, 3 and 15 mg/kg), L-methionine sulfoximine (MS) (0.5, 15 and 45 mg/kg) or tianeptine (Tia) (0.5, 15 and 45mg/kg) compared to vehicle (PCB) on the decrease of surface of CA3 pyramidal neurons induced by a daily administration of 35 mg/kg corticosterone in Wistar rats. All the treatments were administered once a day during 21 days. The animals were decapitated 1 h after the last administration on day 21. GF, MS and Tia were administered intraperitoneally. Corticosterone was administered subcutaneously. Rats administered with both corticosterone's vehicle and treatment's vehicle were tested during the same experiment for comparison (Control).

**Figure 31:** Effect of a repeated administration of glufosinate (GF) (0.1, 3 and 15 mg/kg), L-methionine sulfoximine (MS) (0.5, 15 and 45 mg/kg) or tianeptine (Tia) (0.5, 15 and 45 mg/kg) compared to vehicle (PCB) on whole brain GS activity (FIG.31A) and expression (FIG.31B) in Wistar Rats administered with 35 mg/kg corticosterone. All the

treatments were administered once a day during 21 days. The animals were decapitated 1 h after the last administration on day 21. GF, MS and Tia were administered intraperitoneally. Corticosterone was administered subcutaneously. GS expression was determined using ELISA (absorbance at 405 nm). Rats administered with both corticosterone's vehicle and treatment's vehicle were tested during the same experiment for comparison (Control).

**Figure 32:** Effect of a repeated administration of glufosinate (GF) (0.1, 3 and 15 mg/kg), L-methionine sulfoximine (MS) (0.5, 15 and 45 mg/kg) or tianeptine (Tia) (0.5, 15 and 45 mg/kg) compared to vehicle (PCB) on liver (FIG. 32A) and lung (FIG.32B) GS activity in Wistar Rats administered with 35 mg/kg corticosterone. All the treatments were administered once a day during 21 days. The animals were decapitated 1 h after the last administration on day 21. GF, MS and Tia were administered intraperitoneally. Corticosterone was administered subcutaneously. Rats administered with both corticosterone's vehicle and treatment's vehicle were tested during the same experiment for comparison (Control).

**Figure 33:** Effect of a repeated administration of glufosinate (GF) (0.1, 3 and 15 mg/kg), L-methionine sulfoximine (MS) (0.5, 15 and 45 mg/kg) or tianeptine (Tia) (0.5, 15 and 45 mg/kg) compared to vehicle (PCB) on brain glucocorticoid receptor (GR) expression (FIG.33A) and adrenal weight (FIG.33B) in Wistar rats administered with 35 mg/kg corticosterone. All the treatments were administered once a day during 21 days. The animals were decapitated 1 h after the last administration on day 21. GF, MS and Tia were administered intraperitoneally. Corticosterone was administered subcutaneously. GR expression was determined using ELISA (absorbance at 405 nm). Rats administered with both corticosterone's vehicle and treatment's vehicle were tested during the same experiment for comparison (Control).

## DETAILED DESCRIPTION OF THE INVENTION

The Inventors have surprisingly discovered that tianeptine, an atypical antidepressant with neuroprotective properties, is a specific ligand of the enzyme glutamine synthetase (GS; EC 6.3.1.2). Depending on the concentration/dose and experimental or (patho)physiological condition, tianeptine can activate, inhibit or modulate/regulate GS activity in animals and bacteria like observed previously, only in plants, with GF and MS, and derivatives thereof (Estigneeva et al., 2003). These modulatory/regulatory effects of tianeptine on GS function can likely underlie its already proposed clinical applications (see above) contrary to its unclear effects on 5-HT uptake that are usually proposed. They can also be reconciled with the emerging pharmacological profile of tianeptine, which suggests that tianeptine may serve to balance glutamatergic neurotransmission (Plaisant et al., 2003; Reagan et al., 2004). So, the Inventors have found a solution for one of the classical paradoxes against the monoamine theory of depression: "If blocking serotonin reuptake is important in the treatment of depression, then why is tianeptine, which enhances serotonin reuptake, an effective antidepressant? ", a fair explanation for the neuroprotective effects of tianeptine, a plausible mechanism for its effects on asthma which are usually considered "provocative and worth exploring" when proposed to be related to effects on serotonin, etc..

In vertebrate animals including humans, as well as in other (all) organisms with eukaryotic cells such as algae, invertebrate animals (arthropods, protozoa, shellfish, worms, ...), fungi (yeasts, molds, ...) or plants, but also prokaryotes e.g. algae or bacteria, L-glutamine serves as the most important carrier of nitrogen and one of the major build stones in protein synthesis. Acting as a respiratory fuel, it provides also energy. These explain why glutamine homeostasis is carefully balanced. The equilibrium is due to a precise control of glutamine consumption and production, both within cells, tissues, organs, ... Transport of glutamine into and out of cells plays an important role in the control of this process. However, ultimately, the balance between glutamine anabolism and catabolism, which relies largely on the activity of two enzymes, glutamine synthetase and glutaminase, is crucial.

Glutamine catabolism is initiated through its deamidation to form glutamate. This can proceed via a number of cytosolic transamidase enzymes that use the γ-amido nitrogen of glutamine in a variety of metabolic syntheses (Zalkin and Smith, 1998). The rate at which these reactions utilize glutamine depends upon the metabolic demand for the reaction products and is, therefore, not appropriate for control of glutamine homeostasis. On the contrary, the mitochondrial enzyme glutaminase catalyzes the hydrolysis of the γ-amido group of glutamine to form glutamate and ammonia. Ammonia can be used to form carbamoyl phosphate or can diffuse from the mitochondria and the cell itself. Glutamate can be further deaminated by glutamate dehydrogenase (GDH) to form α-ketoglutarate and thus enter the citric acid cycle. Thus, the increase of glutamine catabolism through glutaminase, under physiological conditions, does not lead to production of excessive amounts of specific metabolites.

In contrast to the many enzymes that utilize glutamine as a substrate, only one enzyme, GS, is responsible for de novo synthesis of glutamine. This ligase catalyses, under normal conditions, the formation of glutamine from glutamate and ammonia.

$$\text{Glutamate} + NH_3 + ATP \overset{Mg^{2+}}{\rightleftharpoons} \text{L-glutamine} + ADP + P_i$$

The glutamine synthetases of animal origin resemble each other with respect to amino acid composition, subunit structure and molecular weight (Meister 1985). Most of animal cells make a version with eight subunits, each of which has an active site for production of glutamine. Briefly, when performing its reaction, the active site of GS binds to glutamate and ammonia, and to an ATP molecule that powers the reaction. *In vitro,* GS converts around 90% of glutamate to glutamine when it is incubated with equal concentrations of L-glutamate, ammonium ion and ATP, in presence of $Mg^{2+}$, at pH 7.0 and 37°C. GS activity depends on the concentrations of its substrates and products, of divalent cations like $Mg^{2+}$ and $Mn^{2+}$, of ADP/ATP, ... Its mechanism can be described as ternary, either order or random ter ter, i.e. the interaction of its substrates at different concentrations and cellular conditions can lead to production of various metabolites. This is schematized below:

Order Ter Ter

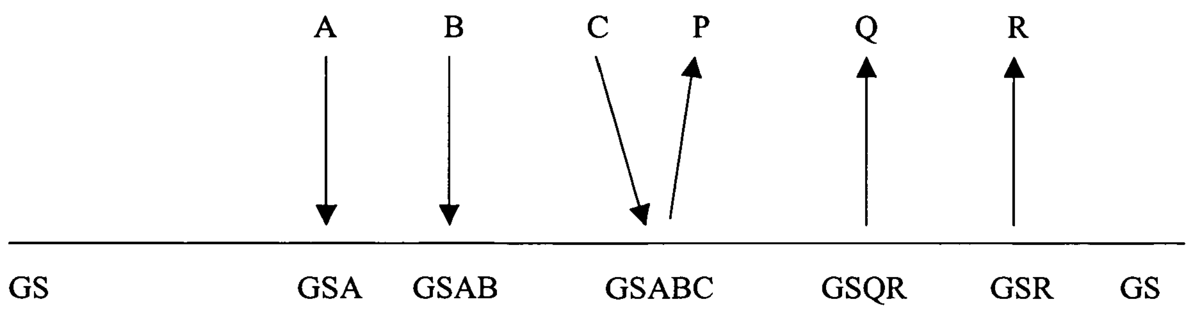


Random Ter Ter

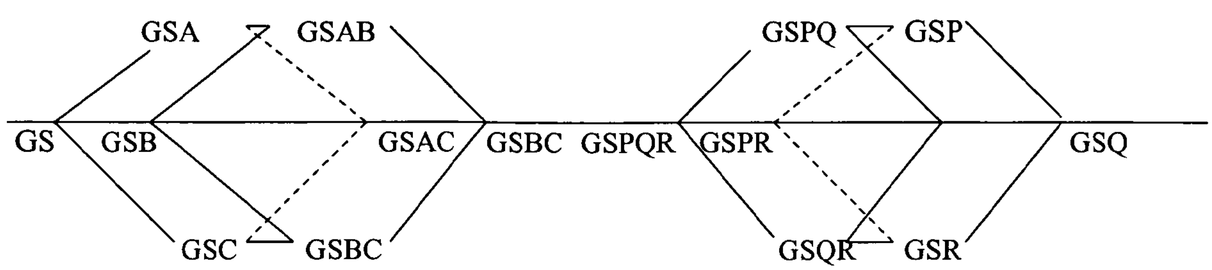


where substrates are A, B, C, and products are P, Q, R.

Partial reactions catalyzed by GS include the $\gamma$-glutamyl transfer reaction, the arsenolysis of glutamine, the synthesis of $\gamma$-glutamyl hydroxamates and $\gamma$-carboxyl-activated glutamate derivatives, ... (Meister, 1985). All these reactions can take place e.g. the latter is activated in the absence of ammonia, where GS catalyses the conversion of glutamate to 5-oxoproline. Glutamate attaches to the enzyme only when both ATP and a divalent cation (usually $Mg^{2+}$) are present. So the reactions catalyzed by GS are clearly not straightforward. The schema below illustrates the mechanism of action of GS with reversal and partial reactions as well as the products of the metabolization of L-methionine-S-sulfoximine (MS; L-MSO) (Meister, 1985).

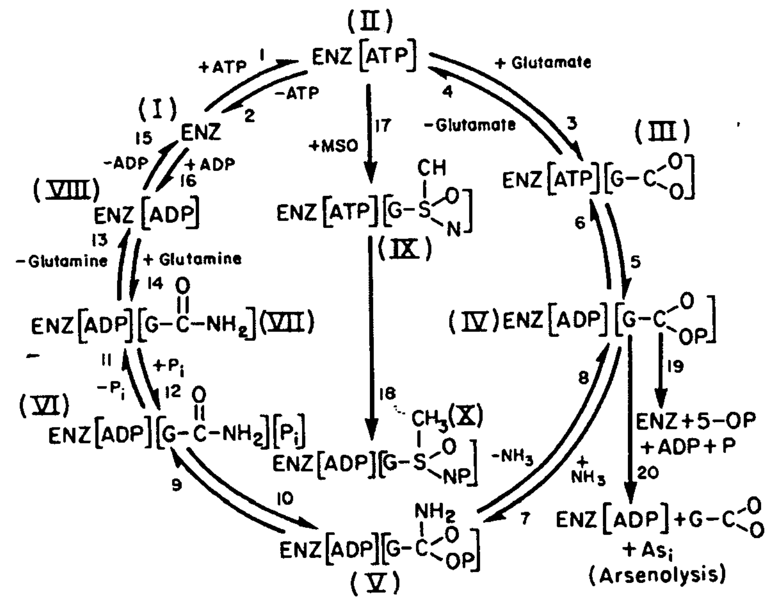


Scheme of the mechanism of action of glutamine synthetase: G, $^{-}OOCCH(NH_3^{+})(CH_2)_2$. Glutamine synthesis: 1, 3, 5, 7, 9, 11, 13, 15. Reversal of synthesis: 16, 14, 12, 10, 8, 6, 4, 2. Partial reactions: γ-Glutamyl transfer: 16, 14, 12, 10, 8, 7 ($+NH_2OH$) 9, 11, 13. Arsenolysis of glutamine: 16, 14, 12 ($+As_i$), 10, 8, 20. Phosphorylation of L-methionine-S-sulfoximine: 1, 17, 18. Cyclization: formation of 5-oxoproline (5-OP): 1, 3, 5, 19. Phosphorylation of glutamate analog (cycloglutamate): 1, 3, 5. Acyl phosphate (β-glutamyl - P, carbamyl - P, acetyl - P) + ADP ⇒ ATP: 6, 4, 2 (adapted from Meister, 1985).

GS active sites can also bind to other compounds, which may partially block the action of the enzyme e.g. to amino acids like alanine, glycine, histidine, serine, tryptophane, ... but also adenosine monophosphate, carbamyl phosphate, cytidine triphosphate, glucosamine 6-phosphate, ... As the concentrations of these "effectors" rise, more and more GS sites are occupied, eventually shutting down the whole enzyme. On the contrary, certain amino acids or xenobiotic compounds such as glufosinate (GF; phosphinothrycin (PPT)) and L-methioninesulfoximine (MS; L-MSO) and derivatives thereof, which are usually known as GS inhibitors, at low concentrations/doses in plants behave as GS activators (Pushkin et al., 1974 and 1975; Evstigneeva et al., 2003). So a large body of evidence indicates that GS effectors can cause subunit but also whole enzyme conformational changes, which result in GS activation and/or inhibition (Eisenberg et al., 2000).

Expression of GS in mammalian is regulated by transcriptional and post-transcriptional mechanisms. In particular increased transcription occurs due to glucocorticoid action and regulation of protein stability is linked to glutamine concentration. Glucocorticoid administration as well as various stresses have been observed to elevate GS mRNA level through glucocorticoid receptor-dependent mechanisms (Abcouwer et al., 1995 and 1996; Ardawi et al., 1990; Ardawi and Majzoub, 1991; Chandrasekhar et al., 1999; Lukaszewicz et al., 1997). However, not always is there a correlation between the expression of GS at both mRNA and protein levels. The ability of glutamine to promote GS protein turnover via a post-transcriptional mechanism, in which the rate of GS protein degradation is altered, is one of the bases of this irregularity. Through such mechanisms, glutamine production is adapted to the glutamine demand in a tissue-specific fashion (Arad et al., 1976; Arad and Kulka, 1978; Milman et al., 1975; Feng et al., 1990; Lacoste et al., 1982; Patel et al., 1986). In first approach, the specific activity of animal GS seems to be less affected by post-translational modifications contrary to bacterial GS. In fact, in animals, GS nitrification is very likely, but no one has found adenylation nor phosphorylation as observed in bacteria (Kosenko et al., 2003).

Xenobiotics have been demonstrated to influence GS expression too. For instance MS (100 mg/kg) and dexamethasone (0.5 mg/kg) were reported individually to augment rat lung GS protein levels 4-fold and 2-fold, respectively, and their combination 12-fold (Labow et al., 1998).

So depending on cell type, tissue, organ, species, (patho)physiological condition or treatment, GS mRNA, GS protein level and GS activity are obviously variable. Because both GS substrates i.e. ammonia and L-glutamate are usually not limiting, the rate of L-glutamine formation is usually highly dependent upon the activity of GS, thus on cofactors and effectors concentrations.

The Inventors have discovered that tianeptine is a selective ligand of the GS subunit catalytic site (Figure 4) and when it binds consequently the catalysing activity of the subunit is impeded (Figures 4 and 5D). However, while

"high concentrations" of tianeptine, i.e. concentrations which occupy (more or less) all the subunits of the enzyme, result "obligatorily" in inhibition of GS activity (Figures 5D, 6 and 7D), "lower concentrations", i.e. concentrations where only one (or few) GS subunit(s), are occupied can result in an increase or a decrease of GS activity, or can be pharmacologically tolerated, depending on the experimental and/or (patho)physiological condition (Figures 7D, 14A and 15A). Considering that tianeptine-induced GS activation can be observed in vitro on purified GS whole enzyme (Figure 7D), but not on isolated subunits (monomer) (Figure 5D), it may be speculated that tianeptine, in (impeding) binding to one (or few) GS subunit(s) can cause conformational changes that result in the activation or inhibition of the other non occupied subunits; since tianeptine seems to bind GS only in its closed form (Figure 4), its modulatory/regulatory activity depends on the number of sites already occupied by the natural substrates, cofactors and effectors. Tianeptine can also protect GS from oxidative stress (Figure 17) and raise GS protein expression in vivo (Figure 14B, 15B and 31B). So, while "high concentrations" of tianeptine result "obligatorily" in an inhibition of GS activity - these concentrations are toxic in eukaryotes and prokaryotes, and can serve as cytolytics e.g. to combat neoplasms in animals, or growth in algae, fungi, plants or bacteria -, "low concentrations" of tianeptine can result in an increase of GS activity, which may be of interest in conditions and disorders demanding an increase of GS activity/response and/or comprising an altered GS activity. "Intermediate concentrations" of tianeptine can result in an increase or a decrease of GS activity or be tolerated depending on the experimental and/or (patho)physiological condition. So tianeptine may influence several aspects of the GS homeostatic activity i.e. can be proposed to modulate/regulate all types of functions which involve or can be influenced by GS activity, directly or indirectly, in particular to be of major therapeutic interest in preventing and/or treating all the conditions and disorders related to (relative or absolute excess of) ammonia, (relative or absolute deficiency or excess of) glutamate, and/or (absolute or relative deficiency or excess of) glutamine. In fact, tianeptine as well as GF and MS (at "low/intermediate concentrations/doses") were observed, in vitro, to prevent cellular apoptosis in conditions of elevated ammonia and/or elevated glutamate concentrations (Figures 25-27) and, in vivo, to counteract hippocampal CA3 structural changes associated with chronic glucorticoid administration (Figure 30), and these effects could be explained by their influence on GS (Figures 18-22, 31).

In addition, the Inventors have discovered that GS ligands which are well established as GS inhibitors in animals like GF and MS, as well as hydrazines and bisphosphonates, can affect GS activity more or less as tianeptine does. Certain hydrazines were already known to bind GS (Rueppel et al., 1972). The Inventors have demonstrated for the first time that it is also the case for the well established antituberculous agent isoniazid and its derivative the typical monoamine oxidase inhibitor (MAOI) antidepressant iproniazid. Certain bisphosphonates like [(5-chloro-pyridin-2-ylamino)-phosphonomethyl]-phosphinic acid were already known to bind GS and to exhibit an herbicidal activity. The Inventors have shown for the first time that bisphosphonates which are usually used for the treatment of osteoporosis and osteolytic disease like pamidronate and risedronate bind GS as well. All these "other GS ligands" present a GS activating and/or modulating/regulating activity in animals when they are used at concentrations/doses lower as compared to the concentrations/doses used to inhibit GS activity, like observed previously, only in plants, with GF, MS and derivatives thereof (Figures 7, 12-17, 31-32): in outline i) at "low concentrations/doses", these GS ligands behave as GS activators; ii) at "intermediate concentrations/doses", they are tolerated, or activate or inhibit GS activity depending on the experimental or (patho)physiological condition, in particular on the concentrations of substrates, cofactors and effectors...; while iii) at "high concentrations/doses", they behave as GS inhibitors. These effects on GS function may underlie, at least in part, the already proposed clinical applications of hydrazines (see Table 6) and bisphosphonates (see Table 7). So there is compelling evidence for the use of GS ligands like GF and MS but also of hydrazines and bisphosphonates, and isomers thereof, pro-drugs thereof, metabolites thereof and structural analogs thereof, either alone or combined, for treating conditions and disorders demonstrated or proposed to be sensitive to tianeptine, even though their selectivity for GS, their interaction with GS or their pharmacokinetics differ to some extent; for instance MS is a non-selective irreversible GS ligand (Rao and Meister, 1972; Meister and Tate, 1976) while tianeptine is a selective, reversible GS ligand (Figures 4, 8-9). There are convergent arguments for the use of GS ligands like GF and MS as well as of bisphosphonates and hydrazines, and isomers thereof, pro-drugs thereof, metabolites thereof and structural analogs thereof, in obtaining activators and/or modulators/regulators of GS activity intended for activating and/or to modulating/regulating all types of functions which involve GS activity or can be influenced by GS activity, directly or indirectly, and/or preventing and/or treating all types of conditions and disorders which involve or can be influenced by GS activity, directly or indirectly, in particular those related to absolute or relative excess of ammonia, those related to absolute or relative deficiency or excess of glutamate, and those related to absolute or relative deficiency or excess of glutamine, either isolated or combined. Conversely there are also reasonable arguments to propose the use of tianeptine, isomers thereof, pro-drugs thereof, metabolites thereof and structural analogs thereof, for preventing and/or treating all conditions and disorders demonstrated or proposed to be sensitive to GS ligands like GF MS, bisphosphonates and hydrazines, or isomers thereof, pro-drugs thereof, metabolites thereof or structural analogs thereof.

Preferably, "GS ligand(s)" encompasses compounds able to bind GS (subunit) in its catalytic site, and more particularly in the glutamate binding site. Preferably, these compounds are able to compete with a compound selected from the group consisting of glutamate, GF, MS, and tianeptine for the catalytic site of GS. In a preferred embodiment,

without to be limited thereto, this compound present an inhibition constant (Ki) equal or less than $10^{-4}$ M.

Preferred examples of GS ligand(s) are selected from the group consisting of tianeptine, GF, MS, hydrazines, bisphosphonates and glutamate analogs, and isomers thereof, pro-drugs thereof, metabolites thereof and structural analogs thereof (Tables 8-13). More preferably, GS ligand(s) are selected from the group consisting of tianeptine, GF and MS, and isomers thereof, pro-drugs thereof, metabolites thereof and structural analogs thereof (Tables 8-10).

Examples of tianeptine isomers, pro-drugs, metabolites and structural analogs include, without to be limited thereto, the compounds disclosed in Table 8.

Examples of GF isomers, pro-drugs, metabolites and (structural) analogs include, without to be limited thereto, the compounds disclosed in Tables 9 and 13, preferably in Table 9. Preferably, GF isomers, pro-drugs, metabolites and structural analogs are selected from the group consisting of glufosinate, gamma-hydroxy phosphinothricin, gamma methyl phosphinothricin, gamma-acetoxy phosphinothricin, alpha-methyl phosphinothricin, alpha-ethyl phosphinothricin, cyclohexane phosphinothricin, cyclopentane phosphinothricin, tetrahydrofuran phosphinothricin, S-phosphonomethyl homocysteine, s-phosphonomethyl homocysteine sulfoxide, s-phosphonomethyl homocystein sulfone, 4-(phosphonoacetyl)-L-alpha-aminobutyrate, threo-4-hydroxy-D-glutamic acid, erythron-4-fluoro-D,L-glutamic acid, 2-amino-4-[(phosphonomethyl)hydroxyphosphinyl]butanoic acid, 2-amino-4-phosphono butanoic acid, 2-amino-2-methyl-4-phosphono butanoic acid, 4-amino-4-phosphono butanoic acid, 4-amino-4-(hydroxymethylphosphinyl)butanoic acid, 4-amino-4-(hydroxymethylphosphinyl)-4-methyl butanoic acid, 4-amino-4-phosphono butanamide, 2-amoido-4-phosphono butanoic acid, 2-methyoxycarbonyl-4-phosphono butanoic acid methyl 4-amino-4-phosphono butanate and 2-amido-4-phosphono butanoic acid, and salts thereof and isomers thereof.

Examples of MS isomers, pro-drugs, metabolites and (structural) analogs include, without to be limited thereto, the compounds disclosed in Tables 10 and 13, preferably in Table 10.

Preferably, "hydrazines" encompasses compounds able to bind GS in its catalytic site, and more particularly in the ammonium binding site. Preferably, this compound is able to compete with a compound selected from the group consisting of iproniazid and isoniazid for the catalytic site of GS. Preferred examples of hydrazines binding to GS are selected from the group of iproniazid and isoniazid, and isomers thereof, pro-drugs thereof, metabolites thereof and structural analogs thereof (Table 11). Preferably, the hydrazine is selected from the group which comprises isoniazid, iproniazid, pyridoxal isonicotinoyl hydrazone, pyridoxal benzoyl hydrazone, salicylaldehyde isonicotinoyl hydrazone, salicyaldehyde benzoyl hydrazone, 2-hydroxy-1-naphthaldehyde isonicotinoyl hydrazone, 2-hydroxy-1-naphthaldehyde benzoyl hydrazone, isonicotinic acid N'-isopropyl-hydrazide, 4-chloro-N(2-morpholinoethyl)benzamide, 5-methylisoxazole-3-carboxylic acid N'-(3-methyl-benzyl)-hydrazide, phenethyl-hydrazine (phenelzine), DL-Serine 2-(2,3,4-trihydroxybenzyl)hydrazide (benserazide), butylmalonic acidmono-(1,2-diphenylhydrazide) (bumadizone), 5-(3,3-dimethyl-1-triazenyl)-1H-imidazole-4-carboxamide (dacarbazine), 1,4-Dihydrazino-5-azaphthalazine (dihydralazine), 1-hydrazinophthalazine (hydralazine), {1-[2-(4-Chlorophenoxy)-ethoxy]-ethyl}-hydrazine (iproclozid), 6-Methyl-[1,2]oxazinane-3-carboxylic acid N'-cyclohexyl-hydrazide (isocarboxazid), pyridine-4-carboxylic 2-[2-(benzylcarbamoyl)ethyl]hydrazide (nialamide), 5-nitro-2-furaldehyde p-hydroxybenzoylhydrazone (nifuroxazide), cyclohexa-2,4-dienecarboxylic acid hydrazide (phenicarbazide), N-(3,4-dihydro-phthalazin-6-yl)-*N'*-(1,2-dihydro-pyridin-4-yl)-hydrazine (picodralazine) and N-isopropyl[(methyl2hydrazino)methyl]-p-toluamide (procarbazine), and salts thereof and isomers thereof.

Preferably, "bisphosphonates" encompasses compounds able to bind GS in its catalytic site, and more particularly in the $Mn^{2+}$ binding sites. Preferably, this compound is able to compete with a compound selected from the group consisting of $Mn^{2+}$, $Mg^{2+}$, pamidronate, risedronate, and [(5-Chloro-pyridin-2-ylamino)-phosphono-methyl]-phosphinic acid for the catalytic site of GS. Preferred examples of bisphosphonates binding to GS are selected from the group consisting of pamidronate, risedronate and [(5-Chloro-pyridin-2-ylamino)-phosphono-methyl]-phosphinic acid, and isomers thereof, pro-drugs thereof, metabolites thereof and structural analogs thereof (Table 12). Preferably, bisphosphonates are selected from the group consisting of 1-phosphono-2-pyridin-2-ylamino)-ethyl]-phosphonic acid, 6-(2-Amino-ethyl)-pyridin-3-Ol-phosphonic acid, [3,4-Dichloro-phenyl)-phosphono-methyl]-phosphonic acid, 2-amino-4-[(phosphonomethyl)hydroxyphosphinyl]butanoic acid, [(5-Chloro-pyridin-2-ylamino)-phosphono-methyl]-phosphonic acid, alendronate, clodronate, etidronate, ibandronate, pamidronate, risedronate and zoledronate, and salts thereof and isomers thereof.

Examples of glutamate analogs include, without to be limited thereto, the compounds disclosed in Table 13.

"Prodrug" of a compound refers to a chemical entity which, after administration to a subject, is converted in the compound and which displays similar therapeutic activity and/or biological effect to the compound.

"Metabolite" of a compound refers to a chemical entity which is obtained following the administration of the compound to a subject and which displays similar therapeutic activity and/or biological effect to the compound.

"Structural analog" of a compound refers to a chemical entity which comprises chemical changes which do not affect substantially the therapeutic properties and/or biological activity of the compound.

In the context of the present invention, when a combination of a GS ligand and an other active ingredient is considered, the composition containing the GS ligand according to the present invention with the other active ingredient can be as a combined preparation for simultaneous, separate or sequential use in the same therapy. It will be appreciated

that the compounds of the combination may be administered simultaneously, either in the same or different pharmaceutical formulations or sequentially. If there is sequential administration, the delay in administering the second active ingredient should not be such as to lose the benefit of the efficacious effect of the combination of the active ingredients.

Whenever within this whole specification "treatment of a condition or disorder" or the like is mentioned with reference to a GS ligand, there is meant:

a) a method for treating a condition or disorder, said method comprising administering a GS ligand to a subject in need of such treatment;
b) the use of a GS ligand for the treatment of a condition or a disorder;
c) the use of a GS ligand for the manufacture of a pharmaceutical preparation for the treatment of a condition or a disorder; and/or
d) a pharmaceutical preparation comprising a dose of a GS ligand that is appropriate for the treatment of a condition or a disorder.

By "treatment" one means in the present application a treatment aiming to prevent or to cure a condition or a disorder. By "cure/treat a condition or a disorder" one means that the treatment alleviates, reduces or abolishes its symptom(s) and/or cause(s) (etiology).

By "low concentrations/doses" one means in the present application that the GS ligand behaves as a GS activator in the target cells/tissues/organs. By "intermediate concentrations/doses" one means that the GS ligand decreases or increases the activity of GS depending on the experimental or (patho)physiological condition in the target cells/tissues/organs. By "high concentrations/doses" is intended that the GS ligand behaves as a GS inhibitor in the target cells/tissues/organs. By "modulate" is preferably intended that the GS activity can be increased or decreased. By "regulate" is preferably intended that the level of GS activity becomes normal.

***Functions, conditions and disorders related to ammonia***

Ammonia is a molecule with many physiological roles. It serves as an important substrate for several enzymatic reactions, and is a normal product of degradation of proteins and other nitrogen compounds.

In mammals, liver usually has the responsibility to detoxify systemic (plasma) ammonia through its incorporation into urea which will be eliminated in urine. Although normally from protein digestion large quantities of ammonia enter the portal vein, arterial ammonia is maintained at a low concentration (50—100 μM range).

Number of conditions/disorders are associated with hyperammonemia i.e. a systemic ammonia concentration increase. In some of these conditions/disorders, hyperammonemia is the primary defect; in others, it is secondary. Liver failure (due to cirrhosis, hepatitis, intoxication, ...), inborn errors of metabolism (urea cycle disorders, transport defects of intermediates in the urea cycle, organic acidurias, ...), porto-systemic shunts as well as "non-hepatic" critical conditions/disorders, alone or combined, can result in a systemic ammonia concentration increase (see Tables 1 and 2 (the "double-underlined" conditions and disorders")) (Jones and Weissenborn, 1997; Hawkes et al., 2001; Felipo and Butterworth, 2002b; Leonard and Morris, 2002). High concentrations of ammonia can be also more localized due to local increased protein degradation and/or deficiency of local detoxification/elimination/use e.g. local critical stress due to anoxia, cancer, degeneration, infection, inflammation, ischaemia, surgery, trauma, ... Potential factors and mechanisms which can underlie these conditions and disorders are disclosed in Table 4, without to be limited thereto.

Increased ammonia concentration has particularly deleterious effects in the central nervous system. Depending upon the species, the age, and the magnitude and duration of exposure, ammonia may result in a spectrum of neuropsychiatric symptoms including asterixis (flapping tremor), irritability, lethargy, mental retardation, somnolence, disorientation, seizure and coma, and in neuronal cell damage or loss; brain edema sufficient to cause increased intracranial pressure and death by brain herniation is a classic complication of severe acute hyperammonemia (Jones and Weissenborn, 1997; Hawkes et al., 2001; Felipo and Butterworth, 2002b; Leonard and Morris, 2002). Clinical signs associated with localized ammonia increases depend on the cell/tissue/organ function(s), and duration, intensity and nature of the local condition/disorder.

The molecular mechanisms of ammonia toxicity with regard to CNS function are still incompletely understood (Jones and Weissenborn, 1997; Hawkes et al., 2001; Felipo and Butterworth, 2002a,b; Kosenko et al., 2003). One hypothesis stresses the interference of ammonia with glutamate/glutamine metabolism, suggesting that toxic effects may be mediated by subsequent ammonia metabolism rather than by ammonia itself (Hawkins et al., 1993; Zwingmann et al., 1998). Since brain lacks an effective urea cycle, cerebral ammonia removal relies mainly on glutamine synthesis (Cooper et al., 1985; Cooper and Plum, 1987). Glutamine concentration in the cerebrospinal fluid correlates reasonably with the clinical grade of hepatic encephalopathy (Hourani et al., 1971). High brain glutamine concentrations are commonly found in patients with hepatic encephalopathy as well as in experimentally induced hyperammonemia (Kanamori et al., 1997; De Graaf et al., 1991; Ross et al., 1996; Vogels et al., 1997). Glutamine synthetase is highly concentrated

in astrocytes (Norenberg and Martinez-Hernandez, 1979) and a considerable body of evidence implicates astrocytes in the pathogenesis of hepatic encephalopathy (Norenberg, 1981 and 1986; Norenberg et al., 1987). GS ligands known as GS inhibitors have been reported to alleviate acute deleterious effects of high ammonia concentration on astrocytes. In glial cell cultures, MS was observed to prevent the cell swelling seen with exposure to 5 mM ammonium salt (Norenberg and Bender, 1994; Isaacks et al., 1999). *In vivo,* MS was observed to prevent the increase in cortical glutamine concentration, water content and intracranial pressure as well as the swelling of astrocyte perivascular processes, that are seen in rats when plasma ammonia concentration is increased to 500-600 μM (Takahashi et al., 1991; Takahashi et al., 1992; Willard-Mack et al., 1996). However, the astrocytic glutamine is partly released into the blood (Norenberg and Martinez-Hernandez, 1979), which means a net reaction to remove excess ammonia from the brain. A rise in brain GS activity may improve the metabolic balance between astrocytes and neurons in hyperammoniemic situations; in particular it may alleviate excessive glutamatergic activation (Tsacopoulos et al., 1997 ; Butterworth, 1993 ; Norenberg et al., 1992; Kosenko et al., 2003). GS is not working at its maximum rate in hyperammoniemic situations (Kosenko et al., 2003). So it is now most often considered useful to increase GS activity in hyperammoniemic situations, at least in conditions of chronic hyperammonemia (Kosenko et al., 2003).

While measures to decrease the quantities of ammonia which enter the circulation from protein digestion in the gastrointestinal tract are most often a comer-stone in the treatment of hyperammoniemic syndromes (for the general principles relevant to the management of hepatic and non-hepatic hyperammonemia see: Jones and Weissenborn, 1997; Hawkes et al., 2001; Felipo and Butterworth, 2002b), altering peripheral GS activity may additionally improve systemic ammonia detoxification/removal. Since skeletal muscle is an important organ involved in systemic ammonia removal, a rise in muscular GS activity may be particularly useful. For instance, zinc supplementation has been proposed in the prevention of hepatic encephalopathy by activating glutamine synthetase; an increased uptake of ammonia by leg skeletal muscle and an increased release of glutamine from leg skeletal muscle were observed after oral supplementation with zinc sulfate in patients with decompensated liver cirrhosis (Yoshida et al., 2001). Activating GS activity may as well improve local ammonia detoxification in case of local increase of ammonia (see also below the importance of local de novo synthesis of glutamine).

Accordingly, the present invention concerns the use of tianeptine, salts thereof, isomers thereof, pro-drugs thereof, metabolites thereof and structural analogs thereof, in the manufacture of medicaments intended for prevention and/or treatment of conditions and disorders related to or resulting in a local excess of ammonia or a systemic excess of ammonia i.e. hyperammon(em)ia. The present invention also concerns a method of treatment of hyperammon(em) ia in a subject comprising administering to said subject an efficient amount of tianeptine or a salt thereof, an isomer thereof, a pro-drug thereof, a metabolite thereof or a structural analog thereof. Preferably, said systemic ammonia concentration increases are related to a severe liver disease (due to cirrhosis, hepatitis, intoxication, ...), an inborn error of metabolism (urea cycle disorder, transport defect of intermediate in the urea cycle, organic aciduria, ...), a porto-systemic shunt, and/or a "non hepatic" (critical) disorder (see Tables 1 and 2 (the "double-underlined" conditions and disorders)). Preferably, said localized ammonia increases are related to a local critical stress due to anoxia, cancer, degeneration, infection, inflammation, ischaemia, surgery, trauma, ... Preferably, are considered by the present invention the use of tianeptine, salts thereof, isomers thereof, pro-drugs thereof, metabolites thereof and structural analogs thereof, in the manufacture of medicaments intended for prevention and/or treatment of conditions and disorders selected from the group consisting of those disclosed in Tables 1 and 2 (the double-underlined conditions and disorders), in particular severe hepatic disorders (resulting from cirrhosis, hepatitis, intoxication, ...), errors of metabolism (urea cycle disorder, transport defect of intermediate in the urea cycle, organic aciduria, ...), porto-systemic shunts, gastrointestinal haemorrhages, transplant rejections and catabolic states, with the exception of the conditions and disorders disclosed in Table 5. Potential factors and mechanisms which can underlie these conditions and disorders are disclosed in Table 4, without to be limited thereto.

For instance, tianeptine metabolites and analogs are described in FR 2 104 728 and FR 2 635 461 (the disclosure thereof being incorporated herein by reference) or in Sanchez-Mateo et al., 2003. In particular, tianeptine analogs can have the following formula:

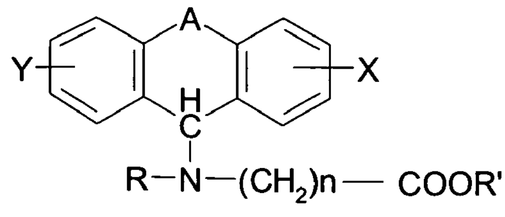

in which A represents a bridge selected from the group consisting of $-(CH_2)_m-$, $-CH=CH-$, $-(CH_2)_p-O-$, $-(CH_2)_p-S-$,

$-(CH_2)_p-SO_2-$, $-(CH_2)_p-NR_1-$, and $-SO_2-NR_2-$; *m* is an integer 1, 2, or 3; *p* is 1 or 2; R1 represents a hydrogen atom or a lower alkyl radical containing 1 to 5 carbon atoms, and R2 represents a lower alkyl radical containing 1 to 5 carbon atoms, X and Y may be the same or different and each represents a hydrogen atom or a halogen atom (e.g., F, Cl, Br); R and R' may be the same or different and each represents a hydrogen atom or a lower alkyl radical containing 1 to 5 carbon atoms; and n is an integer from 1 to 12. Preferably, A is $-SO_2-NR_2-$.

In a preferred embodiment, tianeptine metabolites or structural analogs are selected from the compounds disclosed in Table 8.

Accordingly, the present invention concerns also the use of tianeptine, salts thereof, isomers thereof, pro-drugs thereof, metabolites thereof and structural analogs thereof, in the manufacture of medicaments to be combined with other (etiologic and/or symptomatic) drugs or measures intended i) to prevent/treat hyperammonemia and/or hyperammonemia symptoms, in particular benzodiazepine receptor antagonists (flumazenil, ...), zinc supplementation and measures to minimize absorption of nitrogenous substances e.g. dietary restriction, evacuation of bowel, lactulose (or a related sugar) and/or broad spectrum antibiotics (for example, neomycin) in liver failure; or sodium benzoate and sodium phenylacetate in conjunction with a low protein diet and a specific amino acid supplementation in carbamylphosphate synthetase, ornithine transcarbamylase or arginosuccinate synthetase deficiency; or sodium benzoate, sodium phenylacetate, arginine hydrochloride and/or haemodialysis, after solid-organ transplantation; or ii) resulting in hyperammonemia, in particular certain anaesthetic agents (halothane, enflurane, ...), anti-cancer therapies (5-fluorouracil, asparaginase, ...) and antiepileptics (sodium valproate, primidone, ...) (Jones and Weissenborn, 1997; Hawkes et al., 2001; Felipo and Butterworth, 2002b; Leonard and Morris, 2002). The present invention also concerns a pharmaceutical composition comprising tianeptine or a salt thereof, an isomer thereof, a pro-drug thereof, a metabolite thereof or a structural analog thereof, and at least one other drug intended to prevent/treat hyperammon(em)ia, in particular flumazenil and zinc. Alternatively, the present invention concerns a pharmaceutical composition comprising tianeptine or a salt, an isomer, a pro-drug, a metabolite or a structural analog thereof, and at least one other drug resulting in hyperammonemia e.g. anti-cancer therapies (5-fluorouracil, asparaginase, ...) or antiepileptics (sodium valproate, primidone, ...).

The present invention also concerns a method of treatment of hyperammon(em)ia comprising administering an effective amount of an "other GS ligand" selected from the group consisting of GF, hydrazines and bisphosphonates, and salts thereof, isomers thereof, pro-drugs thereof, metabolites thereof and structural analogs thereof. Preferably, the therapeutical amount of this compound is a "low or intermediate dose" i.e. a dose resulting in concentrations of ligand which can activate or modulate/regulate GS activity in the target cells/tissues/organs (directly or indirectly). The present invention also concerns the use of GS ligands like GF, hydrazines and bisphosphonates, and salts thereof, isomers thereof, pro-drugs thereof, metabolites thereof and structural analogs thereof, for the preparation of medicaments for prevention and/or treatment of hyperammon(em)ia. Furthermore, the present invention contemplates a pharmaceutical composition comprising at least one GS ligand selected from the group of GF, hydrazines and bisphosphonates, at a "low or intermediate dose", wherein said dose result in concentrations of ligand which can activate or modulate/regulate GS activity in the target cells/tissues/organs (directly or indirectly). In a particular embodiment, said GS ligand like GF, hydrazines and bisphosphonates, and salts thereof, isomers thereof, pro-drugs thereof, metabolites thereof and structural analogs thereof, administered at a "low or intermediate dose", can be associated with other measures/drugs intended i) to prevent/treat hyperammonemia and/or hyperammonemia symptoms, in particular benzodiazepine receptor antagonists (flumazenil, ...), zinc supplementation and/or measures to minimize absorption of nitrogenous substances i.e. dietary restriction, evacuation of bowel, lactulose (or a related sugar) and/or broad spectrum antibiotics (for example, neomycin) in liver failure; or sodium benzoate and sodium phenylacetate in conjunction with a low protein diet and a specific amino acid supplementation in carbamylphosphate synthetase, ornithine transcarbamylase or arginosuccinate synthetase deficiency; or sodium benzoate, sodium phenylacetate arginine hydrochloride and/or haemodialysis after solid-organ transplantation; or ii) resulting in hyperammonemia e.g. anaesthetic agents (halothane, enflurane, ...), anti-cancer therapies (5-fluorouracil, asparaginase, ...) or antiepileptics (sodium valproate, primidone, ...) (Jones and Weissenborn, 1997; Hawkes et al., 2001; Felipo and Butterworth 2002b; Leonard and Morris, 2002). Therefore, the present invention concerns a pharmaceutical composition comprising a GS ligand like GF, an hydrazine or a bisphosphonate, at a "low or intermediate dose", and a drug intended to prevent/treat hyperammon(em)ia for instance flumazenil, zinc, sodium benzoate, sodium phenylacetate or arginine hydrochloride, or resulting in hyperammonemia e.g. anti-cancer therapies (5-fluorouracil, asparaginase, ...) or antiepileptics (sodium valproate, primidone, ...).

More particularly, the present invention concerns the use of GF, salts thereof, isomers thereof, pro-drugs thereof, metabolites thereof and structural analogs thereof, for the preparation of medicaments for prevention and/or treatment of hyperammon(em)ia. The present invention also concerns a method of treatment of hyperammon(em)ia in a subject comprising administering to said subject an efficient amount of GF or a salt thereof, an isomer thereof, a pro-drug thereof, a metabolite thereof or an analog thereof. Preferably, said hyperammonemia is related to a severe liver disease (due to cirrhosis, hepatitis, intoxication, ...), an inborn error of metabolism (urea cycle disorder, transport defect of intermediate in the urea cycle, organic aciduria, ...), a porto-systemic shunt and/or a "non hepatic" (critical) disorder (see Table 1 and 2 (the "double-underlined" conditions and disorders). Preferably, said localized ammonia increase is

related to a local critical stress e.g. anoxia, cancer, degeneration, infection, inflammation, ischaemia, surgery, trauma, ... Potential factors and mechanisms which can underlie these conditions and disorders are disclosed in Table 4, without to be limited thereto. In a particular embodiment, GF or salts thereof, isomers thereof, pro-drugs thereof, metabolites thereof or structural analogs thereof, can be combined with other measures/drugs intended i) to prevent/treat hyperammonemia and/or hyperammonemia symptoms, in particular benzodiazepine receptor antagonists (flumazenil, ...), zinc supplementation and/or measures to minimize absorption of nitrogenous substances i.e. dietary restriction, evacuation of bowel, lactulose (or a related sugar) and/or broad spectrum antibiotics (for example, neomycin) in liver failure; or sodium benzoate and sodium phenylacetate in conjunction with a low protein diet and a specific amino acid supplementation in carbamylphosphate synthetase, ornithine transcarbamylase or arginosuccinate synthetase deficiency; or sodium benzoate, sodium phenylacetate, arginine hydrochloride and/or haemodialysis after solid-organ transplantation; or ii) resulting in hyperammonemia e.g. anaesthetic agents (halothane, enflurane, ...), anti-cancer therapies (5-fluorouracil, asparaginase, ...) or antiepileptics (sodium valproate, primidone, ...) (Jones and Weissenborn, 1997; Hawkes et al., 2001; Felipo and Butterworth, 2002b; Leonard and Morris, 2002). Therefore, the present invention concerns a pharmaceutical composition comprising GF or a salt thereof, an isomer thereof, a pro-drug thereof, a metabolite thereof or a structural analog thereof, and a drug intended to prevent/treat hyperammonemia and/or hyperammonemia symptoms, for instance flumazenil, zinc, sodium benzoate, sodium phenylacetate and arginine hydrochloride, or resulting in hyperammonemia e.g. anti-cancer therapies (5-fluorouracil, asparaginase, ...) or antiepileptics (sodium valproate, primidone, ...). In a preferred embodiment, the efficient dose is a "low or intermediate dose" i.e. a dose resulting in concentrations of GF which activate or modulate/regulate GS activity in the target cells/tissues/organs (directly or indirectly).

In a preferred embodiment, GF metabolites or (structural) analogs are selected from the group consisting of the compounds in Tables 9 and 13, preferably Table 9.

In a particular embodiment, the GS ligand can be selected from the group of hydrazines and salts thereof, isomers thereof, pro-drugs thereof, metabolites thereof and structural analogs thereof, preferably those disclosed in Table 11.

In another particular embodiment, the GS ligand can be be selected from the group of bisphosphonates and salts thereof, isomers thereof, pro-drugs thereof, metabolites thereof and structural analogs thereof, preferably selected from those disclosed in Table 12.

In a particular embodiment, the GS ligand can be an analog of L-glutamate or glutamate or a salt thereof, an isomer thereof, a pro-drug thereof, a metabolite thereof or structural analog thereof, preferably selected from those disclosed in Table 13.

***Functions, conditions and disorders related to glutamate***

The amino acid L-glutamate plays an important role both in neuronal and non-neuronal tissues as a fundamental metabolite/substrate of the tricarboxylic acid cycle and amino acid metabolism (Hertz, 1979; Hertz et al., 1999; Hertz and Zielke, 2004). It is also accepted as a specific excitatory amino acid in the nervous tissues, where it is the major excitatory neurotransmitter at synaptic junctions (Orrego and Villanueva, 1993), and as an extracellular autocrine and/or paracrine signal mediator in peripheral nervous and non-nervous tissues (Hinoi et al., 2004).

In the nervous tissues, glutamate acts as an excitatory neurotransmitter via stimulation of a complex multiprotein receptor system composed of several different glutamate receptor subtypes, both ligand gated ion channel receptors (*N*-methyl-D-aspartate (NMDA), $\alpha$-amino-3-hydroxy-5-methyl-4-isoxazole propionic acid (AMPA) and kainate (Ka) receptors) and G-protein coupled (metabotropic) receptors. This complex beside mediating excitatory neurotransmission plays a key role in synaptogenesis and formation of neuronal circuitry. Compelling evidence indicates that it plays a crucial role in how initial alterations in the activity of CNS are transformed into more persistent and sometimes permanent modifications of the neuronal activity and excitability. These mechanisms are thought, in particular, to underlie learning and memory. On the contrary, excessive activation of glutamate receptors is believed to participate in neurodegeneration following a wide range of neurological insults including hypoglycemia, ischaemia, (epileptic) seizures or trauma. Chronic neurodegenerative disorders such as Alzheimer's disease, amyotrophic lateral sclerosis, Huntington's chorea or AIDS encephalopathy are alike assumed to involve neuronal cell death related to excitotoxic properties of glutamate.

In order to use the common amino acid L-glutamate as a neurotransmitter, it has been necessary to construct a discriminating mechanism that greatly restricts the availability of glutamate. The most far-reaching innovations have been: i) to exclude the nervous tissues (brain) from access to glutamate present in the systemic circulation (blood-brain barrier), thereby making it autonomous in the production and disposal of glutamate; ii) to surround glutamatergic synapses with glial cells and endow these cells with much more efficient glutamate uptake carriers than the neurons themselves, so that most released glutamate is rapidly inactivated by uptake in glial cells; iii) to provide glial cells but not neurons the key enzyme (glutamine synthetase) that is involved in the return of accumulated glutamate to neurons by amidation to glutamine, which has no transmitter activity, and can be safely released to the extracellular space, returned to neurons

and deaminated to glutamate (glutamine-glutamate cycle) (Sibson et al., 1997; Zwingmann et al., 1998 and 2002) - in neurons, glutamate serves of course also as a respiratory fuel, gluconeogenic precursor, carrier of nitrogen, precursor of γ-aminobutyric acid (GABA) (Paulsen et al., 1988), etc. (see below) - iv) to restrict to glial cells two key enzymes (pyruvate carboxylase and cytosolic malic enzyme) that are involved, respectively, in de novo synthesis (from glucose) of the carbon skeleton of glutamate, and in the return of the carbon skeleton of excess glutamate to the metabolic pathway for glucose oxidation. As a consequence of these innovations, neurons constantly require new carbon skeletons from glial cells. When these supplies are withdrawn, neurons are unable to generate amino acid transmitters and their rate of oxidative metabolism is impaired. Thus, given the commensalism that exists between neurons and glia, brain function has to be viewed not just as a series of interactions between neurons, but also as a series of interactions between neurons and their collaborating glial cells (Kvamme 1998; Hertz et al., 1999; Hertz and Zielke, 2004).

Clearance of synaptic glutamate by glial cells is required for a normal nervous function ("signal-to-noise ratio"). An increase in excitatory synapses meets an elevation of GS activity in glial cells, which in turn participates in an effective tissue function/protection. This was suggested in different models for instance in established retinal tissue paradigmes for glutamate neurotoxicity (Gorovits et al., 1997; Shaked et al., 2002); in primary culture of retinal tissue i) glutamate serves as a neurotransmitter in photoreceptors, bipolar cells and ganglion cells (Massey and Miller, 1990); ii) insult leads to accumulation of relatively high levels of glutamate in the extracellular fluid (Louzada et al., 1992; Neal et al., 1994); iii) administration of glutamate leads to neuronal cell death (David et al., 1988; Zeevalk et al., 1989; Zeevalk and Nicklas, 1990); and iv) glutamate receptor antagonists protect against neuronal degeneration (Mosinger et al., 1991). Hormonal induction of the endogenous GS gene in retinal glial cells correlates with a decline in neuronal degeneration (Gorovits et al., 1997), whereas inhibition of GS activity by MS leads to increased cell death (Gorovits et al., 1997;. Shaked et al., 2002); finally a supply of purified GS enzyme causes a dose-dependent decline in the extent of cell death (Gorovits et al., 1997).

The Inventors have discovered that tianeptine, depending on the concentration/dose and experimental or (patho)physiological condition, can activate or inhibit GS activity in vitro as well as in vivo (Figures 5-7, 14-16, 31-32). At "low and intermediate concentrations/doses", it behaves as a GS activator or as a GS modulator/regulator. Tianeptine was also observed, *in vitro,* to prevent cellular apoptosis in conditions of elevated ammonia and/or elevated glutamate concentrations (Figures 25-27) and, *in vivo*, to counteract hippocampal CA3 structural changes associated with chronic glucorticoid administration (Figure 30), and these effects could be explained by their influence on GS (Figures 18-22, 31). Tianeptine could also protect GS from oxidative stress (Figure 17) and raise GS protein expression *in vivo* (Figure 14B, 15B and 31 B). These properties of tianeptine can likely participate in its neuroprotective effects (US 6,599,896), its effects on psychoneurotic disorders (FR 2 104 728), stress (FR 2 635 461) and pain (FR 2 104 728; U.S. Pat. No. 3,758,528) but also on asthma, cough (FR 2 104 728), nonulcer dyspepsia (US 6,683,072) and irritable bowel syndrome (US 6,683,072), in increasing/regulating the clearance of glutamate and/or the synthesis of glutamine (see below).

Increasing/balancing the clearance of extracellular glutamate can result in an original pharmacological profile. In particular, all glutamate receptor subtypes may be less activated in situations of high glutamate concentration. Accordingly, the present invention extends the use of tianeptine, salts thereof, isomers thereof, pro-drugs thereof, metabolites thereof and structural analogs thereof, in the manufacture of medicaments to be used in the prevention and/or treatment of all conditions and disorders related to an extracellular excess of glutamate, whatever its cause(s) and the activated glutamate receptor subtype(s), with the exception of the already claimed or proposed clinical applications of tianeptine. In fact, all the already claimed or proposed clinical applications of tianeptine, which are disclosed in Table 5, in particular asthma, cough, depression (major depression with or without melancholia, dysthymic disorders, depressed bipolar disorder, ...), irritable bowel syndrome, mnemo-cognitive disorders, neurodegenerative diseases (such as cerebral hypoxia, cerebral ischaemia, cerebral traumatism, cerebral ageing, Alzheimer's disease, multiple sclerosis, amyotrophic lateral sclerosis, demyelinating pathologies, encephalopathies, myalgic encephalomyelitis, chronic fatigue syndrome, post-viral fatigue syndrome, the state of fatigue and depression following a bacterial or viral infection, the dementia syndrome of acquired immune deficiency syndrome (AIDS), ...), nonulcer dyspepsia, pain, psychoneurotic disorders (neurotic or reactive states of depression, anxiodepressive states with somatic complaints such as digestive problems, anxiodepressive states observed in alcoholic detoxification, ...), stress and stroke, are likely, at least in part, associated and/or related to (absolute or relative) excess of glutamate. Thus, the additional target conditions and disorders complete these already claimed conditions and disorders from the nervous system disorders, psychiatric disorders, gastrointestinal disorders, and respiratory, thoracic and mediastinal disorders SOCs. They comprise in particular conditions and disorders from other "peripheral" SOCs (see Table 2). The present invention also concerns a method of prevention and/or treatment of all these additional conditions and disorders related to excess of glutamate, with the exception of the conditions and disorders disclosed in Table 5, comprising administering an effective amount of tianeptine or of salt thereof, an isomer thereof, pro-drug thereof, a metabolite thereof or a structural analog thereof. Preferably, tianeptine, salts thereof, isomers thereof, pro-drugs thereof, metabolites thereof and structural analogs thereof, are used at "low or intermediate" doses i.e. doses resulting in concentrations of tianeptine which can increase or modulate/regulate GS activity in the target cells/tissues/organs (directly or indirectly). In a particular embodiment, said tianeptine, salts thereof, isomers thereof,

pro-drugs thereof, metabolites thereof and structural analogs thereof, are used at "low doses" i.e. doses resulting in concentrations of tianeptine which increase GS activity in the target cells/tissues/organs. Tianeptine or salts thereof, isomers thereof, pro-drugs thereof, metabolites thereof or structural analogs thereof, may be in particular combined with treatments preventing from excitotoxic properties of glutamate through other mechanisms e.g. with glutamate release inhibitors (lamotrigine, riluzole, ...) or glutamate receptor antagonists (D-AP5, ketamine, memantine, MK-801, ...). Therefore, the present invention also concerns a pharmaceutical composition comprising tianeptine or a salt, an isomer, a pro-drug, a metabolite or a structural analog thereof, and at least one other drug intended to prevent from excitotoxic properties of glutamate through other mechanisms e.g. glutamate release inhibitors (lamotrigine, riluzole, ...) or glutamate receptor antagonists (D-AP5, ketamine, memantine, MK-801, ...).

Additionally, the present invention concerns a method of treatment of conditions and disorders related to an (absolute or relative) excess of glutamate in a subject comprising administering an effective amount of a GS ligand selected from the group consisting of GF, MS, hydrazines and bisphosphonates, and salts thereof, isomers thereof, pro-drugs thereof, metabolites thereof and structural analogs thereof. Preferably, the therapeutical amount of this compound is a "low or intermediate dose" i.e. a dose resulting in concentrations of ligand which can increase or modulate/regulate GS activity in the target cells/tissues/organs (directly or indirectly). In a particular embodiment, the GS ligand is used at "low doses" i.e. doses resulting in concentrations which increase GS activity in the target cells/tissues/organs. The present invention also concerns the use of GS ligands selected from the group consisting of GF, MS, hydrazines and bisphosphonates, and salts thereof, isomers thereof, pro-drugs thereof, metabolites thereof and structural analogs thereof, for the preparation of medicaments for prevention and/or treatment of conditions and disorders related to (absolute or relative) excess of glutamate, with the exception of: if said GS ligand is MS, the conditions and disorders related to cerebral ischemia, hyperammonemia and mycobacterial infectious disorders; if said GS ligand is a hydrazine, the conditions and disorders disclosed in Table 6; and, if said GS ligand is a bisphosphonate, the conditions and disorders disclosed in Table 7. Preferably, the conditions and disorders to be treated are selected from those disclosed in Tables 2 and 3. Furthermore, the present invention contemplates a pharmaceutical composition comprising at least one GS ligand selected from the group consisting of GF, MS, hydrazines and bisphosphonates, at a "low or intermediate dose", wherein said "low or intermediate dose" result in concentrations which can increase or modulate/regulate the GS activity (directly or indirectly), and at least one other drug intended to prevent from excitotoxic properties of glutamate through other mechanisms e.g. glutamate release inhibitors (lamotrigine, riluzole, ...) or glutamate receptor antagonists (D-AP5, ketamine, memantine, MK-801...). In a preferred embodiment, said GS ligand is GF or a salt thereof, an isomer thereof, a pro-drug thereof, a metabolite thereof or a (structural) analog thereof, preferably a compound selected from Tables 9 and 13, more preferably Table 9. In an other embodiment, said GS ligand is MS or a salt thereof, an isomer thereof, a pro-drug thereof, a metabolite thereof or a (structural) analog thereof, preferably a compound selected from Tables 10 and 13, more preferably Table 10. In a preferred embodiment, said hydrazine, salt thereof, isomer thereof, pro-drug thereof, metabolite thereof or structural analog thereof is selected from Table 11. Preferably it is isoniazid or iproniazid, or a salt thereof, an isomer thereof, a pro-drug thereof, a metabolites thereof or a structural analogs thereof. In a preferred embodiment, said bisphosphonate, or salt thereof, isomer thereof, prodrug thereof, metabolite thereof or structural analog thereof is selected from Table 12. Preferably it is pamidronate, risedronate or [(5-Chloro-pyridin-2-ylamino)-phosphonomethyl]-phosphinic acid, or a salt thereof, an isomer thereof, a pro-drug thereof, a metabolite thereof or a structural analogs thereof.

Increasing or modulating/regulating the clearance of extracellular glutamate by an increase in GS activity may also participate to reduce/treat the hyperglutamatergic consequences of NMDA receptor dysfunctions implicated in pathophysiologic processes of neuropsychiatric illnesses such as schizophrenia (Anand et al., 2000). Lamotrigine, a drug inhibiting glutamate release, was observed to reduce the neuropsychiatric effects of ketamine, a NMDA receptor antagonist, in humans. Lamotrigine significantly decreased ketamine-induced perceptual abnormalities, positive and negative symptoms, and learning and memory impairment. On the contrary, it increased the immediate mood-elevating effects of ketamine, which might be not the case with drugs increasing the clearance of extracellular glutamate, in particular those increasing GS activity.

Accordingly, the present invention extends the use of tianeptine, salts thereof, isomers thereof, pro-drugs thereof, metabolites thereof and structural analogs thereof, in the manufacture of medicaments intended to reduce the hyperglutamatergic consequences of glutamate receptor dysfunctions, in particular of NMDA receptor dysfunctions implicated in the pathophysiologic processes of neuropsychiatric illnesses such as schizophrenia, with the exception of the conditions and disorders disclosed in Table 5. Tianeptine, salts thereof, isomers thereof, pro-drugs thereof, metabolites thereof and structural analogs thereof might be combined with treatments altering glutamate receptor(s) function(s), in particular with NMDA glutamate receptor antagonists (D-AP5, ketamine, memantine, MK-801, ...), with the aim to reduce their hyperglutamatergic consequences mediated by non-NMDA glutamate receptors (adverse effects). Therefore, the present invention contemplates a pharmaceutical composition comprising such combinations. Similarly, instead of tianeptine, GS ligands selected from the group consisting of GF, MS, hydrazines and bisphosphonates, and salts thereof, isomers thereof, pro-drugs thereof, metabolites thereof and structural analogs thereof, can be used, preferably

at "low or intermediate doses" i.e. doses resulting in concentrations which increase or modulate/regulate GS activity (directly or indirectly). All these GS ligands can be combined with other treatments preventing (in particular through other mechanisms) from these hyperglutamatergic consequences of glutamate receptor dysfunctions e.g. ampakines, glutamate release inhibitors (lamotrigine, riluzole, ...), glycine, ... Therefore, the present invention contemplates a pharmaceutical composition comprising such combinations.

Increasing the clearance of extracellular glutamate by an increase of GS activity may be also particularly useful in disorders with extracellular glutamate excess where GS protein/activity is altered or insufficiently effective. This was reported for instance for hyperammonemia (Kosenko et al., 2003; see above), temporal epilepsy (Eid et al., 2004) and sepsis (Tumani et al., 2000). A deficiency in glutamine synthetase in astrocytes has been proposed to be the molecular basis for extracellular glutamate accumulation and seizure generation in mesial temporal lobe epilepsy, a drug resistant type of epilepsy (Eid et al., 2004); in more than 40% of cases, this disorder cannot be controlled by medication, and anteromedial temporal lobectomy with hippocampectomy is needed for seizure control in certain of these patients. Apoptosis of dentate granular cells in the hippocampal formation during bacterial meningitis has been proposed to be due to the inability of hippocampal glutamine synthetase to metabolize excess amounts of glutamate (Tumani et al., 2000).

Accordingly, the present invention extends the use of tianeptine, salts thereof, isomers thereof, pro-drugs thereof, metabolites thereof and structural analogs thereof, in the manufacture of medicaments to be used for preventing and/or treating conditions and disorders with high extracellular glutamate where GS protein/activity is altered or insufficiently effective, e.g. in hyperammonemia, epilepsy or critical sepsis, with the exception of the conditions and disorders disclosed in Table 5. Tianeptine, salts thereof, isomers thereof, pro-drugs thereof, metabolites thereof and structural analogs thereof, may be in particular combined with etiologic and symptomatic treatments in these indications e.g. symptomatic ammonia lowering measures/treatments (see above), anti-epileptic treatments (benzodiazepines, carbamazepine, ethosuximide, hydantoins, gabapentin, lamotrigin, phenobarbital, primidone, valproate, ...) or antibiotics (aminoglycosides, cephalosporins, chloramphenicol, macrolides, penicillins, quinolones, rifampicine, sulfonamides, tetracyclines, trimethoprim-sulfamethoxazole, ...). Similarly, instead of tianeptine, GS ligands selected from the group consisting of GF, MS, hydrazines and bisphosphonates, and salts thereof, isomers thereof, pro-drugs thereof, metabolites thereof and structural analogs thereof, also can be used in the manufacture of medicaments intended for preventing and/or treating conditions and disorders with high extracellular glutamate where GS protein/activity is altered or insufficiently effective, preferably at "low or intermediate doses" i.e. doses resulting in concentrations which can increase and/or modulate/regulate GS activity (directly or indirectly), with the exception of: if said GS ligand is MS, the conditions and disorders related to cerebral ischemia, hyperammonemia and mycobacterial infectious disorders; if said GS ligand is a hydrazine, the conditions and disorders disclosed in Table 6; and, if said GS ligand is a bisphosphonate, the conditions and disorders disclosed in Table 7. Thus, the present invention concerns a pharmaceutical composition comprising a drug selected from the group consisting of tianeptine, GF, MS, hydrazines and bisphosphonates, and salts thereof, isomers thereof, pro-drugs thereof, metabolites thereof and structural analogs thereof, and etiologic and symptomatic treatments in these indications e.g. symptomatic ammonia lowering treatments (see above), anti-epileptic treatments (benzodiazepines, carbamazepine, ethosuximide, hydantoins, gabapentin, lamotrigin, phenobarbital, primidone, valproate, ...) or antibiotics (aminoglycosides, cephalosporins, chloramphenicol, macrolides, penicillins, quinolones, rifampicine, sulfonamides, tetracyclines, trimethoprim-sulfamethoxazole, ...).

Endogenous glutamate production can be stimulated or triggered by a variety of inflammatory mediators, including arachidonate metabolites and reactive oxygen species (Lipton and Gendelman, 1995; Harder et al., 1998; Farooqui et al., 1995). There is also a lot of evidence supporting glutamate-induced injury is mediated by nitric oxide and other free radicals, including superoxide anions. NMDA-induced injury in primary cortical cultures (Dawson et al., 1991) as well as in cerebellar (Garthwaite et al., 1986) or hippocampal slices (Izumi et al., 1992) have been reported to be mediated by excessive NO synthesis. Glutamate neurotoxicity is susceptible to modulation by intervention at several levels, which include blockade of the ionotropic receptors, inhibition of NO synthesis and inhibition of NO-induced activation of poly(ADP-ribose) polymerase (PARP). The major role of glutamate in determining neurological disorders is characterized by an increasing damage of cell components, including mitochondria, leading to cell death, and reactive oxygen species are generated in death process. Glucocorticoids as well as various stresses increase GS expression (Abcouwer et al., 1995 and 1996; Ardawi 1990; Ardawi and Majzoub 1991; Chandrasekhar et al., 1999; Lukaszewicz et al., 1997). Cytokines are likely involved in the mechanism(s) of action of tianeptine e.g. tianeptine and its enantiomers S 16190 and S 16191 have neuroprotective effects against deleterious effects of certain cytokines in cortex and white matter (Plaisant et al., 2003). One of these cytokines, IL-1β, which is normally present in the hippocampus, inhibits neuronal plasticity and is believed to be a causative agent for depression (Dantzer et al., 1998 and 1999; Rothwell, 1997). Tianeptine has been shown to antagonise the behavioural effects of lipopolysaccharide (LPS) as well as of chronic administration of IL-1β (Castanon et al., 2001), and to inhibit fos expression induced by LPS in the rat paraventricular nucleus (Castanon et al., 2003).

Accordingly, the present invention extends the use of tianeptine, salts thereof, isomers thereof, pro-drugs thereof, metabolites thereof and structural analogs thereof, in the manufacture of medicaments intended for prevention

and/or treatment of all conditions and disorders with high extracellular glutamate combined with, related to or resulting in an inflammatory process, with the exception of the conditions and disorders disclosed in Table 5. All the already claimed or proposed (central and peripheral) clinical applications of tianeptine likely or possibly, at least in part, are related to or result in an inflammatory process. Thus, additional target conditions and disorders complete these already claimed conditions and disorders. They comprise in particular conditions and disorders from "peripheral" SOCs (see below and Table 2). Tianeptine, salts thereof, isomers thereof, pro-drugs thereof, metabolites thereof and structural analogs thereof, may be in particular combined with anti-inflammatory drugs or drugs preventing from the occurrence of inflammation. Tianeptine may allow to decrease the doses of glucocorticoids in conditions and disorders with high extracellular glutamate combined with, related to or resulting in an inflammatory process - this was for instance reported in asthma (Lechin et al., 1998) - and/or prevent adverse effects of glucocorticoids at high doses e.g. impairement of antioxidant status (Orzechowski et al., 2000). Similarly, instead of tianeptine, GS ligands selected from GF, MS, hydrazines and bisphosphonates, and salts thereof, isomers thereof, pro-drugs thereof, metabolites thereof and structural analogs thereof, can also be used, preferably at "low or intermediate doses" i.e. doses resulting in concentrations which increase or modulate/regulate GS activity in the target cells/tissues/organs (directly or indirectly). Accordingly, the present invention extends the use of a GS ligand selected from GF, MS, hydrazines and bisphosphonates, and salts thereof, isomers thereof, pro-drugs thereof, metabolites thereof and structural analogs thereof, in the manufacture of a medicament intended for prevention and/or treatment of all conditions and disorders with extracellular excess of glutamate combined with, related to or resulting in an inflammatory process, with the exception of: if said GS ligand is MS, the conditions and disorders related to cerebral ischemia, hyperammonemia and mycobacterial infectious disorders; if said GS ligand is a hydrazine, the conditions and disorders disclosed in Table 6; and, if said GS ligand is a bisphosphonate, the conditions and disorders disclosed in Table 7. Thus, the present invention concerns a pharmaceutical composition comprising a drug selected from the group consisting of tianeptine, GF, MS, hydrazines and bisphosphonates, and salts thereof, isomers thereof, pro-drugs thereof, metabolites thereof and structural analogs thereof, and anti-inflammatory drugs or drugs preventing from the occurrence of inflammation.

Finally, neuronal death results from multifactorial processes that are inter-related and vary depending on the (patho)physiological condition. A number of robust and important experimental evidence conflicts with the notion that endogenous glutamate excitotoxicity is the sole contributor to neuronal death. For instance, very high extracellular levels of glutamate, far above those measured in models of neurological disorders, must be reached to initiate neuronal death. Thus, drugs like tianeptine that are able to regulate GS activity depending on the pathophysiological condition can be protective by increasing the resistance of cells e.g. neurons and astrocytes to deleterious mechanisms that are not necessary directly or mainly related to glutamatergic transmission (Obrenovitch et al., 2000; see below). GS activation or GS modulation/regulation would be usually the mechanism underlying their neuroprotective effect(s) but, in certain experimental and pathophysiological conditions, GS inhibition would also be, directly or indirectly, protective, as observed in certain model of hyperammonemia (see above).

In addition to its "excitatory amino acid neurotransmitter" role in the nervous tissues, evidence is emerging for a role of glutamate as an extracellular signal mediator in a myriad of peripheral nervous and non-nervous locations (Skerry and Genever, 2001; Hinoi et al., 2004). Recent reports give support to the expression of glutamate signaling molecules in adrenal gland (Watanabe et al., 1994; Yoneda & Ogita 1986; Kristensen, 1993; Hinoi et al., 2002b,c), bone (osteocyte, osteoblast, and osteoclast) (Mason et al., 1997; Chenu et al., 1998; Hinoi et al., 2002a and 2003), (cerebral) endothelium (Krizbai et al., 1998), autonomic and sensory ganglions (Shigemoto et al., 1992; Watanabe et al., 1994), heart (Gill et al., 1998), liver (Storto et al., 2000b), gastrointestinal tract (Sninsky et al., 1994; Shannon & Sawyer 1989), kidney (Leung et al., 2002, Deng et al., 2002), lung (Said et al., 1996), lymphocytes (Lombardi et al., 2001), macrophages (Dickman et al., 2004), pancreas (Inagaki etal., 1995; Gonoi et al., 1994; Hayashi et al., 2003; Tong et al., 2002), pineal gland (Govitrapong et al., 1986; Sato et al., 1993; Yamada et al., 1998; Yatsushiro et al., 2000), pituitary gland (Kiyama et al., 1993; Hinoi and Yoneda, 2001), platelets (Franconi et al., 1996), skin (Genever et al., 1999a), male lower urogenital tract and testis (Storto et al., 2001; Nagata et al., 1999; Gonzalez-Cadavid et al., 2000), thymus (Storto et al., 2000a), ... Glutamate behaves as a neuro-, auto- and/or paracrine transmitter in these locations; of course it is also an important gustatory stimulus that is believed to signal dietary protein (Chaudhari et al., 2000).

In these peripheral locations, glutamate acts via the same complex multiprotein receptor system as in the CNS i.e. ligand gated ion channels and/or G-protein coupled (metabotropic) receptors. The expression - at least 16 complementary deoxyribonucleic acids encoding for the glutamate receptors have been identified and may be modified further by alternate splicing or ribonucleic acid editing (Gasic and Hollmann, 1992; Hollmann and Heinemann, 1994) - and distribution of these signaling molecules is specific of each species, each location, each condition, ... For instance in rat heart, GluR 2/3, GluR 5/6/7, Ka 2, and NMDAR 1 subunits are expressed within nerve terminals, ganglia, conducting fibers, nerve bundles and some to myocardiocytes (particularly in the atrium), each with a distinct pattern of distribution (Gill et al., 1998; Leung et al., 2002). Glutamate receptors are also expressed in the aorta, pulmonary artery, ... This sophisticated "anatomo-functional" organization - in fact, the receptor complexes of different subunit combinations exhibit varied pharmacological and electrophysiological properties, including ionic channel selectivity to $Na^+$, $K^+$ and $Ca^{2+}$

(Gasic and Hollmann, 1992; Hollmann and Heinemann, 1994) - suggests that glutamate receptors play a specific role in cardiac electrophysiology and pathology; secondarily, glutamate receptors might be involved in cardiac dysfunctions associated with intoxication with excitatory amino acids present in food or food additives such as domoic acid or monosodium L-glutamate. In the (rat) kidney, the NMDAR1 subunit is present in cortex and medulla, and its expression increases with development; of the NMDAR2 subunits, only the NMDAR2C is present (Leung et al., 2002). NMDA receptors have been observed to exert a tonic vasodilatory influence (Deng et al., 2002), this effect being likely related to an effect on the proximal tubule; at this level, GS activity is a key factor that limits the release of ammonia, generated by renal cells, into the urine and/or renal vein (Ferrier et al., 1999).

This complex system confers a finely tuned control over a wide range of physiological functions, conditions and disorders. For instance in bone, where GS expression in osteoblasts can be induced by glucocorticoids (Olkku et al., 2004), glutamate plays a crucial role in influencing proliferation, differentiation, activity as well as apoptosis of both osteoblasts and osteoclasts. An innervation of bone by glutamate-containing nerves has been demonstrated (Chenu, 2002). There is compelling evidence for an osteoblastic source of glutamate. Osteoblasts express all the components of neuronal presynaptic machinery required for glutamate release (Bhangu et al., 2001). Glutamate exocytosis from osteoblasts has been demonstrated, prevention of which inhibits cell survival and differentiation (Hinoi et al., 2002b; Genever and Skerry, 2001). There are also several arguments suggesting osteoblasts use glutamate as a signalling molecule to perceive, record and respond to mechanical stimulation (Spencer and Genever, 2003; Spencer et al., 2004). Osteoporosis has been proposed to result from an age-related failure in these adaptive osteogenic responses (Lanyon and Skerry, 2001; Frost, 2002). NMDA receptors are expressed on osteoblasts and osteoclasts (Patton et al., 1998; Chenu et al., 1998; Peet et al., 1999; Hitchcock et al., 2003). NMDA receptor antagonists can inhibit bone formation as well as bone resorption (Peet et al., 1999; Birch et al., 1997); these effects are likely mostly to be mediated through impaired cell differentiation (Peet et al., 1999; Hinoi et al., 2003; Merle et al., 2003). As such, manipulation of these signalling pathways offers real therapeutic potential for the treatment of bone disorders characterised by inappropriate changes in bone formation and/or bone resorption. Glutamate might participate to prime the skeleton to avoid inappropriate changes in bone formation/resorption ratio, in particular those due to biomechanical factors, using mechanisms similar or identical to neuronal long-term potentiation (Spencer et al., 2004). In that respect, the discovery that bisphosphonates such as pamidronate and risedronate like [(5-Chloro-pyridin-2-ylamino)-phosphono-methyl]-phosphinic acid alter GS activity (probably through docking into GS $Mn^{2+}$ binding sites) fortifies the therapeutic potential of this approach for the treatment of bone disorders (Figures 12-13) (Kafarski et al., 2000). Indeed bisphosphonates are largely employed as therapeutic agents for treatment of bone disorders, in particular of osteolytic bone metastases, osteoporosis associated with postmenopause and long-term corticosteroid therapy, and Paget's disease of bone (see Table 7); and their mode of action remains unclear.

Mechanisms similar or identical to neuronal long-term potentiation likely play an important role in peripheral tissues/organs. For instance, they participate in the prolonged alteration in responsiveness of cerebral endothelial cells after exposure to high glutamate concentrations e.g in ischemic conditions (Krizbai et al., 1998). Exposure of cerebral endothelial cells to glutamate increases the phosphorylation of calcium/calmodulin dependent protein kinase II (Krizbai et al., 1998). In its phosphorylated form, CAM-PKII loses its $Ca^{2+}$ dependency and retains its activity (Bronstein et al., 1993). Thus, vascular glutamate signalling is believed to play an important role in the breakdown of the blood brain barrier in cerebral injuries e.g. in ischemic conditions; this effect is mediated by nitric oxide through NMDA receptors (Fergus and Lee, 1997; Meng et al., 1995).

Over-activation of peripheral glutamate receptors may be also a pathogenic mechanism of injury and inflammation in peripheral locations; conversely, endogenous glutamate production may also be stimulated or triggered by a variety of inflammatory mediators in peripheral locations. For instance, excessive activation of NMDA receptors may be involved in the pathogenesis of some inadequately understood forms of "neurogenic" pulmonary edema like occurring at high altitude as well as of inflammatory injury of asthmatic airways (Said et al., 1996). NMDA receptor activation in perfused, ventilated rat lung triggers acute injury marked by increased pressures needed to ventilate and perfuse the lung, and by high-permeability edema (Said et al., 1996). This injury can be prevented by competitive NMDA receptor antagonists and by NMDA channel-blockers, and is reduced in the presence of $Mg^{2+}$ (Said et al., 1996). As with NMDA-induced injury in CNS, this lung injury requires L-arginine, is associated with increased production of NO, and is attenuated by NO synthase inhibitors (Dawson et al., 1993; Said et al., 1996); inhibitors of NMDA receptors also greatly attenuate oxidant lung injury caused by paraquat or xanthine oxidase, supporting the role for endogenous activation of NMDA receptors in mediating certain forms of lung injury (Said et al., 2000). The neuropeptide vasoactive intestinal peptide and inhibitors of poly(ADP-ribose) polymerase (PARP) can also prevent this injury, but without inhibiting NO synthesis, both acting by inhibiting the toxic action of NO (Said et al., 1996). All these findings provide a molecular-biological basis for the excitotoxic actions of glutamate in (rat) lungs and airways. NMDAR 1 subunits were detected in peripheral, midlung and mainstem lung regions, as well as in the trachea and the alveolar macrophages (Dickman et al., 2004). NMDAR 2C subunits were detected in peripheral and mid-lung (Dickman et al., 2004). NMDAR 2D are the dominant subunits in the peripheral gas-exchange zone of lung and in alveolar macrophages (Dickman et al., 2004).

Peripheral tissues are protected from excess of extracellular glutamate through different mechanisms: barriers which protect from "circumfluent" glutamate, amino acid metabolism ("recycling"), in which glutamine synthetase plays an important role in catalyzing the formation of glutamine from glutamate and ammonia, and glutamate metabolism through the tricarboxylic acid cycle. Clearance of glutamate (and ammonia) is required for the normal function of peripheral tissues and an increase or a modulation/regulation of GS activity may therefore be prone to protect peripheral tissues from high glutamate (plus ammonia) concentrations (Haussinger et al., 1985); it provides also glutamine which plays an important role to maintain normal cell and tissue function (see below).

Tianeptine can behave as a GS activator or as a GS modulator/regulator at "low and intermediate concentrations/doses", respectively. Such an improvement or modulation/regulation of the recycling of glutamate (as well as of the synthesis of glutamine; see below) can likely participate in its already reported peripheral properties e.g. on asthma, cough (FR 2 104 728), irritable bowel syndrome (US 6,683,072), nonulcer dyspepsia (US 6,683,072), pain (FR 2 104 728; U.S. Pat. No. 3,758,528) and stress (FR 2 635 461). For instance, the very impressive clinical effects of tianeptine on asthma i.e. "dramatic and sudden decrease of clinical rating" and "increase in pulmonary function" (Lechin et al., 1998) which were considered "provocative and worth exploring" by most of the scientific community because they were suggested to be related to effects on serotonin, can be reasonably explained by the effects of tianeptine on GS (see above). Glucocorticoids, which are the most effective long-term control medicine currently available in asthma and which increase GS activity and expression in lungs (Labow et al., 1998), could be even omitted in young asthmatic patients treated with tianeptine (Lechin et al., 1998); supplemental glutamine might participate in these protective effects in preventing from oxidative stress (see below).

The clinical effects of tianeptine on irritable bowel syndrome can be alike reasonably explained by activation or modulation/regulation of GS activity. In fact glutamate is likely to play a role as an excitatory neurotransmitter in the enteric nervous system (ENS) (Kirchgessner, 2001). For instance, in the guinea-pig, glutamate immunoreactivity has been detected in subsets of submucosal and myenteric neurons in the ileum. At this level, glutamate is selectively concentrated in terminal axonal vesicles and can be released after application of an appropriate stimulus (Wiley et al., 1991; Liu et al., 1997a; Sinsky and Donnerer, 1998; Reis et al., 2000). Enteric neurons are endowed both with the neuronal transporter excitatory amino acid carrier 1 (EAAC1) and the vesicular glutamate transporter 2 (VGLUT2) (Liu et al., 1997a; Tong et al., 2001). Functional studies are consistent with a role of glutamate in the modulation of motor and secretory functions in the gut (Wiley et al., 1991; Sinsky and Donnerer, 1998; Cosentino et al., 1995; Rhoads et al., 1995). Glutamate may in particular participate in the modulation of the enteric cholinergic function - glutamate receptors are abundantly expressed on enteric cholinergic neurons (Liu et al., 1997a) -, since activation of NMDA receptors enhances acetylcholine release from myenteric neurons in the ileum and colon (Wiley et al., 1991; Cosentino et al., 1995); this latter effect is likely responsible for glutamate-induced contractions of the guinea-pig ileum (Wiley et al., 1991; Sinsky and Donnerer, 1998). So there are structural and functional bases for a glutamatergic modulation of enteric neurons in the ENS (Giaroni et al., 2003). At this level, glutamate receptors of the NMDA type may participate to the facilitatory modulation of excitatory cholinergic pathways, which retain a primary role in the control of motility. Secondarily, glutamate might also have a role in the modulation of sensory pathways, as already suggested in the guinea-pig ileum and rat colon (Kirchgessner, 2001; McRoberts et al., 2001). Hypothetically, in the complex neuronal circuitries, which constitute the ENS, glutamate might also participate to activity-dependent plasticity (Giaroni et al., 1999). Secondarily, glutamate receptors might be involved in alterations of the gastrointestinal functions consequent to an alimentary intoxication, as observed after ingestion of food contaminated with domoic acid, a ionotropic non-NMDA glutamate receptor agonist (Teitelbaum et al., 1990). Finally, we cannot exclude that this system may represent an underlying support for glutamate mediated excitotoxicity in the ENS. Indeed, in the guinea-pig, prolonged stimulation of enteric ganglia by glutamate was observed to cause necrosis and apoptosis in ileal enteric neurons, which seemed primarily mediated via NMDA receptors (Kirchgessner et al., 1997). So excitotoxicity induced by glutamate may be a critical factor in the pathogenesis of some diseases of the gastrointestinal tract, as it is the case for some neurological disorders in the CNS (Obrenovitch and Urenjak, 1997).

The effects of tianeptine on GS can also plausibly explain the clinical effects of tianeptine on pain. Indeed glutamate is expressed in the sensory pathways e.g. in the trigeminal and dorsal root ganglions (Watanabe et al., 1994) in sensory nerve terminals (Ault and Hildebrand, 1993; Carlton et al., 1995). Administration of glutamate into a joint produces a hyperalgesic response, while glutamate receptor antagonists can provide protection against its development (Sluka et al., 1994; McNearney et al., 2000). The concentration of glutamate in the synovial fluid of humans with active arthritis, in particular in Reiter's, systemic lupus erythematosus and infectious arthropathies, is increased, which suggest in addition that glutamate mediated events may contribute to the pathogenesis of arthritic conditions (McNearney et al., 2000). Etc..

Finally, although it is clear that further investigations are required to clarify the role(s) played by glutamate (receptors) and GS in the different peripheral cells/tissues/organs under normal conditions and different types of conditions and disorders, tianeptine, due to its discovered mechanism of action on GS and already demonstrated therapeutic properties, should offer real therapeutic potential in a wide range of additional peripheral disorders associated with an

(absolute or relative) deficiency or excess of glutamate.

Tianeptine may be first useful in varied conditions/disorders associated with (altered) cell differentiation. For instance in bone, tianeptine indications might not only involve bone disorders characterised by inappropriate and excessive changes in bone formation and/or bone resorption (see above) but also haematopoietic disorders. In particular, bone marrow megakaryocytes, which primary function is the production and release of functional platelets, express NMDA subunits (NMDAR1, NMDAR2A and NMDAR2D). Cell lines as well as primary megakaryocytes incubated with a NMDA glutamate receptor antagonist fail to produce proplatelet structures, and lack the cytoplasmic characteristics and organelles essential for the production of functioning platelets (Genever et al., 1999b; Hitchcock et al., 2003).

Tianeptine may be also useful in disorders associated with mucous membrane (barrier) injuries or disruptions e.g. for the treatment of skin diseases and enhancement of wound healing (epidermal renewal). Glutamate plays an important role as a signal of cutaneous barrier homeostasis and epidermal hyperplasia induced by barrier disruption (Genever et al., 1999a; Fuziwara et al., 2003). In resting skin epidermis, immunoreactive NMDA-, AMPA- and metabotropic-type glutamate receptors are colocalized with the specific glutamate transporter EAAC1 in basal layer keratinocytes, and GLT-1 (glutamate transporter type 1), a related transporter, is expressed suprabasally. In full-thickness skin wounds, marked modifications in the distribution of N-methyl-D-aspartate receptors and EAAC1 are observed during re-epithelialization. Topical application of L-glutamic acid, L-aspartic acid (non-specific glutamate receptor agonists) and N-methyl-D-aspartate (NMDA type receptor agonist) delays the barrier recovery rate after barrier disruption with tape stripping. On the other hand, topical application of D-glutamic acid (a non-specific antagonist of glutamate receptor), MK 801 or D-AP5 (NMDA-type receptor specific antagonists) accelerates the barrier repair. Topical application of MK-801 also promotes the healing of epidermal hyperplasia induced by acetone treatment under low environmental humidity. Immediately after barrier disruption on skin organ culture, secretion of glutamic acid from skin is significantly increased.

Tianeptine may be as well useful in immunological disorders. The Inventors already mentioned (see above) the increased glutamate in the synovial fluid of patients with active arthritis, in particular in Reiter's and systemic lupus erythematosus (McNearney et al., 2000). In addition, functional glutamate receptors are present in different thymic cell compartments, and might have a role in the intrathymic lympho-stromal relationships regulating thymocyte differentiation (Storto et al., 2000a). How these receptors are activated is matter of speculation. Glutamate receptors may respond to glutamate, which may either enter the thymus from the bloodstream (where it is present in micromolar concentrations) or may be released from stromal cells or thymocytes. Glutamate receptors are also found on peripheral blood lymphocytes and elevated extracellular glutamate concentrations may inhibit peripheral lymphocyte functions (Kostanyan et al., 1997; Droge et al., 1988; Lombardi et al., 2001); an inverse correlation between extracellular glutamate levels and T lymphocyte proliferation has been observed (Droge et al., 1988). The relevance of this observation could be substantial, because of the evidence that excitatory amino acid neurotransmission is related to many pathological conditions characterized by impaired immune functions (AIDS, Alzheimer disease, hepatic encephalopathy, chronic epilepsy, multiple sclerosis, ...), and that high plasma glutamate concentrations (commonly observed in patients with AIDS and neoplastic diseases) inhibit lymphocyte responses to mitogens and macrophage cysteine release into the extracellular space (Droge et al., 1988; Eck et al., 1989). We can presume that either elevated plasma glutamate concentrations or large glutamate release in tissular extracellular spaces e.g. in nervous tissues (Olney, 1990; Lipton & Rosenberg, 1994) may impair lymphocyte functions and have important secondary immunological consequences.

Tianeptine may be in addition useful in conditions and disorders associated with, or resulting in abnormal regulation of endocrine tissues. Glutamate receptors are believed to participate in the regulation of hormone secretion in varied endocrine tissues e.g. in pancreas, adrenal glands, pineal gland or pituitary gland. Pancreatic islets express both NMDA and non-NMDA ionotropic glutamate receptors. Activation of AMPA receptors can potentiate insulin and glucagon secretion by modulating the intrinsic properties of $\alpha$-and $\beta$-cells (Bertrand et al., 1993; Gonoi et al., 1994; Inagaki et al., 1995; Liu et al., 1997b; Weaver et al., 1996 and 1998). The endocrine pancreas can be influenced by glutamate via activation of metabotropic glutamate receptors (Tong et al., 2002). Etc.. NMDA receptor channels are also likely constitutively expressed in rat adrenal medulla, and adrenal medulla secretion may be regulated by glutamate (Watanabe et al., 1994; Yoneda and Ogita 1986; Kristensen, 1993; Hinoi et al., 2002c). In the pineal gland, pinealocytes accumulate L-glutamate in microvesicles and secrete it through an exocytic mechanism. The secreted glutamate binds to constitutively expressed metabotropic glutamate receptors and inhibit norepinephrine-stimulated melatonin synthesis in neighboring pinealocytes. GluR1, a functional isoform of the AMPA glutamate receptor, might participate in a signaling cascade that enhances and expands a cholinergic and glutamatergic signal throughout the pineal gland (Yatsushiro et al., 2000). These pineal systems are believed to function as a negative regulatory mechanism for melatonin synthesis (Govitrapong et al., 1986; Yamada et al., 1998; Yatsushiro et al., 2000). Finally, in the pituitary gland, GluR6:7 subunits of kainate receptors are likely constitutively expressed in some anterior pituitary cells, and pituicytes in the neural lobe thus may be probably regulated by glutamate (Hinoi and Yoneda, 2001; Kiyama et al., 1993). Etc..

Tianeptine may be even useful in certain sexual disorders. In fact, there is evidence for the expression of glutamate receptors in testis (immunocytochemical analysis of bioptic samples from human testis shows a high expression of mGlu5 receptors inside the seminiferous tubuli, whereas mGlula immunoreactivity is restricted to intertubular

spaces). In mature human sperm, mGlu5 receptors are present and active (Storto et al., 1999 and 2001; Nagata et al., 1999). Some sexual responses, in particular penile erection, are controlled by neural circuits in the brain and spine that are stimulated by the binding of excitatory amino acids to postsynaptic NMDA receptors. In penile, urinary bladder and ventral prostate tissues from adult male rats, and homologous surgical tissues from human male patients, all the essential NMDA receptors subunits are present. These tissues bind NMDA receptor ligands and NMDA receptor antagonists relax strips of these tissues (Gonzalez-Cadavid et al., 2000).

Therefore, the present invention extends the use of tianeptine, salts thereof, isomers thereof, pro-drugs thereof, metabolites thereof and structural analogs thereof, in the preparation of medicaments to be used for prevention and/or treatment of all conditions and disorders which are related to (absolute or relative) deficiency or excess of glutamate, in particular selected from those disclosed in Tables 2 and 3, whatever their cause and their pathophysiology, with the exception of the already proposed clinical applications of tianeptine (disclosed in Table 5). The present invention also concerns methods of treatment for these additional conditions and disorders which are related to or result in (absolute or relative) deficiency or excess of glutamate, comprising administering an efficient amount of tianeptine or a salt thereof, an isomer thereof, a pro-drug thereof, a metabolite thereof or a structural analog thereof. Preferably, said conditions and disorders are selected from those disclosed in Tables 2 and 3, excluding those disclosed in Table 5. In fact, all the already proposed clinical applications of tianeptine, including asthma, cough, depression (major depression with or without melancholia, dysthymic disorders, depressed bipolar disorder, ...), irritable bowel syndrome, mnemo-cognitive disorders, neurodegenerative diseases (such as cerebral hypoxia, cerebral ischaemia, cerebral traumatism, cerebral ageing, Alzheimer's disease, multiple sclerosis, amyotrophic lateral sclerosis, demyelinating pathologies, encephalopathies, myalgic encephalomyelitis, chronic fatigue syndrome, post-viral fatigue syndrome, the state of fatigue and depression following a bacterial or viral infection, the dementia syndrome of AIDS, ...), nonulcer dyspepsia, pain, psychoneurotic disorders (neurotic or reactive states of depression, anxiodepressive states with somatic complaints such as digestive problems, anxiodepressive states observed in alcoholic detoxification, ...), stress and stroke, are likely related to or result in, at least in part, (absolute or relative) deficiency or excess of glutamate. Thus the additional target conditions and disorders for tianeptine complete these already claimed conditions and disorders. They comprise in particular the above mentioned conditions and disorders from "peripheral" SOCs. Considering the large and complex distribution of the different glutamate receptor subtypes, the importance and variety of their already known physiological and pathophysiological roles, and the quasi ubiquitous distribution and critical role of GS in cellular metabolism, a myriad of central and peripheral disorders can be speculated to be related to or result in (absolute or relative) deficiency or excess of glutamate and to benefit from a treatment with tianeptine. Considering that it is almost impossible to clearly distinguish the conditions and disorders which can be the targets of tianeptine due to its effects on glutamate from those due to its effects on de novo synthesis of glutamine, all the potential indications have been gathered in the Tables 2 and 3 which present a list of conditions and disorders which (can) benefit from medications activating, modulating/regulating or inhibiting GS activity. These conditions and disorders are reported according to the MedDRA classification (MedDRA Version 7.1). For most system organ classes (SOC), we have indicated the included high level group terms (HLGT) of particular interest; for certain HLGT, we have indicated the included high level terms (HLT) of particular interest; for certain HLT, we have indicated the included preferred terms (PT) of particular interest. Potential indications, for which we have emphasized that they are or can be related to (absolute or relative) deficiency or excess of glutamate, are marked in "*italic*". All the already claimed or proposed clinical applications of tianeptine are "double-underlined". Potential factors and mechanisms which can underlie these conditions and disorders are disclosed in Table 4, without to be limited thereto.

Similarly, instead of tianeptine, GS ligands selected from the group consisting of GF, MS, hydrazines and bisphosphonates, and salts thereof, isomers thereof, pro-drugs thereof, metabolites thereof and structural analogs thereof, can also be used, "at low or intermediate doses" i.e. doses resulting in concentrations which increase or modulate/regulate GS activity (directly or indirectly) in the target cells/tissues/organs, to prevent and/or treat these conditions and disorders. In particular, GS ligands selected from the group consisting of GF, MS, hydrazines and bisphosphonates, and salts thereof, isomers thereof, pro-drugs thereof, metabolites thereof and structural analogs thereof, can be used in the preparation of medicaments intended for prevention and/or treatment of conditions and disorders which are related to or result in (absolute or relative) deficiency or excess of glutamate, in particular selected from those disclosed in Tables 2 and 3, whatever their cause and their pathophysiology, with the exception of: if said GS ligand is MS, the conditions and disorders related to cerebral ischemia, hyperammonemia and mycobacterial infectious disorders; if said GS ligand is a hydrazine, the conditions and disorders disclosed in Table 6; and, if said GS ligand is a bisphosphonate, the conditions and disorders disclosed in Table 7.

GS ligands selected from the group consisting of tianeptine, GF, MS, hydrazines and bisphosphonates, and salts thereof, isomers thereof, pro-drugs thereof, metabolites thereof and structural analogs thereof, may be in particular combined with treatments preventing from excitotoxic properties of glutamate through other mechanisms e.g. with drugs altering glutamate receptor(s) function(s), in particular with NMDA glutamate receptor antagonists (D-AP5, ketamine, memantine, MK-801, ...), and glucocorticoids (to decrease the doses of glucocorticoids and/or prevent adverse effects

of glucocorticoids. The present invention contemplates also a pharmaceutical composition comprising such combination and its use for the preparation of a medicament or in a method of treatment.

***Functions, conditions and disorders related to glutamine***

In animals, glutamine is the most abundant free amino acid. It has the largest free pool and one of the highest fluxes over organs of all amino acids (Young and Ajami, 2001). Glutamine serves as a fuel source that is more or less important depending on the cell type and (patho)physiological condition; for instance it is crucial in the gastrointestinal tract and immune system (Wilmore, 2001; Newsholme, 2001). It is involved in essential cellular syntheses. It provides nitrogen for the synthesis of purine and pyrimidine nucleotides, which are necessary for the synthesis of new DNA and RNA. It is involved in the synthesis of agents like glutamate, glutathione, gamma aminobutyric acid, glucose but also arginine, citrulline, tryptophane, ... It participates in homeostatic functions e.g. in maintaining normal acid-base balance by providing the ammonia that is necessary to counterbalance acidic compounds and/or in carrying potentially toxic ammonia to the kidneys for excretion. During metabolic acidosis, the kidneys can siphon off large amounts of glutamine. It is involved in the production of the body's most important antioxidants, glutathione (Welbourne and Dass, 1982; Cao et al., 1998; Hong et al., 1992; Manhart et al., 2001) and the amino acid taurine (Boelens et al., 2003). Glutathione is present at high concentrations in most mammalian cells and has many protective and metabolic functions. It is quantitatively the most important endogenous scavenger with the ability to hinder from oxidative injury caused by oxygen-derived free radicals and peroxides (Beutler, 1989). Glutathione protects cell membranes by reducing peroxides. By maintaining the thiol groups of many proteins in the reduced form, it ensures their normal function, thereby influencing the activity of several enzymes (Meister, 1991). Glutathione also prevents activation of transcription factor nuclear factor-kB, a stimulator involved in the synthesis of certain cytokines and adhesion molecules (Roth et al., 2002), and thus inhibits inflammatory responses. Etc.. All this illustrates the critical role of glutamine in vivo (for review see Labow et al., 2001; Hertz and Zielke, 2004; Melis et al., 2004).

Animals have evolved normally effective mechanisms to control glutamine homeostasis. These mechanisms are usually remarkably effective in maintaining a constant plasma glutamine level, even when faced with variable dietary intake and changing glutamine demand. Significant decreases in plasma glutamine concentration are only observed in states of critical illness e.g. cancer with intense chemotherapy and/or radiotherapy, endotoxemia, sepsis, starvation, surgery, trauma, implicating that glutamine may become a conditionally essential amino acid in patients with catabolic disease(s) (Houdijk et al., 1998; Labow et al., 1998; Planas et al., 1993; Eaton, 2003; Miller, 1999; Neu et al., 2002). In catabolic states, which are characterized by a loss tissue/body mass related to the disease itself, to its treatment and/or to physical inactivity (Cuthbertson et al., 1979), the consumption of glutamine exceeds glutamine synthesis and supply from proteolysis, resulting in depletion of the glutamine stores; large amounts of glutamine are released from muscle (Karinch et al., 2001) while glutamine consumption in certain cells and tissues e.g. in the immune system increases (Newsholme, 2001). The goal of nutritional support in catabolic patients is to prevent loss of lean body mass as far as possible. Besides preventing loss of lean body mass, nutritional supplementation with high-doses of glutamine influence the response of the patient to metabolic stress, in particular by protection of body cells from oxidative stress, enhancement of the immune system and enforcement of the gut barrier function (Melis et al., 2004).

In certain cells e.g. in astrocytes, all the glutamine has to be synthesized de novo from glutamate and ammonia by GS (see above). In others e.g. for certain functions in the gastrointestinal tract, de novo synthesized glutamine is more effective as compared to exogenous glutamine (see above; Weiss et al., 1999; Le Bacquer et al., 2001; Li et al., 2004). Consequently, GS activity is or may become conditionally limiting in certain cells, tissues or organs. For instance, GS activity in glial cells is critical for the moment-to-moment sustenance of astrocytic but also neuronal function in the nervous tissue (Barnett et al., 2000). In certain pathologic states, although glutamine plasma level is normal, glutamine deprivation can occur in specific cells/tissues, due to either an increase of local demand and/or insufficient local synthesis of glutamine. It might be the case for the dendritic atrophy of CA3 pyramidal neurons induced by glucocorticoids against which we observed that tianeptine but also GS ligands like GF and MS could be protective (Figure 30).

GS activity and expression are susceptible to inhibition by oxidative stress (Kosenko et al., 2003). During most critical cellular, tissular, organ, multi-organ as well as whole body disorders/failures, a state of oxidative stress occurs due to an imbalance between increased production of reactive oxygen species and natural antioxidant depletion. This can trigger death in certain cells in case of glutamine deprivation (Lee et al., 1998; Shohami et al., 1999; Eaton, 2003). In certain catabolic states, glutamine supplementation can restore decreased glutathione concentrations and, to some extent, prevent ongoing oxidative stress with the subsequent risk of tissue damage (Robinson et al., 1992; Fläring et al., 2003). Glutamine also regulates the production of taurine. One function of taurine is to trap chlorinated oxidants by producing the nontoxic, long-lived, taurine chloroamine, and therefore protect the cell from self-destruction (Marcinkiewicz et al., 1995). As taurine is exceptionally abundant in the cytosol of inflammatory cells, especially in neutrophils, taurine travels with the migrating neutrophils to the damaged tissue to combat free radicals (Koyama et al., 1992). Glutamine supplementation was also observed to prevent extracellular water retention in catabolic patients; and this effect could

be also exercised through taurine, because taurine can serve as an osmoregulator (Scheltinga et al., 1991). Glutamine has been also shown to increase survival of distal pulmonary epithelial cells during hyperoxia; while high oxygen concentrations which are used in the treatment of acute respiratory distress syndrome and hyaline membrane disease are known to damage alveolar epithelial cells through the release of free oxygen radicals (McGrath-Morrow and Stahl, 2002).

So, various agents and mechanisms regulate GS expression and activity in a tissue-specific fashion. For instance, an increase of glutamine export by skeletal muscles and lungs is associated with increased glutamine consumption by other organs such as the gut, liver and immune system, in response to various stresses; unlike skeletal muscle, which contains substantial quantities of free glutamine, the lung primarily contributes glutamine synthesized de novo from glutamate and ammonia (Ardawi, 1990; Labow et al., 1998 and 1999). Glucocorticoid hormones, which are the primary mediator of GS expression during stress, act on the lung and skeletal muscle rather fast. This hormonal regulation allows GS mRNA levels to increase during catabolic states. However, the ultimate level of GS enzyme expression is further governed by a post-transcriptional mechanism regulating GS protein stability. In a unique form of product feedback, GS protein turnover is increased by glutamine. This mechanism appears to provide a way to index the production of glutamine to its intracellular concentration and, therefore, to its systemic demand (Labow et al., 2001). Both GS mRNA and GS protein expression increase markedly in atrophic muscle after denervation (Falduto et al., 1992) like after chronic exposure to exogenous glucocorticoids (Hickson et al., 1996). In addition to produce significant muscle atrophy, both of these stimuli induce glutamine efflux and deplete muscle glutamine stores (Babij et al., 1986; Hundal et al., 1990).

The Inventors already (see above) mentioned that functional glutamate receptors are present in different thymic cell compartments and might have a role in the intrathymic lympho-stromal relationships regulating thymocyte differentiation (Storto et al., 2000a). Glutamate receptors were also found on peripheral blood lymphocytes where elevated extracellular glutamate concentrations inhibit peripheral lymphocyte function (Droge et al., 1988; Kostanyan et al., 1997; Lombardi et al., 2001). In addition, glutamine has been demonstrated to play important roles in the immune response. It provides nitrogen for the synthesis of purine and pyrimidine nucleotides, which are necessary for the synthesis of new DNA and RNA during proliferation of lymphocytes, and for mRNA synthesis and DNA repair in macrophages (Newsholme, 2001; Curi et al., 1999). It is an important source of energy for the white blood cells. Freshly isolated lymphocytes, macrophages and neutrophils utilize glutamine at a rate similar to or greater than glucose (Ardawi and Newsholme, 1983). Neutrophils increase their phagocytic activity and rate of production of superoxide when glutamine is offered, in a dose-dependent manner (Furukawa et al., 2000). Furthermore, glutamine was shown to improve the function of neutrophils by reducing the adrenaline-induced inhibition of superoxide production (Garcia et al., 1999), and improving their mitochondrial functionality, ATP generation and protection from apoptosis (Pithon-Curi et al., 2003); the latter effect may be mediated by glutathione. In patients undergoing surgery, parenteral supplementation with glutamine increase HLA-DR expression on monocytes postoperatively (Spittler et al., 2001). Boelens et al. confirmed this result in trauma patients which was accompanied by a decrease in infectious morbidity (Houdijk et al., 1998; Houdijk et al., 1999; Boelens et al., 2002). Glutamine, therefore, is believed to be crucial for the functionality of the immune system.

The Inventors already mentioned that glutamine is utilized as a major fuel and nucleotide substrate by intestinal mucosal cells and the gut-associated immune system (Scheppach et al., 1994; McCauley et al.,1998; Wiren et al., 1998), and therefore could prevent intestinal atrophy in certain conditions or disorders (Ziegler et al., 2000 and 2003). The barrier function of the gastrointestinal tract can be impaired following stresses due to injury or surgery, as well as inflammation (Bjarnason et al., 1995; Israeli et al., 2004). This loss of barrier function may play a role in the translocation of bacteria and endotoxins across the gut wall, subsequently resulting in sepsis, prolonged systemic inflammatory response or, eventually, multiple organ failure (Deitch, 1992; Mainous et al., 1994). In an animal experiment, Potsic et al. studied the effect of dietary and endogenously produced glutamine on gut integrity in artificially fed baby rats (Potsic et al., 2002). Glutamine supplementation turned out to improve gut wall integrity. Methionine sulfoximine was given as a glutamine synthetase inhibitor and subsequently gut wall integrity was decreased.

So there is compelling evidence indicating that de novo synthesis of glutamine by glutamine synthetase plays an important role to maintain for normal cell, tissue and organ functions.

On the contrary, other works have draw attention to the impact of excess of de novo glutamine synthesis in certain conditions and disorders e.g. in hepatic encephalopathy where increased accumulation of glutamine was observed to aggravate anaerobic glycolysis and energetic failure (Albrecht, 2003; Zwingmann et al., 2003). Excess of glutamine induces mitochondrial permeability transition (MPT) in astrocytes (Rao et al., 2003); astrocytic mitochondria are more vulnerable to the effects of glutamine in induction of the MPT as compared to neurons e.g. to manganese induced MPT (Rao et al., 2004). The mechanism(s) by which glutamine selectively induces MPT in astrocytes is not understood completely. However, increased production of free radicals and associated oxidative stress are generally considered major factors in MPT induction (Castilho et al., 1995; Halestrap et al., 1997). Similarly, treatment of various cells with antioxidants showed attenuation of MPT, including in astrocytes exposed to laser irradiation (Jou et al., 2002) and in rat hepatocytes treated with ethanol (Higuchi et al., 2001). Glutamine has been shown to cause the production of free radicals in astrocytes in a concentration/dose-dependent manner (Jayakumar et al., 2004). So it is likely that glutamine-induced MPT is mediated by oxidative stress. MPT induction by excess of glutamine in astrocytes may be due to increased free radical production by hydrolysis of glutamine, and high ammonia concentrations in mitochondria resulting from glutamine hydrolysis may be responsible for the effects of glutamine; treatment of astrocytes with the mitochondrial glutaminase inhibitor, 6-diazo-5-oxo-L-norleucine, completely blocked free radical formation and MPT (Murthy et al., 2001; Jayakumar et al., 2004; Norenberg et al., 2004). Finally by interfering with mitochondrial functions, excess of de novo synthesis of glutamine may participate to impairment of energy metabolism in susceptible cells in other disorders e.g. inflammatory, immunological or ischaemic disorders.

Accordingly, the present invention concerns the use of tianeptine, salts thereof, isomers thereof, pro-drugs thereof, metabolites thereof and structural analogs thereof, in the preparation of medicaments to be used for prevention and/or treatment of conditions and disorders which are related to (absolute or relative) deficiency or excess of glutamine and/or sensitive to glutamine deprivation or glutamine supplementation, whatever their cause and their pathophysiology, with the exception of the already proposed clinical applications of tianeptine, which are disclosed in Table 5. All the already proposed clinical applications of tianeptine, including asthma, cough, depression (major depression with or without melancholia, dysthymic disorders, depressed bipolar disorder, ...), irritable bowel syndrome, mnemo-cognitive disorders, neurodegenerative diseases (such as cerebral hypoxia, cerebral ischaemia, cerebral traumatism, cerebral ageing, Alzheimer's disease, multiple sclerosis, amyotrophic lateral sclerosis, demyelinating pathologies, encephalopathies, myalgic encephalomyelitis, chronic fatigue syndrome, post-viral fatigue syndrome, the state of fatigue and depression following a bacterial or viral infection, the dementia syndrome of AIDS, ...), nonulcer dyspepsia, pain, psychoneurotic disorders (neurotic or reactive states of depression, anxiodepressive states with somatic complaints such as digestive problems, anxiodepressive states observed in alcoholic detoxification, ...), stress and stroke, might be related to a (absolute or relative) deficiency or excess of glutamine. Thus, the additional target conditions and disorders for tianeptine complete these already claimed conditions and disorders. They comprise in particular the above-mentioned conditions and disorders from "peripheral" SOCs. Considering the critical role of glutamine in vivo and the quasi ubiquitous distribution of GS, a myriad of central and peripheral disorders can be speculated to be related to an (absolute or relative) deficiency or excess of glutamine and to benefit from a treatment with tianeptine. Since it is quasi impossible to distinguish the conditions and disorders which can benefit from the effects of tianeptine on de novo synthesis of glutamine from those which can benefit from its effects on glutamate metabolism, all these potential indications have been gathered in the Tables 2 and 3. These tables present a list of conditions and disorders which can benefit from drugs activating, modulating/regulating or inhibiting GS activity. As above-mentioned, hyperammon(em)ia can result in an excess of glutamine. Therefore the conditions and disorders which (can) result, alone or combined, in a localized or a systemic ammonia increase are also claimed (see below; Table 1 and 4). All these conditions and disorders are reported according to the MedDRA classification (MedDRA Version 7.1). For most system organ classes (SOC), we have indicated the included high level group terms (HLGT) of particular interest; for certain HLGT, we have indicated the included high level terms (HLT) of particular interest; for certain HLT, we have indicated the included preferred terms (PT) of particular interest. All the already claimed or proposed clinical applications of tianeptine are "double-underlined". Potential factors and mechanisms which can underlie these conditions and disorders are disclosed in Table 4, without to be limited thereto.

Similarly, instead of tianeptine, GS ligands selected from the group consisting of GF, MS, hydrazines and bisphosphonates, and salts thereof, isomers thereof, pro-drugs thereof, metabolites thereof and structural analogs thereof, can also be used in the preparation of a medicament intended for prevention and/or treatment of conditions and disorders which are related to (absolute or relative) deficiency or excess of glutamine and/or sensitive to glutamine deprivation or glutamine supplementation, whatever their cause and their pathophysiology, at "low or intermediate doses" i.e. doses resulting in concentrations which activate or modulate/regulate GS activity in the target cells/tissues/organs (directly or indirectly), with the exception of: if said GS ligand is MS, the conditions and disorders related to cerebral ischemia, hyperammonemia and mycobacterial infectious disorders; if said GS ligand is a hydrazine, the conditions and disorders disclosed in Table 6; and, if said GS ligand is a bisphosphonate, the conditions and disorders disclosed in Table 7. For these ligands, all the already proposed clinical applications of tianeptine i.e. asthma, cough, depression (major depression with or without melancholia, dysthymic disorders, depressed bipolar disorder, ...), irritable bowel syndrome, mnemo-cognitive disorders, neurodegenerative diseases (such as cerebral hypoxia, cerebral ischaemia, cerebral traumatism, cerebral ageing, Alzheimer's disease, multiple sclerosis, amyotrophic lateral sclerosis, demyelinating pathologies, encephalopathies, myalgic encephalomyelitis, chronic fatigue syndrome, post-viral fatigue syndrome, the state of fatigue and depression following a bacterial or viral infection, the dementia syndrome of AIDS, ...), nonulcer dyspepsia, pain, psychoneurotic disorders (neurotic or reactive states of depression, anxiodepressive states with somatic complaints such as digestive problems, anxiodepressive states observed in alcoholic detoxification, ...), stress and stroke, which might be related to a (absolute or relative) deficiency or excess of glutamine, are claimed.

Thus "low or intermediate doses" of a GS ligand selected from the group consisting of tianeptine, GF, MS, hydrazines and bisphosphonates, and salts thereof, isomers thereof, pro-drugs thereof, metabolites thereof and structural analogs thereof, may serve to activate or modulate/regulate GS homeostatic role in preventing and/or treating conditions and disorders related to critical cellular stresses, in particular in conditions of hyperammon(em)ia and/or excess of

glutamate, or in catabolic states. Potential factors and mechanisms which can underlie these conditions and disorders are disclosed in Table 4, without to be limited thereto.

Preferably, a "low or intermediate dose" of a GS ligand selected from the group consisting of tianeptine, GF, MS, hydrazines and bisphosphonates, and salts thereof, isomers thereof, pro-drugs thereof, metabolites thereof and structural analogs thereof, will be combined with other etiologic and/or symptomatic measures e.g. glutamine nutritional supplementation in catabolic states including AIDS, cancer, heart failure, infection, inflammation, ischaemia, trauma, ..., in intensive care unit patients, subjects engaged in intense exercise, ..., to help in preserving the immune and gut barrier functions, and accelerate their rate of recovery, or measures to control nitrogenous supply in certain conditions and disorders associated with excess of glutamine.Therefore, the present invention concerns a pharmaceutical composition comprising a GS ligand selected from the group consisting of tianeptine, GF, MS, hydrazines and bisphosphonates, and salts thereof, isomers thereof, pro-drugs thereof, metabolites thereof and structural analogs thereof, and other etiologic and/or symptomatic drugs. In particular, GS ligands selected from the group consisting of tianeptine, GF, MS, hydrazines and bisphosphonates, and salts thereof, isomers thereof, pro-drugs thereof, metabolites thereof and structural analogs thereof, may be associated with glucocorticoids to improve their efficacy and/or prevent their adverse effects.

Inhibition of glutamine synthesis has the potential to reduce cell growth and to inhibit glutathione synthesis in tumors; if so, such inhibition should increase oxidative stress and hinder the ability of tumor cells to resist the effects of both chemical and radiation therapy.

Accordingly, the present invention concerns the use of GS ligands selected from the group consisting of tianeptine, GF, MS, hydrazines and bisphosphonates, and salts thereof, isomers thereof, pro-drugs thereof, metabolites thereof and structural analogs thereof, for the preparation of cytolytics medicaments, for instance to combat neoplasms or certain inflammatory diseases. GS ligands selected from the group consisting of tianeptine, GF, MS, hydrazines and bisphosphonates, and salts thereof, isomers thereof, pro-drugs thereof, metabolites thereof and structural analogs thereof, are administered to obtain a target-specific drug delivery i.e. concentrations which "saturate" the subunits of the enzyme in the targeted cells/tissues/organs (administration with a targeted carrier, local administration, ...), and/or combined with anti-proliferative (5-fluorouracil, asparaginase, ...) and/or anti-inflammatory therapies. The therapeutical amount result in concentrations of compound which can activate or modulate/regulate GS activity in the normal cells/tissues/organs.

***Others organisms with eukaryotic cells and prokaryotes***

Others (all) organisms with eukaryotic cells such as invertebrate animals (arthropods, protozoa, shellfish, worms, ...), algae, fungi (yeasts, molds, ...) or plants, and also prokaryotes e.g. algae or bacteria, express GS (iso) enzyme(s). In these (lower) organisms GS proteic structure(s), regulation(s) and (patho)physiological role(s) can differ to some extent compared to those observed in vertebrate animals.

For instance in plants, GS occurs in two major octameric forms, one in the cytosol (GS1) and one in the chloroplast (GS2), with a highly sophisticated pattern of distribution and regulation (Lea, 1997; Miflin and Habash, 2002; Ishiyama et al., 2004). GS1 may be either homomeric or heteromeric; molecular biological studies have identified a number of genes encoding GS1 subunits from various plant species. The isoenzymes of GS1 show organ- and cell-specific patterns of expression and are developmentally regulated. Edwards et al. using promoter analysis of the *GS3A* gene of pea, suggested that cytosolic GS is preferentially expressed in the vascular tissue of leaves (Edwards *et al.*, 1990). Many subsequent studies have confirmed the importance of the location of GS1 in the phloem and related vascular tissues (Tobin and Yamaya, 2001). GS1 is also localized in roots, and in a number of specialist tissues and organs involved in the generation and transport of reduced nitrogen. For instance, a nodule-specific GS1 isoenzyme is formed during the onset of nitrogen fixation (Lara et al., 1983) and one of the maize GS1 genes is preferentially highly expressed in the pedicels of developing kernels (Rastogi et al., 1998). GS1 diversity leads to very sophisticated changes in the nature of GS as the plant and its individual organs pass through different development stages. For instance, the GS1 subunit composition of sugar beet changes with respect to nitrogen nutrition and organ ontogeny (Brechlina et al., 2000). Changes in subunit composition in *Phaseolus vulgaris* leaves are due to the differential expression of the various GS1 genes during development and ageing (Cock et al., 1991). On the contrary, the plastidic form of GS (GS2), which is widely distributed in the chloroplast, is generally regarded as universal; GS2 is also present in plastids in roots and other non-green tissues, this distribution differing between species and with respect to plastid subtypes (Tobin and Yamaya, 2001). Finally in plants, the majority of primary nitrogen enters through the roots as nitrate, and nitrate reductase and nitrite reductase sequentially reduce the nitrogen to ammonium. GS1 is the major form of GS in roots, and so the ammonium taken up from the soil is directly converted to glutamine by its reaction; the role of GS2 in leaves is to reassimilate the $NH_3$ generated by photorespiration. The amide group from glutamine can be then transferred to glutamate by the action of the glutamate synthase (GOGAT). So ammonium is assimilated into glutamine and glutamate through a consecutive reaction of GS and glutamate synthase (GOGAT). This so-called GS/GOGAT cycle is of crucial importance for growth and development in plants; in fact, glutamine and glutamate are the donors for the biosynthesis of major nitrogen-containing compounds, including amino acids, nucleotides, chlorophylls, polyamines and alkaloids (Miflin and

Lea, 1980; Lam et al., 1996; Lea et al., 1997; Miflin and Habash, 2002).

The nature of the metabolism occurring via GS depends on the environment of the plant, which may act directly or through the metabolic status of the plant and its different tissues, and varies over the course of the day (Stitt et al., 2002). The reactions and the forms of GS involved differ according to the plant organ under consideration. Within an organ, the role of GS and the metabolism in progress differ according to the tissue, cell or subcellular compartment. Within any location, the metabolism differs according to the developmental stage of that part of the plant. In this regard, it is important to realize that developmental stages such as vegetative and reproductive growth are not linear but overlapping. Thus, the nature of GS and its regulation has to be approached by taking into account the multidimensional nature of nitrogen metabolism and appreciating the large differences that occur in glutamine metabolism between various locations in the matrix. The plant has evolved mechanisms to enable it to cope with this complexity and which enable it to survive in competition with other plants in its environment. In seed plants, this must place the greatest importance on the success of the seed, because mechanisms that do not support effective reproduction will not have been maintained during evolution.

Because engineering of nitrogen assimilation is very important agriculturally, improvement of nitrogen assimilation by molecular approach has been attempted for several years. Recent reports have shown that overproduction of GS can lead to a better performance of nitrogen utilization through the promoted recycling of ammonia released during photorespiration but can not enhance net nitrogen assimilation. GS transgenic plants showed improved growth under nitrogen-limiting conditions only when they were grown initially under nitrogen-sufficient conditions (Migge et al., 2000; Fuentes et al., 2001). On the contrary, overexpressing GS activity in roots can lead to a decrease in plant biomass production due to a lower nitrate uptake accompanied by a redistribution to the shoots of the newly absorbed nitrogen which can not be reduced due to the lack of nitrate reductase activity in this organ (Limami et al., 1999). Nitrogen assimilation requires not only inorganic nitrogen but also the carbon skeleton 2-oxoglutarate (2-OG) that is produced through sequential reactions from photoassimilated carbohydrates (Gallardo et al., 1999; Migge et al., 2000; Fuentes et al., 2001; Oliveira et al., 2002; Limami et al., 2002).

While MS and GF, and derivatives thereof exhibit herbicidal activity at "high concentrations" (this effect is accounted for by impairment of nitrogen metabolism, resulting from inhibition of GS; excess ammonium and glutamine deficiency act in concert to cause plant death) (Lea and Ridley, 1989; Sadunishvili et al., 1996), lower concentrations of MS and GF, and derivatives thereof have been reported recently to have an opposite effect; glutamine synthetase was observed to be activated with a concomitant stimulation of plant growth and productivity (Evstigneeva et al., 2003). Accordingly, the present invention concerns also the use of tianeptine, salts thereof, isomers thereof, pro-drugs thereof, metabolites thereof and structural analogs thereof, in the manufacture of treatments intended for the activation, modulation/regulation or inhibition of all types of functions which involve GS activity or can be influenced by GS activity, directly or indirectly, with the aim to facilitate plant growth, protect plants from certain stresses and disorders, or combat plant growth, respectively. Therefore, in a particular embodiment, the present invention concerns the use of tianeptine, salts thereof, isomers thereof, pro-drugs thereof, metabolites thereof and structural analogs thereof to facilitate plant growth or protect plants from certain stresses and disorders. In an alternative embodiment, the present invention concerns the use of tianeptine, salts thereof, isomers thereof, pro-drugs thereof, metabolites thereof and structural analogs thereof as herbicides.

In particular, tianeptine, a salt thereof, an isomer thereof, a pro-drug thereof, a metabolite thereof or a structural analog thereof, may be associated crop tolerant to glufosinate i.e. expressing the gene coding for phosphinothricin tolerance (*bar*), which encodes the enzyme phosphinothricin acetyltransferase (PAT) (PAT is derived from *Streptomyces hygroscopicus and Streptomyces viridochromogenes*; EP 275957 was filed in 1987 by Aventis CropScience (former AgrEvo), with the aim to facilitate their growth, protect them from certain stresses and disorders, or combat their growth.

According to recent reports (United Nations Programme) worldwide nitrogen pollution is one of the main threats to human survival and environment. Sixty per cent of nitrogen resulting from human activities is inorganic crop fertiliser, the global use of which has increased 10-fold in the last half century (UNEP, 1999). Plants typically take up less than half of the N fertiliser applied with the majority lost to the atmosphere or dissolved in surface of groundwater (Vitousek et al., 1997). Nitrogen is a rate-limiting element in plant growth. Although the ability of plants to take up nitrate or reduce nitrate uptake is not limited, it is the ability to incorporate nitrogen into proteins which appear to be limited (Lam et al., 1995). Nitrogenous fertiliser accounts for 40% of costs associated with crops (Sheldrick et al., 1987). Increasing the efficiency of nitrogen use would be cost effective and would minimise problems of ground water contamination by excess nitrate application (Sheldrick et al., 1987). Conversely in starving condition, when plants suffer from low concentration of nitrogen or light (in photosynthetic plants), activation of GS would facilitate assimilation of $NH_4$ and change plants homeostasis, which would help them better resist the stress (Glevarec et al., 2004).

Accordingly, the present invention concerns also the use of tianeptine or salts thereof, isomers thereof, prodrugs thereof, metabolites thereof or structural analogs thereof, in the manufacture of treatments intended to reduce inorganic crop fertilizer requirement. Tianeptine, salts thereof, isomers thereof, pro-drugs thereof, metabolites thereof, and structural analogs thereof might be in particular associated with crop with a low N requirement (Foyer and Ferrario,

1994).

Conversely, high doses tianeptine can be used as a herbicide, since acute inhibition of GS by tianeptine at normal growing conditions decreases the production of glutamine and increases the accumulation of ammonia resulting in death of plant.

Depending on the type of cell, tissue, organ, organism, species, physiological or pathophysiological condition, ..., GS activity and de novo synthesis of glutamine can be more or less limiting. For instance, GS has been proposed to play a crucial role in the synthesis of a poly-L-glutamate-glutamine cell wall component which is found exclusively in pathogenic mycobacteria. Treatment of *Mycobacterium tuberculosis* with MS or with antisense oligodeoxyribonucleotides specific to *Mycobacterium tuberculosis* GS mRNA is associated with an inhibition of the formation of this poly-L-glutamate-glutamine cell wall structure. Paralleling this effect, these agents inhibit bacterial growth, indicating that GS plays an important role in these bacteria homeostasis. MS can block the growth in broth cultures of pathogenic mycobacteria, including *M tuberculosis, M. bovis, M. avium,* but has no effect on non-pathogenic mycobacteria as well as non-mycobacterial microorganisms at the doses used. It blocks the growth of *M. tuberculosis* and *M. avium* within human mononuclear phagocytes, the primary host cells of these pathogens, and at the concentrations which are effective are completely non-toxic to the mammalian cells, likely reflecting a 100-fold greater sensitivity to MS of bacterial GS than of mammalian GS. Finally MS protects guinea pigs challenged by aerosol with a highly virulent strain of *M. tuberculosis* from i) death, ii) disease, as manifested by protection against weight loss, and iii) growth and dissemination of *M. tuberculosis* in animal organs, as manifested by decreased CFU in the lungs and spleen. MS acts synergistically with the major antituberculosis drug isoniazid (INH), reducing CFU in guinea pig organs by ~1.5 log units below the level attained with INH alone. In comparison, GF was less selective and had only a very minor inhibitory effect on mycobacterium growth (US 6,013,660), so as it has never been proposed to be used as a drug.

Accordingly, the present invention concerns the use of tianeptine, salts thereof, isomers thereof, pro-drugs thereof, metabolites thereof and structural analogs thereof, in the preparation of medicaments for the treatment of pathogenic mycobacterium growth. The present invention also concerns a method of treatment of pathogenic mycobacterium growth in a subject comprising administering to said subject an efficient amount of tianeptine or a salt thereof, an isomer thereof, a pro-drug thereof, a metabolite thereof or a structural analog thereof. Preferably said pathogenic mycobacterium is selected from the group consisting of *M. tuberculosis, M. bovis, M. avium*, atypical mycobacteria (*M. avium Complex, M. chelonae*, *M. fortuitum, M. kansasii, M. marinum, M. scrofulaceum, M. ulcerans*), *M. leprae* more preferably the group consisting of *M. tuberculosis, M. bovis,* and *M. avium.* More preferably, said pathogenic mycobacterium is *M. tuberculosis.* More particularly, said treatment of pathogenic mycobacterium growth can be used as treatment of leprosy, paratuberculosis or tuberculosis.

More than 50 years after its discovery, isoniazid remains one of the primary drug for the chemotherapy of tuberculosis. Although several hypotheses have been proposed to explain its efficacy, its mechanism of action remains unclear. Its isopropyl derivative, iproniazid, is a well-established antidepressant. Developed in 1951 with isoniazid, it was found to have mood-elevating effects in patients with tuberculosis. In 1952, Zeller and Barsky found that iproniazid, in contrast to isoniazid, was capable of inhibiting the enzyme monoamine oxydase (MAO). Since then, iproniazid has been used for the treatment of depressed patients. MAO inhibitors had an important impact on the development of modem biological activity.

Based on the above-mentioned observations related to MS and tianeptine, the Inventors have tested isoniazid and iproniazid on GS activity and have found that both these drugs are GS ligands. Isoniazid and iproniazid are powerful competitive inhibitors of GS in its ammonia binding site.

Accordingly, the present invention in particular concerns a method of treatment of pathogenic mycobacterium growth in a subject comprising administering to said subject an efficient amount of tianeptine or a salt thereof, an isomer thereof, a pro-drug thereof, a metabolite thereof or a structural analog thereof, preferably associated with an other antituberculous agents e.g. isoniazid, ethambutol, pyrazinamide, rifampicin or streptomycin. Preferably said pathogenic mycobacterium is selected from the group consisting of *M. tuberculosis, M. bovis* and *M. avium.* More preferably, said pathogenic mycobacterium is *M. tuberculosis.* The present invention also concerns a pharmaceutical composition comprising such combinations.

On the contrary, activation of the GS is a feasible therapeutic strategy to protect bacteria from stress or facilitate their growth.

Finally, the present invention concerns the use of tianeptine, salts thereof, isomers thereof, pro-drugs thereof, metabolites thereof and structural analogs thereof, in obtaining activators, inhibitors and/or modulators of GS activity intended for the activation, inhibition and/or modulation of all types of functions which involve GS activity or can be influenced by GS activity, directly or indirectly, in non-vertebrate organisms with eukaryotic cells such as invertebrate animals (arthropods, protozoa, shellfish, worms, ...), algae, fungi (yeasts, molds, ...) or plants, and also prokaryotes e.g. in algae or bacteria, aiming to facilitate their growth or protect them from stresses and disorders, or, inversely, aiming to combat their growth. The present invention concerns methods to facilitate the growth, protect from stresses and disorders, or, inversely, combat growth, of non-vertebrate organisms with eukaryotic cells such as invertebrate animals

(arthropods, protozoa, shellfish, worms, ...), algae, fungi (yeasts, molds, ...) or plants, and also of prokaryotes e.g. algae or bacteria, comprising the administration an efficient amount of tianeptine or a salt thereof, an isomer thereof, a pro-drug thereof, a metabolite thereof or a structural analog thereof.

***Pharmaceutical form***

The pharmaceutical composition according to the present invention can be presented in pharmaceutical forms that are suitable for administration by the oral, parenteral (intramuscular, intravenous, intratissular, intratumoral, subcutaneous, ...), per- or transcutaneous, nasal, rectal, perlingual, ocular or respiratory route, especially injectable preparations, aerosols, eye or nose drops, sublingual tablets, glossettes, soft gelatin capsules, hard gelatin capsules, lozenges, suppositories, creams, ointments, dermal gels, dermal patchs, etc., those forms allowing the immediate release or delayed and controlled release, and/or targeted distribution of the active ingredient.

As basic salts, there may be used, without any limitation, sodium hydroxide, potassium hydroxide, calcium hydroxide or aluminium hydroxide, alkali metal or alkaline earth metal carbonates, or organic bases such as triethylamine, benzylamine, diethanolamine, tert-butylamine, dicyclohexylamine and arginine. As acid salts, there may be used without any limitation, hydrochloric acid, sulphuric acid, phosphoric acid, tartaric acid, malic acid, maleic acid, fumaric acid, oxalic acid, methanesulphonic acid, ethanesulphonic acid, camphoric acid and citric acid. A preferred salt of tianeptine is the sodium salt.

The dosage of the pharmaceutical composition according to the present invention varies principally according to the nature of the therapeutic indication. For instance, tianeptine, which p.o. dosage ranges from 12.5 mg to 300 mg per dose in its usual indications in human, could be effective at lower doses (<12.5 mg) in certain new indications, and GF within estimate of acceptable daily intake (<0.02 mg/kg) in certain indications.

***Screening methods***

In an other aspect of the present invention, the discovery of the target of tianeptine, and that tianeptine but also GS ligands such as GF, MS, hydrazines and bisphosphonates can behave as activator or modulators/regulators of GS activity i.e. can activate, inhibit or be tolerated depending on the dose and experimental or (patho)physiological condition, allows to design methods for screening, identifying and/or developing new drugs for prevention and/or treatment of all conditions and disorders previously proposed as clinical applications of tianeptine i.e. those disclosed in Table 5 and in particular asthma, cough, depression (major depression with or without melancholia, dysthymic disorders, depressed bipolar disorder, ...), irritable bowel syndrome, mnemo-cognitive disorders, neurodegenerative diseases (such as cerebral hypoxia, cerebral ischaemia, cerebral traumatism, cerebral ageing, Alzheimer's disease, multiple sclerosis, amyotrophic lateral sclerosis, demyelinating pathologies, encephalopathies, myalgic encephalomyelitis, chronic fatigue syndrome, post-viral fatigue syndrome, the state of fatigue and depression following a bacterial or viral infection, the dementia syndrome of AIDS, ...), nonulcer dyspepsia, pain, psychoneurotic disorders (neurotic or reactive states of depression, anxiodepressive states with somatic complaints such as digestive problems, anxiodepressive states observed in alcoholic detoxification, ...), stress and stroke.

Preferred therapies according to the invention involve activation, inhibition and/or modulation of GS, in particular of astrocytic GS. The invention is generally useful for identifying and developing drugs intended to treat patients affected by conditions and disorders which involve or can be influenced by GS activity, directly or indirectly, in particular those related to absolute or relative excess of ammonia, those related to absolute or relative deficiency or excess of glutamate, and those related to absolute or relative deficency or excess of glutamine, either isolated or associated, for instance those mentioned in Tables 1-4.

Preferred inhibitors of enzymatic conversion of glutamate to glutamine by GS are: drugs binding at the GS catalytic site such as tianeptine or ligands like GF, MS and glutamate analogs, drugs which compete with amine at the amine binding site of the enzyme such as hydrazines like iproniazid and isoniazid, and drugs docking into GS $Mn^{2+}$ binding sites such as bisphosphonates like pamidronate, risedronate and [(5-Chloro-pyridin-2-ylamino)-phosphono-methyl]-phosphinic acid.

The present invention concerns a method for screening, identifying and/or developing a new drug active in at least one of the conditions disclosed in Table 5, in particular asthma, cough, depression (major depression with or without melancholia, dysthymic disorders, depressed bipolar disorder, ...), irritable bowel syndrome, mnemo-cognitive disorders, neurodegenerative diseases (such as cerebral hypoxia, cerebral ischaemia, cerebral traumatism, cerebral ageing, Alzheimer's disease, multiple sclerosis, amyotrophic lateral sclerosis, demyelinating pathologies, encephalopathies, myalgic encephalomyelitis, chronic fatigue syndrome, post-viral fatigue syndrome, the state of fatigue and depression following a bacterial or a viral infection, the dementia syndrome of AIDS, ...), nonulcer dyspepsia, pain, psychoneurotic disorders (neurotic or reactive states of depression, anxiodepressive states with somatic complaints such as digestive problems, anxiodepressive states observed in alcoholic detoxification, ...), stress and stroke, wherein the method comprises the

screening and the identification of compounds activating, inhibiting and/or modulating/regulating GS activity.

In a first embodiment, the method comprises:

a) contacting in vitro a candidate compound with GS or a fragment thereof; and,
b) determining the ability of said candidate compound to bind said glutamine synthetase or a fragment thereof.

Binding to said glutamine synthetase or a fragment thereof provides an indication of the compound's ability to modulate the activity of said glutamine synthetase.

In a second embodiment, the method comprises:

a) contacting in vitro a candidate compound with GS in conditions allowing GS activity; and,
b) determining the ability of said candidate compound to activate, inhibit and/or modulate the activity of said GS.

In a third embodiment, the method comprises:

a) adding to a cell expressing GS a candidate compound; and,
b) determining the ability of said candidate compound to activate, inhibit and/or modulate the activity of said GS.

Optionally, said screening method is performed in vivo, ex vivo or in vitro.

Preferably, said cell is an astrocyte. More preferably, said astrocyte is incubated with the candidate compound in absence of glutamine. The contacting step is performed for a period of time and under culture conditions adequate to expose the cell to glutamate and the candidate compound. The ability to activate and/or inhibit the GS activity can be performed by determining the cellular survival in the culture or the increase of glutamine in the extracellular medium. Optionally, said astrocytes can be in culture in the presence of neurones.

In a preferred embodiment of the screening methods according to the present invention, said GS fragment comprises or consists of the catalytic site, the amine binding site and/or the GS $Mn^{2+}$ binding site. By fragment is intended for instance a peptide from 5 to 100, preferably 10 to 50 amino acids. More preferably, said fragment comprises or consists of more or less one part of the sequence ITGTNAEVMPAQWEFQIGPCEGIR (SEQ ID No 1).

In a preferred embodiment of the screening methods according to the present invention, said GS activity is the conversion of glutamate to glutamine, or of glutamine to glutamate.

In a fourth embodiment, the method uses at least one functional antibody directed against said GS. A functional antibody refers to an antibody, a fragment thereof or a derivative thereof, which specifically binds GS and is able to modify its activity. In a preferred embodiment of the screening methods according to the present invention, said GS fragment comprises or consists of the catalytic site or the amine binding site. By fragment is intended for instance a peptide from 5 to 100, preferably 10 to 50 amino acids. More preferably, said fragment comprises or consists of more or less one part of the sequence of SEQ ID No 1. This method of screening comprises contacting a candidate compound with a functional antibody and a target molecule, wherein the target molecule comprises an epitope of GS which is recognized by said functional antibody, and determining whether said candidate compound inhibits the binding of said functional antibody to said target molecule. Preferably, inhibition of the binding of said functional antibody to said target molecule is determined by inhibition ELISA. This method is detailed in the PCT patent application n° WO2005040820.

In a fifth embodiment, the method comprises a step of molecular modeling using the 3D structure of GS (Gill and Eisenberg, 2001). The molecular modeling allows the selection and/or the design of drug which binds GS, more particularly in its catalytic site, in its amine binding site or in its $Mn^{2+}$ binding site(s).

In a further embodiment, the method comprises determining the effect of said candidate compound on one or several exhibited symptoms in a subject. Said subject can be an animal or a human model for a condition or a disorder. Said subject can also be a patient.

In an additional embodiment, the method combines several above-mentioned methods. For instance, the method can comprise several steps selecting from the group consisting of a step of molecular modeling, a step of in vitro binding assay, a step of in vitro functional assay, a step of toxicity evaluation, and a step of in vivo functional assay.

The present invention also concerns a method for screening, identifying and/or developing a new biologically active compound (drug) comprising a competition assay on GS with a candidate compound and tianeptine. The new biologically active compounds (drugs) are those that show a capacity to compete with tianeptine for the binding to GS. Competition assays are well-known by the man skilled in the art. For instance, said method comprises the following steps:

a) contacting in vitro, GS or a fragment thereof with a candidate compound;
b) adding tianeptine; and,
c) determining the ability of said candidate compound to bind GS or a fragment thereof.

Preferably, said GS fragment comprises the catalytic site. More preferably, said fragment comprises or consists of the sequence SEQ ID No 1. Preferably, said tianeptine is labeled (e.g. by fluorescence or radioactivity). The ability of said candidate compound to bind GS or a fragment thereof is determined by the measure of the released tianeptine. This method can comprise additional steps such as determining the ability of the selected compound to activate, inhibit and/or modulate GS (in vitro and/or in vivo), determining the toxicity of the selected compound and determining the biological activity of the selected compound on a animal model, a healthy subject or a patient.

The present invention also concerns a method for screening, identifying and/or developing a new biologically active drug comprising a competition assay on GS with a candidate compound and a hydrazine like iproniazid or isoniazid. The new biologically active drugs are those that show a capacity to compete with a hydrazine for the binding to GS. Competitive assays are well-known by the man skilled in the art. For instance, said method comprises the following steps :

a) contacting in vitro GS or a fragment thereof with a candidate compound;
b) adding a hydrazine; and
c) determining the ability of said candidate compound to bind GS or a fragment thereof.

Preferably, said GS fragment comprises the amine binding site. Preferably, said hydrazine is labeled, (i.e., by fluorescence or radioactivity). The ability of said candidate compound to bind GS or a fragment thereof is determined by the measure of the released hydrazine.

The present invention also concerns a method for screening, identifying and/or developing a new biologically active drug comprising a competition assay on GS with a candidate compound and a bisphosphonate like pamidronate or risedronate. The new biologically active drugs are those that show a capacity to compete with a bisphosphonate for the binding to GS. Competitive assays are well-known by the man skilled in the art. For instance, said method comprises the following steps :

a) contacting in vitro GS or a fragment thereof with a candidate compound;
b) adding a bisphosphonate; and
c) determining the ability of said candidate compound to bind GS or a fragment thereof.

Preferably, said GS fragment comprises a $Mn^{2+}$ binding site. Preferably, said bisphosphonate is labeled (e.g. by fluorescence or radioactivity). The ability of said candidate compound to bind GS or a fragment thereof is determined by the measure of the released bisphosphonate.

The methods include binding assays and/or functional assays, and may be performed in vitro, in cell systems, in vertebrates, etc.. The above screening assays may be performed in any suitable device, such as dishes, flasks, plates, tubes, etc.. Typically, the assay is performed in multi-wells plates. Several test compounds can be assayed in parallel. Furthermore, the test compound may be of various origin, nature and composition. It may be any organic or inorganic substance, such as a lipid, peptide, polypeptide, nucleic acid, small molecule, etc., in isolated or in mixture with other substances. The compounds may be all or part of a combinatorial library of products, for instance.

Further aspects and advantages of the present invention will be disclosed in the following experimental section, which should be regarded as illustrative and not limiting the scope of the present invention.

## EXAMPLES

## MATERIAL AND METHODS

Most of chemical products including glufosinate, iproniazid, isoniazid, L-methionine sulfoximine, pamidronate and tianeptine were provided by Sigma-Aldrich. Mice and rats were purchased from Charles River Laboratories and Harlan.

### 1 Screening of tianeptine's target protein

1.1 Biotinylation of tianeptine

Tianeptine was chemically coupled to a long chain EZ-Linked-biotin-PEO-amine (PEO: polyethylene oxide) according to the manufacturer recommendations (Pierce). Biotin conjugated tianeptine was separated from unconjugated ones by using a phase inverse C18 column and eluted with acetonitrile (0-100% gradient). The purity of the biotinylated tianeptine was checked using mass spectrometry.

1.2 Isolation and identification of tianeptine's target protein

*1.2.1 Membrane preparation*

Based on literature and on a preliminary immuno-histological study in mice administered intraperitonealy (i.p.) with biotinylated tianeptine, we choose to work on hippocampus membrane proteins. Hippocampi from 10 BALB/c mice were isolated, minced and left for 1 h in 300 mM Tris HCl solution buffer (TBS) on ice. Thereafter, the preparation was homogenised and centrifuged at 10400 g for 20 min at 4°C. The supernatant was centrifuged at 141400 g for 1h at 4°C. The latter was repeated twice. Finally, the resultant pellet was diluted in 500 μl of TBS and stored at -20°C until use.

*1.2.2 SDS-PAGE and western blotting*

Mice hippocampus membrane proteins (twenty μg per well) were separated on a 12% sodium dodecyle sulfate polyacrylamide electrophoresis (SDS-PAGE) gel in presence of 2β-mercaptoethanol. This was performed in absence and in presence of 100 mM n-octyl glucoside. The separated proteins were thereafter transferred to a nitrocellulose membrane (pore size 0.20 μm). Non-specific binding sites were blocked with 5% low fat skimmed milk in TBS buffer supplemented with 0.1 % tween 20. The transferred proteins were allowed to react with biotinylated tianeptine (100 mM) for 2 h at room temperature. After one quick wash with TBS 0.1% tween 20, horseradish peroxidase conjugated streptavidin was subjected to the transferred proteins for 2 h at room temperature. Finally, the bound tianeptine, after several washes, was revealed using an electro chemiluminescence procedure (ECL).

*1.2.3 2D gel electrophoresis and western blotting*

Mice hippocampus membrane proteins were first focalized on a pH strip (range 3-10) over night at room temperature according to the standard procedure. The strip was then placed over a 12% acrylamide/bisacrylamide gel in presence of PDEA (2-(2PyridinylDithiol) Ethane Amine) and separated proteins were transferred to a nitrocellulose membrane (pore size 0.20 μm). Non-specific binding sites were blocked with 5% low fat skimmed milk in TBS buffer supplemented with 0.1% tween 20. Transferred proteins were allowed to react with biotinylated tianeptine (100 mM) for 2h at room temperature. After one quick wash with TBS 0.1% tween 20, horseradish peroxidase conjugated streptavidin was subjected to the transferred proteins for 2 h at room temperature. Finally, after several wash, the bound tianeptine was revealed using ECL.

*1.2.4 Mass spectrometry analysis - Sequence determination*

The spots issued from 2D gel electrophoresis/western blotting were digested and analysed for identifying the protein involved. The peptide sequences obtained form the latter were compared with sequences from protein banks.

1.3 In silico study of the interaction between tianeptine and GS

Starting from the glufosinate (phosphinotricin) - glutamine synthetase complex structure (PDB 1FPY) (Gill and Eisenberg, 2001), a glutamine synthetase monomer was created in which glufosinate and $Mn^{2+}$ were deleted. The tianeptine molecule was constructed *in silico* and minimalized using the AMPAC/MOPAC module of Accelerys (San Diego, California). It was manually docked in the glutamine binding site of glutamine synthetase and the complex minimalized by conjugated gradient minimalization of the DISCOVER module fixing the atoms of the glutamine synthetase molecule. To explore the position of tianeptine in the binding site, a molecular dynamic simulation was performed at 300°K for 100 psec after an equilibration phase of 10 psec. A second complex was obtained with a lower potential energy than the minimalized one. After minimalization of this complex by conjugated gradient, a further approach was used to take into account the possible charge effects on the complex. The complex was charged at a pH of 7.4 and subjected to a new round of molecular dynamics using a distance dependent dielectric constant. Three conformations with the lowest potential energy were chosen for further minimalization by conjugated gradient using a distance dependent dielectric constant.

## 2 Charaterization of GS ligands pharmacological effects

2.1 Study of the effects of GS ligands in vitro

*2.1.1 Effects of GF, iproniazid, MS and tianeptine on purified sheep brain GS activity*

The effects of glufosinate (GF), iproniazid, L-methionine sulfoximine (MS) and tianeptine on purified sheep brain GS activity were assessed according to Meister's method (Meister 1985). Briefly the assay mixture (final volume 0.5 ml) contained 0.1 M imidazole-HCl buffer (pH 7.2), 10 mM sodium arsenate, 20 mM $MnCl_2$, 125 mM hydroxylamine sodium, different concentrations of L-glutamine (0.1-50 mM), 20 μM ADP (sample buffer) and purified sheep brain GS. To study the effects on GS subunits (monomers), 25 mM 2β-mercaptoethanol (β-ME) was added to the assay mixture, with the exception of the experiments with tianeptine. In fact, tianeptine lost its activity in presence of β-ME due to its interaction with $SO_2$; thus β-ME had to be replaced by DTT (dithiothreitol) (25 mM). After 15 min incubation at 37°C, 0.5 ml of ferric chloride reagent (ferric [II] chloride 0.37 M with trichloroacetic acid 0.2 M) was added to stop the reaction. The formation of gamma-glutamylhydroxamate was assessed by reading the absorbance of the resultant solution at 535 nm against reagent blanks (the same sample buffer without either hydroxylamine, L-glutamine or ADP).

**L-Glutamine + $NH_2OH$ ----------> L- γ-glutamyl-hydroxamate + $NH_3$**

(in presence of sodium arsenate, $Mn^{2+}$ and ADP)

*2.1.2 Nature of the interaction between tianeptine and GS purified from sheep brain*

*2.1.2.1 Effect of a over-night dialysis on the binding/effect of tianeptine on purified sheep brain GS*

Two different concentrations of tianeptine (1 and 5 nM) were allowed to react with 3.4 units of GS purified from sheep brain. The reaction samples were prepared as follow: 200 μl of sample buffer (see above 2.1.1), 50 μl of tianeptine solution and 10 μl (3.4 units) of GS with ADP (20 μM).

Half of the samples were immediately incubated for 15 min at 37 °C. Before stopping the reaction with ferric chloride solution, a few μl of each sample were taken to be subjected to thin layer chromatography with a migration buffer composed of 9:1 DCM (dichloromethane) / methanol V/V. GS activity was assessed on the rest as mentioned above for purified sheep brain GS.

The second half of the samples was removed to microtubes, which were dialysed against the sample buffer over night at 4°C. Before stopping the reaction, the day after, a few μl of each sample were taken to be subjected to thin layer chromatography with a migration buffer composed of 9:1 DCM (dichloromethane) / methanol V/V, and GS activity was assessed on the rest.

*Effect of L-glutamate on the effect of tianeptine on GS activity*

The effects of increasing concentrations of L-glutamate (0.016-10 mM) on the inhibitory effect of 5 μM tianeptine were assessed on GS purified from sheep brain. GS activity was assessed as mentioned above in 2.1.1. Higher concentration than 10 mM of L-glutamate (100 and 500 mM) increased background activity so no specific activity could be measured.

The effect of tianeptine (100 nM) on extracellular free amine induced by glutamate (1, 10, 100, 500 μM) after 1 h incubation in C6 cells in serum free HBSS was also assessed. The effect of tianeptine (100 nM) in presence of N-methyl-D-aspartate (NMDA) (1, 10, 100, 500 μM) on extracellular free amine after 1 h incubation in C6 cells in serum free HBSS were tested for comparison. These experiments were performed according to the method mentioned below in 3.2.1.

*2.1.3 Effects of pamidronate and risedronate on GS activity in rat brain homogenates*

Wistar rat brain homogenates were incubated with pamidronate and risedronate ($2.27.10^{-9}$ to $7.96.10^{-21}$ or $8.75.10^{-20}$ M) in presence or absence of 2-βME. GS activity was assessed as mentioned above for purified sheep brain GS.

2.2 Study of the effects of GS ligands on GS activity and expression in vivo

*2.2.1 Effects of GF, MS and tianeptine on brain GS activity and expression in mice*

The effects of GF, MS and/or tianeptine on whole brain GS activity and expression were assessed i) 24 h after a single intraperitoneal (i.p.) administration in ICR mice [MS (0.078, 0.312, 1.25, 5, 20 mg/kg); tianeptine (0.078, 0.3125, 1.25, 5, 20 mg/kg); GS protein expression determined using western blot], and ii) 1 h after the last administration of a repeated (for 7 days) daily i.p. administration in BALB/c mice [GF (0.1, 3, 15 mg/kg); MS (0.5, 15, 45 mg/kg); tianeptine (0.5, 15, 45 mg/kg); GS protein expression determined by ELISA].

*2.2.1.1 Determination of GS activity*

The brain's right hemisphere from each mouse was deep frosted in liquid nitrogen immediately after euthanasia and kept at -80°C until use. On the day of assay, membranes were prepared in a chilled extraction buffer composed of HEPES (5 mM), sucrose (320 mM) and EDTA (1 mM). The pH was set to 7.4 with TRIS (100 mM). Ten μl of each brain mixture was incubated for 15 min at 37°C in presence of 250 μl of sample buffer (see above 2.1.1). The enzymatic reaction was stopped using 250 μl of ferric chloride reagent. The absorbance was measured at 535 nm after centrifugation at 10000 g for 10 min, and the protein concentration determined using a BCA (bicinchoninic acid) kit (read at 562 nm; Pierce). GS activity results were expressed as absorbance at 535 nm per μg of protein.

*2.2.1.2 Determination of GSprotein expression by western blot*

The brain's left hemisphere of each mouse was homogenised at 4°C in a buffer consisting of 50 mM Tris, 350 mM glucose and 5 mM $MgCl_2$ (pH = 7). The homogenate was centrifuged at 10000 g for 20 min at 4°C. The supernatant was centrifuged for 3 x 1 h at 20000 g at 4 °C. The resulting pellet was suspended in TBS (pH = 7) and the quantity of protein was determined using a BCA kit.

Twenty μg per well of protein from each sample were applied to a 12% acryl/bisacrylamid gel in presence of SDS and 2β-mercaptoethanol. Separated proteins were thereafter transferred to a 0.2 μm PVDF membrane. After saturation with 5% low fat milk in TBS (pH = 7) supplemented with 0.1% tween 20, membranes were incubated with goat monoclonal anti-GS antibodies (1/5000; AbCam) over night at 4°C. After several washes, the membranes were subjected to anti-goat antibodies conjugated to peroxidase (1/10000) for 1 h. Membranes were extensively washed and the complex of GS/antibody was revealed using an ECL method. Revealed western blots were analysed by densitometry (MacBas software). The results were expressed in absorbance unit per $pixel^2$ and μg of protein.

*2.2.1.3 Determination of GS protein expression by ELISA*

Brain homogenate (prepared as above mentioned for GS activity) were tested for GS expression. After centrifugation at 10500 g for 20 min at 4°C, the supernatant was collected. Its protein concentration was determined using a BCA kit. Fifty μl of different dilutions (0.2 to 0.025) of each sample in coating buffer (sodium carbonate buffer; pH 9.6) were incubated on microtitre plates (Falcon, USA) over a night at 4°C. After a single wash with TBS tween 0.1% (washing buffer), the plates were saturated with TBS tween 0.1% supplemented with 3% low fat graded dried milk (Biorad) for 1 h at 37°C. Anti-GS antibodies raised in goat (1/1000 dilution) were allowed to react for 1 h at 37°C. After 3 washes with washing buffer, the plates were incubated with peroxidase conjugated anti-goat antibodies for 1 h at 37°C, and washed 3 times with washing buffer and 4 times with just TBS. The bound antibodies were revealed with TMB (trimethyl benzidine) and $H_2O_2$ as substrates. The reaction was stopped after incubating the microtitre plates for 15 min at 37°C with HCl 1 M. The absorbance was read at 405 nm on a microtitre plate reader. The results were expressed in absorbance per μg of protein.

2.2.2 Effects of GF and tianeptine on GS activity in cortex, hippocampus and thalamus/hypothalamus in ICR mice

The effects of GF (0.1, 3 and 15 mg/kg), tianeptine (0.5, 15, 45 mg/kg) and placebo on GS activity in cortex, hippocampus and thalamus/ hypothalamus were assessed in ICR mice 24 h after a single i.p. administration. Cortex, hippocampi and thalamus/hypothalamus were carefully isolated immediately after euthanasia (as mentioned above for hippocampi). Membranes were prepared and the activity of GS determined in these different structures (as mentioned above for the whole brain).

**3 Evaluation of the therapeutic potential of GS ligands**

3.1 Effect of tianeptine on rat brain GS in condition of oxidative stress

Rat brain membrane preparation was pre-incubated for 2 min in sample buffer (see above 2.1.1) in presence of L-glutamine (50 mM) and ammonium dioxy persulfate (APS) (1.5%). Different concentrations of tianeptine ($1.1.10^{-4}$ to $2.3.10^{-12}$ M) were added. GS activity was assessed as mentioned above for purified sheep brain GS.

3.2 Effects of MS and tianeptine on C6 cells in condition of excess of ammonia and/or glutamate

*3.2.1 Effects on extracellular ammonium ion and free amine concentrations*

C6 glioblastoma cells were sealed to 6 wells culture plates at $10^5$ cells per well in presence of DMEM (Dulbecco's modified Egle's medium) and 10% fetal calf serum (FCS), and incubated 24 h at 37°C with 5% $CO_2$ before the start of the assay. Thereafter, after discarding DMEM medium, they were washed 3 times with HBSS (Hank's Balanced salt solution), distributed at $10^5$ cells per well into 6 wells culture plates in FCS free HBSS, and incubated for 1 h at 37°C and 5% $CO_2$ with either glutamate or glutamine and/or $NH_4Cl$, with or without MS or tianeptine. Media free of cells after 1 h were collected centrifuged and kept in -20°C until analysis.

*Determination of ammonium ion concentration:* the ammonium ion concentration was determined using Nessler's quantitative method (Zhloba et al., 1968). Supernatants collected from treated cell cultures were mixed with NaOH, KI and HgCl. The amount of ammonium ion was reflected by the amount of $NH_2Hg_2I_3$ formed. The samples were centrifuged and the supernatants were carefully removed. The pellets were collected, washed three times with mili Q water and left to dry at 100°C overnight before weighting them on a high precision balance.

*Determination of free amine concentration:* free amine concentrations were determined using Kaiser's quantitative ninhydrin technique (Sarin et al., 1981). The culture mediums were mixed with 3 different solutions i.e. phenol/ethanol, KSCN/pyridine and ninhidrin/ethanol and heated at 100°C during 7 min. These mixtures were read at 570 nm. The amount of free amine for each sample was calculated according to the following formula:

$$\text{Free amines (mol/l)} = \frac{\text{(absorbance of sample - absorbance of blank) x dilution x } 10^6}{\text{Extinction coefficient x sample volume in ml}}$$

where extinction coefficient is 1.5 x $10^4$ in $m^{-1}$ $cm^{-1}$

*3.2.2 Effects on apoptosis*

C6 glioblastoma cells were sealed to 6 wells culture plates at $10^5$ cells per well in presence of DMEM (Dulbecco's modified Egle's medium) and 10% fetal calf serum (FCS), and incubated 24 h at 37°C with 5% $CO_2$ before addition of glutamate and/or $NH_4Cl$, with or without MS or tianeptine. The plates were incubated either for 24 h or 72 h at 37°C and 5% $CO_2$. Apoptosis was evaluated using a fluocytometric technique. Briefly, medium from each well was first collected. The cells were washed twice with

HBSS. After a brief incubation with child trypsin / EDTA (1 min), they were collected and centrifuged at 250 g for 10 min. They were then incubated with FITC conjugated annexin V for 15 min in complete obscurity. Just before counting the cells, propidium iodide was added to each sample. The (total) apoptosis was the sum of early and late apoptosis (FACScalibur 4 colors, BD Bioscience).

3.3 Effects of GF, MS and tianeptine in conditions of high glucocorticoid exposure

Wistar rats were administered during 21 days once a day with corticosterone (35 mg/kg) s.c. plus GF (0.1, 3 and 15 mg/kg), MS (0.5, 15 and 45 mg/kg), tianeptine (0.5, 15 and 45 mg/kg) or vehicle (PCB) i.p., or with both corticosterone's vehicle and treatment's vehicle (Control).

*3.3.1 Behavioral explorations*

Rat's global activity was explored through an open field task after 15 days of administration of corticosterone plus GF, MS, tianeptine or vehicle (PCB), or of both corticosterone's vehicle and treatment's vehicle (Control). One hour before the experiment, the animal was administered its treatment and placed into the experimental room. At the time of the experiment, the animal was gently placed in the center of the arena (78 cm x 78 cm x 49 cm) and left free to explore it (10 min session). Its behavior was recorded with a numeric video camera. The video records were analyzed offline using the SMART automated tracking program (Panlab). Resting time was the sum of the time spend immobile; immobility was defined as no movement with a speed exceeding 2.5 cm/sec.

*A forced swimming task* was also done after 19 days of administration of corticosterone plus GF, MS, tianeptine or vehicle (PCB), or of both corticosterone's vehicle and treatment's vehicle (Control). One hour before the experiment, the animal was administered its treatment and placed into the experimental room. At the time of experiment, it was gently placed in an individual cylinder (50 cm x 20 cm in diameter) filled with water (25 ˚C) up to 30 cm. A rat was judged to be immobile when it floated and made only movements necessary to keep the head above the water. At the end of swimming session, the rat was removed from the cylinder, dried with towels, placed in a cage for 15 min rest and recovery, and then returned to its home cage.

*3.3.2 Golgi procedure to assess the surface of CA3 pyramidal neurons*

One hour after the last drug(s)/vehicle(s) administration on day 21, each rat was sacrificed and its brain was collected. The right hemisphere was fixed in 4% formaldehyde and the left hemisphere was frozen in liquid nitrogen. The latter was kept at -80˚C until use (see below 3.3.3). The right hemisphere fixed in formaldehyde was cut with a vibratome (100 μm slices) in a bath of 3% potassium dichromate and processed according to a modified version of the single-section Golgi impregnation procedure of Gabbott & Somogyi (Gabbott & Somogyi, 1984). The slices were incubated over a night in 3% potassium dichromate in distilled water, then rinsed in distilled water and mounted on plain slides. A coverslip was glued over the sections at the four corners. The slide assemblies were incubated in darkness over night in a 5% silver nitrate water solution. Two days later the slide assemblies were dismounted, and the tissue sections were rinsed in distilled water and mounted according to the standard procedures.

Golgi-impregnated neurons according to Magarinos and McEwen (Magarinos and McEwen, 1995) had to possess the following characteristics to be included in the analysis: a) location within the CA3c subregion of the dorsal hippocampus; b) dark and consistent impregnation throughout the extent of all the dendrites; c) relative isolation from neighboring impregnated cells, which could interfere with the analysis; and d) cell bodies in the middle third of the tissue section, to avoid analysis of impregnated neurons that extended well into other sections. For each brain 6-10 pyramidal cells at 400 x magnification were digitalized and traced. Each digitalized traced pyramidal neuron was extracted from the raw image and its surface area was measured using the Scion Image software. The slides were coded before analysis and code was broken only after the analysis was completed.

*3.3.3 Determination of GS activity in whole brain, liver and lungs*

Brain's left hemisphere, liver and lungs were collected immediately after euthanasia and frosted in liquid nitrogen. These organs were kept at -80˚C until use. On the day of the assay, they were homogenised in sample buffer HEPES (5 mM) with (EDTA) (1 mM). The pH was set to 7.4 with TRIS (100 mM). GS activity was assessed as mentioned above for purified sheep brain GS (see 2.1.1).

*3.3.4 Determination of the expression of GS and glucocorticoid receptor by ELISA*

Brain's left hemisphere homogenates from rats (prepared as above mentioned in 2.1.1) were tested for expression of GS and/or glucocorticoids receptors. After centrifugation at 10500 g for 20 min at 4˚C, the supernatant was collected and its protein concentration was determined using a BCA kit. Fifty μl of different dilutions (0.2 to 0.025) of each sample in coating buffer (sodium carbonate buffer; pH 9.6) were incubated on microtitre plates (Falcon, USA) over a night at 4˚C. After a single wash with TBS tween 0.1% (washing buffer), the plates were saturated with TBS tween 0.1% supplemented with 3% low fat graded dried milk (Biorad) for 1 h at 37˚C. Either anti-GS antibodies raised in goat or goat anti-rabbit anti-glucocorticoids receptor antibodies (1/1000 dilution) were allowed to react for 1 h at 37˚C. After 3 washes with washing buffer, the plates were either incubated with peroxidase conjugated anti-goat or goat anti rabbit antibodies for 1 h at 37˚C, and washed 3 times with washing buffer and 4 times with just TBS. The bound antibodies were revealed with TMB (trimethyl benzidine) and $H_2O_2$ as substrates. The reaction was stopped after an incubation of 15 min at 37˚C with HCl 1 M. The absorbance was read at 405 nm on a microtitre plate reader. The result was expressed in absorbance per μg of protein.

## MAIN RESULTS

### 1 Screening of tianeptine's target protein

1.1 Biotinylation of tianeptine (Figure 1)

The biotinylation of tianeptine required a 3 days reaction period with regular addition of *N*-(3-Dimethylaminopropyl)-*N*'-ethylcarbodiimide (EDC). Biotin conjugated tianeptine was separated from unconjugated ones by using a phase inverse C18 column; conjugated tianeptine was eluted at 60 % acetonitrile (retention time 11.7 min) (Figure 1A). The yield of biotinylation was estimated 76 %. The mass of biotinylated tianeptine was checked by mass spectrometry (Figure 1B).

1.2 Isolation and identification of tianeptine's target protein (Figures 2-3)

Western blot on mouse hippocampus membrane proteins in absence of n-octyl glucoside revealed a bond at 45 kDa (Figure 2). In the presence of n-octyl glucoside the bond vanished (Figure 2), which indicated this 45 kDa protein revealed by peroxydase conjugated streptavidin was probably a cytoplasmic rather than membrane anchored protein.

The same membrane preparation with 2D gel electrophoresis followed by western blotting revealed two neighboring spots at 42.146 kDa with an isoelectric point at pH 6.5 (Figure 3 A and B). Mass spectrometry confirmed the former and the sequence obtained from the digested spots matched (with scores 1.897 x $10^6$ and 6,6 x $10^5$ respectively for mouse glutamine synthetase. The interaction between biotinylated tianeptine and GS, based on the latter analyses, appeared to be at the enzyme's catalytic site with peptide sequence ITGTNAEVMPAQWEFQIGPCEGIR (for both spots).

1.3 In silico study of the interaction between tianeptine and bovine brain GS (Figure 4)

In silico study of the interaction between tianeptine and bovine brain GS was almost identical with the one between glufosinate and the same GS (Gill and Eisenberg, 2001).The three conformations with the lowest potential energy, which were chosen for the final minimalization by conjugated gradient using a distance dependent dielectric constant, resulted in the same complex after minimalization.

### 2 Charaterization of GS ligands pharmacological effects

2.1 In vitro effects of GS ligands

*2.1.1 <u>Effects of GF, iproniazid, MS and tianeptine on purified sheep brain GS activity (Figures 5, 6, 7,</u>* <u>9)</u>

In presence of 2β-ME, GF, iproniazid, MS and tianeptine could inhibit GS activity at "high" concentrations (Figure 5-7). GF, MS and tianeptine are competitive with L-glutamine (Figure 6; Meister 1985; Gill and Eisenberg, 2001), while iproniazid competes with hydroxylamine on the $NH_4$ site (Rueppel et al., 1972).

In absence of 2β-ME, GF and tianeptine could inhibit GS activity at "high" concentrations but also activate GS activity at "low concentrations" ($10^{-4}$ to $10^{-11}$ and $10^{-13}$ to $10^{-16}$ M, respectively) (Figure 7).

*2.1.2 <u>Effects of dialysis on the effect of tianeptine on purified sheep brain GS activity (Figures 8-9)</u>*

The interaction between tianeptine and GS was reversible as it is illustrated in figures 8 and 9 where decreased GS activity due to 1 and 5 nM tianeptine is reversed after over night dialysis against the reaction buffer. GF and MS are known to bind GS irreversibly (Meister 1985).

*2.1.3 <u>Effects of L-glutamate on the effect of tianeptine (Figure 10-11)</u>*

The effect of increasing concentration of L-glutamate (0.0016-10 mM) were assessed on purified sheep brain GS in presence of 5 μM tianeptine (Figure 10). The inhibitory effect of tianeptine on GS activity decreased as the concentration of L-glutamate increased. Higher concentration than 10 mM of L-glutamate (100 and 500 mM) increased background activity so no specific activity could be measured.

The inhibitory effect of tianeptine on extracellular free amine induced by glutamate after 1 h incubation in C6 cells in serum free HBSS decreased also as the concentration of L-glutamate increased (Figure 11). At the highest concentration (500 μM), tianeptine (100 nM) was even observed to increase extracellular free amine induced by glutamate. Tianeptine (100 nM) and/or N-methyl-D-aspartate (NMDA) (1, 10, 100, 500 μM) had no effect on extracellular

free amine after 1 h incubation in C6 cells in serum free HBSS.

*2.1.4 Effects of pamidronate and risedronate on rat brain GS activity (Figure 12-13)*

In presence of 2β-ME, pamidronate and risedronate could inhibit GS activity at "high" concentrations (Figure 12A and 13A). In absence of 2β-ME, pamidronate and risedronate could inhibit GS activity at "high" concentrations but also activate GS activity at "low concentrations" ($10^{-12}$ to $10^{-17}$ M) (Figures 12B and 13B)

2.2 Study of the effects of GS ligands on GS activity and expression in vivo

*2.2.1 Effects of GS ligands on whole brain GS activity and expression*

*2.2.1.1 Effects of MS and tianeptine on whole brain GS activity and expression 24h after a single i.p. administration in BALB/c mice (Figure 14)*

The effects of different doses of MS (0.078, 0.312, 1.25, 5, 20 mg/kg) and tianeptine (0.078, 0.312, 1.25, 5, 20 mg/kg) were assessed. Although there was no direct correlation between GS activity and GS expression, the effects of MS and tianeptine on both GS activity and GS expression were dose-dependent. MS and tianeptine could increase GS activity, the highest activity being observed at 0.312 mg/kg for MS and 1.25 mg/kg for tianeptine. The differences between doses as regard to GS expression were less pronounced for both molecules.

*2.2.1.2 Effects of GF, MS and tianeptine on whole brain GS activity and expression after a 7 days repeated i.p. administration in BALB/c mice (Figure 15)*

The effects of different doses of GF (0.1, 3, 15 mg/kg), MS (0.5, 15, 45 mg/kg) and tianeptine (0.5, 15, 45 mg/kg) were assessed. Although there were no direct correlation between GS activity and GS expression, the effects of GF, MS and tianeptine were dose-dependent on both GS activity and GS expression. GF and tianeptine could increase GS activity at the 3 mg/kg and 15 mg/kg doses, respectively. At these doses GF and tianeptine increased also GS expression in the brain. At higher doses, GF and tianeptine decreased GS activity, as this was also the case for the three doses of MS, which decreased GS activity proportionally the dose.

*2.2.2 Effects of GF and tianeptine on cortex, hippocampus and thalamus /hypothalamus GS activity after a single administration in ICR mice (Figure 16)*

The effects of different doses of GF (0.1, 3 and 15 mg/kg) and tianeptine (0.5, 15, 45 mg/kg) were assessed. Twenty four hours after administration, GF and tianeptine had no significant effect on GS activity in the cortex, while they dose-dependently increased GS activity in the hippocampus and decreased GS activity in the thalamus/hypothalamus.

**3 Evaluation of the therapeutic potential of GS ligands**

3.1 Effect of tianeptine on rat brain GS in condition of oxidative stress (Figure 17)

GS activity in presence of APS (1.5%) decreased about 35 %. Tianeptine at concentrations between $10^{-6}$ and $10^{-11}$ M protected GS activity from APS. It was even able to increase GS activity from concentrations $10^{-8}$ to $10^{-10}$ M.

3.2 Effects of MS and tianeptine on C6 cells in condition of excess of ammonia and/or glutamate

*3.2.1 Effects on extracellular ammonium ion and free amine (Figures 11, 18-24)*

Different concentrations of MS (0.06, 0.12, 0.25 and 0.5 μM) and tianeptine (0.05, 0.5, 5 and 100 nM) were tested in cultures of C6 glioblastoma cells in serum free HBSS supplemented with either glutamate (50 mM) and/or $NH_4Cl$ (10 mM).

No extracellular $NH_4^+$ could be detected after addition of L-glutamate (50 mM). On the contrary, addition of $NH_4Cl$ (10 mM) clearly increased the extracellular concentration of $NH_4^+$; and MS and tianeptine at low concentrations (0.06 nM and 0.05 nM, respectively) could blunt this increase (Figure 18). The latter was emphasized when culture medium was supplemented both with L-glutamate (50mM) and NH4Cl (10mM) (Figure 19).

The production of free amine increased in cultures of C6 glioblastoma cells in serum free HBSS supplemented

with glutamate (Figure 11, 20). In comparison, the extracellular concentrations of free amine in cultures supplemented with $NH_4Cl$ (10 mM) were only slightly increased; and those in the cultures supplemented both with glutamate (50 mM) and $NH_4Cl$ (10 mM) were intermediate. MS and tianeptine could blunt these increases (Figure 20-22). The nature of the dose-effect relationship appeared to be condition-dependent. MS alone was observed to interact with the Kaiser's method (Figure 23). Thus, it was difficult to conclude about the "biphasic" dose-effect relationship which was observed with MS. For tianeptine, the blunting effect was clearly proportional to the dose.

Glutamine (2 mM) did not alter the effects MS (5 mM) and tianeptine (100 nM) on extracellular ammonium ion and free amine concentrations induced by $NH_4Cl$ (10 mM) after 1 h in C6 cells in serum free HBSS (Figure 24).

*3.2.2 Effect of MS and tianeptine on apoptosis in C6 cells (Figures 25-27)*

Total apoptosis (early apoptosis and necrosis) induced by hyperammonia ($NH_4Cl$ 10 mM) was assessed after 72 h in C6 glioblastoma cells cultured in DMEM supplemented with 10% fetal calf serum (FCS). Both MS and tianeptine decreased total apoptosis at low concentrations (0.125 $\mu$M and 0.25 nM respectively) (Figure 25).

Total apoptosis induced by L-glutamate (Glu 50 mM) was assessed after 24 h in C6 glioblastoma cells cultured in DMEM supplemented with 10% FCS. Both MS and tianeptine decreased total apoptosis at low concentrations (0.125 $\mu$M and 0.25 nM respectively) (Figure 26).

Total apoptosis induced by the association hyperammonia (NH4Cl 10 mM) plus glutamate (Glu 50 mM) was assessed after 24 h in C6 glioblastoma cells cultured in DMEM supplemented with 10% FCS. Both MS and tianeptine decreased total apoptosis at low concentrations (0.25 $\mu$M and 0.25 nM respectively) (Figure 27).

3.3 Effects of GF, MS and tianeptine in conditions of high glucocorticoid exposure

Wistar rats were administered during 21 days once a day with corticosterone (35 mg/kg) s.c. plus GF (0.1, 3 and 15 mg/kg), MS (0.5, 15 and 45 mg/kg), tianeptine (0.5, 15 and 45 mg/kg) or vehicle (PCB) i.p., or with both corticosterone's vehicle (s.c.) and treatment's vehicle (i.p.) (Control).

*3.3.1 Behavioral explorations (Figures 28-29)*

Behavior was assessed in an open field task at day 15 (Figure 28) and in a forced swimming task at day 19 (Figure 29).

Resting time in the open field task was observed to be higher in the rats administered with corticosterone plus GF, MS or tianeptine compared to the rats administered with corticosterone and vehicle (PCB) and those administered with both corticosterone's vehicle and treatment's vehicle (Control), whose resting time remained comparable. This increase in resting time was only proportional to the dose for MS.

Immobility in the forced swimming test was observed to be comparable or lower in the rats administered with corticosterone plus GF, MS or tianeptine compared to the rats administered with corticosterone and vehicle (PCB) and the rats administered with both corticosterone's vehicle and treatment's vehicle (Control), whose duration of immobility remained comparable. A clear decrease in immobility was observed in the animals administered with 0.1 mg/kg of GF and 0.5 mg/kg of tianeptine.

3.3.2 *Morphology of CA3 pyramidal neurons (Figure 30)*

Rats administered during 21 days with corticosterone plus GF, MS or tianeptine showed morphological changes of pyramidal neurons in the hippocampus CA3 region compared to the rats administered with corticosterone and vehicle (PCB) and those administered with both corticosterone's vehicle and treatment's vehicle (Control) (Figure 30). GF at the doses 0.1 and 3 mg/kg could prevent in part the deleterious effects of corticosterone on CA3 pyramidal neurons. For MS and tianeptine, the best protective effects were observed at the 15 mg/kg dose, and 0.5 and 15 mg/kg doses, respectively (Figure 30). Only GF at the 15 mg/kg dose failed to blunt the effects of corticosterone.

*3.3.3 GS activity and expression in the whole brain (Figure 31)*

A dose-dependent decrease in brain GS activity was observed in the rats administered with corticosterone plus GF, MS or tianeptine compared to the rats administered with corticosterone and vehicle (PCB) and those administered with both corticosterone's vehicle and treatment's vehicle (Control); brain GS activities in the PCB and control groups were comparable (Figure 31A). GS activity in the rats treated with 0.5 mg/kg of tianeptine remained in the same range as observed in the rats from the PCB and Control groups. The lowest GS activities were observed with MS at the 15 and 45 mg/kg doses.

Brain GS expression was observed to be higher in the rats treated with corticosterone plus GF at the doses of 0.1 and 3 mg/kg, MS at the dose of 45 mg/kg or tianeptine at the dose of 15 mg/kg compared to the rats administered with corticosterone and vehicle (PCB) or, to a lesser extent, those administered with both corticosterone's vehicle and treatment's vehicle (Control) (Figure 31B). Brain GS expression in the rats administered with corticosterone and vehicle (PCB) was clearly lower compared to this in the rats administered with both corticosterone's vehicle and treatment's vehicle (Control).

*3.3.4 Determination of GS activity in liver and lungs (Figure 32)*

A clear decrease in liver GS activity was observed in the rats administered with corticosterone plus GF, MS or tianeptine compared to the rats administered with corticosterone and vehicle (PCB) and those administered with both corticosterone's vehicle and treatment's vehicle (Control); liver GS activities in the PCB and Control groups were comparable (Figure 32A). Only GS activity in the rats treated with 0.5 mg/kg tianeptine remained in the same range as observed in the rats from the PCB and Control groups.

Lung GS activity in the rats administered with corticosterone and vehicle (PCB) was increased compared to the activity in the rats administered with both corticosterone's vehicle and treatment's vehicle (Control). A decrease in lung GS activity was observed in most rats administered with corticosterone plus GF, MS or tianeptine compared to the rats administered with corticosterone and vehicle (PCB) (Figure 32B). Only GS activity in the rats treated with 3 mg/kg GF and 15 mg/kg tianeptine remained in the same range as observed in the rats from the PCB group.

*3.3.5 Glucocorticoid receptor (GR) expression in the whole brain and adrenal weight (Figure 33)*

Brain GR expression was observed to be higher in the rats treated with corticosterone plus GF at the doses of 0.1 and 3 mg/kg, MS at the doses of 0.5, 15 and 45 mg/kg or tianeptine at the dose of 0.5 mg/kg compared to the rats administered with corticosterone and vehicle (PCB) (Figure 33A). This increase in GR expression appeared to be proportional to the dose for MS and inversely proportional to the dose for tianeptine.

Repeated administration of corticosterone decreased the adrenal weight. Adrenal weight was observed to be higher in the rats treated with corticosterone plus GF, MS or tianeptine compared to the rats administered with corticosterone and vehicle (PCB) (Figure 33B); however, it remained lower compared to the rats administered with corticosterone's vehicle and treatment's vehicle (Control). Notably the dose-effect patterns were similar to those observed for brain GR expression (Figure 33A).

Table 1: Conditions and disorders which (can) result in a systemic ammonia concentration increase (hyperammonemia), reported according to the MedDRA classification (MedDRA Version 7.1). For certain system organ classes (SOC), the included high level group terms (HLGT) of particular interest are indicated; for certain HLGT, the included high level terms (HLT) of particular interest are indicated; for certain HLT, the included preferred terms (PT) of particular interest are indicated; for certain PT, the included low level terms (LLT) of particular interest are indicated. Certain conditions and disorders could not be reported according to the MedDRA classification and are reported according their usual denomination used in the literature.

- ➢ Hepatobiliary disorders (SOC)
  In particular
  - o Hepatic and hepatobiliary disorders (HLGT)
    In particular
    - ■ Cholestasis and jaundice (HLT)
      In particular Hepatitis cholestatic (PT)
    - ■ Hepatic and hepatobiliary disorders NEC (HLT)
      In particular Cerebrohepatorenal syndrome (PT), Complications of transplanted liver (PT), Hepatic infection (PT), Hepatic lesion (PT), Hepatic trauma (PT), Liver and pancreas transplant rejection (PT), Liver disorder (PT), Liver transplant rejection (PT), Polycystic liver disease (PT)
    - ■ Hepatic enzymes and function abnormalities (HLT)
    - ■ Hepatic failure and associated disorders (HLT)
      In particular

- Hepatic failure (PT)
  In particular Acute hepatic failure (LLT), Acute liver failure (LLT), Chronic hepatic failure (LLT), End stage liver disease (LLT), Failure liver (LLT), Fulminant hepatic failure (LLT), Hepatic failure (LLT), Hepatic insufficiency (LLT), Hepatobiliary insufficiency (LLT), Subacute hepatic failure (LLT)
- Hepatorenal failure (PT)
- Hepatorenal syndrome (PT)

■ Hepatic fibrosis and cirrhosis (HLT)
Biliary cirrhosis (PT), Biliary cirrhosis primary (PT), Biliary fibrosis (PT), Cardiac cirrhosis (PT), Cirrhosis alcoholic (PT), Congenital hepatic fibrosis (PT), Cryptogenic cirrhosis (PT), Hepatic cirrhosis (PT), Hepatic fibrosis (PT), Lupoid hepatic cirrhosis (PT)
■ Hepatic infections (excl viral) (HLT)
■ Hepatic metabolic disorders (HLT)
In particular Alpha-1 anti-trypsin deficiency (PT), Haemochromatosis (PT), Hepatic siderosis (PT), Hepatolenticular degeneration (PT), Hereditary haemochromatosis (PT)
■ Hepatic vascular disorders (HLT)
In particular Budd-Chiari syndrome (PT), Portal vein occlusion (PT), Portal vein phlebitis (PT), Portal vein stenosis (PT)
■ Hepatic viral infections (HLT)
In particular Hepatitis B (PT), Hepatitis C (PT), Hepatitis D (PT), Hepatitis non-A nonB non-C (PT)
■ Hepatocellular damage and hepatitis NEC (HLT)
In particular Alcoholic liver disease (PT), Chronic hepatitis (PT), Hepatic necrosis (PT), Hepatitis alcoholic (PT), Hepatitis chronic active (PT), Hepatitis chronic persistent (PT), Hepatitis fulminant (PT), Hepatitis toxic (PT), Peliosis hepatitis (PT), Reye's syndrome (PT)

o Hepatobiliary neoplasms (HLGT)

➢ Metabolic and nutritional disorders congenital (HLGT)

o Urea cycle enzyme disorders
In particular Arginase deficiency (PT) (argininaemia), Argininosuccinate synthetase deficiency (PT) (Citrullinaemia), Argininosuccinate lyase deficiency (PT) (Argininosuccinic aciduria), Carbamoyl phosphate synthetase deficiency (PT), N-acetylglutamate synthetase deficiency, Ornithine transcarbamoylase deficiency (PT)
o Transport defects of intermediates in the urea cycle
In particular Lysinuric protein intolerance (PT), Hyperammonaemia-hyperomithinaemia-homocitrullinuria syndrome
o Organic acidurias
Methylmalonic aciduria (PT) and other organic aciduria, Propionic aciduria
o Lipid metabolism disorders
Medium-chain acetyl-coenzyme A dehydrogenase deficiency (PT); (Congenital) carnitine deficiency (PT), more particularly because of mutation of the gene encoding carnitine transporter; Long chain fatty acid oxidation defects and other related disorders
o Other inborn errors
Pyruvate carboxylase deficiency; Ornithine aminotransferase deficiency

➢ Other metabolic causes

o (Distal) Renal tubular acidosis (PT); Hyperinsulinaemic hypoglycaemia

➢ Porto-systemic shunts
In particular due to Hepatobiliary disorders (SOC) (see above); Congenital absence of the portal vein; Surgical
➢ Blood and lymphatic system disorders (SOC)
In particular

o Haematopoietic neoplasms (excl leukaemias and lymphomas) (HLGT)
o Leukaemias (HLGT)
In particular Leukaemias acute myeloid (HLT); Leukemia chronic myeloid (HLT)
o Plasma cell neoplasms (HLGT), in particular multiple myelomas (HLT)

o Allogenic bone marrow transplantation therapy (PT)
o Allogenic peripheral blood progenitor cell transplantation
o Drugs related hyperammonaemia
In particular Intensive chemotherapy, more particularly with 5-fluorouracil and asparaginase; Anaesthetic agents, more particularly halothane and enflurane; Sodium valproate; Primidone

➢ Infections and infestations (SOC)
In particular with Herpes simplex in newborn (systemic infection); Urea splitting organisms; Urease positive bacteria
➢ Renal and urinary disorders (SOC)
In particular Subureteric injection for vesicoureteric reflux; Urinary diversion, more particularly with ureterosigmoidostomy and ileal conduit; Urinary tract infections, more particularly with urease positive bacteria
➢ Other causes
Muscular disorder e.g. rhabdomyolysis due to carnitine palmitoyltransferase deficiency; Parenteral nutrition, in particular arginine deficient total parenteral nutrition; Solid organ transplantation; Transient hyperammonaemia of the newborn; Upper gastrointestinal bleeding; Critical systemic illnesses and injuries; Idiopathic; Etc.

Abbreviations: HLGT: high level group term; HLT: high level term; LLT: low level term; NEC: not elsewhere classified; PT: preferred term; SOC: system organ class.

Table 2: Conditions and disorders which can benefit directly or indirectly from medications activating, modulating/regulating or inhibiting GS activity, reported according to the MedDRA classification (MedDRA Version 7.1). For certain system organ classes (SOC), the included high level group terms (HLGT) of particular interest are indicated; for certain HLGT, the included high level terms (HLT) of particular interest are indicated; for certain HLT, the included preferred terms (PT) of particular interest are indicated; for certain PT, the included low level terms (LLT) of particular interest are indicated. Those conditions and disorders which are "double/**underlined**" may in particular be related to an excess of ammonia. Those conditions and disorders marked in "***italic***" may in particular be related to an excess of glutamate. Those conditions and disorders marked in "bold/underlined" are already proposed clinical applications of tianeptine. Those conditions and disorders marked in "grey" are already claimed or proposed clinical applications of bisphophonates:

➢ **Blood and lymphatic system disorders (SOC)**
Anaemias nonhaemolytic and marrow depression (HLGT); Bleeding tendencies and purpuras (excl thrombocytopenic) (HLGT); Coagulopathies and bleeding diatheses (HLGT); Haematological disorders NEC (HLGT); Haematopoietic neoplasms (excl leukaemias and lymphomas) (HLGT); Haemoglobinopathies (HLGT); Haemolyses and related conditions (HLGT); Leukaemias (HLGT); Lymphomas Hodgkin's disease (HLGT); Lymphomas NEC (HLGT); Lymphomas non-Hodgkin's B-cell (HLGT); Lymphomas non-Hodgkin's T-cell (HLGT); Lymphomas non-Hodgkin's unspecified histology (HLGT); Plasma cell neoplasms (HLGT) (in particular multiple myeloma); ***Platelet disorders (HLGT)***; Red blood cell disorders (HLGT); Spleen, lymphatic and reticuloendothelial system disorders (HLGT); White blood cell disorders (HLGT)
➢ **Cardiac disorders (SOC)**

*o Cardiac arrhythmias (HLGT)*
*Cardiac conduction disorders (HLT); Rate and rhythm disorders NEC (HLT); Supraventricular arrhythmias (HLT); Ventricular arrhythmias and cardiac arrest (HLT)*
o Cardiac disorder signs and symptoms (HLGT)
Cardiac disorders NEC (HLT); Cardiac hypertensive complications (HLT); Cardiac infections and inflammations NEC (HLT); Dyspnoeas (HLT)
o Cardiac neoplasms (HLGT)
o Cardiac valve disorders (HLGT)
o Congenital cardiac disorders (HLGT)
o Coronary artery disorders (HLGT)
Coronary artery disorders NEC (HLT); Ischaemic coronary artery disorders (HLT)
o Endocardial disorders (HLGT)
Endocardial bacterial infections (HLT); Endocardial disorders NEC (HLT); Endocardial fungal infections (HLT); Endocardial viral infections (HLT); Endocarditis NEC (HLT)
o Heart failures (HLGT)
Heart failure signs and symptoms (HLT); Heart failures NEC (HLT); Left ventricular failures (HLT); Right ventricular failures (HLT)

o Myocardial disorders (HLGT)

Cardiomyopathies (HLT), in particular Diabetic cardiomyopathy (PT) and Ischaemic cardiomyopathy (PT); Infectious myocarditis (HLT); Myocardial disorders NEC (HLT); Noninfectious myocarditis (HLT)

o Pericardial disorders (HLGT)

➢ **Congenital, familial and genetic disorders (SOC)**

In particular Metabolic and nutritional disorders congenital (HLGT); Neurological disorders congenital (HLGT)

➢ **Ear and labyrinth disorders (SOC)**

In particular Aural disorders NEC (HLGT); Congenital ear disorders (excl deafness) (HLGT); External ear disorders (HLGT); Hearing disorders (HLGT); Inner ear and VIIIth cranial nerve disorders (HLGT); Middle ear disorders (HLGT)

➢ **Endocrine disorders (SOC)**

o Adrenal gland disorders (HLGT)

In particular Adrenal cortical hyperfunctions (HLT), more particularly Hypercorticoidism (PT); Adrenal cortical hypofunctions (HLT); Adrenal gland disorders NEC (HLT); Adrenal medulla disorders (HLT); Adrenal neoplasms (HLT)

o Diabetic complications (HLGT)

Diabetic complications cardiovascular (HLT); Diabetic complications dermal (HLT); Diabetic complications gastrointestinal (HLT); Diabetic complications NEC (HLT); Diabetic complications neurological (HLT); Diabetic complications ophthalmic (HLT); Diabetic complications renal (HLT)

o Endocrine and glandular disorders (HLGT)

Endocrine disorders NEC (HLT), in particularl Endocrine pancreatic disorder (PT); Polyglandular endocrine disorders (HLT)

o Endocrine disorders of gonadal function (HLGT)

o Glucose metabolism disorders (incl diabetes mellitus) (HLGT)

Diabetes mellitus (incl subtypes) (HLT); Hyperglycaemic conditions NEC (HLT); Hypoglycaemic conditions NEC (HLT)

o Hypothalamus and pituitary gland disorders (HLGT)

o Neoplastic and ectopic endocrinopathies (HLGT)

o Parathyroid gland disorders (HLGT)

o Thyroid gland disorders (HLGT)

Acute and chronic thyroiditis (HLT); Thyroid disorders NEC (HLT); Thyroid hyperfunction disorders (HLT); Thyroid hypofunction disorders (HLT); Thyroid neoplasms (HLT)

➢ Eye disorders (SOC)

In particular Anterior eye structural change, deposit and degeneration (HLGT); Eye disorders NEC (HLGT); Glaucoma and ocular hypertension (HLGT); Ocular haemorrhages and vascular disorders (HLGT); Ocular infections, irritations and inflammations (HLGT); Ocular injuries (HLGT); Ocular neoplasms (HLGT); Ocular neuromuscular disorders (HLGT); Ocular sensory symptoms (HLGT); Ocular structural change, deposit and degeneration (HLGT); Retina, choroid and vitreous haemorrhages and vascular disorders (HLGT); Vision disorders (HLGT)

➢ **Gastrointestinal disorders (SOC)**

In particular

o Anal and rectal conditions (HLGT)

In particular **Anal and rectal pains (HLT);** Anal and rectal signs and symptoms (HLT)

o Benign neoplasms gastrointestinal (HLGT)

o Dental and gingival conditions (HLGT)

In particular Dental and periodontal infections and inflammations (HLT); **Dental pain and sensation disorders (HLT); Gingival pains (HLT)**

o Diverticular disorders (HLGT)

o Exocrine pancreas conditions (HLGT),

o In particular Acute and chronic pancreatitis (HLT)

o Gastrointestinal conditions NEC (HLGT)

In particular Gastrointestinal disorders NEC (HLT); **Neurogenic bowel (HLT); Intestinal functional disorder (HLT);** Gastrointestinal mucosal dystrophies and secretion disorders (HLT)

o Gastrointestinal haemorrhages NEC (HLGT)

Gastric and oesophageal haemorrhages (HLT); Intestinal haemorrhages (HLT); Non-site specific gastrointestinal haemorrhages (HLT)
o Gastrointestinal infections (HLGT)
o Gastrointestinal inflammatory conditions (HLGT)
In particular Gastrointestinal inflammatory disorders NEC (HLT), more particularly **Inflammatory bowel disease (PT)**
o Gastrointestinal motility and defaecation conditions (HLGT)
In particular Gatrointestinal spastic and hypermotility disorders (HLT), more particularly **Frequent bowel movements (PT)** and **Irritable bowel syndrome (PT);** Gastrointestinal dyskinetic disorders (HLT); Gastrointestinal atonic and hypomotility disorders NEC (HLT), more particularly **Infrequent bowel movements (PT);** Non-mechanical ileus (HLT)
o Gastrointestinal signs and symptoms (HLGT)
In particular Dyspeptic signs and symptoms (HLT), more particularly **Dyspepsia (PT);** Flatulence, bloating and distension (HLT); **Gastrointestinal and abdominal pains (excl oral and throat) (HLT);** Gastrointestinal signs and symptoms NEC (HLT), more particularly **Bowel movement irregularity (PT)**
o Gastrointestinal stenosis and obstruction (HLGT)
o Gastrointestinal ulceration and perforation (HLGT)
o Gastrointestinal vascular conditions (HLGT)
o Malabsorption conditions (HLGT)
o Malignant and unspecified neoplasms gastrointestinal NEC (HLGT)
o Oral soft tissue conditions (HLGT)
In particular **Oral soft tissue pain and paraesthesia (HLT)**
o Peritoneal and retroperitoneal conditions (HLGT)
o Salivary gland conditions (HLGT)
o Tongue conditions (HLGT)

➢ General disorders and administration site conditions (SOC)
In particular

o Administration site reactions (HLGT)
o General system disorders NEC (HLGT)
In particular Asthenic conditions (HLT); Inflammations (HLT); Mucosal findings abnormal (HLT); **Pain and discomfort NEC (HLT)**
o Tissue disorders NEC (HLGT)
In particular Necrosis NEC (HLT), more particularly Cell death (PT) and Wound necrosis (PT); Trophic disorders (HLT), more particularly Atrophy (PT) and Denervation atrophy (PT)

➢ **Hepatobiliary disorders (SOC)**
In particular

o Bile duct disorders (HLGT)
In particular Bile duct infections and inflammations (HLT)
o Gallbladder disorders (HLGT)
o Hepatic and hepatobiliary disorders (HLGT) (in particular see Table 1)
o Hepatobiliary neoplasms (HLGT)

➢ **Immune system disorders (SOC)**

o Allergic conditions (HLGT)
In particular Allergic conditions NEC (HLT), more particularly Allergic bronchitis (PT), **Allergic cough (PT)** and **Asthma (PT);** Allergies to foods, food additives, drugs and other chemicals (HLT); Atopic disorders (HLT)
o Autoimmune disorders (HLGT)
In particular Autoimmune disorders NEC (HLT), more particularly *Reiter's syndrome (PT)*; Hepatic autoimmune disorders (HLT); *Lupus erythematosus and associated conditions (HLT);* Muscular autoimmune disorders (HLT); *Rheumatoid arthritis and associated conditions (HLT);* Scleroderma and associated disorders (HLT)
o Immune disorders NEC (HLGT)
In particular Acute and chronic sarcoidosis (HLT); Immune and associated conditions NEC (HLT), more particularly Chronic inflammatory demyelinating polyradiculoneuropathy (PT), Colitis ulcerative (PT) and Crohn's disease (PT); Transplant rejections (HLT); Vasculitides (HLT)

o Immunodeficiency syndromes (HLGT)
o In particular Acquired immunodeficiency syndromes (HLT)

➢ **Infections and infestations (SOC)**
In particular

o Ancillary infectious topics (HLGT)
In particular Inflammatory disorders following infection (HLT), more particularly *Reiter's syndrome (PT)* and Reye's syndrome (PT)
o Bacterial infectious disorders (HLGT)
o Chlamydial infectious disorders (HLGT)
o Ectoparasitic disorders (HLGT)
o Fungal infectious disorders (HLGT)
o Helminthic disorders (HLGT)
o Infections - pathogen class unspecified (HLGT),
In particular Central nervous system and spinal infections (HLT)
o Mycobacterial infectious disorders (HLGT)
o In particular Tuberculous infections (HLT)
o Mycoplasmal infectious disorders (HLGT)
o Protozoal infectious disorders (HLGT)
o Rickettsial infectious disorders (HLGT)
o Viral infectious disorders (HLGT)

➢ **Injury, poisoning and procedural complications (SOC)**
o Administration site reactions (HLGT)
*o Bone and joint injuries (HLGT)*
o Chemical injury, overdose and poisoning (HLGT)
o Injuries by physical agents (HLGT)
o Injuries NEC (HLGT),
In particular Cerebral injuries NEC (HLT); Non-site specific injuries NEC (HLT); Post-traumatic osteoporosis (HLT)
o Procedural and device related injuries and complications NEC (HLGT), I
In particular Anaesthetic complications (HLT); Neurological and psychiatric procedural complications (HLT)

➢ **Metabolism and nutrition disorders (SOC)**
In particular

o Acid-base disorders (HLGT)
o Appetite and general nutritional disorders (HLGT)
o Bone, calcium, magnesium and phosphorus metabolism disorders (HLGT)
In particular Bone metabolism disorders (HLT), more particularly *Osteoporosis (PT), Osteoporosis circumscripta cranii (PT), Osteoporosis postmenopausal (PT)* and *Senile osteoporosis (PT)*
o Diabetic complications (HLGT)
o Glucose metabolism disorders (incl diabetes mellitus) (HLGT)
o Inborn errors of metabolism (HLGT)
In particular Inborn errors of amino acid metabolism (HLT) (more particularly see Table 1)
o Iron and trace metal metabolism disorders (HLGT)
o Metabolism disorders NEC (HLGT)
Metabolic disorders NEC (HLT), more particularly Catabolic state (PT), Cushing's syndrome (PT), Hypercatabolism (PT) and Hypercorticoidism (PT)
o Protein and amino acid metabolism disorders NEC (HLGT)
In particular Amino acid metabolism disorder NEC (HLT), more particularly Disorders of urea cycle metabolism (PT); Protein metabolism disorders NEC (HLT), more particularly Hyperammonemia (PT)
o Purine and pyrimidine metabolism disorders (HLGT)

➢ **Musculoskeletal and connective tissue disorders (SOC)**

o Bone disorders (excl congenital and fractures) (HLGT)

In particular Bone and joint infections (excl arthritis) (HLT), more particularly Bone tuberculosis (PT) and Joint tuberculosis (PT); Bone disorders NEC (HLT), more particularly Bone erosion (PT), Osteolysis (PT) and *Post-traumatic osteoporosis (PT);* Bone related signs and symptoms (HLT), more particularly Bone pain (PT); Metabolic bone disorders (HLT), more particularly *Osteoporosis (PT), Oesteoporosis circumscripta cranii (PT), Osteoporosis postmenopausal (PT) and Senile osteoporosis (PT)*

o Connective tissue disorders (excl congenital) (HLGT)

Connective tissue disorders (excl LE) (HLT); Lupus erythematosus (incl subtypes) (HLT)

o Fractures (HLGT)

o Joint disorders (HLGT)

o Muscle disorders (HLGT)

In particular Muscle injuries (HLT); Muscle pains (HLT); Muscle related signs and symptoms NEC (HLT), more particularly Muscle fatigue (PT)

o Musculoskeletal and connective tissue disorders NEC (HLGT).

o Musculoskeletal and connective tissue neoplasms (benign/malignant) (HLGT)

o Synovial and bursal disorders (HLGT)

o Tendon, ligament and cartilage disorders (HLGT)

- **Neoplasms benign, malignant and unspecified (incl cysts and polyps) (SOC)**
- **Nervous system disorders (SOC) (see Table 3)**
- **Pregnancy, puerperium and perinatal conditions (SOC)**
- **Psychiatric disorders (SOC)**

**o Adjustment disorders (incl subtypes) (HLGT)**

**o Anxiety disorders and symptoms (HLGT)**

In particular **Anxiety disorders NEC (incl obsessive compulsive disorder) (HLT); Anxiety symptoms (HLT); Stress disorders (HLT)**

o Changes in physical activity (HLGT)

o **Cognitive and attention disorders and disturbances (HLGT)**

o Communication disorders and disturbances (HLGT)

o Deliria (incl confusion) (HLGT)

o **Dementia and amnestic conditions (HLGT)**

In particular **Alzheimer's disease (incl subtypes) (HLT); Amnestic symptoms (HLT); Dementia NEC (HLT); Vascular dementia disorders (HLT)**

o **Depressed mood disorders and disturbances (HLGT)**

o Developmental disorders NEC (HLGT)

Developmental motor skills disorders (HLT); Pervasive developmental disorders NEC (HLT)

o Dissociative disorders (HLGT)

o Disturbances in thinking and perception (HLGT)

o Eating disorders and disturbances (HLGT)

o Impulse control disorders NEC (HLGT)

o **Manic and bipolar mood disorders and disturbances (HLGT)**

o **Mood disorders and disturbances NEC (HLGT)**

**Affect alterations NEC (HLT); Emotional and mood disturbances NEC (HLT); Mood disorders due to a general medical condition (HLT); Mood disorders NEC (HLT)**

o Personality disorders and disturbances in behaviour (HLGT)

o Psychiatric and behavioural symptoms NEC (HLGT)

o Psychiatric disorders NEC (HLGT)

In particular Mental disorders due to a general medical condition NEC (HLT)

o *Schizophrenia and other psychotic disorders (HLGT)*

*Brief psychotic disorder (HLT); Delusional disorders (HLT); Psychotic disorder NEC (HLT); Schizoaffective and schizophreniform disorders (HLT); Schizophrenia NEC (HLT)*

o Sexual dysfunctions, disturbances and gender identity disorders (HLGT)

o Sleep disorders and disturbances (HLGT)

o Somatoform and factitious disorders (HLGT)

In particular Somatoform disorders (HLT), more particularly **Irritable bowel syndrome (PT)**

o Suicidal and self-injurious behaviours NEC (HLGT)

- **Renal and urinary disorders (SOC)**

In particular Bladder and bladder neck disorders (excl calcul) (HLGT); Genitourinary tract disorders NEC (HLGT); Nephropathies (HLGT); Renal disorders (excl nephropathies) (HLGT); Ureteric disorders (HLGT); Urethral disorders (excl calculi) (HLGT); Urinary tract signs and symptoms (HLGT); Urolithiases (HLGT)

➢ **Reproductive system and breast disorders (SOC)**
In particular

o Menopause and related conditions (HLGT)
In particular Menopausal effects NEC (HLT), more particularly Osteoporosis postmenopausal (PT)
o *Penile and scrotal disorders (excl infections and inflammations) (HLGT)*
o *Sexual function and fertility disorders (HLGT)*

➢ **Respiratory, thoracic and mediastinal disorders (SOC)**
In particular

o Bronchial disorders (excl neoplasms) (HLGT)
Bronchial conditions NEC (HLT), in particularl Allergic bronchitis (PT) and Bronchitis chronic (PT); Bronchospasm and obstruction (HLT), more particularly **Asthma (PT)**
o Lower respiratory tract disorders (excl obstruction and infection) (HLGT)
Lower respiratory tract inflammatory and immunologic conditions (HLT); Lower respiratory tract radiation disorders (HLT); Occupational parenchymal lung disorders (HLT); Parenchymal lung disorders NEC(HLT); Pulmonary oedemas (HLT)
o Neonatal respiratory disorders (HLGT)
Neonatal hypoxic conditions (HLT); Newborn respiratory disorders NEC (HLT)
o Pleural disorders (HLGT)
o Pulmonary vascular disorders (HLGT)
o Respiratory disorders NEC (HLGT)
In particular Conditions associated with abnormal gas exchange (HLT), more particularly Anoxia (PT), **Brain hypoxia (PT),** Hyperoxia (PT), Hypoxia (PT) and **Hypoxic encephalopathy (PT);** Coughing and associated symptoms (HLT), more particularly Allergic cough (PT) and **Cough (PT);** Lower respiratory tract signs and symptoms (HLT); Respiratory tract disorders NEC (HLT); Upper respiratory tract signs and symptoms (HLT)
o Respiratory tract infections (HLGT), in particular
In particular Bacterial lower respiratory tract infections (HLT), more particularly Pulmonary tuberculosis (PT)
o Respiratory tract neoplasms (HLGT)
o Thoracic disorders (excl lung and pleura) (HLGT)
o Upper respiratory tract disorders (excl infections) (HLGT)

➢ **Skin and subcutaneous tissue disorders (SOC)**
Angioedema and urticaria (HLGT); Cornification and dystrophic skin disorders (HLGT); Cutaneous neoplasms benign (HLGT); Epidermal and dermal conditions (HLGT); Pigmentation disorders (HLGT); Skin and subcutaneous tissue disorders NEC (HLGT); Skin and subcutaneous tissue infections and infestations (HLGT); Skin appendage conditions (HLGT); Skin neoplasms malignant and unspecified (HLGT); Skin vascular abnormalities (HLGT)

➢ **Surgical and medical procedures (SOC)**
Due to the quasi ubiquitous distribution of GS, of its important homeostatic roles and, sometimes, pathophysiological roles, medications activating, modulating/regulating or inhibiting GS activity might be theoretically associated with all surgical and/or medical procedures.
For instance with

o *Bone and joint therapeutic procedures (HLGT)*
o Therapeutic procedures and supportive care NEC (HLGT)
In particular Prophylactic procedures NEC (HLT), more particularly *Osteoporosis prophylaxis (PT)*

➢ **Vascular disorders including the high level terms (SOC)**
In particular

o Aneurysms and artery dissections (HLGT)
o Arteriosclerosis, stenosis, vascular insufficiency and necrosis (HLGT)

In particular Cerebrovascular and spinal necrosis and vascular insufficiency (HLT), more particularly **Hypoxic encephalopathy (PT)**, **Vascular dementia (PT)**, **Vascular encephalopathy (PT)**, **Ischaemic stroke (PT)**
o Decreased and nonspecific blood pressure disorders and shock (HLGT)
In particular Circulatory collapse and shock (HLT), more particularly **Heat stroke (PT);** Vascular hypotensive disorders (HLT)
o Embolism and thrombosis (HLGT)
In particular Cerebrovascular embolism and thrombosis (HLT), more particularly **Embolic stroke (PT)**, **Thromboembolic stroke (PT)** and Thrombotic stroke (PT)
o Lymphatic vessel disorders (HLGT)
o Vascular disorders NEC (HLGT)
In particular Cerebrovascular and spinal vascular disorders NEC (HLT), more particularly *Blood brain barrier defect (PT)*; Ocular vascular disorders NEC (HLT)
o Vascular haemorrhagic disorders (HLGT)
In particular Nervous system haemorrhagic disorders (HLT), more particularly **Haemorrhagic stroke (PT)**
o Vascular hypertensive disorders (HLGT)
o Vascular inflammations (HLGT)
o Vascular injuries (HLGT)
In particular Arterial inflammations (HLT) and Vasculitides NEC (HLT)
o Venous varices (HLGT)

Abbreviations: HLGT: high level group term; HLT: high level term; NEC: not elsewhere classified; PT: preferred term; SOC: system organ class

**Table 3:** Conditions and disorders of the nervous system which (can) benefit directly or indirectly from medications activating, modulating/regulating or inhibiting GS activity (reported according to the MedDRA classification; MedDRA Version 7.1). For certain system organ classes (SOC), the included high level group terms (HLGT) of particular interest are indicated; for certain HLGT, the included high level terms (HLT) of particular interest are indicated; for certain HLT, the included preferred terms (PT) of particular interest are indicated; for certain PT, the included low level terms (LLT) of particular interest are indicated. All these conditions and disorders are, more or less, related to or result in absolute or relative deficiency or excess of glutamate. Those conditions and disorders marked in "double/underlined" are related to or result in an excess of ammonia Those conditions and disorders marked in "**bold/underlined**" are already proposed clinical applications of tianeptine.

- **Nervous system disorders (SOC)**

o Central nervous system infections and inflammations (HLGT)
Central nervous system abscesses (HLT); Central nervous system inflammatory disorders NEC (HLT); Encephalitis NEC (HLT); Encephalitis nonviral infectious (HLT); Encephalitis of viral origin (HLT); Meningeal bacterial infections (HLT); Meningeal fungal infections (HLT); Meningeal viral infections (HLT); Meningitis NEC (HLT); Myelitis (incl infective) (HLT); Nervous system infections NEC (HLT)
o Central nervous system vascular disorders (HLGT)

■ Central nervous system aneurysms (HLT) ;
■ **Central nervous system haemorrhages and cerebrovascular accidents (HLT)**
**In particular Embolic stroke (PT), Haemorrhagic stroke (PT), Ischaemic stroke (PT), Thromboembolic stroke (PT) and Thrombotic stroke (PT)**
■ Central nervous system vascular disorders NEC (HLT)
In particular Bood brain barrier defect (PT)
■ Cerebrovascular venous and sinus thrombosis (HLT)
■ Transient cerebrovascular events (HLT)
■ **Traumatic central nervous system haemorrhages (HLT)**

o Congenital and peripartum neurological conditions (HLGT)
o Cranial nerve disorders (excl neoplasms) (HLGT)
o **Demyelinating disorders (HLGT)**
In particular **Multiple sclerosis acute and progressive (HLT)**

o Encephalopathies (HLGT)

- Encephalopathies NEC (HLT), in particular **AIDS encephalopathy (PT), Anoxic encephalopathy (PT),** Encephalopathy allergic (PT), Encephalopathy neonatal (PT), **Hypertensive encephalopathy (PT), Hypoxic encephalopathy (PT)**
- Encephalopathies toxic and metabolic (HLT), in particular Hepatic encephalopathy (PT), Hypoglycaemic encephalopathy (PT), Reye's syndrome (PT) and Toxic induced encephalopathy (PT)

o Headaches (HLGT)
Headaches NEC (HLT); Migraine headaches (HLT)
o Increased intracranial pressure and hydrocephalus (HLGT)
Hydrocephalic conditions (HLT); Increased intracranial pressure disorders (HLT)
o **Mental impairment disorders (HLGT)**
**Alzheimer's disease (incl subtypes) (HLT); Dementia (excl Alzheimer's type) (HLT); Developmental disorders cognitive (HLT); Memory loss (excl dementia) (HLT); Mental impairment (excl dementia and memory loss) (HLT); Mental retardations (HLT)**
o Movement disorders (incl Parkinsonism) (HLGT)
Choreiform movements (HLT); Dyskinesias and movement disorders NEC (HLT); Dystonias (HLT); Paralysis and paresis (excl congenital and cranial nerve) (HLT); Parkinson's disease and parkinsonism (HLT); Tremor (excl congenital) (HLT); Nervous system neoplasms benign (HLGT); Gliomas benign (HLT); Meningiomas benign (HLT); Nervous system neoplasms benign NEC (HLT); Neuromas (HLT)
o Nervous system neoplasms malignant and unspecified NEC (HLGT)
Central nervous system neoplasms malignant NEC (HLT); Glial tumours malignant (HLT); Meningiomas malignant (HLT); Nervous system neoplasms malignant NEC (HLT); Nervous system neoplasms unspecified malignancy NEC (HLT); Pineal parenchymal neoplasms (HLT)
o Neurological disorders NEC (HLGT)
Abnormal reflexes (HLT); Cerebellar coordination and balance disturbances (HLT); Coma states (HLT), in particular Coma hepatic (PT); Cortical dysfunction NEC (HLT), in particular Cognitive deterioration (PT) and Confusion postoperative (PT); Disturbances in consciousness NEC (HLT); Nervous system disorders NEC (HLT), in particular Anaesthetic complication neurological (PT), Central nervous system lesion (PT) and **Neurodegenerative disorder (PT);** Neurological signs and symptoms NEC (HLT); Paraesthesias and dysaesthesias (HLT); Pupillary signs (HLT); Sensory abnormalities NEC (HLT); Speech and language abnormalities (HLT); Vertigos NEC (HLT)
o Neurological disorders of the eye (HLGT)
Neurologic visual problems NEC (HLT); Ocular signs and symptoms NEC (HLT)
o Neuromuscular disorders (HLGT)
Autonomic nervous system disorders (HLT), in particular Neurogenic bowel (PT) and Stress incontinence (PT); Motor neurone diseases (HLT), in particular **Amyotrophic lateral sclerosis (PT);** Muscle tone abnormal (HLT); Neuromuscular disorders NEC (HLT); Neuromuscular junction dysfunction (HLT)
o Peripheral neuropathies (HLGT)
Acute polyneuropathies (HLT), in particular Guillain-Barre syndrome (PT); Chronic polyneuropathies (HLT); Inherited neuropathies (HLT); Mononeuropathies (HLT); Peripheral neuropathies NEC (HLT)
o Seizures (incl subtypes) (HLGT)
Absence seizures (HLT); Generalised tonic-clonic seizures (HLT); Partial complex seizures (HLT), in particular Temporal lobe epilepsy (PT); Partial simple seizures NEC (HLT); Seizures and seizure disorders NEC (HLT)
o Sleep disturbances (incl subtypes) (HLGT)
Abnormal sleep-related events (HLT); Disturbances in initiating and maintaining sleep (HLT); Disturbances in sleep phase rhythm (HLT); Narcolepsy and hypersomnia (HLT); Sleep apnoeas (HLT); Sleep disturbances NEC (HLT)
o Spinal cord and nerve root disorders (HLGT)
Cervical spinal cord and nerve root disorders (HLT); Lumbar spinal cord and nerve root disorders (HLT); Spinal cord and nerve root disorders NEC (HLT); Spinal cord and nerve root disorders traumatic (HLT)
o Structural brain disorders (HLGT)
Structural brain disorders (HLT), in particular Brain contusion (PT) and Brain damage (PT)

Abbreviations: HLGT: high level group term; HLT: high level term; NEC: not elsewhere classified; PT: preferred term; SOC: system organ class.

**Table 4:** Factors and mechanisms which underlie conditions and disorders which can be prevented or treated, alone or in combination, with medications activating, modulating/regulating or inhibiting GS activity. Certain of these factors and mechanisms are reported according to the MedDRA classification (MedDRA Version 7.1).

- ■ Age related factors (HLT) e.g. elderly (PT), menopause (PT), post menopause (PT)
- ■ Allergic factors
- ■ Biomechanical factors
- ■ Catabolic states
- ■ Chemical factors e.g. acids, ammonia, bases, ions (bivalent metal ions, ...)
- ■ Degenerative processes
- ■ Dietary factors e.g. alcohol, monosodium L-glutamate, protein deficiency, starvation
- ■ Drugs e.g. chemotherapies, corticosteroids, sodium valproate
- ■ Environmental issues (HLT)
- ■ Exercise e.g. extremely taxing exercice
- ■ Haemorrhagia
- ■ Hyperoxia
- ■ Hypoxia
- ■ Immune causes e.g. immunodeficiency, hypersensitivity, transplantation
- ■ Inflammatory processes e.g. connective tissue diseases, inflammatory bowel diseases, myositis
- ■ Infections e.g. due to bacteria, fungi, protozoaires, viri, worms, ..., or endotoxemia
- ■ Ischaemia
- ■ Metabolic stresses e.g. abnormal acid-base balance, hyperammonemia, hypercorticism, hypoglycemia/hyperglycemia, siderosis
- ■ Neoplasm (benign/malignant)
- ■ Oxidative stresses e.g. free oxygen radicals, chlorinated oxidants
- ■ Physical stresses e.g. electricity, heat/cold, pressure, radiations/radiotherapy
- ■ Surgery
- ■ Toxic factors e.g. antiseptic agents, detergents, paraquat
- ■ Trauma
- ■ ...

**Table 5:** Conditions and disorders already claimed or proposed as clinical applications of tianeptine (reported according to the MedDRA classification; MedDRA Version 7.1).

Anal and rectal pains (HLT); Dental pain and sensation disorders (HLT); Gingival pains (HLT); Neurogenic bowel (HLT); Intestinal functional disorder (HLT); Inflammatory bowel disease (PT); Frequent bowel movements (PT); Irritable bowel syndrome (PT); Infrequent bowel movements (PT); Dyspepsia (PT); Gastrointestinal and abdominal pains (excl oral and throat) (HLT); Bowel movement irregularity (PT); Oral soft tissue pain and paraesthesia (HLT); Pain and discomfort NEC (HLT); Allergic cough (PT); Asthma (PT); Adjustment disorders (incl subtypes) (HLGT); Anxiety disorders and symptoms (HLGT); Anxiety disorders NEC (incl obsessive compulsive disorder) (HLT); Anxiety symptoms (HLT); Stress disorders (HLT); Cognitive and attention disorders and disturbances (HLGT); Dementia and amnestic conditions (HLGT); Alzheimer's disease (incl subtypes) (HLT); Amnestic symptoms (HLT); Dementia NEC (HLT); Vascular dementia disorders (HLT); Depressed mood disorders and disturbances (HLGT); Manic and bipolar mood disorders and disturbances (HLGT); Mood disorders and disturbances NEC (HLGT); Affect alterations NEC (HLT); Emotional and mood disturbances NEC (HLT); Mood disorders due to a general medical condition (HLT); Mood disorders NEC (HLT); Brain hypoxia (PT); Cough (PT); Hypoxic encephalopathy (PT); Vascular dementia (PT); Vascular encephalopathy (PT); Ischaemic stroke (PT); Heat stroke (PT); Embolic stroke (PT); Thromboembolic stroke (PT); Thrombotic stroke (PT); Haemorrhagic stroke (PT); Central nervous system haemorrhages and cerebrovascular accidents (HLT); Traumatic central nervous system haemorrhages (HLT); Demyelinating disorders (HLGT); Multiple sclerosis acute and progressive (HLT); AIDS encephalopathy (PT); Anoxic encephalopathy (PT); Hypertensive encephalopathy (PT); Mental impairment disorders (HLGT); Dementia (excl Alzheimer's type) (HLT); Developmental disorders cognitive (HLT); Memory loss (excl dementia) (HLT); Mental impairment (excl dementia and memory loss) (HLT); Mental retardations (HLT); Neurodegenerative disorder (PT); Amyotrophic lateral sclerosis (PT)

Abbreviations: HLGT: high level group term; HLT: high level term; NEC: not elsewhere classified; PT: preferred term.

**Table 6:** Conditions and disorders already proposed as clinical applications of hydrazines (reported according to the MedDRA classification; MedDRA Version 7.1).

- ■ If said hydrazine is benserazide: Parkinson's disease and parkinsonism (HLT) (known inhibitory properties on the aromatic L-aminoacid decarboxylase)
- ■ If said hydrazine is bumadizone: conditions and disorders sensitive to analgesic-antipyretic and anti-inflammatory agents
- ■ If said hydrazine is dacarbazine: neoplasms (known antineoplastic properties)
- ■ If said hydrazine is dihydralazine: Heart failure (HLGT), Pre-eclampsia (PT), Vascular hypertensive disorders (HLGT) and conditions and disorders sensitive to vasodilators
- ■ If said hydrazine is hydralazine: Heart failure (HLGT), Pre-eclampsia (PT), Vascular hypertensive disorders (HLGT) and conditions and disorders sensitive to vasodilators
- ■ If said hydrazine is iproclozide: Mood alterations with depressive symptoms (HLGT) and Depressive disorders (HLT) (known inhibitory properties on the monoamine oxidase), and Ischaemic coronary artery disorders (HLT)
- ■ If said hydrazine is iproniazid: Mood alterations with depressive symptoms (HLGT) and Depressive disorders (HLT) (known inhibitory properties on the monoamine oxidase), and Vascular hypertensive disorders (HLGT)
- ■ If said hydrazine is isocarboxazide: Mood alterations with depressive symptoms (HLGT) and Depressive disorders (HLT) (known inhibitory properties on the monoamine oxidase), Ischaemic coronary artery disorders (HLT), Vascular hypertensive disorders (HLGT) and conditions and disorders sensitive to vasodilators
- ■ If said hydrazine is isoniazid: Mycobacterial infectious disorders (HLGT) and Crohn's disease (PT)
- ■ If said hydrazine is nialamide: Mood alterations with depressive symptoms (HLGT) and Depressive disorders (HLT) (known inhibitory properties on the monoamine oxidase), Ischaemic coronary artery disorders (HLT), Vascular hypertensive disorders (HLGT) and conditions and disorders sensitive to vasodilators
- ■ If said hydrazine is nifuroxazide: Gastrointestinal infections (HLGT)
- ■ If said hydrazine is phenicarbazide: Mood alterations with depressive symptoms (HLGT), Depressive disorders (HLT), Migraine headaches (HLT) and Crohn's disease (PT)
- ■ If said hydrazine is picadralazine: vascular hypertensive disorders (HLGT), and conditions and disorders sensitive to vasodilators
- ■ If said hydrazine is procarbazine: neoplasms (antineoplastic properties)

Abbreviations: HLGT: high level group term; HLT: high level term; PT: preferred term.

**Table 7:** Conditions and disorders already proposed as clinical applications of bisphosphonates (reported according to the MedDRA classification; MedDRA Version 7.1).

Bone and joint injuries (HLGT); Post-traumatic osteoporosis (PT); Osteoporosis (PT); Osteoporosis circumscripta cranii (PT); Osteoporosis postmenopausal (PT); Senile osteoporosis (PT); Bone disorders (excl congenital and fractures) (HLGT); Bone and joint infections (excl arthritis) (HLT); Bone disorders NEC (HLT); Bone erosion (PT); Osteolysis (PT); Post-traumatic osteoporosis (PT); Bone related signs and symptoms (HLT); Bone pain (PT); Metabolic bone disorders (HLT); Fractures (HLGT); Musculoskeletal and connective tissue neoplasms (benign/malignant) (HLGT); Bone cancer metastatic (PT); Metastases to bone (PT); Gaucher's disease (PT); Protozoal infectious disorders (HLGT); Vascular calcification (PT)

Abbreviations: HLGT: high level group term; HLT: high level term; NEC: not elsewhere classified; PT: preferred term.

**Table 8:** Tianeptine, metabolites and analogs.

| | |
|---|---|
| 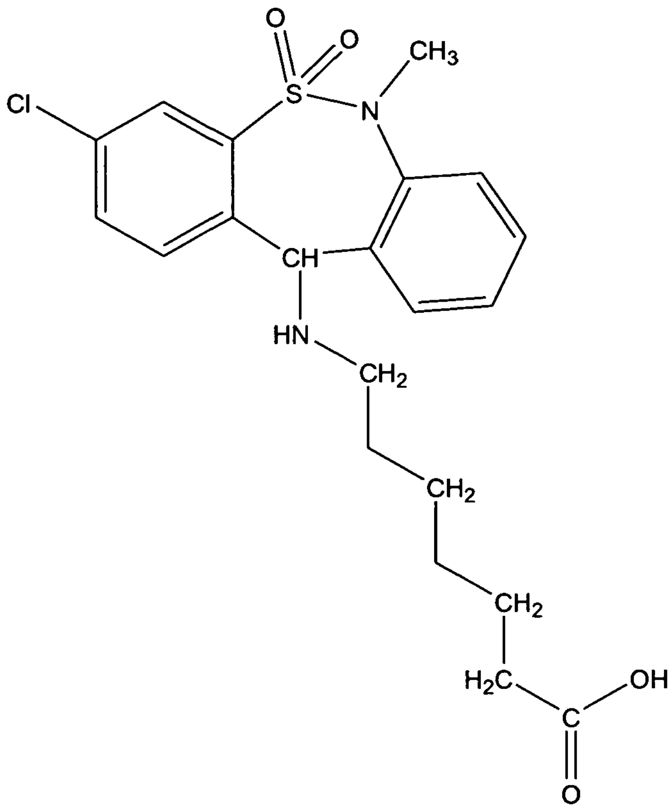 7-(8-Chloro-11-methyl-10,10-dioxo-10,11-dihydro-5*H*-10$\lambda^6$-thia-11-aza-dibenzo[*a*,*d*]cyclohepten-5-ylamino)-heptanoic acid | Tianeptine |
| 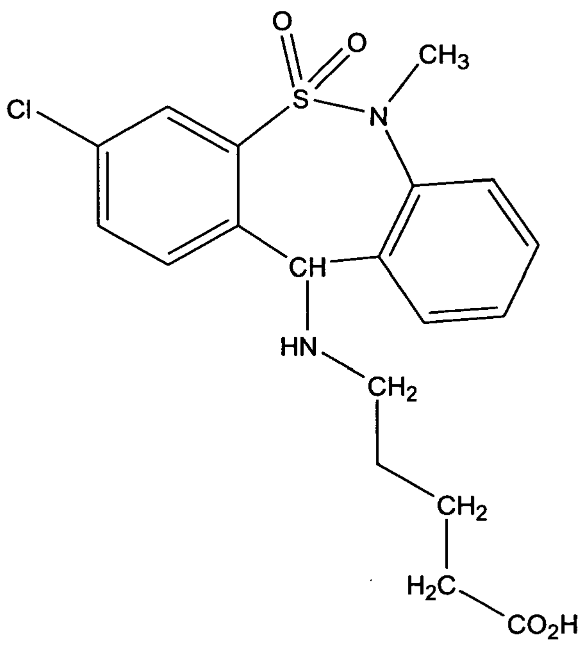 5-(8-Chloro-11-methyl-10,10-dioxo-10,11-dihydro-5*H*-10$\lambda^6$-thia-11-aza-dibenzo[*a*,*d*]cyclohepten-5-ylamino)-pentanoic acid | Metabolite |

(continued)

| | |
|---|---|
| 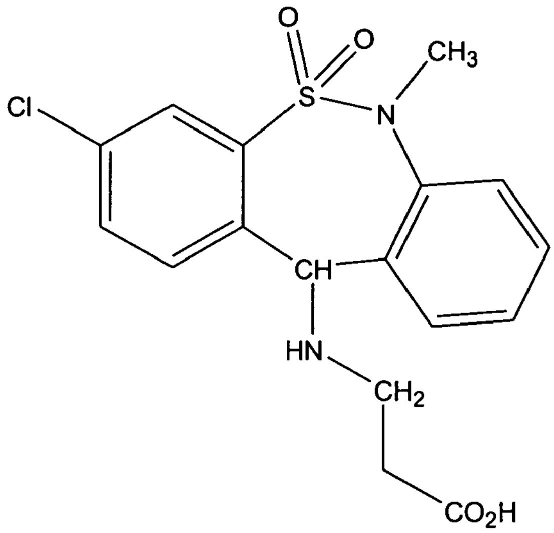 3-(8-Chloro-11-methyl-10,10-dioxo-10,11-dihydro-5*H*-10$\lambda^6$-thia-11-aza-dibenzo[*a*,*d*]cyclohepten-5-ylamino)-propionic acid | Metabolite |
| 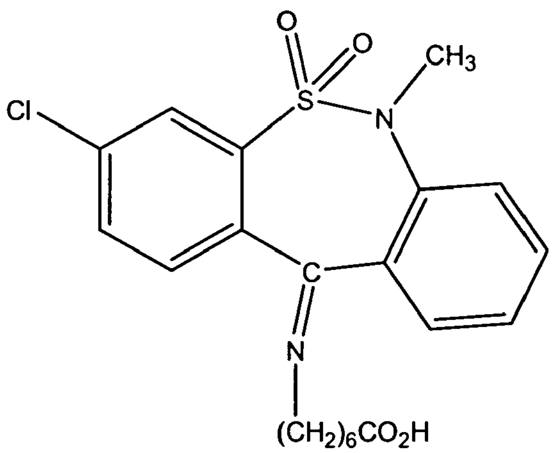 7-(8-Chloro-11-methyl-10,10-dioxo-10,11-dihydro-10$\lambda^6$-thia-11-aza-dibenzo[*a*,*d*]cyclohepten-5-ylideneamino)-heptanoic acid | Analog |
| 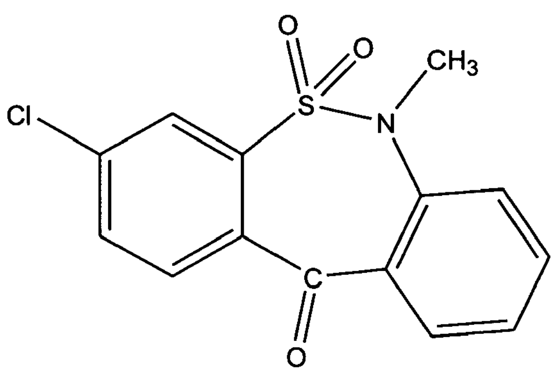 8-Chloro-11-methyl-10,10-dioxo-10,11-dihydro-10$\lambda^6$-thia-11-aza-dibenzo[*a*,*d*]cyclohepten-5-one | Analog |

(continued)

| | |
|---|---|
| 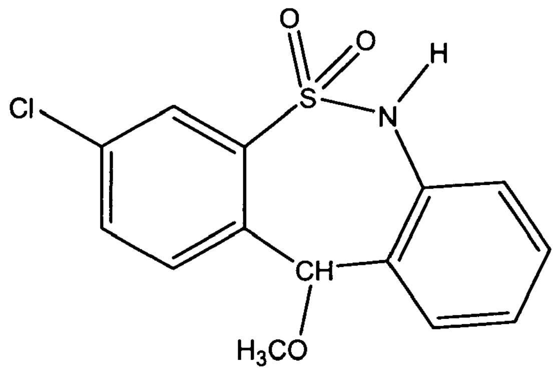 8-Chloro-5-methoxy-5,11-dihydro-10-thia-11-aza-dibenzo[*a*,*d*]cycloheptene 10,10-dioxide | Analog |
| 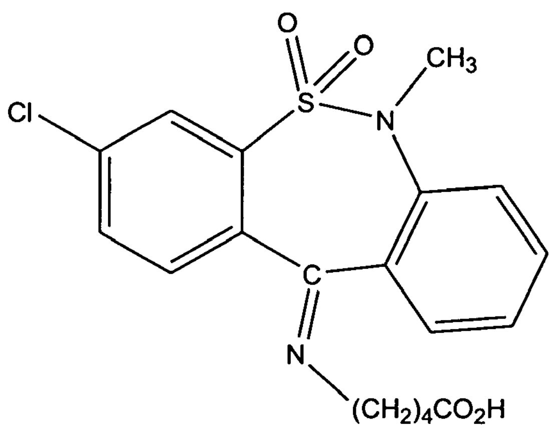 5-(8-Chloro-11-methyl-10,10-dioxo-10,11-dihydro-10$\lambda^6$-thia-11-aza-dibenzo[*a*,*d*]cyclohepten-5-ylideneamino)-pentanoic acid | Analog |
| 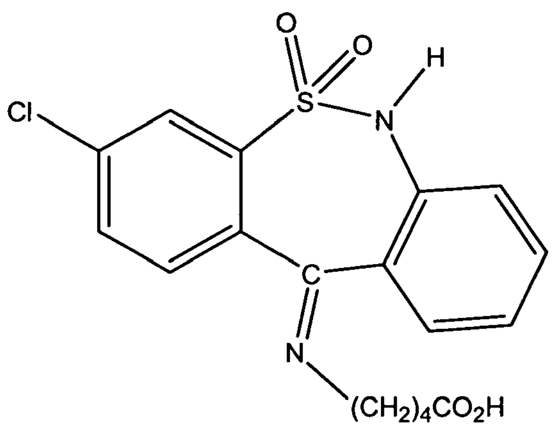 5-(8-Chloro-10,10-dioxo-10,11-dihydro-10$\lambda^6$-thia-11-aza-dibenzo[*a*,*d*]cyclohepten-5-ylideneamino)-pentanoic acid | Analog |

(continued)

| | |
|---|---|
| 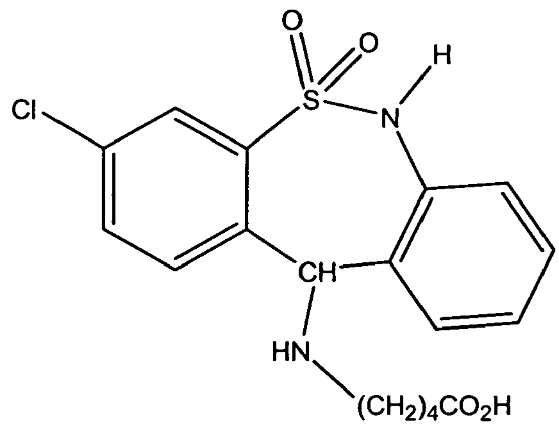<br>5-(8-Chloro-10,10-dioxo-10,11-dihydro-5*H*-10λ⁶-thia-11-aza-dibenzo[*a*,*d*]cyclohepten-5-ylamino)-pentanoic acid; compound with methane | Analog |
| 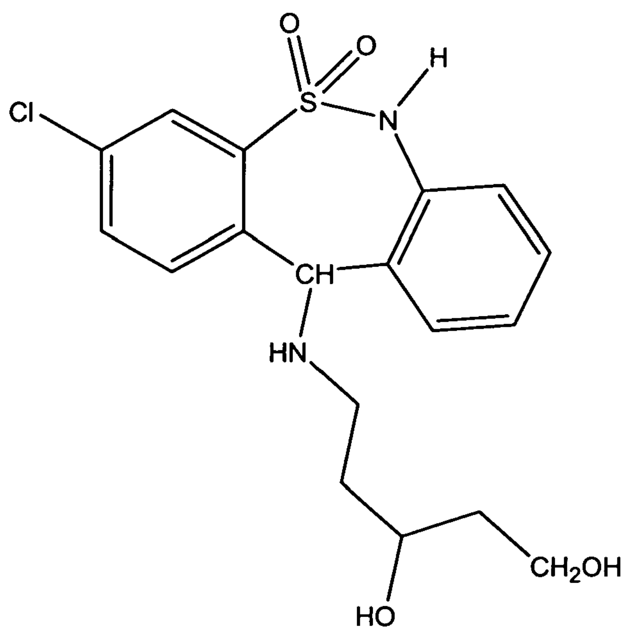<br>5-(8-Chloro-10,10-dioxo-10,11-dihydro-5*H*-10λ⁶-thia-11-aza-dibenzo[*a*,*d*]cyclohepten-5-ylamino)-pentane-1,3-diol | Analog |

(continued)

| | |
|---|---|
| 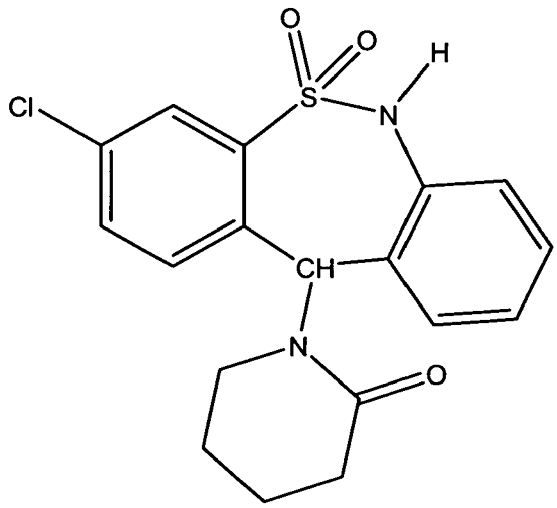<br>1-(8-Chloro-10,10-dioxo-10,11-dihydro-5*H*-10λ$^6$-thia-11-aza-dibenzo[*a*,*d*]cyclohepten-5-yl)-piperidin-2-one | Analog |

**Table 9:** GF, metabolites and analogs

| | |
|---|---|
| 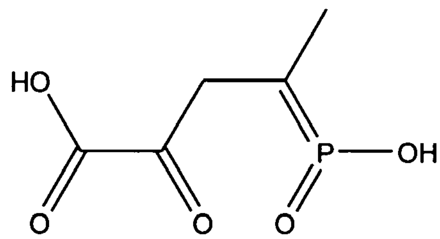<br>4-Methylphosphinico-2-oxo-butanoic acid | Metabolite<br>Ruhland et al., 2002 |
| 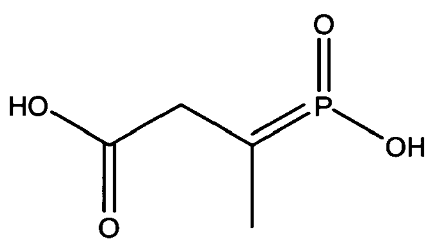<br>3-Methylphosphinicopropionic acid | Metabolite<br>Ruhland et al., 2002 |
| 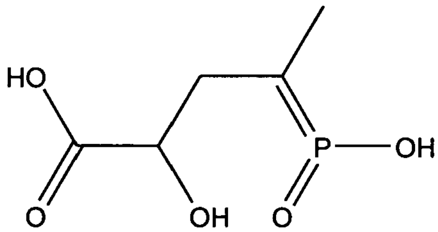<br>4-Methylphosphinico-2-hydroxybutanoic acid | Metabolite<br>Ruhland et al., 2002 |

(continued)

| | |
|---|---|
| 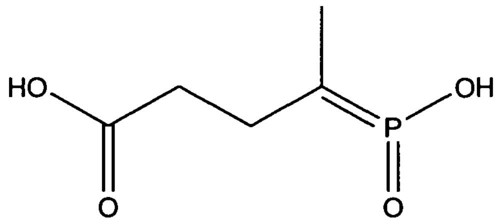<br>4-Methylphosphinicobutanoic acid | Metabolite<br>Ruhland et al., 2002 |
| 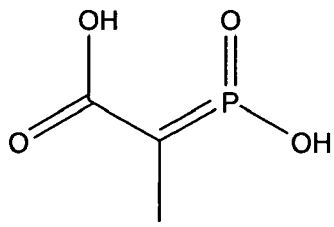<br>2-Methylphosphinicoacetic acid | Metabolite<br>Ruhland et al., 2002 |
| 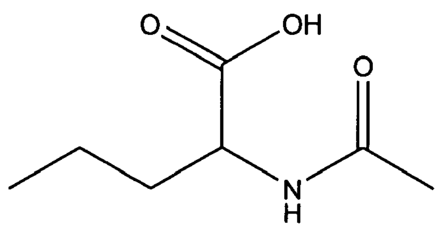<br>2-Acetamido-4-methylbutanoic acid | Metabolite<br>Ruhland et al., 2002 |
| 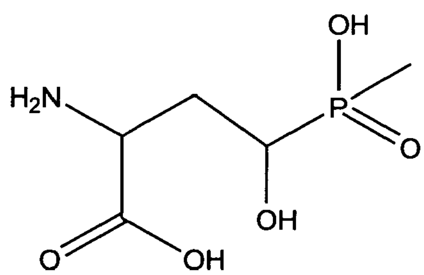<br>Gamma-hydroxy phosphinothricin | Synthetic inhibitor Ki = 47 μM (sheep brain)<br>Logusch et al., 1989 Eisenberg et al., 2000 |
| 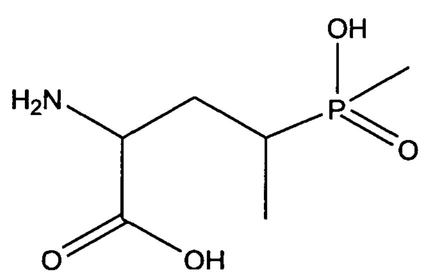<br>Gamma methyl phosphinothricin | Synthetic inhibitor Ki = 407 μM (sheep brain)<br>Logusch et al., 1989 Eisenberg et al., 2000 |

(continued)

| | |
|---|---|
| 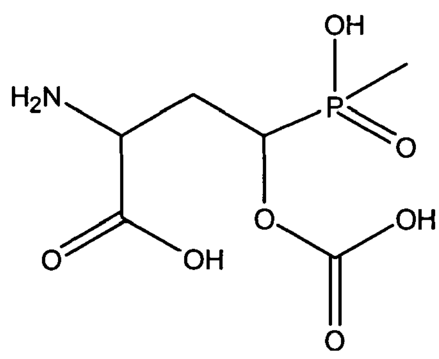<br>Gamma-acetoxy phosphinothricin | Synthetic inhibitor Ki = 33 μM (*E.coli*)<br><br>Walker et al., 1987 Eisenberg et al., 2000 |
| 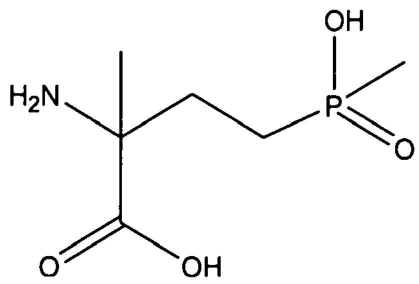<br>Alpha-methyl phosphinothricin | Synthetic inhibitor Ki = 125 μM (sheep brain)<br><br>Logusch et al., 1988 Eisenberg et al., 2000 |
| 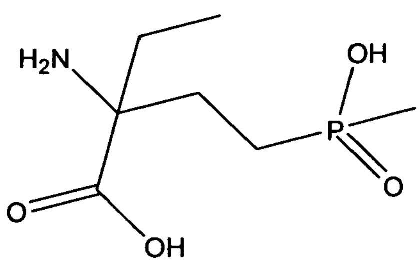<br>Alpha-ethyl phosphinothricin | Synthetic inhibitor Ki = 111 μM (sheep brain)<br><br>Logusch et al., 1988 Eisenberg et al., 2000 |
| 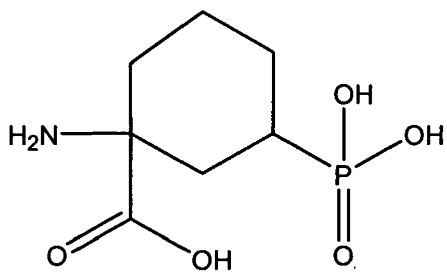<br>1-Amino-3-phosphono-cyclohexanecarboxylic acid<br>Cyclohexane phosphinothricin | Synthetic inhibitor Ki = 125 μM (sheep brain)<br><br>Logusch et al., 1988 Eisenberg et al., 2000 |

(continued)

| | |
|---|---|
| 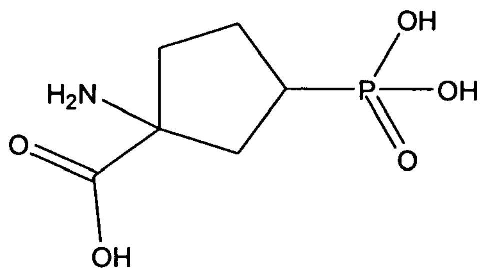<br>1-Amino-3-phosphono-cyclopentanecarboxylic acid<br>Cyclopentane phosphinothricin | Synthetic inhibitor Ki = 0.47 μM (mung bean)<br>Johnson et al., 1990 Eisenberg et al., 2000 |
| 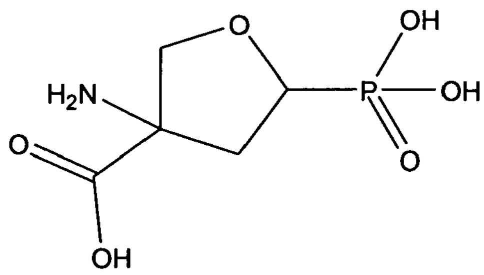<br>3-Amino-5-phosphono-tetrahydro-furan-3-carboxylic acid<br>Tetrahydrofuran phosphinothricin | Synthetic inhibitor Ki = 5 μM (mung bean)<br>Johnson et al., 1990 Eisenberg et al., 2000 |
| 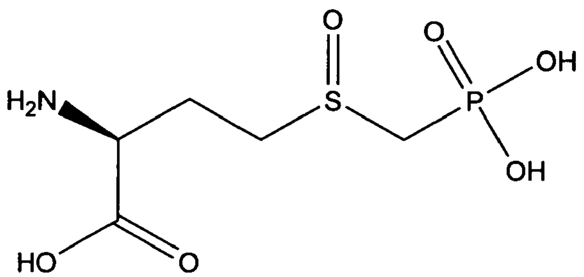<br>s-Phosphonomethyl homocysteine sulfoxide | Synthetic inhibitor<br>Logusch et al., 1988 Eisenberg et al., 2000 |
| 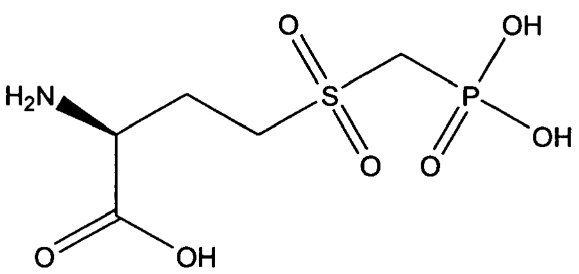<br>s-Phosphonomethyl homocysteine sulfone | Synthetic inhibitor Ki = 1.4 mM<br>Logusch et al., 1988 Eisenberg et al., 2000 |

(continued)

| | |
|---|---|
| 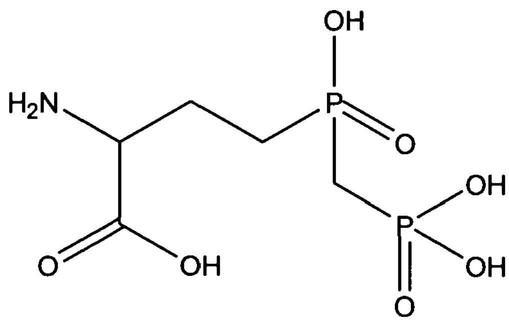<br>2-Amino-4-[(phosphonomethyl)hydroxyphosphinyl]butanoic acid | Synthetic inhibitor Ki = 750 mM (sheep brain)<br>Farrington et al., 1987 Eisenberg et al., 2000 |
| 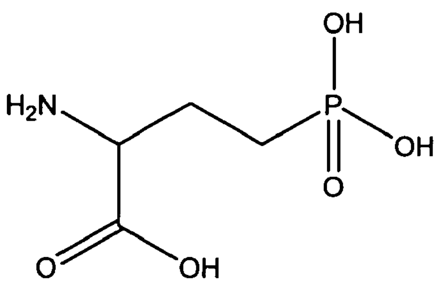<br>2-Amino-4-phosphono butanoic acid | Synthetic inhibitor Ki = 880 μM (rat liver)<br>Lejczak et al., 1981 Eisenberg et al., 2000 |
| 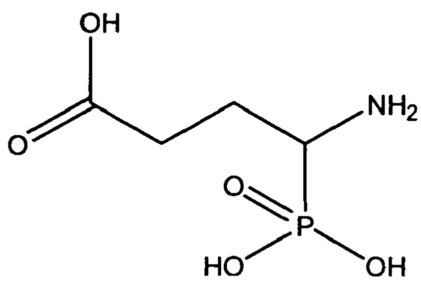<br>4-Amino-4-phosphono butanoic acid | Synthetic inhibitor Ki = 1.3 mM (rat liver)<br>Lejczak et al., 1981 Eisenberg et al., 2000 |
| 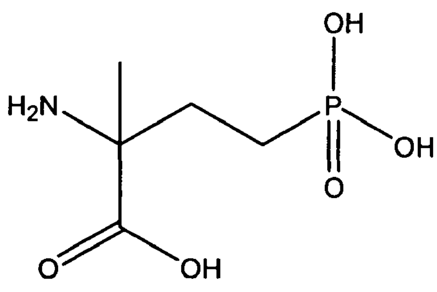<br>2-Amino-2-methyl-4-phosphono butanoic acid | Synthetic inhibitor with Ki = 6.3mM (rat liver)<br>Lejczak et al., 1981 Eisenberg et al., 2000 |

(continued)

| | |
|---|---|
| 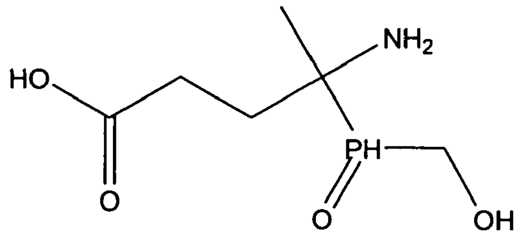<br>4-Amino-4-(hydroxymethylphosphinyl)-4-methyl butanoic acid | Synthetic inhibitor Ki = 9.5 mM (rat liver)<br>Lejczak et al., 1981 Eisenberg et al., 2000 |
| 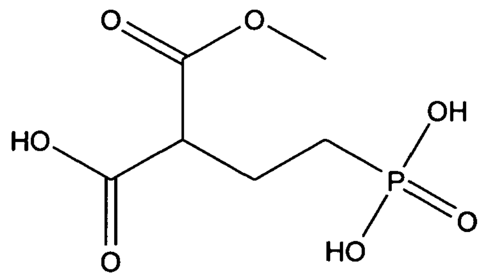<br>2-Methoxycarbonyl-4-phosphono butanoic acid | Synthetic inhibitor Ki = 8.7 mM (rat liver)<br>Lejczak et al., 1981 Eisenberg et al., 2000 |
| 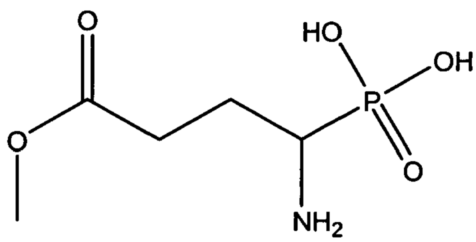<br>Methyl 4-amino-4-phosphono butanate | Synthetic inhibitor Ki = 2.1 mM (rat liver)<br>Lejczak et al., 1981 Eisenberg et al., 2000 |
| 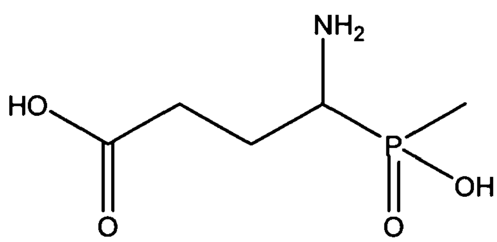<br>4-Amino-4-(hydroxymethylphosphinyl)butanoic acid | Synthetic inhibitor Ki = 0.8 mM (rat liver)<br>Lejczak et al., 1981 Eisenberg et al., 2000 |
| 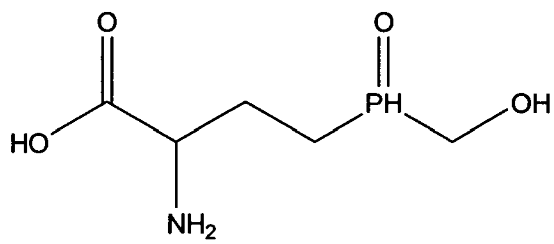<br>2-Amino-4-(hydroxymethylphosphinyl)butanoic acid | Lejczak et al., 1981 Eisenberg et al., 2000 |

(continued)

| | |
|---|---|
| 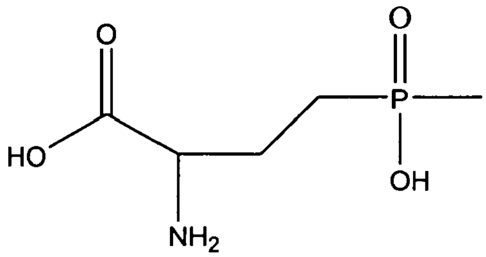 Phosphinothricin | Bayer et al., 1972 |
| 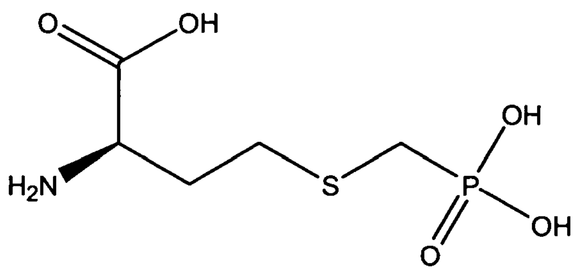 s-Phosphonomethyl homocysteine | Logusch et al., 1988 Eisenberg et al., 2000 |
| 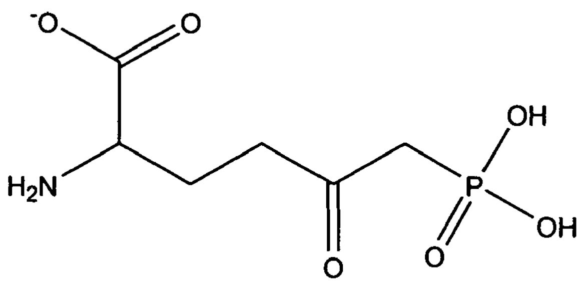 4-(Phosphonoacetyl)-L-alpha-aminobutyrate | Wedler et al., 1980 |
| 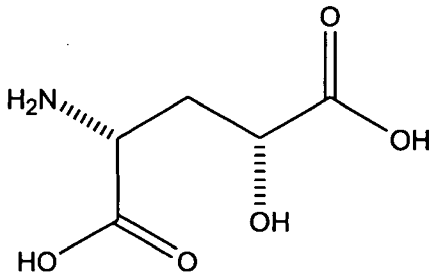 Threo-4-hydroxy-D- glutamic acid | Firsova et al., 1986 |

(continued)

| | |
|---|---|
| 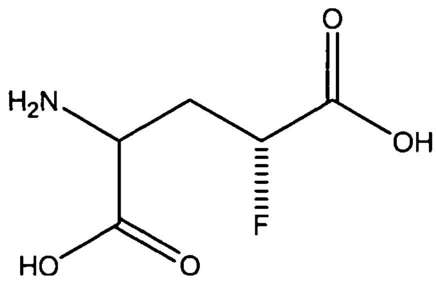<br>Erythro-4-fluoro-D,L-glutamic acid | Firsova et al., 1986 |
| 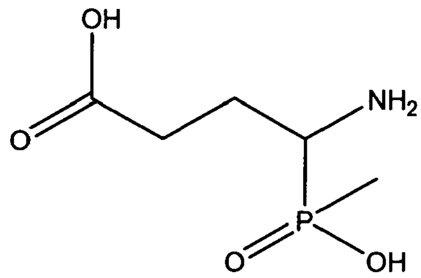<br>4-Amino-4-(hydroxy-methyl-phosphinoyl)-butyric acid | Lejczak et al., 1981 |
| 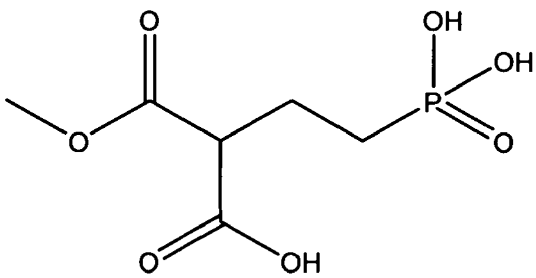<br>2-Methoxycarbonyl-4-phosphono butanoic acid | Lejczak et al., 1981 |
| 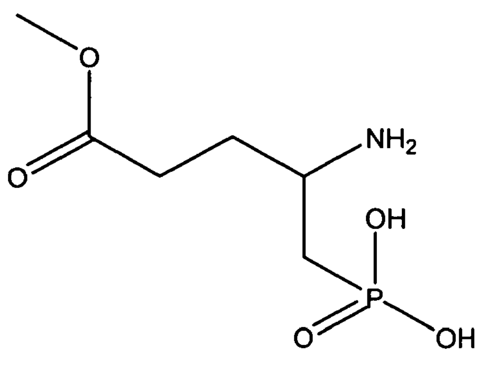<br>Methyl 4-amino-4-phosphono butanoate | Lejczak et al., 1981 |

(continued)

| | |
|---|---|
| 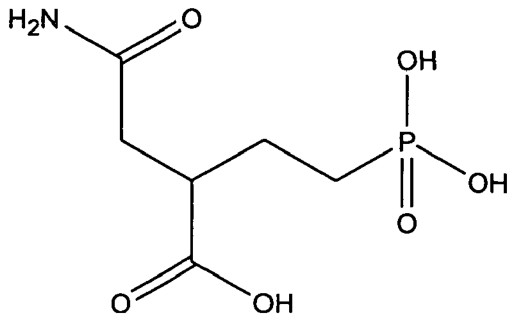 2-Amido-4-phosphono butanoic acid | Synthetic inhibitor Ki = 6,2 mM (rat liver)<br>Eisenberg et al., 2000 |

**Table 10:** MS and analogs

| | |
|---|---|
| 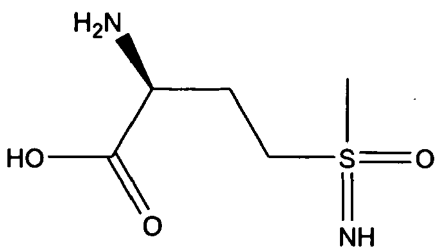 Methionine sulfoximine | Eisenberg et al., 2000 |
| 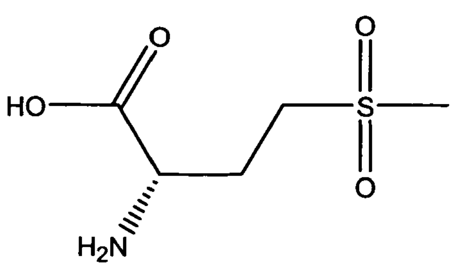 Methionine sulfone | Very potent ATP-dependent inhibitor of GS activity in a number of species. First discovered in nitrogen chloride treated zein.<br>Eisenberg et al., 2000 |
| 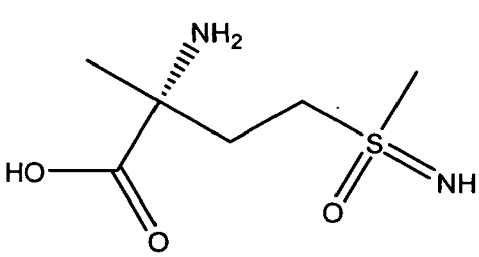 Alpha-methyl methionine sulfoximine | Slightly less inhibitory synthetic MS derivative<br>Eisenberg et al., 2000 |

(continued)

| | |
|---|---|
| 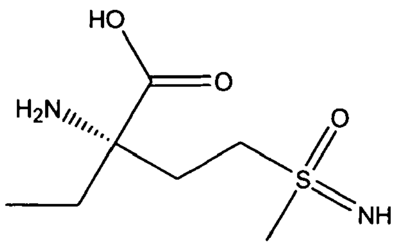 Alpha-ethyl methionine sulfoximine | Eisenberg et al., 2000 |
| 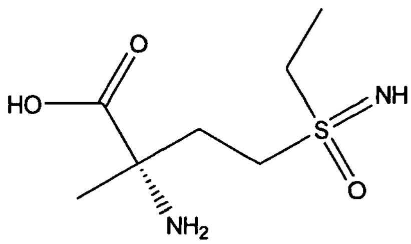 Alpha-methyl ethionine sulfoximine | Eisenberg et al., 2000 |
| 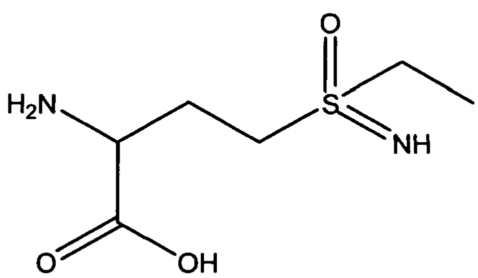 Ethionine sulfoximine | Eisenberg et al., 2000 |
| 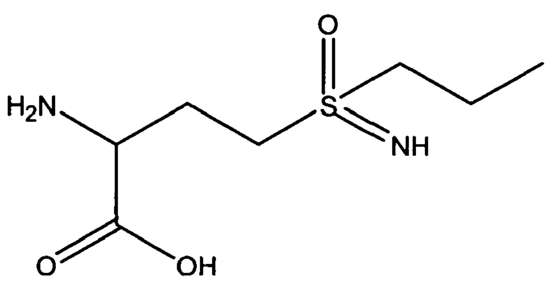 Prothionine sulfoximine | Eisenberg et al., 2000 |

(continued)

| | |
|---|---|
| 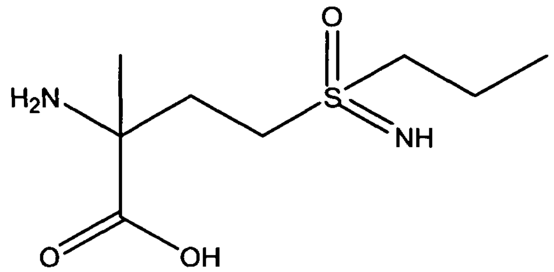<br>Alpha-methyl prothionine sulfoximine | Eisenberg et al., 2000 |
| 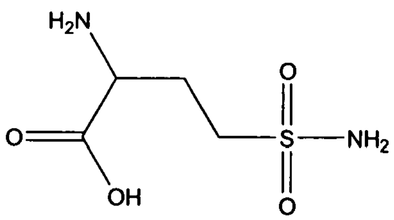<br>3-Amino-3-carboxypropane-sulfonamide | Eisenberg et al., 2000 |
| 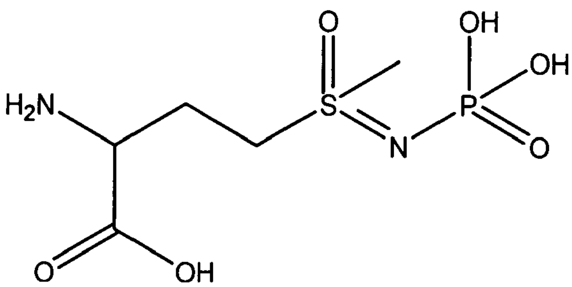<br>Methionine sulfoximine phosphate | Rowe et al., 1969 |

**Table 11:** Hydrazines

| | |
|---|---|
| 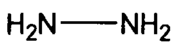<br>Hydrazine | |

(continued)

| | |
|---|---|
| 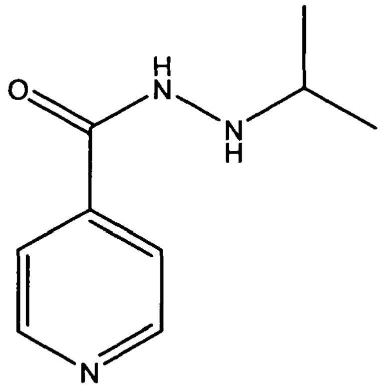 Iproniazid | Ito et al., 1992 |
| 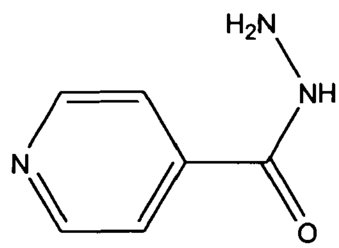 Isoniazid | Ito et al., 1992 |
| 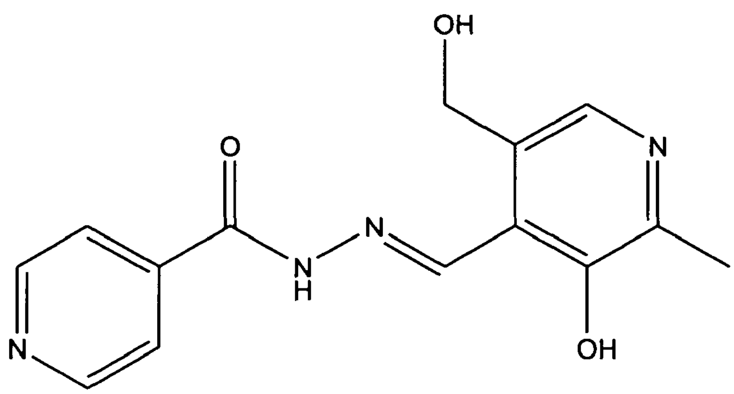 Pyridoxal isonicotinoyl hydrazone | Buss et al., 2004 |

(continued)

| | |
|---|---|
| 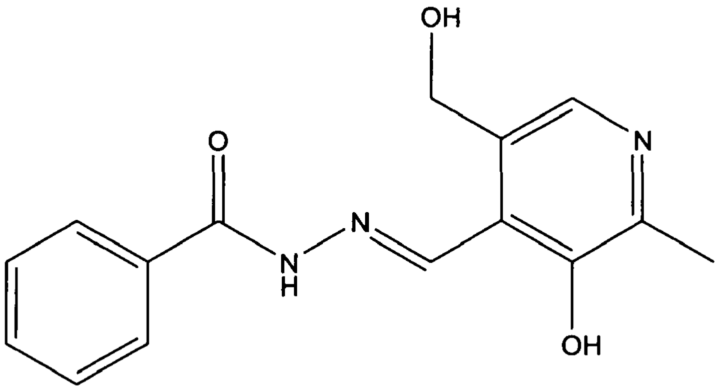 Pyridoxal benzoyl hydrazone | Buss et al., 2004 |
| 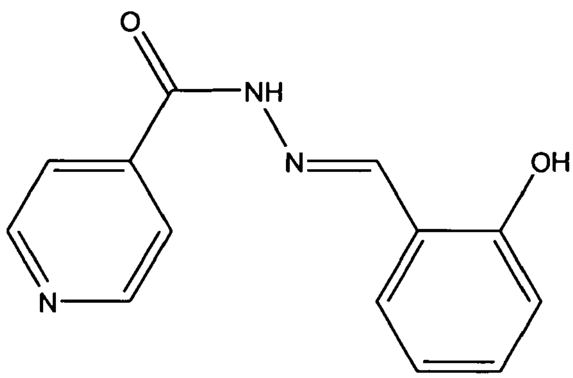 Salicylaldehyde isonicotinoyl hydrazone | Buss et al., 2004 |
| 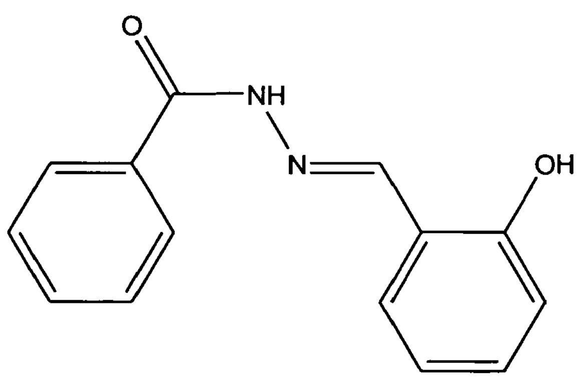 Salicylaldehyde benzoyl hydrazone | Buss et al., 2004 |

(continued)

| | |
|---|---|
| 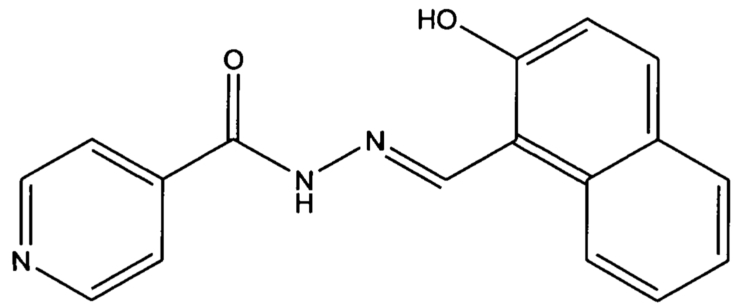<br>2-Hydroxy-1-naphthaldehyde isonicotinoyl hydrazone | Ito et al., 1992 |
| 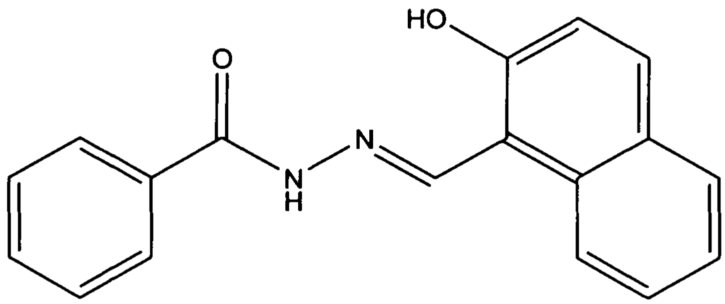<br>2-Hydroxy-1-naphthaldehyde benzoyl hydrazone | Ito et al., 1992 |
| 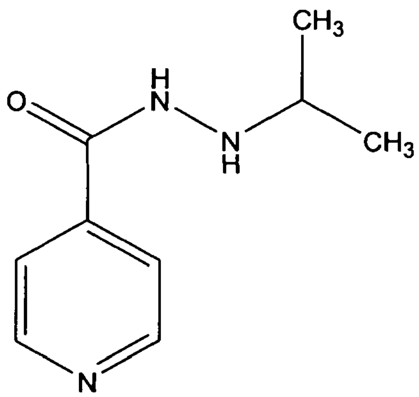<br>Isonicotinic acid *N'*-isopropyl-hydrazide | Ito et al., 1992 |
| 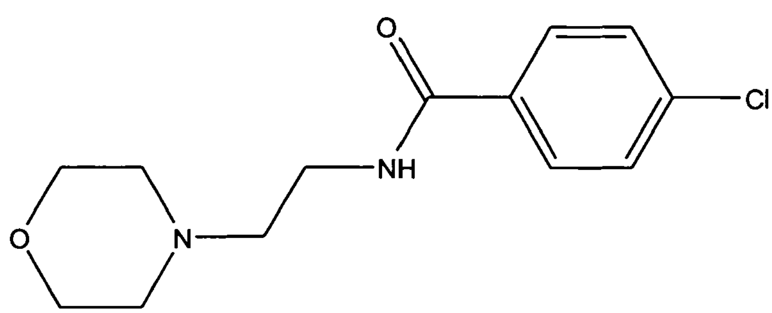<br>4-Chloro-N(2-morpholinoethyl)benzamide | King 1952 |

(continued)

| | |
|---|---|
| 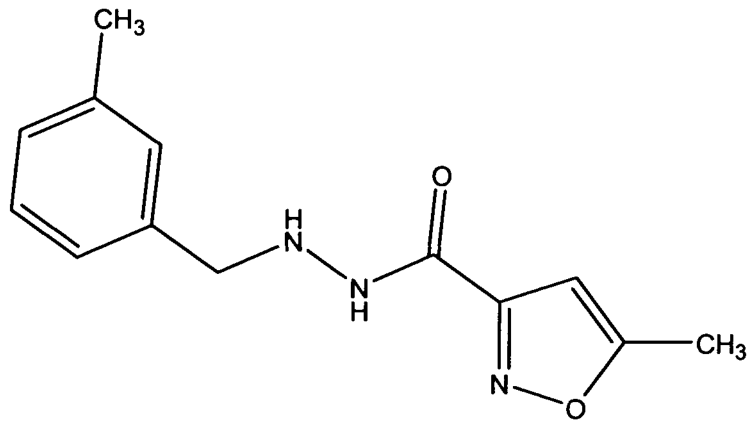 5-Methyl-isoxazole-3-carboxylic acid *N'*-(3-methyl-benzyl)-hydrazide | Darling 1959 |
| 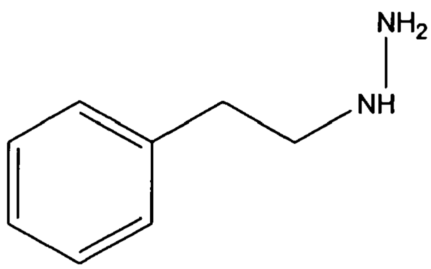 Phenethyl-hydrazine | Phenelzine Roh et al., 1994 |
| 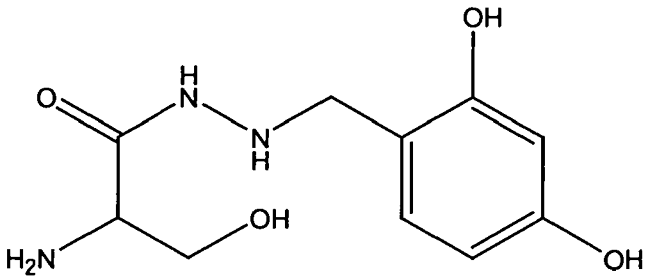 DL-Serine 2-(2,3,4-trihydroxybenzyl)hydrazide | Benserazide |

(continued)

| | |
|---|---|
| 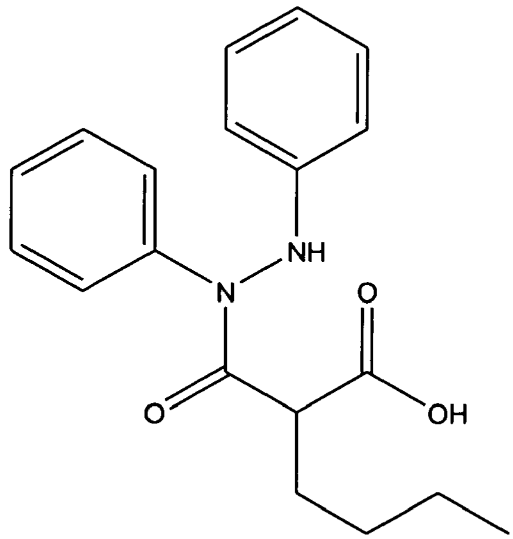<br>Butylmalonic acidmono-(1,2-diphenylhydrazide) | Bumadizone |
| 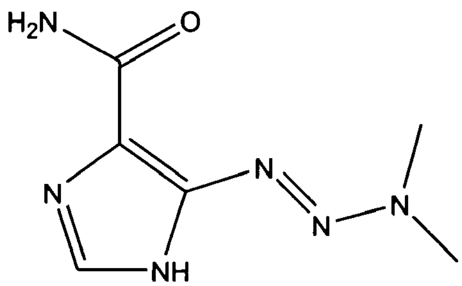<br>5-(3,3-Dimethyl-1-triazenyl)-1H-imidazole-4-carboxamide | Dacarbazine |
| 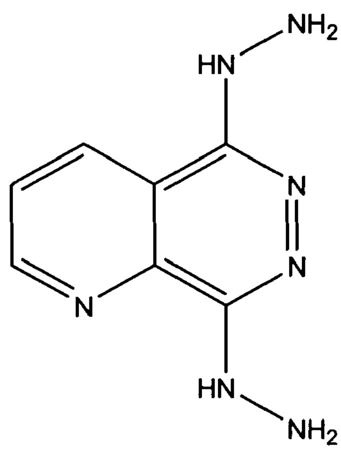<br>1,4-Dihydrazino-5-azaphthalazine | Dihydralazine |

(continued)

| | |
|---|---|
| 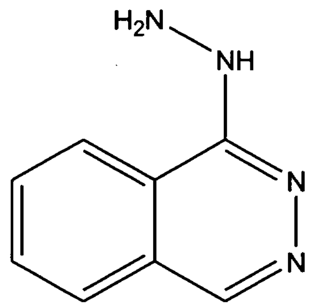 1-Hydrazinophthalazine | Hydralazine |
| 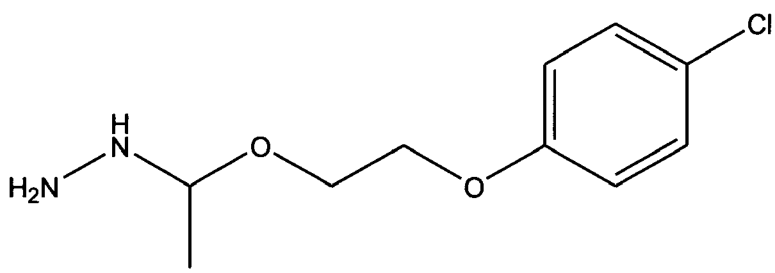 {1-[2-(4-Chloro-phenoxy)-ethoxy]-ethyl}-hydrazine | Iproclozid |
| 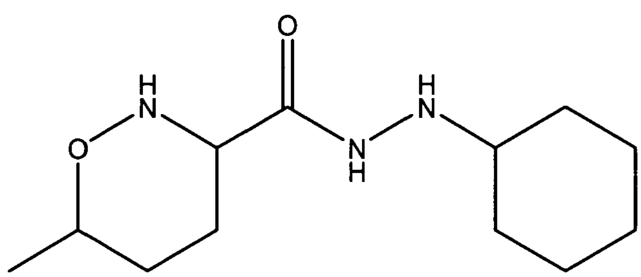 6-Methyl-[1,2]oxazinane-3-carboxylic acid *N'*-cyclohexyl-hydrazide | Isocarboxazid |
| 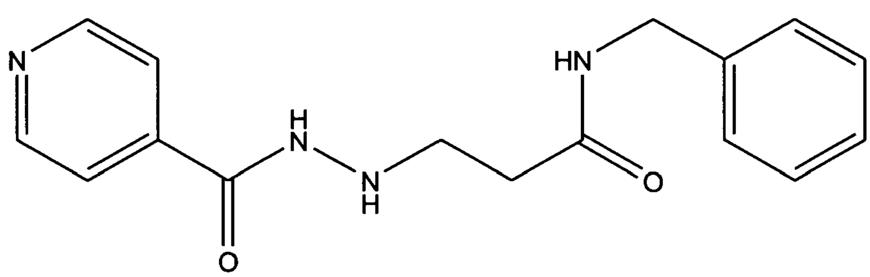 Pyridine-4-carboxylic 2-[2-(benzylcarbamoyl)ethyl]hydrazide | Nialamide |

(continued)

| | |
|---|---|
| 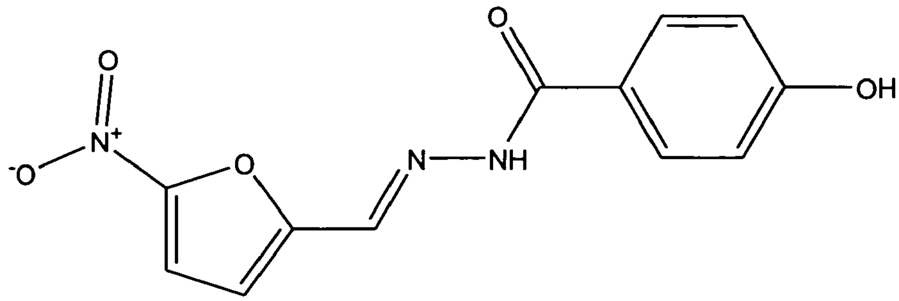 5-Nitro-2-furaldehyde p-hydroxybenzoylhydrazone | Nifuroxazide |
| 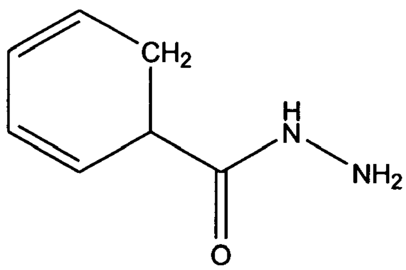 Cyclohexa-2,4-dienecarboxylic acid hydrazide | Phenicarbazide |
| 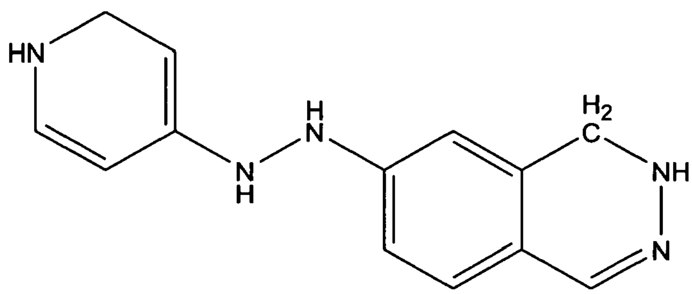 *N*-(3,4-Dihydro-phthalazin-6-yl)-*N'*-(1,2-dihydro-pyridin-4-yl)-hydrazine | Picodralazine |
| 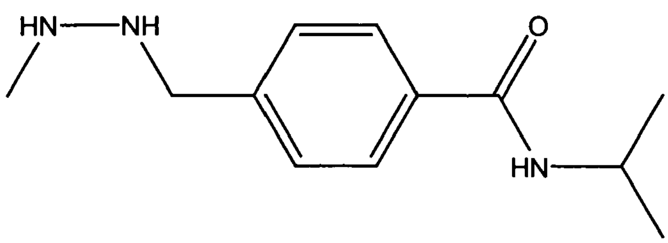 N-Isopropyl[(methyl-2hydrazino)methyl]-p-toluamide | Procarbazine |

**Table 12:** Bisphosphonates

| | |
|---|---|
| 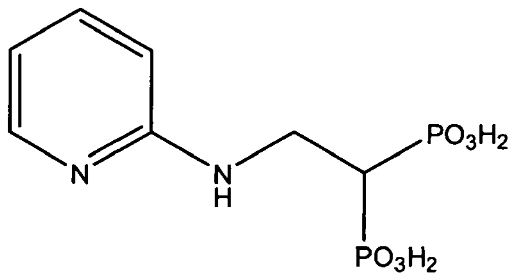 [1-Phosphono-2-(pyridin-2-ylamino)-ethyl]-phosphonic acid | Obojska et al., 2004 |
| 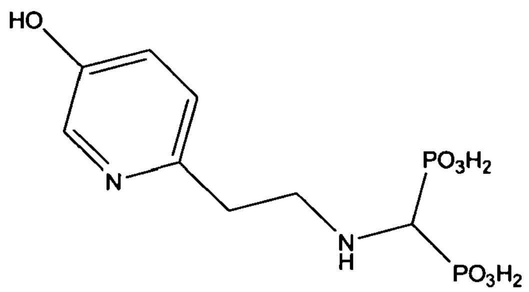 {[2-(5-Hydroxy-pyridin-2-yl)-ethylamino]-phosphono-methyl}-phosphonic acid | Obojska et al., 2004 |
| 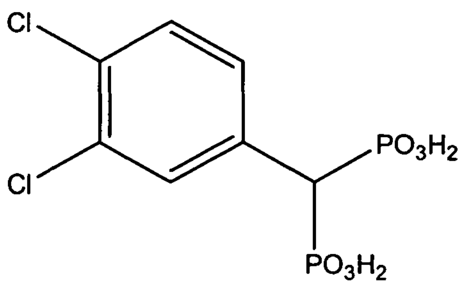 [(3,4-Dichloro-phenyl)-phosphono-methyl]-phosphonic acid | Obojska et al., 2004 |
| 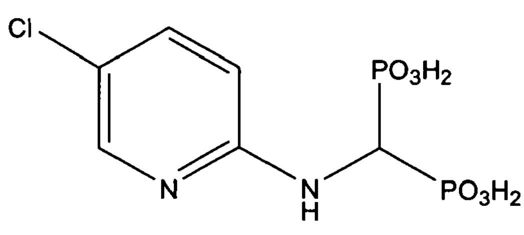 [(5-Chloro-pyridin-2-ylamino)-phosphono-methyl]-phosphonic acid | Kafarski et al., 2001 |

(continued)

| | |
|---|---|
| 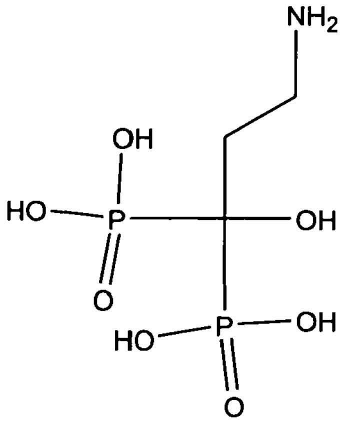<br>3-Amino-1-hydroxy-1-phosphonopropyl phosphonic acid | Pamidronate Dunford et al., 2001 |
| 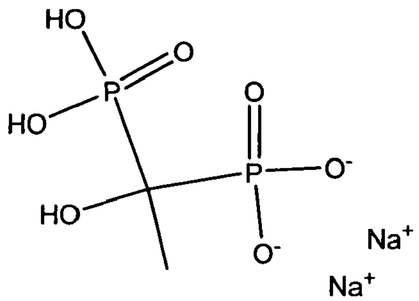<br>Dihydrogen (1-hydroxyethylidene)bisphosphonate disodium | Etidronate Reitsma et al., 1980 |
| 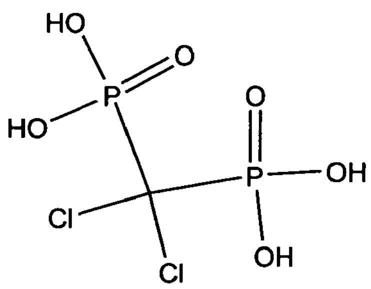<br>Dichloromethylene bisphosphonate | Clodronate Wronski 1991 |
| 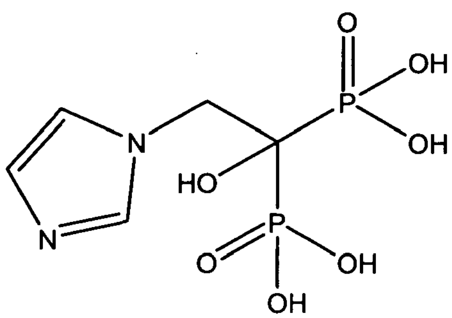<br>(1-Hydroxy-2-imidazol-1-yl-1-phosphono-ethyl)-phosphonic acid | Zolendronate<br>Neville-Webbe et al., 2002 |

(continued)

| | |
|---|---|
| 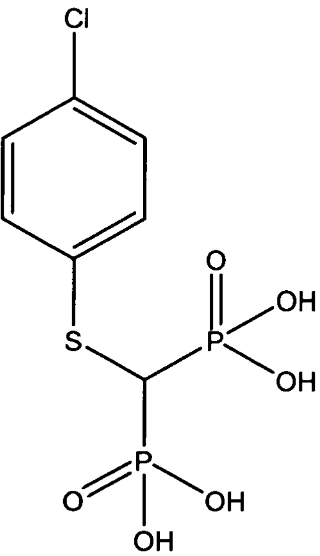<br>[(4-Chloro-phenylsulfanyl)-phosphono-methyl]-phosphonic acid | Tiludronate Morales-Piga 1999 |
| 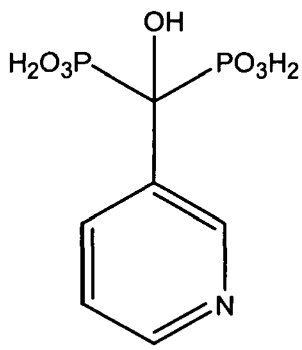<br>(Hydroxy-phosphono-pyridin-3-yl-methyl)-phosphonic acid | Risedronate Wronski et al., 1991 |
| 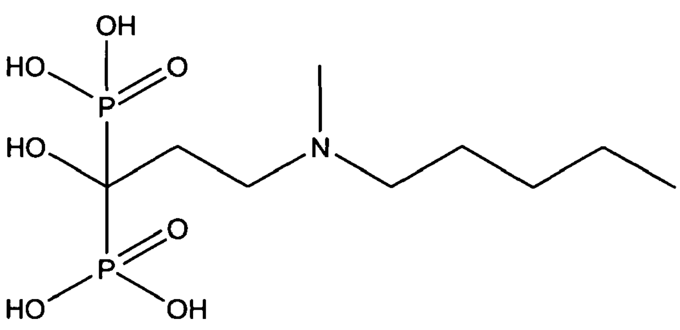<br>(Hydroxy-phosphono-pyridin-3-yl-methyl)-phosphonic acid | Ibandronate Muhlbauer et al., 1991 |

(continued)

| | |
|---|---|
| 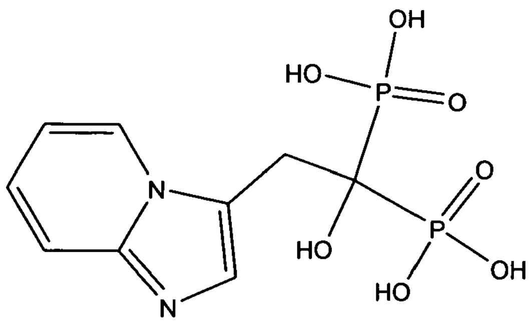 [1-hydroxy-2-(imidazo[1,2-a]pyridin-3-yl)ethylidene]bisphosphonic acid | Minodronate Takeuchi et al., 1998 |
| 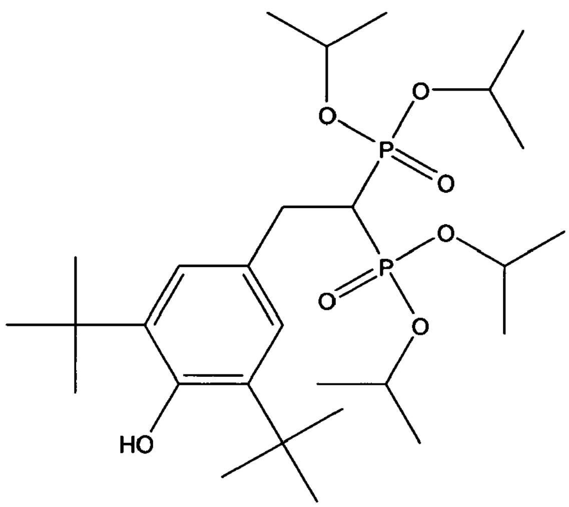 Tetra-iso-propyl 2-(3,5-di-tert-butyl-4-hydroxyphenyl)ethyl-1,1-diphosphonate | Apomine Jackson et el., 2000 |

**Table 13:** Other structural analogs of glutamate

| | |
|---|---|
| 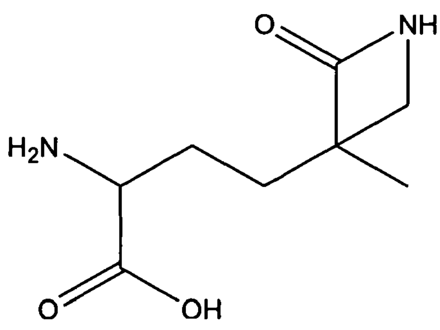 Tabtoxinine-β-lactam | Langston-Unkefer et al., 1987 |

(continued)

| | |
|---|---|
| 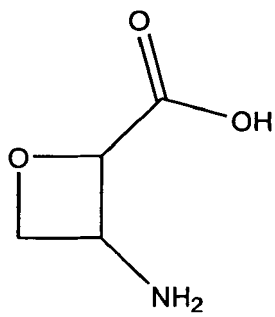 Oxetin | Omura et al., 1984 |
| 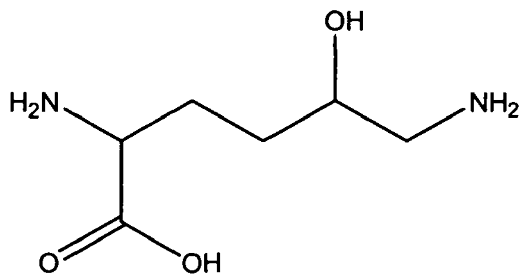 5-Hydroxylysine | Rabinovitz et al., 1957 |
| 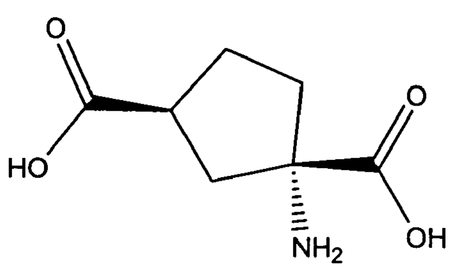 1-Aminocyclopentane-cis-1,3-dicarboxylic acid | Group I/II glutamate metabotropic receptor agonist<br>Olverman et al., 1983 |
| 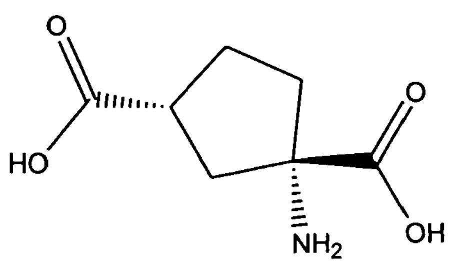 1-Aminocyclopentane-trans-1,3-dicarboxylic acid | Monaghan et al., 1989 |

(continued)

| | |
|---|---|
| 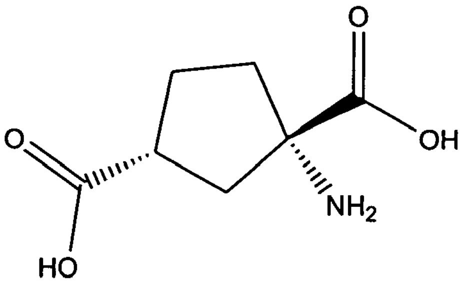<br>(1S,3R)-Aminocyclopentane-1,3-dicarboxylic acid | Irving et al., 1990 |
| 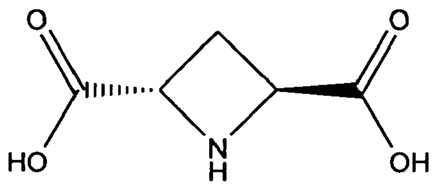<br>Trans-Azetidine-2,4-dicarboxylic acid | Group I selective glutamate metabotropic receptor agonist. (activates $mGlu_1$ and $mGlu_5$)<br><br>Kozikowski et al., 1993 |
| 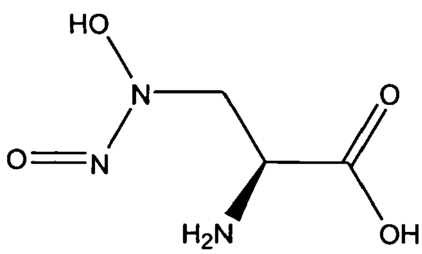<br>Alanosine | Antibiotic produced by *Streptomyces alansinicus.*<br><br>Anandaraj et al., 1980 |
| 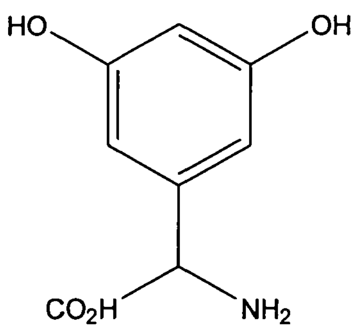<br>(RS)-3,5-Dihydroxyphenylglycine | Group I selective glutamate metabotropic receptor agonist (activates $mGlu_1$ and $mGlu_5$)<br><br>Schoepp et al., 1994 |

(continued)

| | |
|---|---|
| 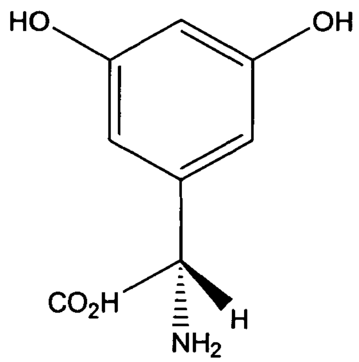<br>(S)-3,5-Dihydroxyphenylglycine | Schoepp et al., 1994 |
| 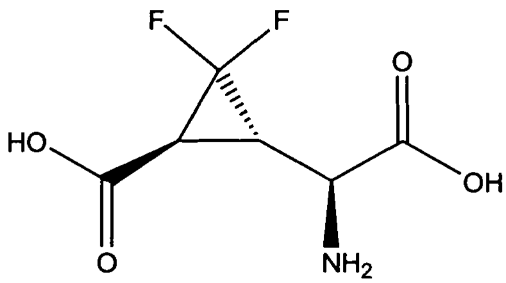<br>(2S,1'S,2'S)-2-(2'-Carboxy-3',3'-difluorocyclopropyl)glycine | Glutamate metabotropic receptor agonist<br>Shinozaki et al., 1996 |
| 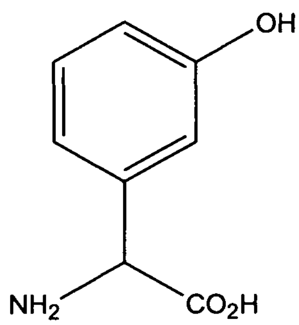<br>(RS)-3-Hydroxyphenylglycine | Glutamate metabotropic receptor agonist<br>Thomsen and Suzdak, 1993 |
| 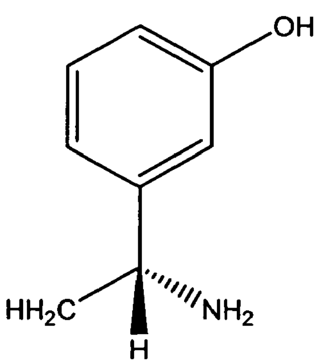<br>S-3-Hydroxyphenylglycine | Selective $mGlu_1$ glutamate metabotropic receptor agonist<br>Thomsen et al., 1993 |

(continued)

| | |
|---|---|
| 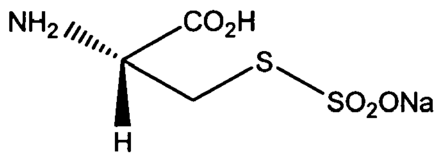<br>S-Sulfo-L-cysteine sodium salt | $mGlu_{1a}$ and $mGlu_{5a}$ glutamate metabotropic receptor agonist<br>Mewett et al., 1983 |
| 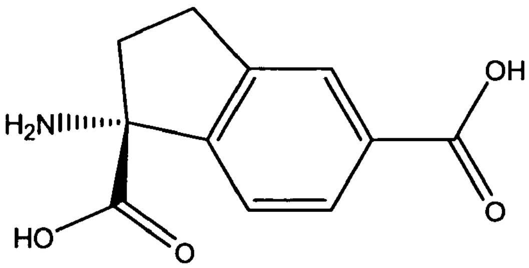<br>(RS)-1-Aminoindan-1,5-dicarboxylic acid | $mGlu_1$ glutamate metabotropic receptor antagonist<br>Pellicciari et al., 1995 |
| 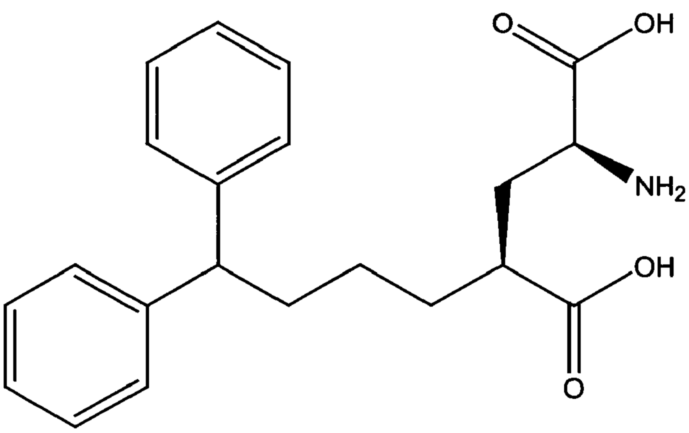<br>(4S)-4-(4,4-Diphenylbutyl)-L-glutamic acid | Selective and competitive $mGlu_2$ glutamate metabotropic receptor antagonist<br>Wermuth et al., 1996 |
| 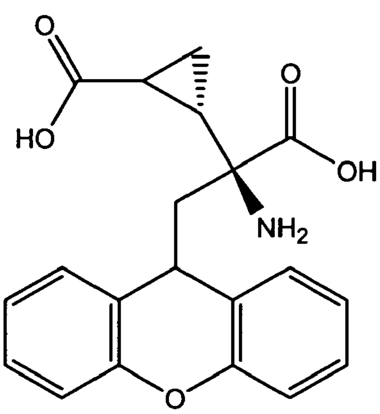<br>(2S)-2-Amino-2-[(1S,2S)-2-carboxycycloprop-1-yl]-3-(xanth-9-yl) propanoic acid | Highly potent $mGlu_2$ glutamate metabotropic receptor antagonist<br>Ornstein et al., 1998 |

(continued)

| | |
|---|---|
| 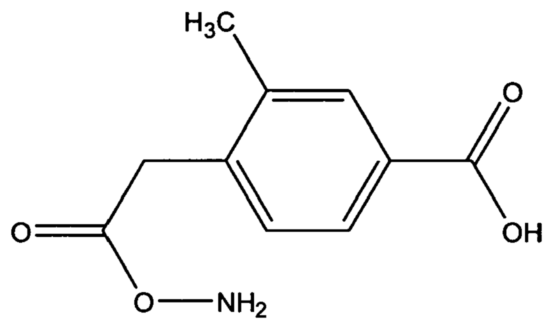 (S)-(+)-Amino-4-carboxy-2-methylbenzeneacetic acid | Selective $mGlu_{1a}$ glutamate metabotropic receptor antagonist<br>Clarke et al., 1997 |
| 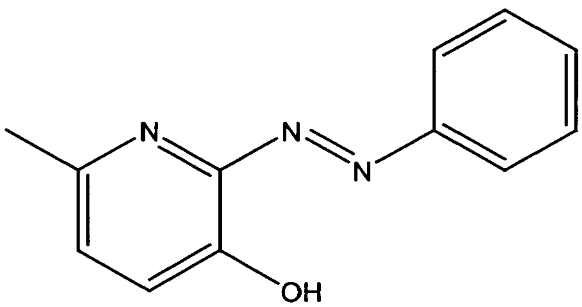 6-Methyl-2-(phenylazo)-3-pyridinol | Selective $mGlu_5$ glutamate metabotropic receptor antagonist<br>Varney et al., 1999 |
| 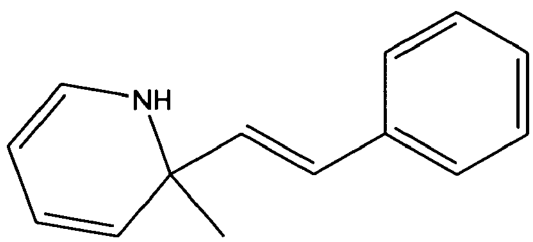 2-Methyl-2-(phenylethenyl)pyridine | Selective $mGlu_5$ glutamate metabotropic receptor antagonist<br>Varney et al., 1999 |
| 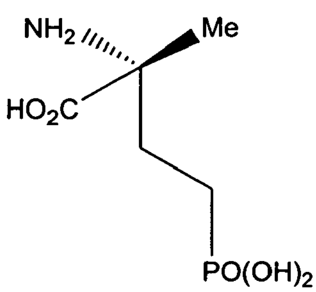 2-Amino-2-methyl-4-phosphono-butyric acid | Selective $mGlu_3$ glutamate metabotropic receptor antagonist<br>Sekiyama et al., 1996 |

(continued)

| | |
|---|---|
| 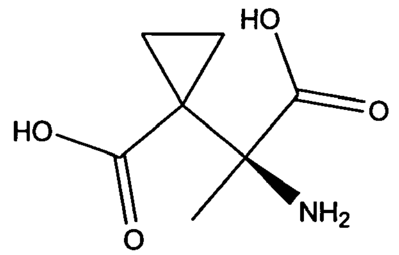<br>(2S,3S,4S)-2-Methyl-2-(carboxycyclopropyl)glycine | $mGlu_2$ glutamate metabotropic receptor antagonist<br>Jane et al., 1994 |
| 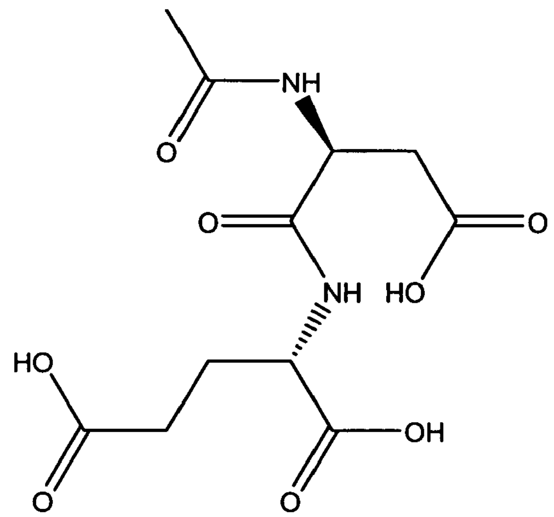<br>N-Acetyl-L-aspartyl-L-glutamic acid | Highly selective $mGlu_3$ glutamate metabotropic receptor agonist<br>Morris et al., 1965 |
| 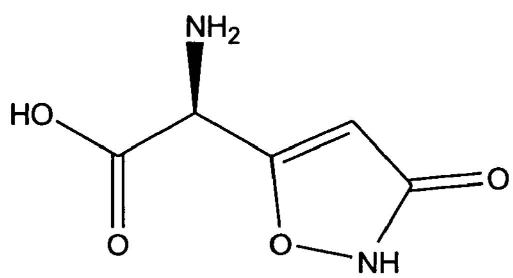<br>Ibotenic acid | Glutamate metabotropic receptor agonist<br>Johnston, 1968 |
| 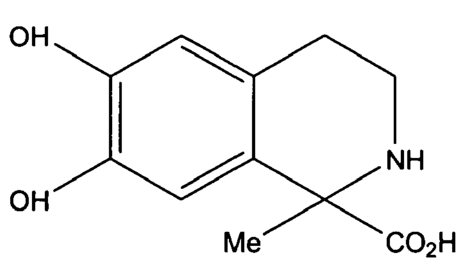<br>Salsolinol-1-carboxylic acid | Natural amino acid in the CNS affecting glutamate receptors<br>Siggins et al., 1982 |

(continued)

| | |
|---|---|
| 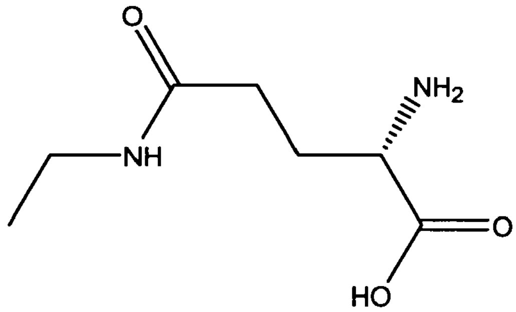 Theanine | Sugiyama and Sadzuka, 1998 |

## REFERENCES

ABCOUWER, S.F., LUKASCEWICZ, G.C., RYAN, U.S. & SOUBA, W.W. (1995). Molecular regulation of lung endothelial glutamine synthetase expression. Surgery, 118, 325-34.

ABCOUWER, S.F., LUKASZEWICZ, G.C. & SOUBA, W.W. (1996). Glucocorticoids regulate glutamine synthetase expression in lung epithelial cells. Am J Physiol, 270, L141-51.

ABELL, L.M., SCHINELLER, J., KECK, P.J. & VILLAFRANCA, J.J. (1995). Effect of metal-ligand mutations on phosphoryl transfer reactions catalyzed by Escherichia coli glutamine synthetase. Biochemistry, 34, 16695-702.

ABELL, L.M. & VILLAFRANCA, J.J. (1991). Investigation of the mechanism of phosphinothricin inactivation of Escherichia coli glutamine synthetase using rapid quench kinetic technique. Biochemistry, 30, 6135-41.

ALBRECHT, J. (2003). Glucose-derived osmolytes and energy impairment in brain edema accompanying liver failure: the role of glutamine reevaluated Gastroenterology, 125, 976-78.

ANAND, A., CHARNEY, D.S., OREN, D.A., BERMAN, R.M., Hu, X.S., CAPPIELLO, A. & KRYSTAL, J.H. (2000). Attenuation of the neuropsychiatric effects of ketamine with lamotrigine: support for hyperglutamatergic effects of N-methyl-D-aspartate receptor antagonists. Arch Gen Psychiatry, 57, 270-6.

ANANDARAJ, S.J., JAYARAM, H.N., COONEY, D.A., TYAGI, A.K., HAN, N., THOMAS, J.H., CHITNIS, M. & MONTGOMERY, J.A. (1980). Interaction of L-alanosine (NSC 153, 353) with enzymes metabolizing L-aspartic acid, L-glutamic acid and their amides. Biochem Pharmacol, 29, 227-45.

ARAD, G., FREIKOPF, A. & KULKA, R.G. (1976). Glutamine-stimulated modification and degradation of glutamine synthetase in hepatoma tissue culture cells. Cell, 8, 95-101.

ARAD, G. & KULKA, R.G. (1978). Effects of glutamine, methionine sulfone and dexamethasone on rates of synthesis of glutamine synthetase in cultured hepatoma cells. Biochim Biophys Acta, 544, 153-62.

ARDAWI, M.S. (1990) Glutamine-synthesizing activity in lungs of fed, starved, acidotic, diabetic, injured and septic rats. Biochem J, 270, 829-32.

ARDAWI, M.S. & MAJZOUB, M.F. (1991). Glutamine metabolism in skeletal muscle of septic rats. Metabolism, 40, 155-64.

ARDAWI, M.S. & NEWSHOLME, E.A. (1983). Glutamine metabolism in lymphocytes of the rat. Biochem J, 212, 835-42.

AULT, B. & HILDEBRAND, L.M. (1993). L-glutamate activates peripheral nociceptors. Agents Actions, 39 Spec No, C142-4.

BABIJ, P., HUNDAL, H.S., RENNIE, M.J. & WATT P.W. (1986). Effects of corticosteroids on glutamine transport in rat skeletal muscle. J Physiol Lond, 347, 35P.

LE BACQUER, O., NAZIH, H., BLOTTIERE, H., MEYNIAL-DENIS, D., LABOISSE, C. & DARMAUN, D. (2001). Effects of glutamine deprivation on protein synthesis in a model of human enterocytes in culture. Am J Physiol Gastrointest Liver Physiol, 281, G 1340-7.

BAINS, J.S. & SHAW, C.A. (1997). Neurodegenerative disorders in humans: the role of glutathione in oxidative stress-mediated neuronal death. Brain Res Brain Res Rev, 25, 335-58.

BARNETT, N.L., POW, D.V. & ROBINSON, S.R. (2000). Inhibition of Muller cell glutamine synthetase rapidly impairs the retinal response to light. Glia, 30, 64-73.

BAYER, E., GUGEL, K.H., HAGELE, K., HAGENMAIER, H., JESSIPOW, S., KONIG, W.A. & ZAHNER, H. (1972).

[Metabolic products of microorganisms. 98. Phosphinothricin and phosphinothricyl-alanyl-analine]. Helv Chim Acta, 55, 224-39.
BEAL, M.F. (1992). Mechanisms of excitotoxicity in neurologic diseases. FASEB J, 6, 3338-44.
BERTRAND, G., GROSS, R., PUECH, R., LOUBATIERES-MARIANI, M.M. & BOCKAERT, J. (1993). Glutamate stimulates glucagon secretion via an excitatory amino acid receptor of the AMPA subtype in rat pancreas. Eur J Pharmacol, 237, 45-50.
BEUTLER, E. (1989). Nutritional and metabolic aspects of glutathione. Annu Rev Nutr, 9, 287-302.
BHANGU, P.S., GENEVER, P.G., SPENCER, G.J., GREWAL, T.S. & SKERRY, T.M. (2001). Evidence for targeted vesicular glutamate exocytosis in osteoblasts. Bone, 29, 16-23.
BIRCH, M.A., GENEVER, P.G., LAKETIC-LJUBOJEVIC, I., PATTON, A.J., PEET, N.M. & SKERRY, T.M. (1997). Glutamate receptor activation is necessary for bone formation in vitro. J Bone Miner Res, 12, 010.
BJARNASON, I., MACPHERSON, A. & HOLLANDER, D. (1995). Intestinal permeability: an overview. Gastroenterology, 108, 1566-81.
BOELENS, P.G., HOUDIJK, A.P., DE THOUARS, H.N., TEERLINK, T., VAN ENGELAND, M.I., HAARMAN, H.J. & VAN LEEUWEN, P.A. (2003). Plasma taurine concentrations increase after enteral glutamine supplementation in trauma patients and stressed rats. Am J Clin Nutr, 77, 250-6.
BRECHLINA, P., UNTERHALT, A., TISCHNER, R. & MACK, G. (2000). Cytosolic and chloroplastic glutamine synthetase of sugarbeet (Beta vulgaris) respond differently to organ ontogeny and nitrogen source. Physiologia Plantarum, 108, 263-69.
BREMNER, J.D., KRYSTAL, J.H., SOUTHWICK, S.M. & CHARNEY, D.S. (1995). Functional neuroanatomical correlates of the effects of stress on memory. J Trauma Stress, 8, 527-53.
BRONSTEIN, J.M., FARBER, D.B. & WASTERLAIN, C.G. (1993). Regulation of type-II calmodulin kinase: functional implications. Brain Res Brain Res Rev, 18, 135-47.
BUSS, J.L., NEUZIL, J. & PONKA, P. (2004). Oxidative stress mediates toxicity of pyridoxal isonicotinoyl hydrazone analogs. Arch Biochem Biophys, 421, 1-9.
BUTTERWORTH, R.F. (1993). Portal-systemic encephalopathy: a disorder of neuron-astrocytic metabolic trafficking. Dev Neurosci, 15, 313-9.
CAMPBELL, P.N., WORK, T.S. & MELLANBY, E. (1950). Isolation of a crystalline toxic factor from agenized wheat flour. Nature (London), 165: 345-46.
CAO, Y., FENG, Z., HOOS, A. & KLIMBERG, V.S. (1998). Glutamine enhances gut glutathione production. JPENJParenter Enteral Nutr, 22, 224-7.
CARLTON, S.M., HARGETT, G.L. & COGGESHALL, R.E. (1995). Localization and activation of glutamate receptors in unmyelinated axons of rat glabrous skin. Neurosci Lett, 197, 25-8.
CASTANON, N., BLUTHE, R.M. & DANTZER, R. (2001). Chronic treatment with the atypical antidepressant tianeptine attenuates sickness behavior induced by peripheral but not central lipopolysaccharide and interleukin-1beta in the rat. Psychopharmacology (Berl), 154, 50-60.
CASTANON, N., KONSMAN, J.P., MEDINA, C., CHAUVET, N. & DANTZER, R. (2003). Chronic treatment with the antidepressant tianeptine attenuates lipopolysaccharide-induced Fos expression in the rat paraventricular nucleus and HPA axis activation. Psychoneuroendocrinology, 28, 19-34.
CASTILHO, R.F., KOWALTOWSKI, A.J., MEINICKE, A.R., BECHARA, E.J. & VERCESI, A.E. (1995). Permeabilization of the inner mitochondrial membrane by Ca2+ ions is stimulated by t-butyl hydroperoxide and mediated by reactive oxygen species generated by mitochondria. Free Radic Biol Med, 18, 479-86.
CHANDRASEKHAR, S., SOUBA, W.W. & ABCOUWER, S.F. (1999). Identification of glucocorticoid-responsive elements that control transcription of rat glutamine synthetase. Am J Physiol, 276, L319-31.
CHAUDHARI, N., LANDIN, A.M. & ROPER, S.D. (2000). A metabotropic glutamate receptor variant functions as a taste receptor. Nat Neurosci, 3, 113-9.
CHENU, C. (2002). Glutamatergic innervation in bone. Microsc Res Tech, 58, 70-6.
CHENU, C., SERRE, C.M., RAYNAL, C., BURT-PICHAT, B. & DELMAS, P.D. (1998). Glutamate receptors are expressed by bone cells and are involved in bone resorption. Bone, 22, 295-9.
CLARKE, V.R., BALLYK, B.A., HOO, K.H., MANDELZYS, A., PELLIZZARI, A., BATH, C.P., THOMAS, J., SHARPE, E.F., DAVIES, C.H., ORNSTEIN, P.L., SCHOEPP, D.D., KAMBOJ, R.K., COLLINGRIDGE, G.L., LODGE, D. & BLEAKMAN, D. (1997). A hippocampal GluR5 kainate receptor regulating inhibitory synaptic transmission. Nature, 389, 599-603.
COCK, J.M., BROCK, I.W., WATSON, A.T., SWARUP, R., MORBY, A.P. & CULLIMORE, J.V. (1991). Regulation of glutamine synthetase genes in leaves of Phaseolus vulgaris. Plant Mol Biol, 17, 761-71.
CONRAD, C.D., GALEA, L.A., KURODA, Y. & MCEWEN, B.S. (1996). Chronic stress impairs rat spatial memory on the Y maze, and this effect is blocked by tianeptine pretreatment. BehavNeurosci, 110, 1321-34.
CONVIT, A., DE LEON, M.J., TARSHISH, C., DE SANTI, S., TSUI, W., RUSINEK, H. & GEORGE, A. (1997).

Specific hippocampal volume reductions in individuals at risk for Alzheimer's disease. Neurobiol Aging, 18, 131-8.
COOPER, A.J., MORA, S.N., CRUZ, N.F. & GELBARD, A.S. (1985). Cerebral ammonia metabolism in hyperammonemic rats. J Neurochem, 44, 1716-23.
COOPER, A.J. & PLUM, F. (1987). Biochemistry and physiology of brain ammonia. Physiol Rev, 67, 440-519.
COSENTINO, M., DE PONTI, F., MARINO, F., GIARONI, C., LEONI, O., LECCHINI, S. & FRIGO, G. (1995). N-methyl-D-aspartate receptors modulate neurotransmitter release and peristalsis in the guinea pig isolated colon. Neurosci Lett, 183, 139-42.
CURI, R., NEWSHOLME, P., PITHON-CURI, T.C., PIRES-DE-MELO, M., GARCIA, C., HOMEM-DE-BITTENCOURT JUNIOR, P.I. & GUIMARAES, A.R. (1999). Metabolic fate of glutamine in lymphocytes, macrophages and neutrophils. Braz J Med Biol Res, 32, 15-21.
CUTHBERTSON, D.P. (1979). Second annual Jonathan E. Rhoads Lecture. The metabolic response to injury and its nutritional implications: retrospect and prospect. JPEN J Parenter Enteral Nutr, 3, 108-29.
DANTZER, R., BLUTHE, R.M., LAYE, S., BRET-DIBAT, J.L., PARNET, P. & KELLEY, K.W. (1998). Cytokines and sickness behavior. Ann N YAcad Sci, 840, 586-90.
DANTZER, R., WOLLMAN, E., VITKOVIC, L. & YIRMIYA, R. (1999). Cytokines and depression: fortuitous or causative association? Mol Psychiatry, 4, 328-32.
DARLING, H.F. (1959). Isocarboxazid (marplan) in ambulatory psychiatric patients. Am J Psychiatry, 116, 355-6.
DAVID, P., LUSKY, M. & TEICHBERG, V.I. (1988). Involvement of excitatory neurotransmitters in the damage produced in chick embryo retinas by anoxia and extracellular high potassium. Exp Eye Res, 46, 657-62.
DAWSON, V.L., DAWSON, T.M., BARTLEY, D.A., UHL G.R. & SNYDER SH. (1993). Mechanisms of nitric oxide-mediated neurotoxicity in primary brain cultures. J Neurosci, 13, 2651-61.
DAWSON, V.L., DAWSON, T.M., LONDON, E.D., BREDT, D.S. & SNYDER, S.H. (1991). Nitric oxide mediates glutamate neurotoxicity in primary cortical cultures. Proc Natl Acad Sci U S A, 88, 6368-71.
DE GRAAF, A.A., DEUTZ, N.E., BOSMAN, D.K., CHAMULEAU, R.A., DE HAAN, J.G. & BOVEE, W.M. (1991). The use of in vivo proton NMR to study the effects of hyperammonemia in the rat cerebral cortex. NMR Biomed, 4, 31-7.
DEITCH, E.A. (1992). Multiple organ failure. Pathophysiology and potential future therapy. Ann Surg, 216, 117-34.
DELBENDE, C., CONTESSE, V., MOCAER, E., KAMOUN, A. & VAUDRY, H. (1991). The novel antidepressant, tianeptine, reduces stress-evoked stimulation of the hypothalamo-pituitary-adrenal axis. Eur J Pharmacol, 202, 391-6.
DENG, A., VALDIVIELSO, J.M., MUNGER, K.A., BLANTZ, R.C. & THOMSON, S.C. (2002). Vasodilatory N-methyl-D-aspartate receptors are constitutively expressed in rat kidney. JAm Soc Nephrol, 13, 1381-4.
DICKMAN, K.G., YOUSSEF, J.G., MATHEW, S.M. & SAID, S.I. (2004). Ionotropic glutamate receptors in lungs and airways: molecular basis for glutamate toxicity. Am J Respir Cell Mol Biol, 30, 139-44.
DROGE, W., ECK, H.P., BETZLER, M., SCHLAG, P., DRINGS, P. & EBERT, W. (1988). Plasma glutamate concentration and lymphocyte activity. J Cancer Res Clin Oncol, 114, 124-8.
DUNFORD, J.E., THOMPSON, K., COXON, F.P., LUCKMAN, S.P., HAHN, F.M., POULTER, C.D., EBETINO, F.H. & ROGERS, M.J. (2001). Structure-activity relationships for inhibition of farnesyl diphosphate synthase in vitro and inhibition of bone resorption in vivo by nitrogen-containing bisphosphonates. J Pharmacol Exp Ther, 296, 235-4
EATON, S. (2003). Impaired energy metabolism during neonatal sepsis: the effects of glutamine. Proc Nutr Soc, 62, 745-51.
ECK, H.P., FREY, H. & DROGE, W. (1989). Elevated plasma glutamate concentrations in HIV-1-infected patients may contribute to loss of macrophage and lymphocyte functions. Int Immunol, 1, 367-72.
EDWARDS, J.W., WALKER, E.L. & CORUZZI, G.M. (1990). Cell-specific expression in transgenic plants reveals nonoverlapping roles for chloroplast and cytosolic glutamine synthetase. Proc Natl Acad Sci U S A, 87, 3459-63.
EID, T., THOMAS, M.J., SPENCER, D.D., RUNDEN-PRAN, E., LAI, J.C., MALTHANKAR, G.V., KIM, J.H., DANBOLT, N.C., OTTERSEN, O.P. & DE LANEROLLE, N.C. (2004). Loss of glutamine synthetase in the human epileptogenic hippocampus: possible mechanism for raised extracellular glutamate in mesial temporal lobe epilepsy. Lancet, 363, 28-37.
EISENBERG, D., GILL, H.S., PFLUEGL, G.M. & ROTSTEIN, S.H. (2000). Structure-function relationships of glutamine synthetases. Biochim Biophys Acta, 1477, 122-45.
EVANS, P.H. (1993). Free radicals in brain metabolism and pathology. Br Med Bull, 49, 577-87. EVSTIGNEEVA, Z.G., SOLOV'EVA, N.A. & SIDEL'NIKOVA, L.I. (2003). [Methionine sulfoximine and phosphinothricin--glutamine synthetase inhibitors and activators and their herbicidal activity (A review)]. Prikl Biokhim Mikrobiol, 39, 613-8.
FALDUTO, M.T., YOUNG, A.P. & HICKSON, R.C. (1992). Exercise inhibits glucocorticoid-induced glutamine synthetase expression in red skeletal muscles. Am J Physiol, 262, C214-20.
FAROOQUI, A.A., WELLS, K. & HORROCKS, L.A. (1995). Breakdown of membrane phospholipids in Alzheimer disease. Involvement of excitatory amino acid receptors. Mol Chem Neuropathol, 25, 155-73.
FARRINGTON, G.K., KUMAR, A. & WEDLER, F.C. (1987). Design and synthesis of phosphonates inhibitors of

glutamine synthetase. J Med Chem, 30, 2062-7.
FELIPO, V. & BUTTERWORTH, R.F. (2002a). Mitochondrial dysfunction in acute hyperammonemia. Neurochem Int, 40, 487-91.
FELIPO, V. & BUTTERWORTH, R.F. (2002b). Neurobiology of ammonia. Prog Neurobiol, 67, 259-79.
FENG, B., SHIBER, S.K. & MAX, S.R. (1990). Glutamine regulates glutamine synthetase expression in skeletal muscle cells in culture. J Cell Physiol, 145, 376-80.
FERGUS, A. & LEE, K.S. (1997). Regulation of cerebral microvessels by glutamatergic mechanisms. Brain Res, 754, 35-45.
FERRIER, B., CONJARD, A., MARTIN, M. & BAVEREL, G. (1999). Glutamine synthesis is heterogeneous and differentially regulated along the rabbit renal proximal tubule. Biochem J, 337 (Pt 3), 543-50.
FINCH, C.E. & COHEN, D.M. (1997). Aging, metabolism, and Alzheimer disease: review and hypotheses. Exp Neurol, 143, 82-102.
FIRSOVA, N.A., SELIVANOVA, K.M., ALEKSEEVA, L.V. & EVSTIGNEEVA, Z.G. (1986). [Inhibition of glutamine synthetase activity by biologically active derivatives of glutamic acid]. Biokhimiia, 51, 850-5.
FLARING, U.B., ROOYACKERS, O.E., WERNERMAN, J. & HAMMARQVIST, F. (2003). Glutamine attenuates post-traumatic glutathione depletion in human muscle. Clin Sci (Lond), 104, 275-82.
FOYER, C.H. & FERRARIO, S. (1994). Modulation of carbon and nitrogen metabolism in transgenic plants with a view to improved biomass production. Biochem Soc Trans, 22, 909-15.
FRANCONI, F., MICELI, M., DE MONTIS, M.G., CRISAFI, E.L., BENNARDINI, F. & TAGLIAMONTE, A. (1996). NMDA receptors play an anti-aggregating role in human platelets. Thromb Haemost, 76, 84-7.
FROST, H.M. (2002). Emerging views about "osteoporosis", bone health, strength, fragility, and their determinants. J Bone Miner Metab, 20, 319-25.
FUENTES, S.I., ALLEN, D.J., ORTIZ-LOPEZ, A. & HERNANDEZ, G. (2001). Over-expression of cytosolic glutamine synthetase increases photosynthesis and growth at low nitrogen concentrations. J Exp Bot, 52, 1071-81.
FURUKAWA, S., SAITO, H., INOUE, T., MATSUDA, T., FUKATSU, K., HAN, I., IKEDA, S. & HIDEMURA, A. (2000). Supplemental glutamine augments phagocytosis and reactive oxygen intermediate production by neutrophils and monocytes from postoperative patients in vitro. Nutrition, 16, 323-9.
FUZIWARA, S., INOUE, K. & DENDA, M. (2003). NMDA-type glutamate receptor is associated with cutaneous barrier homeostasis. J Invest Dermatol, 120, 1023-9.
GABBOTT, P.L. & SOMOGYI, J. (1984). The 'single' section Golgi-impregnation procedure: methodological description. J Neurosci Methods, 11, 221-30.
GALLARDO, F., FU, J., CANTON, F.R., GARCIA-GUTIERREZ, A., CANOVAS, F.M. & KIRBY, E.G. (1999). Expression of a conifer glutamine synthetase gene in transgenic poplar. Planta, 210, 19-26.
GARCIA, C., PITHON-CURI, T.C., DE LOURDES FIRMANO, M., PIRES DE MELO, M., NEWSHOLME, P. & CURI, R. (1999). Effects of adrenaline on glucose and glutamine metabolism and superoxide production by rat neutrophils. Clin Sci (Lond), 96, 549-55.
GARTHWAITE, G., HAJOS, F. & GARTHWAITE, J. (1986). Ionic requirements for neurotoxic effects of excitatory amino acid analogues in rat cerebellar slices. Neuroscience, 18, 437-47.
GASIC, G. P. & HOLLMANN, M. (1992). Molecular neurobiology of glutamate receptors. Ann Rev Physiol, 54, 507-36.
GENEVER, P.G., MAXFIELD, S.J., KENNOVIN, G.D., MALTMAN, J., BOWGEN, C.J., RAXWORTHY, M.J. & SKERRY, T.M. (1999a). Evidence for a novel glutamate-mediated signaling pathway in keratinocytes. J Invest Dermatol, 112, 337-42.
GENEVER, P.G., WILKINSON, D.J., PATTON, A.J., PEET, N.M., HONG,Y., MATHUR, A., ERUSALIMSKY, J.D. & SKERRY, T.M. (1999b). Expression of a functional N-methyl-D-aspartate-type glutamate receptor by bone marrow megakaryocytes. Blood, 93, 2876-83.
GENEVER, P.G. & SKERRY, T.M. (2001). Regulation of spontaneous glutamate release activity in osteoblastic cells and its role in differentiation and survival: evidence for intrinsic glutamatergic signaling in bone. Faseb J, 15, 1586-8.
GIARONI, C., DE PONTI, F., COSENTINO, M., LECCHINI, S. & FRIGO, G. (1999). Plasticity in the enteric nervous system. Gastroenterology, 117, 1438-58.
GIARONI, C., ZANETTI, E., CHIARAVALLI, A.M., ALBARELLO, L., DOMINIONI, L., CAPELLA, C., LECCHINI, S. & FRIGO, G. (2003). Evidence for a glutamatergic modulation of the cholinergic function in the human enteric nervous system via NMDA receptors. Eur J Pharmacol, 476, 63-9.
GILL, S.S., PULIDO, O.M., MUELLER, R.W. & McGUIRE, P.F. (1998). Molecular and immunochemical characterization of the ionotropic glutamate receptors in the rat heart. Brain Res Bull, 46, 429-34.
GILL, H.S. & EISENBERG, D. (2001). The crystal structure of phosphinothricin in the active site of glutamine synthetase illuminates the mechanism of enzymatic inhibition. Biochemistry, 40, 1903-12.
GLEVAREC, G., BOUTON, S., JASPARD, E., RIOU, M.T., CLIQUET, J.B., SUZUKI, A. & LIMAMI, A.M. (2004).

Respective roles of the glutamine synthetase/glutamate synthase cycle and glutamate dehydrogenase in ammonium and amino acid metabolism during germination and post-germinative growth in the model legume Medicago truncatula. Planta, 219, 286-97.

GONOI, T., MIZUNO, N., INAGAKI, N., KUROMI, H., SEINO, Y., MIYAZAKI, J. & SEINO, S. (1994). Functional neuronal ionotropic glutamate receptors are expressed in the non-neuronal cell line MIN6. J Biol Chem, 269, 16989-92.

GONZALEZ-CADAVID, N.F., RYNDIN, I., VERNET, D., MAGEE, T.R. & RAJFER, J. (2000). Presence of NMDA receptor subunits in the male lower urogenital tract. J Androl, 21, 566-78.

GOROVITS, R., AVIDAN, N., AVISAR, N., SHAKED, I. & VARDIMON, L. (1997). Glutamine synthetase protects against neuronal degeneration in injured retinal tissue. Proc Natl Acad Sci U S A, 94, 7024-9.

GOVITRAPONG, P., EBADI, M. & MURRIN, L.C. (1986). Identification of a Cl-/Ca2+-dependent glutamate (quisqualate) binding site in bovine pineal organ. J Pineal Res, 3, 223-34.

GRIFFITH, O.W., ANDERSON, M.E. & MEISTER, A. (1979). Inhibition of glutathione biosynthesis by prothionine sulfoximine (S-n-propyl homocysteine sulfoximine), a selective inhibitor of gamma-glutamylcysteine synthetase. J Biol Chem, 254, 1205-10.

GURVITS, T.V., SHENTON, M.E., HOKAMA, H., OHTA, H., LASKO, N.B., GILBERTSON, M.W., ORR, S.P., KIKINIS, R., JOLESZ, F.A., MCCARLEY, R.W. & PITMAN, R.K. (1996). Magnetic resonance imaging study of hippocampal volume in chronic, combat-related posttraumatic stress disorder. Biol Psychiatry, 40, 1091-9.

HACK, R., EBERT, E., EHLING, G. & LEIST, K.H. (1994). Glufosinate ammonium--some aspects of its mode of action in mammals. Food Chem Toxicol, 32, 461-70.

HALESTRAP, A.P., WOODFIELD, K.Y. & CONNERN, C.P. (1997). Oxidative stress, thiol reagents, and membrane potential modulate the mitochondrial permeability transition by affecting nucleotide binding to the adenine nucleotide translocase. J Biol Chem, 272, 3346-54.

HARDER, D.R., ALKAYED, N.J., LANGE, A.R., GEBREMEDHIN, D. & ROMAN, R.J. (1998). Functional hyperemia in the brain: hypothesis for astrocyte-derived vasodilator metabolites. Stroke, 29, 229-34.

HAUSSINGER, D., SIES, H. & GEROK, W. (1985). Functional hepatocyte heterogeneity in ammonia metabolism. The intercellular glutamine cycle. J Hepatol, 1, 3-14.

HAWKES, N.D., THOMAS, G.A., JUREWICZ, A., WILLIAMS, O.M., HILLIER, C.E., MCQUEEN, I.N. & SHORTLAND, G. (2001). Non-hepatic hyperammonaemia:an important,potentially reversible cause of encephalopathy. Postgard Med J, 77, 316-7.

HAWKINS, R.A., JESSY, J., MANS, A.M. & DE JOSEPH, M.R. (1993). Effect of reducing brain glutamine synthesis on metabolic symptoms of hepatic encephalopathy. J Neurochem, 60, 1000-6.

HAYASHI, M., YAMADA, H., UEHARA, S., MORIMOTO, R., MUROYAMA, A., YATSUSHIRO, S., TAKEDA, J., YAMAMOTO, A. & MORIYAMA, Y. (2003). Secretory granule-mediated co-secretion of L-glutamate and glucagon triggers glutamatergic signal transmission in islets of Langerhans. J Biol Chem, 278, 1966-74.

HERTZ, L. (1979). Functional interactions between neurons and astrocytes I. Turnover and metabolism of putative amino acid transmitters. Prog Neurobiol, 13, 277-323.

HERTZ, L., DRINGEN, R., SCHOUSBOE, A. & ROBINSON, S.R. (1999). Astrocytes: glutamate producers for neurons. J Neurosci Res, 57, 417-28.

HERTZ, L. & ZIELKE, H.R. (2004). Astrocytic control of glutamatergic activity: astrocytes as stars of the show. Trends Neurosci, 27, 735-43.

HICKSON, R.C., WEGRZYN, L.E., OSBORNE, D.F. & KARL, I.E. (1996). Glutamine interferes with glucocorticoid-induced expression of glutamine synthetase in skeletal muscle. Am J Physiol, 270, E912-7.

HIGUCHI, H., ADACHI, M., MIURA, S., GORES, G.J. & ISHII, H. (2001). The mitochondrial permeability transition contributes to acute ethanol-induced apoptosis in rat hepatocytes. Hepatology, 34, 320-8.

HINOI, E., FUJIMORI, S., NAKAMURA, Y., BALCAR, V.J., KUBO, K., OGITA, K. & YONEDA, Y. (2002c) Constitutive expression of heterologous N-methyl-D-aspartate receptor subunits in rat adrenal medulla. J Neurosci Res, 68, 36-45.

HINOI E, FUJIMORI S, TAKARADA T, TANIURA H & YONEDA Y (2002a). Facilitation of glutamate release by ionotropic glutamate receptors in osteoblasts. Biochem Biophys Res Commun, 297, 452-8.

HINOI, E., FUJIMORI, S. & YONEDA, Y. (2003). Modulation of cellular differentiation by N-methyl-D-aspartate receptors in osteoblasts. FASEB J, 17, 1532-4.

HINOI, E., FUJIMORI, S., YONEYAMA, M. & YONEDA, Y. (2002b). Blockade by N-methyl-D-aspartate of elevation of activator protein-1 binding after stress in rat adrenal gland. J Neurosci Res, 70, 161-71.

HINOI, E., TAKARADA, T., UESHIMA, T., TSUCHIHASHI, Y. & YONEDA, Y. (2004). Glutamate signaling in peripheral tissues. Eur J Biochem, 271, 1-13.

HINOI, E. & YONEDA, Y. (2001). Expression of GluR6/7 subunits of kainate receptors in rat adenohypophysis. Neurochem Int, 38, 539-47.

HITCHCOCK, I.S., SKERRY, T.M., HOWARD, M.R. & GENEVER, P.G. (2003). NMDA receptor-mediated regulation of human megakaryocytopoiesis. Blood, 102, 1254-9.
HOLLMANN, M. & HEINEMANN, S. (1994). Cloned glutamate receptors. Annu Rev Neurosci, 17, 31-108.
HONG, R.W., ROUNDS, J.D., HELTON, W.S., ROBINSON, M.K. & WILMORE, D.W. (1992). Glutamine preserves liver glutathione after lethal hepatic injury. Ann Surg, 215, 114-9.
HOUDIJK, A.P., NIJVELDT, R.J. & VAN LEEUWEN, P.A. (1999). Glutamine-enriched enteral feeding in trauma patients: reduced infectious morbidity is not related to changes in endocrine and metabolic responses. JPEN J Parenter Enteral Nutr, 23, S52-8.
HOUDIJK, A.P., RIJNSBURGER, E.R., JANSEN, J., WESDORP, R.I., WEISS, J.K., MCCAMISH, M.A., TEERLINK, T., MEUWISSEN, S.G., HAARMAN, H.J., THIJS, L.G. & VAN LEEUWEN, P.A. (1998). Randomised trial of glutamine-enriched enteral nutrition on infectious morbidity in patients with multiple trauma. Lancet, 352, 772-6.
HOURANI, B.T., HAMLIN, E.M. & REYNOLDS, T.B. (1971). Cerebrospinal fluid glutamine as a measure of hepatic encephalopathy. Arch Intern Med, 127, 1033-6.
HOYER, S. (1994). Possible role of ammonia in the brain in dementia of Alzheimer type. Adv Exp Med Biol, 368, 197-205.
HREBICEK, J. & KOLOUSEK, J. (1968). Preparoxysmal changes of spontaneous and evoked electrical activity of the cat brain after administration of methionine sulphoximine. Epilepsia, 9, 145-62.
HUNDAL, H.S., BABIJ, P., WATT, P.W., WARD, M.R. & RENNIE, M.J. (1990). Glutamine transport and metabolism in denervated rat skeletal muscle. Am J Physiol, 259, E148-54.
INAGAKI, N., KUROMI, H., GONOI, T., OKAMOTO, Y., ISHIDA, H., SEINO, Y., KANEKO, T., IWANAGA, T. & SEINO, S. (1995). Expression and role of ionotropic glutamate receptors in pancreatic islet cells. Faseb J, 9, 686-91.
IRVING, A.J., SCHOFIELD, J.G., WATKINS, J.C., SUNTER, D.C. & COLLINGRIDGE, G.L. (1990). 1S,3R-ACPD stimulates and L-AP3 blocks Ca2+ mobilization in rat cerebellar neurons. Eur J Pharmacol, 186, 363-5.
ISAACKS, R.E., BENDER, A.S., KIM, C.Y., SHI, Y.F. & NORENBERG, M.D. (1999). Effect of ammonia and methionine sulfoximine on myo-inositol transport in cultured astrocytes. Neurochem Res, 24, 51-9.
ISHIYAMA, K., INOUE, E., TABUCHI, M., YAMAYA, T. & TAKAHASHI, H. (2004). Biochemical background and compartmentalized functions of cytosolic glutamine synthetase for active ammonium assimilation in rice roots. Plant Cell Physiol, 45, 1640-7.
ISRAELI, E., BERENSHTEIN, E., WENGROWER, D., APTEKAR, L., KOHEN, R., ZAJICEK, G. & GOLDIN, E. (2004). Prophylactic administration of topical glutamine enhances the capability of the rat colon to resist inflammatory damage. Dig Dis Sci, 49, 1705-12.
ITO, K., YAMAMOTO, K. & KAWANISHI, S. (1992). Manganese-mediated oxidative damage of cellular and isolated DNA by isoniazid and related hydrazines: non-Fenton-type hydroxyl radical formation. Biochemistry, 31, 11606-13.
IZUMI, Y., BENZ, A.M., CLIFFORD, D.B. & ZORUMSKI, C.F. (1992). Nitric oxide inhibitors attenuate N-methyl-D-aspartate excitotoxicity in rat hippocampal slices. Neurosci Lett, 135, 227-30.
JACKSON, B., GEE, A.N., GUYON-GELLIN, Y., NIESOR, E., BENTZEN, C.L., KERNS, W.D. & SUCKLING, K.E. (2000). Hypocholesterolaemic and antiatherosclerotic effects of tetra-iso-propyl 2-(3,5-di-tert-butyl-4-hydroxyphenyl)ethyl-1,1-diphosphonate (SR-9223i). Arzneimittelforschung, 50, 380-6.
JANE, D.E., JONES, P.L., POOK, P.C., TSE, H.W. & WATKINS, J.C. (1994). Actions of two new antagonists showing selectivity for different sub-types of metabotropic glutamate receptor in the neonatal rat spinal cord. Br J Pharmacol, 112, 809-16.
JAYAKUMAR, A.R., RAMA RAO, K.V., SCHOUSBOE, A. & NORENBERG, M.D. (2004). Glutamine-induced free radical production in cultured astrocytes. Glia, 46, 296-301.
JOHNSON, C.R., BOETTCHER, B.R., CHERPECK, R.E., & DOLSON, M.G. (1990). Design and synthesis of potent inhibitors of glutamine synthetase: 1. Cyclic analogs of phosphonothricin. Bioorg Chem, 18, 154-9.
JOHNSTON, G.A. (1968). The intraspinal distribution of some depressant amino acids. J Neurochem, 15, 1013-7.
JONES, E.A. & WEISSENBORN, K. (1997). Neurology and the liver. J Neurol Neurosurg Psychiatry, 63, 279-93.
JOU, M.J., JOU, S.B., CHEN, H.M., LIN, C.H. & PENG, T.I. (2002). Critical role of mitochondrial reactive oxygen species formation in visible laser irradiation-induced apoptosis in rat brain astrocytes (RBA-1). J Biomed Sci, 9, 507-16.
KAFARSKI, P., LEJCZAK, B. & FORLANI, G. (2000). Herbicidally active aminomethylenebisphophonic acids. Heteroatom Chemistry, 11, 449-53.
KAFARSKI, P. & LEJCZAK, B. (2001). Aminophosphonic acids of potential medical importance. Curr Med Chem Anti-Canc Agents, 1, 301-12.
KANAMORI, K., BLUML, S. & ROSS, B. (1997). Magnetic resonance spectroscopy in the study of hyperammonemia and hepatic encephalopathy. Adv Exp Med Biol, 420, 185-94.
KARINCH, A.M., PAN, M., LIN, C.M., STRANGE, R. & SOUBA, W.W. (2001). Glutamine metabolism in sepsis and infection. J Nutr, 131, 2535S-8S.

KING, D.S. (1952). Tuberculosis. N Engl J Med, 247, 718-25.
KIRCHGESSNER, A.L. (2001). Glutamate in the enteric nervous system. Curr Opin Pharmacol, 1, 591-6.
KIRCHGESSNER, A.L., LIU, M.T. & ALCANTARA, F. (1997). Excitotoxicity in the enteric nervous system. J Neurosci, 17, 8804-16.
KIYAMA, H., SATO, K. & TOHYAMA, M. (1993). Characteristic localization of non-NMDA type glutamate receptor subunits in the rat pituitary gland. Brain Res Mol Brain Res, 19, 262-8.
KOLLEGGER, H., McBEAN, G.J. & TIPTON, K.F. (1991). The inhibition of glutamine synthetase in rat corpus striatum in vitro by methionine sulfoximine increases the neurotoxic effects of kainate and N-methyl-D-aspartate. Neurosci Lett, 130, 95-8.
KOSENKO, E., LLANSOLA, M., MONTOLIU, C., MONFORT, P., RODRIGO, R., HERNANDEZ-VIADEL, M., ERCEG, S., SANCHEZ-PEREZ, A.M. & FELIPO, V. (2003). Glutamine synthetase activity and glutamine content in brain: modulation by NMDA receptors and nitric oxide. Neurochem Int, 43, 493-9.
KOSOWER, N.S. & KOSOWER, E.M. (1978). The glutathione status of cells. Int Rev Cytol, 54, 109-60.
KOSTANYAN, I.A., MERKULOVA, M.I., NAVOLOTSKAYA, E.V. & NURIEVA, R.I. (1997). Study of interaction between L-glutamate and human blood lymphocytes. Immunol Lett, 58, 177-80.
KOYAMA, I., NAKAMURA, T., OGASAWARA, M., NEMOTO, M. & YOSHIDA, T. (1992). The protective effect of taurine on the biomembrane against damage produced by the oxygen radical. Adv Exp Med Biol, 315, 355-9.
KOZIKOWSKI, A.P., TUCKMANTEL, W., LIAO, Y., MANEV, H., IKONOMOVIC, S. & WROBLEWSKI, J.T. (1993). Synthesis and metabotropic receptor activity of the novel rigidified glutamate analogues (+)- and (-)-trans-azetidine-2,4-dicarboxylic acid and their N-methyl derivatives. J Med Chem, 36, 2706-8.
KRISTENSEN, P. (1993). Differential expression of AMPA glutamate receptor mRNAs in the rat adrenal gland. FEBS Lett, 332, 14-8.
KRIZBAI, I.A., DELI, M.A., PESTENACZ, A., SIKLOS, L., SZABO, C.A., ANDRAS, I. & JOO, F. (1998). Expression of glutamate receptors on cultured cerebral endothelial cells. J Neurosci Res, 54, 814-9.
KVAMME, E. (1998). Synthesis of glutamate and its regulation. Prog Brain Res, 116, 73-85.
LABOW, B.I., ABCOUWER, S.F., LIN, C.M. & SOUBA, W.W. (1998). Glutamine synthetase expression in rat lung is regulated by protein stability. Am J Physiol, 275, L877-86.
LABOW, B. I., SOUBA, W.W. & ABCOUWER, S.F. (1999). Glutamine synthetase expression in muscle is regulated by transcriptional and posttranscriptional mechanisms. Am J Physiol, 276, E1136-45.
LABOW, B.I., SOUBA, W.W. & ABCOUWER, S.F. (2001). Mechanisms governing the expression of the enzymes of glutamine metabolism--glutaminase and glutamine synthetase. J Nutr, 131, 2467S-74S.
LACOSTE, L., CHAUDHARY, K.D. & LAPOINTE, J. (1982). Derepression of the glutamine synthetase in neuroblastoma cells at low concentrations of glutamine. J Neurochem, 39, 78-85.
LAM, H.M., COSCHIGANO, K., SCHULTZ, C., MELO-OLIVEIRA, R., TJADEN, G., OLIVEIRA, I., NGAI, N., HSIEH, M.H. & CORUZZI, G. (1995). Use of Arabidopsis mutants and genes to study amide amino acid biosynthesis. Plant Cell, 7, 887-98.
LAM, H.M., COSCHIGANO, K.T., OLIVEIRA, I.C., MELO-OLIVEIRA, R. & CORUZZI, G.M. (1996). The Molecular-Genetics of Nitrogen Assimilation into Amino Acids in Higher Plants. Annu Rev Plant Physiol Plant Mol Biol, 47, 569-93.
LANGSTON-UNKEFER, P.J., ROBINSON, A.C., KNIGHT, T.J. & DURBIN, R.D. (1987). Inactivation of pea seed glutamine synthetase by the toxin, tabtoxinine-beta-lactam. J Biol Chem, 262, 1608-13.
LANYON, L. & SKERRY, T. (2001). Postmenopausal osteoporosis as a failure of bone's adaptation to functional loading: a hypothesis. J Bone Miner Res, 16, 1937-47.
LAPOUBLE, E., MONTECOT, C., SEVESTRE, A. & PICHON, J. (2002). Phosphinothricin induces epileptic activity via nitric oxide production through NMDA receptor activation in adult mice. Brain Res, 957, 46-52.
LARA, M., CULLIMORE, J.V., LEA, P.J., MIFLIN, B.J., JOHNSTON, A.W.B. & LAMB J.W. (1983). Appearance of a novel form of plant glutamine synthetase during nodule development in Phaseolus vulgaris. L. Planta, 157, 254-258.
LEA, P.J. & RIDDLEY S.M. (1989). Glutamine synthetase and its inhibition. In: Herbicides and plant metabolism (Seminar series / Society for Experimental Biology; 38). AD Dodge (ed) Cambridge University Press, Cambridge, UK, 137-70.
LEA, P.J. (1997). Primary nitrogen metabolism. In: Plant biochemistry. PM Dey, JB Harborne (eds) Academic Press, London, UK, 273-306.
LECHIN, F., VAN DER DIJS, B., OROZCO, B., JARA, H., RADA, I., LECHIN, M.E. & LECHIN, A.E. (1998). Neuropharmacologic treatment of bronchial asthma with the antidepressant tianeptine: a double-blind, crossover placebo-controlled study. Clin Pharmacol Ther, 64, 223-32.
LEE, S.K., MBWAMBO, Z.H., CHUNG, H., LUYENGI, L., GAMEZ, E.J., MEHTA, R.G., KINGHORN, A.D. & PEZZUTO, J.M. (1998). Evaluation of the antioxidant potential of natural products. Comb Chem High Throughput Screen, 1, 35-46.

LEJCZAK, B., STARZEMSKA, H. & MASTALERZ, P. (1981). Inhibition of rat liver glutamine synthetase by phosphonic analogues of glutamic acid. Experientia, 37, 461-2.
LEONARD, J.V. & MORRIS, A.A. (2002). Urea cycle disorders. Semin Neonatol, 7, 27-35.
LEUNG, J.C., TRAVIS, B.R., VERLANDER, J.W., SANDHU, S.K., YANG, S.G., ZEA, A.H., WEINER, I.D. & SILVERSTEIN, D.M. (2002). Expression and developmental regulation of the NMDA receptor subunits in the kidney and cardiovascular system. Am J Physiol Regul Integr Comp Physiol, 283, R964-71.
LI, N., LEWIS, P., SAMUELSON, D., LIBONI, K. & NEU, J. (2004) Glutamine regulates Caco-2 cell tight junction proteins Am J Physiol Gastrointest Liver Physiol, 287, G726-33.
LIMAMI, A.M., PHILLIPSON, B., AMEZIANE, R., PERNOLLET, N., JIANG, Q., ROY, R., DELEENS, E., CHAUMONT-BONNET, M., GRESSHOFF, P.M. & HIREL, B. (1999). Does root glutamine synthetase control plant biomass production in Lotus japonicus L.? Planta, 209, 495-502.
LIMAMI, A.M., ROUILLON, C., GLEVAREC, G., GALLAIS, A. & HIREL, B. (2002). Genetic and physiological analysis of germination efficiency in maize in relation to nitrogen metabolism reveals the importance of cytosolic glutamine synthetase. Plant Physiology, 130, 1860-70.
LIPTON, S.A. & GENDELMAN, H.E. (1995). Seminars in medicine of the Beth Israel Hospital, Boston. Dementia associated with the acquired immunodeficiency syndrome. N Engl J Med, 332, 934-40.
LIPTON, S.A. & ROSENBERG, P.A. (1994). Excitatory amino acids as a final common pathway for neurologic disorders. N Engl J Med, 330, 613-22.
LIU, H.P., TAY, S.S.W. & LEONG S.K. (1997b). Localization of glutamate receptor subunits of the $\alpha$-amino-3-hydroxy-5-methyl-D-aspartate (AMPA) type in the pancreas of newborn guinea pigs. Pancreas, 14, 360-68.
LIU, M.T., ROTHSTEIN, J.D., GERSHON, M.D. & KIRCHGESSNER, A.L. (1997a). Glutamatergic enteric neurons. J Neurosci, 17, 4764-84.
LODIN, Z. (1958). [Analysis of changes of the central nervous system induced by MSI. J. Cesk Fysiol, 7, 122-8.
LODIN, Z. & KOLOUSEK, J. (1956). [Epileptic symptoms induced by methionine sulfoximine. J. Cesk Neurol, 19, 83-9.
LOGUSCH, E.W. (1988). A simple preparation of S-alkyl homocysteine derivatives: S-phosphonomethyl homocysteines as inhibitors of glutamine synthetase. Tetrahedron Lett, 29, 6055-58.
LOGUSCH, E.W., WALKER, D.M., McDONALD, J.F. & FRANZ, J.E. (1988). Synthesis of alpha and gamma alkyl-substituted phosphinothricin: potent new inhibitors of glutamine synthetase. J Org Chem., 53, 4069-4074.
LOGUSCH, E.W., WALKER, D.M., MCDONALD, J.F. & FRANZ, J.E. (1989). Substrate variability as a factor in enzyme inhibitor design: inhibition of ovine brain glutamine synthetase by alpha- and gamma-substituted phosphinothricins. Biochemistry, 28, 3043-51.
LOGUSCH, E.W., WALKER, D.M., MCDONALD, J.F., FRANZ, J.E., VILLAFRANCA, J.J., DIIANNI, C.L., COLANDUONI, J.A., LI, B. & SCHINELLER, J.B. (1990). Inhibition of Escherichia coli glutamine synthetase by alpha- and gamma-substituted phosphinothricins. Biochemistry, 29, 366-72.
LOMBARDI, G., DIANZANI, C., MIGLIO, G., CANONICO, P.L. & FANTOZZI, R. (2001). Characterization of ionotropic glutamate receptors in human lymphocytes. Br J Pharmacol, 133, 936-44.
LOUZADA-JUNIOR, P., JR., DIAS, J. J., SANTOS, W. F., LACHAT, J. J., BRADFORD, H. F. & COUTINHO NETTO, J. (1992) Glutamate release in experimental ischaemia of the retina: an approach using microdialysis. J. Neurochem. 59, 358-363.
LUINE (1992). Stress, Serotonin and Tianeptine. Symposium at the 8th World Congress of Psychiatry. Athens. Br J Psychiatry Suppl, 6-75.
LUKASZEWICZ, G.C., SOUBA, W.W. & ABCOUWER, S.F. (1997). Induction of muscle glutamine synthetase gene expression during endotoxemia is adrenal gland dependent. Shock, 7, 332-8.
MAINOUS, M.R., BLOCK, E.F. & DEITCH, E.A. (1994). Nutritional support of the gut: how and why. New Horiz, 2, 193-201.
MAGARINOS, A.M. & MCEWEN, B.S. (1995). Stress-induced atrophy of apical dendrites of hippocampal CA3c neurons: involvement of glucocorticoid secretion and excitatory amino acid receptors. Neuroscience, 69, 89-98.
MANHART, N., VIERLINGER, K., SPITTLER, A., BERGMEISTER, H., SAUTNER, T. & ROTH, E. (2001). Oral feeding with glutamine prevents lymphocyte and glutathione depletion of Peyer's patches in endotoxemic mice. Ann Surg, 234, 92-7.
MARCINKIEWICZ, J., GRABOWSKA, A., BERETA, J. & STELMASZYNSKA, T. (1995). Taurine chloramine, a product of activated neutrophils, inhibits in vitro the generation of nitric oxide and other macrophage inflammatory mediators. JLeukoc Biol, 58, 667-74.
MASON, D.J., SUVA, L.J., GENEVER, P.G., PATTON, A.J., STEUCKLE, S., HILLAM, R.A. & SKERRY, T.M. (1997). Mechanically regulated expression of a neural glutamate transporter in bone: a role for excitatory amino acids as osteotropic agents? Bone, 20, 199-205.
MASSEY, S.C. & MILLER, R.F. (1990). N-methyl-D-aspartate receptors of ganglion cells in rabbit retina. J Neurophysiol, 63, 16-30.
MATSUMURA, N., TAKEUCHI, C., HISHIKAWA, K., FUJII, T. & NAKAKI, T. (2001). Glufosinate ammonium induces convulsion through N-methyl-D-aspartate receptors in mice. Neurosci Lett, 304, 123-5.
MCCAULEY, R., KONG, S.E. & HALL, J. (1998). Glutamine and nucleotide metabolism within enterocytes. JPEN J Parenter Enteral Nutr, 22, 105-11.
McGRATH-MORROW, S.A. & STAHL, J. (2002). Inhibition of glutamine synthetase in a549 cells during hyperoxia. Am JRespir Cell Mol Biol, 27, 99-106.
MCNEARNEY, T., SPEEGLE, D., LAWAND, N., LISSE, J. & WESTLUND, K.N. (2000). Excitatory amino acid profiles of synovial fluid from patients with arthritis. J Rheumatol, 27, 739-45.
MCROBERTS, J.A., COUTINHO, S.V., MARVIZON, J.C., GRADY, E.F., TOGNETTO, M., SENGUPTA, J.N., ENNES, H.S., CHABAN, V.V., AMADESI, S., CREMINON, C., LANTHORN, T., GEPPETTI, P., BUNNETT, N.W. & MAYER, E.A. (2001). Role of peripheral N-methyl-D-aspartate (NMDA) receptors in visceral nociception in rats. Gastroenterology, 120, 1737-48.
MEISTER, A. (1985). Glutamine synthetase from mammalian tissues. Methods Enzymol, 113, 185-99.
MEISTER, A. (1991). Glutathione deficiency produced by inhibition of its synthesis, and its reversal; applications in research and therapy. Pharmacol Ther, 51, 155-94.
MEISTER, A. & TATE, S.S. (1976). Glutathione and related gamma-glutamyl compounds: biosynthesis and utilization. Annu Rev Biochem, 45, 559-604.
MELIS, G.C., TER WENGEL, N., BOELENS, P.G. & VAN LEEUWEN, P.A. (2004). Glutamine: recent developments in research on the clinical significance of glutamine. Curr Opin Clin Nutr Metab Care, 7, 59-70.
MENG, W., TOBIN, J.R. & BUSIJA, D.W. (1995). Glutamate-induced cerebral vasodilation is mediated by nitric oxide through N-methyl-D-aspartate receptors. Stroke, 26, 857-62.
MERLE, B., ITZSTEIN, C., DELMAS, P.D. & CHENU, C. (2003). NMDA glutamate receptors are expressed by osteoclast precursors and involved in the regulation of osteoclastogenesis. J Cell Biochem, 90, 424-36.
MEWETT, K.N., OAKES, D.J., OLVERMAN, H.J., SMITH, D.A. & WATKINS, J.C. (1983). Pharmacology of the excitatory actions of sulphonic and sulphinic amino acids. Adv Biochem Psychopharmacol, 37, 163-74.
MIFLIN, B.J. & HABASH, D.Z. (2002). The role of glutamine synthetase and glutamate dehydrogenase in nitrogen assimilation and possibilities for improvement in the nitrogen utilization of crops. J Exp Bot, 53, 979-87.
MIFLIN B.J. & LEA P.J. (1980). Ammonia assimilation. In: The biochemistry of plants. Vol. 5. Amino acids and their derivatives. BJ Miflin (ed) Academic Press, New York, USA,169-202.
MIGGE, A., CARRAYOL, E., HIREL, B. & BECKER, T.W. (2000). Leaf-specific overexpression of plastidic glutamine synthetase stimulates the growth of transgenic tobacco seedlings. Planta, 210, 252-60.
MILLER, A.L. (1999). Therapeutic considerations of L-glutamine: a review of the literature. Altern Med Rev, 4, 239-48.
MILMAN, G., PORTNOFF, L.S. & TIEMEIER, D.C. (1975). Immunochemical evidence for glutamine-mediated degradation of glutamine synthetase in cultured Chinese hamster cells. J Biol Chem, 250, 1393-9.
MONAGHAN, D.T., BRIDGES, R.J. & COTMAN, C.W. (1989). The excitatory amino acid receptors: their classes, pharmacology, and distinct properties in the function of the central nervous system. Annu Rev Pharmacol Toxicol, 29, 365-402.
MORALES-PIGA, A. (1999). Tiludronate. A new treatment for an old ailment: Paget's disease of bone. Expert Opin Pharmacother, 1, 157-70.
MORRIS, D. & STRAUGHAN, D.W. (1965). The interaction of alpha- and beta -L-aspartyl-L-glutamic acid and of their N-acetyl derivatives on central neurones and certain isolated peripheral tissues. Biochem Pharmacol, 14, 1679-81.
MOSINGER, J.L., PRICE, M.T., BAI H.Y., XIAO, H., WOZNIAK, D.F. & OLNEY, J.W. (1991). Blockade of both NMDA and non-NMDA receptors is required for optimal protection against ischemic neuronal degeneration in the in vivo adult mammalian retina. Exp Neurol, 113, 10-7.
MUHLBAUER, R.C., BAUSS, F., SCHENK, R., JANNER, M., BOSIES, E., STREIN, K. & FLEISCH, H. (1991). BM 21.0955, a potent new bisphosphonate to inhibit bone resorption. J Bone Miner Res, 6, 1003-11.
MURTHY, C.R., RAMA RAO, K.V., BAI, G. & NORENBERG, M.D. (2001). Ammonia-induced production of free radicals in primary cultures of rat astrocytes. J Neurosci Res, 66, 282-8.
NAGATA, Y., HOMMA, H., LEE, J.A. & IMAI, K. (1999). D-Aspartate stimulation of testosterone synthesis in rat Leydig cells. FEBS Lett, 444, 160-4.
NAKAKI, T., MISHIMA, A., SUZUKI, E., SHINTANI, F. & FUJII, T. (2000). Glufosinate ammonium stimulates nitric oxide production through N-methyl D-aspartate receptors in rat cerebellum. Neurosci Lett, 290, 209-12.
NEAL, M.J., CUNNINGHAM, J.R., HUTSON, P.H. & HOGG, J. (1994). Effects of ischaemia on neurotransmitter release from the isolated retina. J Neurochem, 62, 1025-33.
NEU, J., DEMARCO, V. & LI, N. (2002). Glutamine: clinical applications and mechanisms of action. Curr Opin Clin Nutr Metab Care, 5, 69-75.

NEVILLE-WEBBE, H.L., HOLEN, I. & COLEMAN, R.E. (2002). The anti-tumour activity of bisphosphonates. Cancer Treat Rev, 28, 305-19.

NEWSHOLME, P. (2001). Why is L-glutamine metabolism important to cells of the immune system in health, postinjury, surgery or infection? J Nutr, 131, 2515S-22S.

NORENBERG, M.D. (1981). The astrocyte in liver disease. Adv Cell Neurobiol, 2: 303-51.

NORENBERG, M.D. (1986). Hepatic encephalopathy: a disorder of astrocytes. In Astrocytes. S Fedoroff, A Vemadakis (eds) Academic Press, New York, USA, 425-460.

NORENBERG, M.D. (1987). The role of astrocytes in hepatic encephalopathy. Neurochem Pathol, 6, 13-33.

NORENBERG, M.D. & BENDER, A.S. (1994). Astrocyte swelling in liver failure: role of glutamine and benzodiazepines. Acta Neurochir Suppl (Wien), 60, 24-7.

NORENBERG, M.D. & MARTINEZ-HERNANDEZ, A. (1979). Fine structural localization of glutamine synthetase in astrocytes of rat brain. Brain Res, 161, 303-10.

NORENBERG, M.D., NEARY, J.T., BENDER, A.S. & DOMBRO, R.S. (1992). Hepatic encephalopathy: a disorder in glial-neuronal communication. Prog Brain Res, 94, 261-9.

NORENBERG, M.D., RAMA RAO, K.V. & JAYAKUMAR, A.R. (2004). Ammonia neurotoxicity and the mitochondrial permeability transition. J Bioenerg Biomembr, 36, 303-7.

OBOJSKA, A., BERLICKI, L., KAFARSKI, P., LEJCZAK, B., CHICCA, M. & FORLANI, G. (2004). Herbicidal pyridyl derivatives of aminomethylene-bisphosphonic acid inhibit plant glutamine synthetase. J Agric Food Chem, 52, 3337-44.

OBRENOVITCH, T.P. & URENJAK, J. (1997). Altered glutamatergic transmission in neurological disorders: from high extracellular glutamate to excessive synaptic efficacy. Prog Neurobiol, 51, 39-87.

OBRENOVITCH, T.P., URENJAK, J., ZILKHA, E. & JAY, T.M. (2000). Excitotoxicity in neurological disorders--the glutamate paradox. Int J Dev Neurosci, 18, 281-7.

OHTAKE, T., YASUDA, H., TAKAHASHI, H., GOTO, T., SUZUKI, K., YONEMURA, K. & HISHIDA, A. (2001). Decreased plasma and cerebrospinal fluid glutamine concentrations in a patient with bialaphos poisoning. Hum Exp Toxicol, 20, 429-34.

OLIVEIRA, I.C., BREARS, T., KNIGHT T.J., CLARK, A. & CORUZZI, G.M. (2002). Overexpression of cytosolic glutamine synthetase. Relation to nitrogen, light, and photorespiration. Plant Physiology, 129, 1170-80.

OLKKU, A., BODINE, P.V., LINNALA-KANKKUNEN, A. & MAHONEN, A. (2004). Glucocorticoids induce glutamine synthetase expression in human osteoblastic cells: a novel observation in bone. Bone, 34, 320-9.

OLNEY, J.W. (1990). Excitotoxin-mediated neuron death in youth and old age. Prog Brain Res, 86, 37-51.

OLVERMAN, H.J. & SHARMAN, D.F. (1983). Dopamine in the blood platelets of the sheep. Comp Biochem Physiol C, 75, 223-5.

OMURA, S., MURATA, M., IMAMURA, N., IWAI, Y., TANAKA, H., FURUSAKI, A. & MATSUMOTO, H. (1984). Oxetin, a new antimetabolite from an actinomycete. Fermentation, isolation, structure and biological activity. J Antibiot (Tokyo), 37, 1324-32.

ORNSTEIN, P.L., BLEISCH, T.J., ARNOLD, M.B., KENNEDY, J.H., WRIGHT, R.A., JOHNSON, B.G., TIZZANO, J.P., HELTON, D.R., KALLMAN, M.J., SCHOEPP, D.D. & HERIN, M. (1998). 2-substituted (2SR)-2-amino-2-((1SR, 2SR)-2-carboxycycloprop-1-yl)glycines as potent and selective antagonists of group II metabotropic glutamate receptors. 2. Effects of aromatic substitution, pharmacological characterization, and bioavailability. J Med Chem, 41, 358-78.

ORREGO, F. & VILLANUEVA, S. (1993). The chemical nature of the main central excitatory transmitter: a critical appraisal based upon release studies and synaptic vesicle localization. Neuroscience, 56, 539-55.

ORZECHOWSKI , O.P., BRODNICKA A, WILCZAK J, JANK M, BALASINSKA B, GRZELKOWSKA K, PLOSZAJ T, OLCZAK J, MROWCZYNSKA A. (2000). Excess of glucocorticoids impairs whole-body antioxidant status in young rats. relation to the effect of dexamethasone in soleus muscle and spleen. Horm Metab Res, 32, 174-80.

PATEL, A.J., HUNT, A. & FARAJI-SHADAN, F. (1986). Effect of removal of glutamine and addition of dexamethasone on the activities of glutamine synthetase, ornithine decarboxylase and lactate dehydrogenase in primary cultures of forebrain and cerebellar astrocytes. Brain Res, 391, 229-38.

PATTON, A.J., GENEVER, P.G., BIRCH, M.A., SUVA, L.J. & SKERRY, T.M. (1998). Expression of an N-methyl-D-aspartate-type receptor by human and rat osteoblasts and osteoclasts suggests a novel glutamate signaling pathway in bone. Bone, 22, 645-9.

PAULSEN, R.E., ODDEN, E. & FONNUM, F. (1988). Importance of glutamine for gamma-aminobutyric acid synthesis in rat neostriatum in vivo. J Neurochem, 51, 1294-9.

PEET, N.M., GRABOWSKI, P.S., LAKETIC-LJUBOJEVIC, I. & SKERRY, T.M. (1999). The glutamate receptor antagonist MK801 modulates bone resorption in vitro by a mechanism predominantly involving osteoclast differentiation. Faseb J, 13, 2179-85.

PELLICCIARI, R., LUNEIA, R., COSTANTINO, G., MARINOZZI, M., NATALINI, B., JAKOBSEN, P., KANSTRUP,

A., LOMBARDI, G., MORONI, F. & THOMSEN, C. (1995). 1-Aminoindan-1,5-dicarboxylic acid: a novel antagonist at phospholipase C-linked metabotropic glutamate receptors. J Med Chem, 38, 3717-9.
PITHON-CURI, T.C., SCHUMACHER, R.I., FREITAS, J.J., LAGRANHA, C., NEWSHOLME, P., PALANCH, A.C., DOI, S.Q. & CURI, R. (2003). Glutamine delays spontaneous apoptosis in neutrophils. Am J Physiol Cell Physiol, 284, C 1355-61.
PLAISANT, F., DOMMERGUES, M.A., SPEDDING, M., CECCHELLI, R., BRILLAULT, J., KATO, G., MUNOZ, C. & GRESSENS, P. (2003). Neuroprotective properties of tianeptine: interactions with cytokines. Neuropharmacology, 44, 801-9.
PLANAS, M., SCHWARTZ, S., ARBOS, M.A. & FARRIOL, M. (1993). Plasma glutamine levels in septic patients. JPEN J Parenter Enteral Nutr, 17, 299-300.
POTSIC, B., HOLLIDAY, N., LEWIS, P., SAMUELSON, D., DEMARCO, V. & NEU, J. (2002). Glutamine supplementation and deprivation: effect on artificially reared rat small intestinal morphology. Pediatr Res, 52, 430-6.
PUSHKIN, A.V., EVSTIGNEEVA, Z.G. & KRETOVICH, V.L. (1974). Fiziol. Rast. (Moscow), 21, 512-7.
PUSHKIN, A.V., EVSTIGNEEVA, Z.G. & KRETOVICH, V.L. (1975). Biokhimiya (Moscow), 40, 53-6.
RABINOVITZ, M., OLSON, M.E. & GREENBERG, D.M. (1957). 8-Hydroxylysine: an inhibitor of glutamine and protein synthesis by the Ehrlich ascites carcinoma cell. Cancer Res, 17, 885-9.
RAO, K.V. & NORENBERG, M.D. (2004). Manganese induces the mitochondrial permeability transition in cultured astrocytes. J Biol Chem, 279, 32333-8.
RAO, K.V., JAYAKUMAR, A.R. & NORENBERG, M.D. (2003). Induction of the mitochondrial permeability transition in cultured astrocytes by glutamine. Neurochemistry International, 43, 517-23.
RAO, S.L. & MEISTER, A. (1972). In vivo formation of methionine sulfoximine phosphate, a protein-bound metabolite of methionine sulfoximine. Biochemistry, 11, 1123-7.
RASTOGI, R., CHOUREY, P.S. & MUHITCH, M.J. (1998). The maize glutamine synthetase GS1-2 gene is preferentially expressed in kernel pedicels and is developmentally-regulated. Plant Cell Physiol, 39, 443-6.
REAGAN, L.P., ROSELL, D.R., WOOD, G.E., SPEDDING, M., MUNOZ, C., ROTHSTEIN, J. & MCEWEN, B.S. (2004). Chronic restraint stress up-regulates GLT-1 mRNA and protein expression in the rat hippocampus: reversal by tianeptine. Proc Natl Acad Sci U S A, 101, 2179-84.
REIS, H.J., MASSENSINI, A.R., PRADO, M.A., GOMEZ, R.S., GOMEZ, M.V. & ROMANO-SILVA, M.A. (2000). Calcium channels coupled to depolarization-evoked glutamate release in the myenteric plexus of guinea-pig ileum. Neuroscience, 101, 237-42.
REITER, R.J. (1995). Oxidative processes and antioxidative defense mechanisms in the aging brain. Faseb J, 9, 526-33.
REITSMA, P.H., FRIJLINK, W.B. & BIJVOET, O.L. (1980). Dichloromethylene diphosphonate and calcium and phosphate metabolism. N Engl J Med, 303, 162-3.
ROH, J.H., SUZUKI, H., AZAKAMI, H., YAMASHITA, M., MUROOKA, Y. & KUMAGAI H. (1994). Purification, characterization, and crystallization of monoamine oxidase from Escherichia coli K-12. Biosci Biotechnol Biochem, 58, 1652-6.
RHOADS, J.M., ARGENZIO, R.A., CHEN, W. & GOMEZ, G.G. (1995). Asparagine stimulates piglet intestinal Cl-secretion by a mechanism requiring a submucosal glutamate receptor and nitric oxide. J Pharmacol Exp Ther, 274, 404-12.
RICHIE, J.P., JR., MILLS, B.J. & LANG, C.A. (1987). Correction of a glutathione deficiency in the aging mosquito increases its longevity. Proc Soc Exp Biol Med, 184, 113-7.
ROBINSON, M.K., AHN, M.S., ROUNDS, J.D., COOK, J.A., JACOBS, D.O. & WILMORE, D.W. (1992). Parenteral glutathione monoester enhances tissue antioxidant stores. JPEN J Parenter Enteral Nutr, 16, 413-8.
ROSS, B.D., DANIELSEN, E.R. & BLUML, S. (1996). Proton magnetic resonance spectroscopy: the new gold standard for diagnosis of clinical and subclinical hepatic encephalopathy? Dig Dis, 14 Suppl 1, 30-9.
ROTH, E., OEHLER, R., MANHART, N., EXNER, R., WESSNER, B., STRASSER, E. & SPITTLER, A. (2002). Regulative potential of glutamine--relation to glutathione metabolism. Nutrition, 18, 217-21.
ROTHWELL, N., ALLAN, S. & TOULMOND, S. (1997). The role of interleukin 1 in acute neurodegeneration and stroke: pathophysiological and therapeutic implications. J Clin Invest, 100, 2648-52.
ROWE, W.B., RONZIO, R.A. & MEISTER, A. (1969). Inhibition of glutamine synthetase by methionine sulfoximine. Studies on methionine sulfoximine phosphate. Biochemistry, 8, 2674-80.
RUEPPEL, M.L., LUNDT, S.L., GASS, J.D. & MEISTER, A. (1972). Specific synthesis of 1-(5-glutamyl)-2-methylhydrazine by glutamine synthetase. Biochemistry, 11, 2839-44.
RUHLAND, M., ENGELHARDT, G. & PAWLIZKI, K. (2002). A comparative investigation of the metabolism of the herbicide glufosinate in cell cultures of transgenic glufosinate-resistant and non-transgenic oilseed rape (Brassica napus) and corn (Zea mays). Environ Biosafety Res, 1, 29-37.
SADUNISHVILI, T., GVARLIANI, N., NUTSUBIDZE, N. & KVESITADZE, G. (1996). Effect of methionine sulfoximine

on nitrogen metabolism and externally supplied ammonium assimilation in kidney bean. Ecotoxicol Environ Saf, 34, 70-5.
SAID, S.I., BERISHA, H.I. & PAKBAZ, H. (1996). Excitotoxicity in the lung: N-methyl-D-aspartate-induced, nitric oxide-dependent, pulmonary edema is attenuated by vasoactive intestinal peptide and by inhibitors of poly(ADP-ribose) polymerase. Proc Natl Acad Sci USA, 93, 4688-92.
SAID, S. I., PAKBAZ, H., BERISHA, H. I., RAZA, S. AND FODA, H. D. (2000). NMDA receptor activation: critical role in oxidant tissue injury. Free Radic Biol Med, 28, 1300-2.
SANCHEZ-MATEO, C.C., DARIAS, V., ALBERTOS, L.M. & EXPOSITO-ORTA, M.A. (2003). Psychopharmacological effects of tianeptine analogous hetero[2,1] benzothiazepine derivatives. Arzneimittelforschung, 53, 12-20.
SARIN, V.K., KENT, S.B., TAM, J.P. & MERRIFIELD, R.B. (1981). Quantitative monitoring of solid-phase peptide synthesis by the ninhydrin reaction. Anal Biochem, 117, 147-57.
SATO, K., KIYAMA, H., SHIMADA, S. & TOHYAMA, M. (1993). Gene expression of KA type and NMDA receptors and of a glycine transporter in the rat pineal gland. Neuroendocrinology, 58, 77-9.
SCHELTINGA, M.R., YOUNG, L.S., BENFELL, K., BYE, R.L., ZIEGLER, T.R., SANTOS, A.A., ANTIN, J.H., SCHLOERB, P.R. & WILMORE, D.W. (1991). Glutamine-enriched intravenous feedings attenuate extracellular fluid expansion after a standard stress. Ann Surg, 214, 385-93.
SCHEPPACH, W., BINGHAM, S., BOUTRON-RUAULT, M.C., GERHARDSSON DE VERDIER, M., MORENO, V., NAGENGAST, F.M., REIFEN, R., RIBOLI, E., SEITZ, H.K. & WAHRENDORF, J. (1999). WHO consensus statement on the role of nutrition in colorectal cancer. Eur J Cancer Prev, 8, 57-62.
SCHEPPACH, W., LOGES, C., BARTRAM, P., CHRISTL, S.U., RICHTER, F., DUSEL, G., STEHLE, P., FUERST, P. & KASPER, H. (1994). Effect of free glutamine and alanyl-glutamine dipeptide on mucosal proliferation of the human ileum and colon. Gastroenterology, 107, 429-34.
SCHOEPP, D.D., GOLDSWORTHY, J., JOHNSON, B.G., SALHOFF, C.R. & BAKER, S.R. (1994). 3,5-dihydroxyphenylglycine is a highly selective agonist for phosphoinositide-linked metabotropic glutamate receptors in the rat hippocampus. J Neurochem, 63, 769-72.
SEILER, N. (1993). Is ammonia a pathogenetic factor in Alzheimer's disease? Neurochem Res, 18, 235-45.
SEKIYAMA, N., HAYASHI, Y., NAKANISHI, S., JANE, D.E., TSE, H.W., BIRSE, E.F. & WATKINS, J.C. (1996). Structure-activity relationships of new agonists and antagonists of different metabotropic glutamate receptor subtypes. Br J Pharmacol, 117, 1493-503.
SHAKED, I., BEN-DROR, I. & VARDIMON, L. (2002). Glutamine synthetase enhances the clearance of extracellular glutamate by the neural retina. J Neurochem, 83, 574-80.
SHANNON, H.E. & SAWYER, B.D. (1989). Glutamate receptors of the N-methyl-D-aspartate subtype in the myenteric plexus of the guinea pig ileum. J Pharmacol Exp Ther, 251, 518-23.
SHAW, C.A., BAINS, J.S., PASQUALOTTO, B.A. & CURRY, K. (1999). Methionine sulfoximine shows excitotoxic actions in rat cortical slices. Can J Physiol Pharmacol, 77, 871-7.
SHELDRICK, W.F. (1987). World Nitrogen Survey. Technical paper no. 59, World Bank, Washington, DC.
SHELINE, Y.I., WANG, P.W., GADO, M.H., CSERNANSKY, J.G. & VANNIER, M.W. (1996). Hippocampal atrophy in recurrent major depression. Proc Natl Acad Sci U S A, 93, 3908-13.
SHIGEMOTO, R., OHISHI, H., NAKANISHI, S. & MIZUNO, N. (1992). Expression of the mRNA for the rat NMDA receptor (NMDAR1) in the sensory and autonomic ganglion neurons. Neurosci Lett, 144, 229-32.
SHINOZAKI, Y., TOBITA, T., MIZUTANI, M. & MATSUZAKI, T. (1996). Isolation and identification of two new diterpene glycosides from Nicotiana tabacum. Biosci Biotechnol Biochem, 60, 903-5.
SHOHAMI, E., GATI, I., BEIT-YANNAI, E., TREMBOVLER, V. & KOHEN, R. (1999). Closed head injury in the rat induces whole body oxidative stress: overall reducing antioxidant profile. J Neurotrauma, 16, 365-76.
SIBSON, N.R., DHANKHAR, A., MASON, G.F., BEHAR, K.L., ROTHMAN, D.L. & SHULMAN, R.G. (1997). In vivo 13C NMR measurements of cerebral glutamine synthesis as evidence for glutamate-glutamine cycling. Proc Natl Acad Sci U S A, 94, 2699-704.
SIGGINS, G.R., BERGER, T., FRENCH, E.D., SHIER, T. & BLOOM, F.E. (1982). Ethanol, salsolinol and tetrahydropapaveroline alter the discharge of neurons in several brain regions: comparison to opioid effects. Prog Clin Biol Res, 90, 275-87.
SINSKY, M. & DONNERER, J. (1998). Evidence for a neurotransmitter role of glutamate in guinea pig myenteric plexus neurons. Neurosci Lett, 258, 109-12.
SKERRY, T.M. & GENEVER, P.G. (2001). Glutamate signalling in non-neuronal tissues. Trends Pharmacol Sci, 22, 174-81.
SLUKA, K.A., JORDAN, H.H., WILLIS, W.D. & WESTLUND, K.N. (1994). Differential effects of N-methyl-D-aspartate (NMDA) and non-NMDA receptor antagonists on spinal release of amino acids after development of acute arthritis in rats. Brain Res, 664, 77-84.
SNINSKY, C. A., BROODERSON, R. J., BROOME, T. A. & BERGERON, R.J. (1994) Gastroenterology, 106, A569

(abstr).
SPENCER, G.J. & GENEVER, P.G. (2003). Long-term potentiation in bone: a role for glutamate in strain-induced cellular memory? BMC Cell Biol, 4, 9.
SPENCER, G.J., HITCHCOCK, I.S. & GENEVER, P.G. (2004). Emerging neuroskeletal signalling pathways: a review. FEBS Lett, 559, 6-12.
SPITTLER, A., SAUTNER, T., GORNIKIEWICZ, A., MANHART, N., OEHLER, R., BERGMANN, M., FUGGER, R. & ROTH, E. (2001). Postoperative glycyl-glutamine infusion reduces immunosuppression: partial prevention of the surgery induced decrease in HLA-DR expression on monocytes. Clin Nutr, 20, 37-42.
STITT, M., MULLER, C., MATT, P., GIBON, Y., CARILLO, P., MORCUENDE, R., SCHEIBLE, W.R. & KRAPP, A. (2002). Steps towards an integrated view of nitrogen metabolism. J Exp Bot, 53, 959-70.
STORTO, M., DE GRAZIA, U., BATTAGLIA, G., FELLI, M.P., MARODER, M., GULINO, A., RAGONA, G., NICOLETTI, F., SCREPANTI, I., FRATI, L. & CALOGERO, A. (2000a). Expression of metabotropic glutamate receptors in murine thymocytes and thymic stromal cells. J Neuroimmunol, 109, 112-20.
STORTO, M., DE GRAZIA, U., KNOPFEL, T., CANONICO, P.L., COPANI, A., RICHELMI, P., NICOLETTI, F. & VAIRETTI, M. (2000b). Selective blockade of mGlu5 metabotropic glutamate receptors protects rat hepatocytes against hypoxic damage. Hepatology, 31, 649-55.
STORTO, M., SALLESE, M., SALVATORE, L., POULET, R., CONDORELLI, D.F., DELL'ALBANI, P., MARCELLO, M.F., ROMEO, R., PIOMBONI, P., BARONE, N., NICOLETTI, F. & DE BLASI, A. (1999). Expression of metabotropic glutamate receptors in the rat and human testis. FEBS Letters, 444, 160-4.
STORTO, M., SALLESE, M., SALVATORE, L., POULET, R., CONDORELLI, D.F., DELL'ALBANI, P., MARCELLO, M.F., ROMEO, R., PIOMBONI, P., BARONE, N., NICOLETTI, F. & DE BLASI, A. (2001). Expression of metabotropic glutamate receptors in the rat and human testis. J Endocrinol, 170, 71-8.
SUGIYAMA, T. & SADZUKA, Y. (1998). Enhancing effects of green tea components on the antitumor activity of adriamycin against M5076 ovarian sarcoma. Cancer Lett, 133, 19-26.
SWANSON, R.A., SHIRAISHI, K., MORTON, M.T. & SHARP, F.R. (1990). Methionine sulfoximine reduces cortical infarct size in rats after middle cerebral artery occlusion. Stroke, 21, 322-7.
TAKEUCHI, M., SAKAMOTO, S., KAWAMUKI, K., KURIHARA, H., NAKAHARA, H. & ISOMURA, Y. (1998). Studies on novel bone resorption inhibitors. II. Synthesis and pharmacological activities of fused aza-heteroarylbisphosphonate derivatives. Chem Pharm Bull (Tokyo), 46, 1703-9.
TAKAHASHI, H., KOEHLER, R.C., BRUSILOW, S.W. & TRAYSTMAN, R.J. (1991). Inhibition of brain glutamine accumulation prevents cerebral edema in hyperammonemic rats. Am J Physiol, 261, H825-9.
TAKAHASHI, H., KOEHLER, R.C., HIRATA, T., BRUSILOW, S.W. & TRAYSTMAN, R.J. (1992). Restoration of cerebrovascular CO2 responsivity by glutamine synthesis inhibition in hyperammonemic rats. Circ Res, 71, 1220-30.
TEITELBAUM, J.S., ZATORRE, R.J., CARPENTER, S., GENDRON, D., EVANS, A.C., GJEDDE, A. & CASHMAN, N.R. (1990). Neurologic sequelae of domoic acid intoxication due to the ingestion of contaminated mussels. N Engl J Med, 322, 1781-7.
THOMSEN, C. & SUZDAK, P.D. (1993). 4-Carboxy-3-hydroxyphenylglycine, an antagonist at type I metabotropic glutamate receptors. Eur J Pharmacol, 245, 299-301.
TOBIN, A.K. & YAMAYA, T. (2001). Cellular compartmentation of ammonium assimilation in rice and barley. J Exp Bot, 52, 591-604.
TONG, Q., MA, J. & KIRCHGESSNER, A.L. (2001). Vesicular glutamate transporter 2 in the brain-gut axis. Neuroreport, 12, 3929-34.
TONG, Q., OUEDRAOGO, R. & KIRCHGESSNER, A.L. (2002). Localization and function of group III metabotropic glutamate receptors in rat pancreatic islets. Am J Physiol Endocrinol Metab, 282, E1324-33.
TSACOPOULOS, M., POITRY-YAMATE, C.L., POITRY, S., PERROTTET, P. & VEUTHEY, A.L. (1997). The nutritive function of glia is regulated by signals released by neurons. Glia, 21, 84-91.
TUMANI, H., SMIRNOV, A., BARCHFELD, S., OLGEMOLLER, U., MAIER, K., LANGE, P., BRUCK, W. & NAU, R. (2000). Inhibition of glutamine synthetase in rabbit pneumococcal meningitis is associated with neuronal apoptosis in the dentate gyrus. Glia, 30, 11-8.
VARNEY, M.A., COSFORD, N.D., JACHEC, C., RAO, S.P., SACAAN, A., LIN, F.F., BLEICHER, L., SANTORI, E.M., FLOR, P.J., ALLGEIER, H., GASPARINI, F., KUHN, R., HESS, S.D., VELICELEBI, G. & JOHNSON, E.C. (1999). SIB-1757 and SIB-1893: selective, noncompetitive antagonists of metabotropic glutamate receptor type 5. J Pharmacol Exp Ther, 290, 170-81.
VITOUSEK, P.W., ABER, J.D., HOWARTH, R.W., LIKENS, G.E., MATSON, P.A., SCHINDLER, D.W., SCHLESINGER, W.H. & TILMAN, D.G. (1997). Human alteration of the global nitrogen cycle: sources and consequences. Ecol Appl, 7, 737-50.
VOGELS, B.A., VAN STEYNEN, B., MAAS, M.A., JORNING, G.G. & CHAMULEAU, R.A. (1997). The effects of ammonia and portal-systemic shunting on brain metabolism, neurotransmission and intracranial hypertension in

hyperammonaemia-induced encephalopathy. J Hepatol, 26, 387-95.
WADA, J.A., IKEDA, H. & BERRY, K. (1967). Reversible behavioral and electrographic manifestations induced by methionine sulfoximine. Neurology, 17, 854-68.
WALKER, D.M., McDONALD, J.F. & LOGUSCH E.W. (1987). J Chem Soc Chem Commun, 1710-11.
WATANABE, M., MISHINA, M. & INOUE, Y. (1994). Distinct gene expression of the N-methyl-D-aspartate receptor channel subunit in peripheral neurons of the mouse sensory ganglia and adrenal gland. Neurosci Lett, 165, 183-6.
WATANABE, Y., GOULD, E., DANIELS, D.C., CAMERON, H. & MCEWEN, B.S. (1992). Tianeptine attenuates stress-induced morphological changes in the hippocampus. Eur J Pharmacol, 222, 157-62.
WEAVER, C.D., YAO, T.L., POWERS, A.C. & VERDOORN, T.A. (1996). Differential expression of glutamate receptor subtypes in rat pancreatic islets. J Biol Chem, 271, 12977-84.
WEAVER, C.D., PARTRIDGE, J.G., YAO, T.L., MOATES, M., MAGNUSON, M.A. & VERDOORN, T.A. (1998). Activation of glycine and glutamate receptors increases intracellular calcium in cells derived from the endocrine pancreas. Mol Pharmacol, 54, 639-46.
WEDLER, F.C., HORN, B.R. & ROBY, W.G. (1980). Interaction of a new gamma-glutamyl-phosphate analog, 4-(phosphonoacetyl)-L-alpha-aminobutyrate, with glutamine synthetase enzymes from Escherichia coli, plant, and mammalian sources. Arch Biochem Biophys, 202, 482-90.
WEISS, M.D., DEMARCO, V., STRAUSS, D.M., SAMUELSON, D.A., LANE, M.E. & NEU, J. (1999). Glutamine synthetase: a key enzyme for intestinal epithelial differentiation? JPEN J Parenter Enteral Nutr, 23, 140-6.
WELBOURNE, T.C. & DASS, P.D. (1982). Function of renal gamma-glutamyltransferase: significance of glutathione and glutamine interactions. Life Sci, 30, 793-801.
WERMUTH, C.G., MANN, A., SCHOENFELDER, A., WRIGHT, R.A., JOHNSON, B.G., BURNETT, J.P., MAYNE, N.G. & SCHOEPP, D.D. (1996). (2S,4S)-2-amino-4-(4,4-diphenylbut-1-yl)- pentane-1,5-dioic acid: a potent and selective antagonist for metabotropic glutamate receptors negatively linked to adenylate cyclase. J Med Chem, 39, 814-6.
WHITE, F.P., DUTTON, G.R. & NORENBERG, M.D. (1981). Microvessels isolated from rat brain: localization of astrocyte processes by immunohistochemical techniques. J Neurochem, 36, 328-32.
WILEY, J.W., LU, Y.X. & OWYANG, C. (1991). Evidence for a glutamatergic neural pathway in the myenteric plexus. Am JPhysiol, 261, G693-700.
WILLARD-MACK, C.L., KOEHLER, R.C., HIRATA, T., CORK, L.C., TAKAHASHI, H., TRAYSTMAN, R.J. & BRUSILOW, S.W. (1996). Inhibition of glutamine synthetase reduces ammonia-induced astrocyte swelling in rat. Neuroscience, 71, 589-99.
WILMORE, D.W. (2001). The effect of glutamine supplementation in patients following elective surgery and accidental injury. J Nutr, 131, 2543S-9S.
WIREN, M., MAGNUSSON, K.E. & LARSSON, J. (1998). The role of glutamine, serum and energy factors in growth of enterocyte-like cell lines. Int J Biochem Cell Biol, 30, 1331-6.
WRONSKI, T.J., YEN, C.F. & SCOTT, K.S. (1991). Estrogen and diphosphonate treatment provide long-term protection against osteopenia in ovariectomized rats. J Bone Miner Res, 6, 387-94.
YAMADA, H., YATSUSHIRO, S., ISHIO, S., HAYASHI, M., NISHI, T., YAMAMOTO, A., FUTAI, M., YAMAGUCHI, A. & MORIYAMA, Y. (1998). Metabotropic glutamate receptors negatively regulate melatonin synthesis in rat pinealocytes. J Neurosci, 18, 2056-62.
YATSUSHIRO, S., YAMADA, H., HAYASHI, M., YAMAMOTO, A. & MORIYAMA, Y. (2000). Ionotropic glutamate receptors trigger microvesicle-mediated exocytosis of L-glutamate in rat pinealocytes. J Neurochem, 75, 288-97.
YONEDA, Y. & OGITA, K. (1986). Localization of [3H]glutamate binding sites in rat adrenal medulla. Brain Res, 383, 387-91.
YOSHIDA, Y., HIGASHI, T., NOUSO, K., NAKATSUKASA, H., NAKAMURA, S.I., WATANABE, A. & TSUJI, T. (2001). Effects of zinc deficiency/zinc supplementation on ammonia metabolism in patients with decompensated liver cirrhosis. Acta Med Okayama, 55, 349-55.
YOUNG, V.R. & AJAMI, A.M. (2001). Glutamine: the emperor or his clothes? J Nutr, 131, 2449S-59S.
ZALKIN, H. & SMITH, J.L. (1998). Enzymes utilizing glutamine as an amide donor. Adv Enzymol Relat Areas Mol Biol, 72, 87-144.
ZEEVALK, G.D., HYNDMAN, A.G. & NICKLAS, W.J. (1989). Excitatory amino acid-induced toxicity in chick retina: amino acid release, histology, and effects of chloride channel blockers. JNeurochem, 53, 1610-9.
ZEEVALK, G.D. & NICKLAS, W.J. (1990). Chemically induced hypoglycemia and anoxia: relationship to glutamate receptor-mediated toxicity in retina. J Pharmacol Exp Ther, 253, 1285-92.
ZELLER, E.A. & BARSKY, J. (1952). In vivo inhibition of liver and brain monoamine oxidase by 1-Isonicotinyl-2-isopropyl hydrazine. Proc Soc Exp Biol Med, 81, 459-61.
ZHLOBA, A.F., RABOTNOVA, V.F. & KIRILLOVA, M.S. (1968). [On the quantitative determination of ammonia in biological fluids under clinical conditions]. Lab Delo, 10, 621-2.

ZIEGLER, T.R., BAZARGAN, N., LEADER, L.M. & MARTINDALE, R.G. (2000). Glutamine and the gastrointestinal tract. Curr Opin Clin Nutr Metab Care, 3, 355-62.
ZIEGLER, T.R., EVANS, M.E., FERNANDEZ-ESTIVARIZ, C. & JONES, D.P. (2003). Trophic and cytoprotective nutrition for intestinal adaptation, mucosal repair, and barrier function. Annu Rev Nutr, 23, 229-61.
ZWINGMANN, C., BRAND, A., RICHTER-LANDSBERG, C. & LEIBFRITZ, D. (1998). Multinuclear NMR spectroscopy studies on NH4Cl-induced metabolic alterations and detoxification processes in primary astrocytes and glioma cells. Dev Neurosci, 20, 417-26.
ZWINGMANN, C., SHOKATI, T., BUTTERWORTH, R.F. & LEIBFRITZ D (2003). Use of NMR spectroscopy for the study of ammonia metabolism in astrocytes and neurons: Role of glutamine synthesis in astrocytes. In: Encephalopathy and Nitrogen Metabolism in Liver Failure. EA Jones, RAFM Chamuleau, AJ Meijer (eds) Kluwer, Dordrecht, Netherlands, 299-311.
ZWINGMANN, C., CHATAURET., N., LEIBFRITZ, D. & BUTTERWORTH, R.F. (2003). Selective increase of brain lactate synthesis in experimental acute liver failure: results of a [1H-13C] nuclear magnetic resonance study. Hepatology, 37, 420-28.

## Claims

1. A pharmaceutical composition comprising glufosinate (GF), a salt, an isomer, a prodrug, a metabolite or a structural analog thereof, in a pharmaceutically acceptable carrier.

2. The pharmaceutical composition according to claim 1, comprising a compound selected from the group consisting of glufosinate, 4-methylphosphinico-2-oxo-butanoic acid, 3-methylphosphinicopropionic acid, 4-methylphosphinico-2-hydroxybutanoic acid, 4-methylphosphinicobutanoic acid, 2-methylphosphinicoacetic acid, 2-acetamido-4-methylbutanoic acid, gamma-hydroxy phosphinothricin, gamma-methyl phosphinothricin, gamma-acetoxy phosphinothricin, alpha-methyl phosphinothricin, alpha-ethyl phosphinothricin, cyclohexane phosphinothricin, cyclopentane phosphinothricin, tetrahydrofuran phosphinothricin, s-phosphonomethyl homocysteine sulfoxide, s-phosphonomethyl homocysteine sulfone, 2-amino-4-[(phosphonomethyl)hydroxyphosphinyl-1]butanoic acid, 2-amino-4-phosphono butanoic acid, 4-amino-4-phosphono butanoic acid, 2-amino-2-methyl-4-phosphono butanoic acid, 4-amino-4-(hydroxymethylphosphinyl)-4-methyl butanoic acid, 2-methoxycarbonyl-4-phosphono butamoic acid, methyl 4-amino-4-phosphono butanate, 4-amino-4-(hydroxymethylphosphinyl)butanoic acid, 2-amino-4-(hydroxymethylphosphinyl)butanoic acid, phosphinothricin, s-phosphonomethyl homocysteine, 4-(phosphonoacetyl)-L-alpha-aminobutyrate, threo-4-hydroxy-D-glutamic acid, erythro-4-fluoro-D,L-glutamic acid, 4-amino-4-(hydroxy-methylphosphinoyl)-butyric acid, 2-methoxycarbonyl-4-phosphono butanoic acid, methyl 4-amino-4-phosphono butanoate and 2-amido-4-phosphono butanoic acid, and salts thereof and isomers thereof.

3. The pharmaceutical composition according to claims 1, comprising glufosinate (GF) or a salt thereof or an isomer thereof.

4. The pharmaceutical composition according to anyone of claims 1-3, wherein the therapeutical amount is a "low or intermediate dose" i.e. a dose resulting in concentrations of compound which activate or modulate/regulate GS activity in the target cells/tissues/organs.

5. The pharmaceutical composition according to anyone of claims 1-4, further comprising an other drug intended to prevent and/or treat conditions and disorders which involve or can be influenced by GS activity, directly or indirectly, in particular those related to absolute or relative excess of ammonia, those related to absolute or relative deficiency or excess of glutamate, and those related to absolute or relative deficiency or excess of glutamine.

6. The pharmaceutical composition according to claim 5, wherein said drug is selected from the group consisting of i) drugs intended to prevent/treat hyperammon(em)ia and/or hyperammon(em)ia symptoms, ii) drugs resulting in hyperammon(em)ia, iii) drugs altering glutamate receptor(s) function(s), iv) drugs intended to prevent/treat disorders where GS protein/activity is altered or relatively ineffective, v) drugs intended to combat neoplasms and/or inflammatory diseases, and vi) anti-inflammatory drugs or drugs preventing from the occurrence of inflammation, in particular glucocorticoids.

7. Glufosinate (GF), a salt, an isomer, a pro-drug, a metabolite or a structural analog thereof, as a medicament.

8. A medicament according to claim 7, wherein the therapeutical amount is a "low or intermediate dose" i.e. a dose resulting in concentrations of medicament which can activate or modulate/regulate GS activity in the target cells/tissues/organs

9. Use of a pharmaceutical composition according to anyone of claims 1-6 in the manufacture of a medicament for preventing and/or treating conditions and disorders which involve or can be influenced by GS activity, directly or indirectly, in particular those related to absolute or relative excess of ammonia, those related to absolute or relative deficiency or excess of glutamate, and those related to absolute or relative deficiency or excess of glutamine.

10. The use according to claim 9, wherein said conditions or disorders are selected from those disclosed in Tables 1-4, preferably Tables 2-3.

11. The use according to claim 9, wherein the condition or disorder is hyperammon(em)ia.

12. The use according to claim 9, wherein said conditions or disorders are selected from the group consisting of asthma, cough, depression (major depression with or without melancholia, dysthymic disorders, depressed bipolar disorder, ...), irritable bowel syndrome, mnemo-cognitive disorders, neurodegenerative diseases (such as cerebral

hypoxia, cerebral ischaemia, cerebral traumatism, cerebral ageing, Alzheimer's disease, multiple sclerosis, amyotrophic lateral sclerosis, demyelinating pathologies, encephalopathies, myalgic encephalomyelitis, chronic fatigue syndrome, post-viral fatigue syndrome, the state of fatigue and depression following a bacterial or viral infection, the dementia syndrome of AIDS, ...), nonulcer dyspepsia, pain, psychoneurotic disorders (neurotic or reactive states of depression, anxiodepressive states with somatic complaints such as digestive problems, anxiodepressive states observed in alcoholic detoxification, ...), stress and stroke.

**13.** Use of tianeptine or a salt, an isomer, a pro-drug, a metabolite or a structural analog thereof, in the manufacture of a medicament for preventing and/or treating all types of conditions and disorders which involve or can be influenced by GS activity, directly or indirectly, in particular those related to absolute or relative excess of ammonia, those related to absolute or relative deficiency or excess of glutamate, and those related to absolute or relative deficiency or excess of glutamine, with the exception of those mentioned in Table 5.

**14.** The use according to claim 13, wherein said conditions or disorders are selected from those disclosed in Tables 1-4, preferably Tables 2-3.

**15.** The use according to claim 13 or 14, wherein the disorder or the disease is hyperammon(em)ia.

**16.** The use according to anyone of claims 13-15, wherein said tianeptine or salt, isomer, pro-drug, metabolite or structural analog thereof, is used in combination with an other drug intended to prevent and/or treat conditions and disorders which involve or can be influenced by GS activity, directly or indirectly, in particular those related to absolute or relative excess of ammonia, those related to absolute or relative deficiency or excess of glutamate, and those related to absolute or relative deficiency or excess of glutamine.

**17.** The use according to claim 16, wherein said drug is selected from the group consisting of i) drugs intended to prevent/treat hyperammon(em)ia and/or hyperammon(em)ia symptoms, ii) drugs resulting in hyperammon(em)ia, iii) drugs altering glutamate receptor(s) function(s), iv) drugs intended to prevent/treat disorders where GS protein/activity is altered or relatively ineffective, v) drugs intended to combat neoplasms and/or inflammatory diseases and vi) anti-inflammatory drugs or drugs preventing from the occurrence of inflammation, in particular glucocorticoids.

**18.** A pharmaceutical composition comprising tianeptine or a salt, an isomer, a prodrug, a metabolite or a structural analog thereof, in combination with a drug selected from the group consisting of other drug intended to prevent/treat hyperammon(emi)a or resulting in hyperammon(em)ia, or an other drug intended to prevent/treat conditions and disorders related to (absolute or relative) deficiency or excess of glutamate or resulting in (absolute or relative) deficiency or excess of glutamate, or an other drug intended to prevent/treat conditions and disorders related to (absolute or relative) deficiency or excess of glutamine or resulting in (absolute or relative) deficiency or excess of glutamine, in a pharmaceutically acceptable carrier.

**19.** The pharmaceutical composition according to claim 18, wherein said drug is selected from the group consisting of i) drugs intended to prevent/treat hyperammon(em)ia and/or hyperammon(em)ia symptoms, ii) drugs resulting in hyperammon(em)ia, iii) drugs altering glutamate receptor(s) function(s), iv) drugs intended to prevent/treat disorders where GS protein/activity is altered or relatively ineffective, v) drugs intended to combat neoplasms and/or inflammatory diseases and vi) anti-inflammatory drugs or drugs preventing from the occurrence of inflammation, in particular glucocorticoids, in a pharmaceutically acceptable carrier.

**20.** Use of a glutamine synthetase (GS) ligand, with the exception of tianeptine, for the preparation of a medicament for preventing and/or treating conditions and disorders selected from the group consisting of asthma, cough, depression (major depression with or without melancholia, dysthymic disorders, depressed bipolar disorder, ...), irritable bowel syndrome, mnemo-cognitive disorders, neurodegenerative diseases (such as cerebral hypoxia, cerebral ischaemia, cerebral traumatism, cerebral ageing, Alzheimer's disease, multiple sclerosis, amyotrophic lateral sclerosis, demyelinating pathologies, encephalopathies, myalgic encephalomyelitis, chronic fatigue syndrome, post-viral fatigue syndrome, the state of fatigue and depression following a bacterial or viral infection, the dementia syndrome of AIDS, ...), nonulcer dyspepsia, pain, psychoneurotic disorders (neurotic or reactive states of depression, anxiodepressive states with somatic complaints such as digestive problems, anxiodepressive states observed in alcoholic detoxification, ...), stress and stroke.

**21.** Use according to claim 20, wherein said GS ligand is selected from the group consisting of glufosinate (GF), L-methionine sulfoximine (MS), hydrazines and bisphosphonates, and salts, isomers, pro-drugs, metabolites and

structural analogs thereof, provided that:

- if said GS ligand is MS, the conditions and disorders related to cerebral ischemia and hyperammonemia are excluded;
- if said GS ligand is a hydrazine, the conditions and disorders disclosed in Table 6 are excluded;
- if said GS ligand is a bisphosphonate, the conditions and disorders disclosed in Table 7 are excluded.

**22.** Use according to claim 20 or 21, wherein said GS ligand is used at a "low or intermediate dose" i.e. a dose resulting in concentrations which activate or modulate/regulate GS activity in the target cells/tissues/organs.

**23.** Use of a glutamine synthetase (GS) ligand, with the exception of tianeptine, for the preparation of a medicament for preventing and/or treating conditions and disorders which involve or can be influenced by GS activity, directly or indirectly, in particular those related to absolute or relative excess of ammonia, those related to absolute or relative deficiency or excess of glutamate, and those related to absolute or relative deficiency or excess of glutamine, wherein said GS ligand is used at a "low or intermediate dose" i.e. a dose resulting in concentrations which activate or modulate/regulate GS activity in the target cells/tissues/organs..

**24.** Use according to claim 23, wherein said GS ligand is selected from the group consisting of glufosinate (GF), L-methionine sulfoximine (MS), hydrazines and bisphosphonates, and salts, isomers, pro-drugs, metabolites and structural analogs thereof, provided that:

- if said GS ligand is MS, the conditions and disorders related to cerebral ischemia, hyperammonemia and mycobacterial infectious disorders are excluded;
- if said GS ligand is a hydrazine, the conditions and disorders disclosed in Table 6 are excluded;
- if said GS ligand is a bisphosphonate, the conditions and disorders disclosed in Table 7 are excluded.

**25.** Use according to claim 23 or 24, wherein said condition or disorder is selected from the group consisting of those disclosed in Tables 1-4, preferably Tables 2-3.

**26.** Use of tianeptine or a salt, an isomer, a pro-drug, a metabolite or a structural analog thereof, for the preparation of a composition aiming to facilitate the growth of non-vertebrate organisms with eukaryotic cells such as invertebrate animals (arthropods, protozoa, shellfish, worms, ...), algae, fungi (yeasts, molds, ...) or plants, or prokaryotes e.g. algae or bacteria, or to protect them from stresses and disorders or, inversely, aiming to combat their growth.

**27.** Use of a GS ligand selected from the group consisting of glufosinate (GF), L-methionine sulfoximine (MS), hydrazines and bisphosphonates, and salts, isomers, pro-drugs, metabolites and structural analogs thereof, for the preparation of a composition aiming to facilitate the growth of non-vertebrate organisms with eukaryotic cells such as invertebrate animals (arthropods, protozoa, shellfish, worms, ...), algae, fungi (yeasts, molds, ...) or plants, or prokaryotes e.g. algae or bacteria, or to protect them from stresses and disorders, wherein said GS ligand is used at "low or intermediate dose" i.e. a dose resulting in compound concentrations which activate or modulate/regulate GS activity, provided that if said GS ligand is GF, MS, or a bisphosphonate, plants are excluded.

**28.** A method for screening, identifying and/or developing a new active compound, activating and/or modulating/regulating GS activity comprising an assay on glutamine synthetase or a fragment thereof with a candidate compound and a GS ligand selected from the group consisting of tianeptine, glufosinate (GF), L-methionine sulfoximine (MS), hydrazines and bisphosphonates, and salts thereof, isomers thereof, pro-drugs thereof, metabolites thereof and structural analogs thereof.

**29.** The method according to claim 28, comprising a competition assay on glutamine synthetase or a fragment thereof with a candidate compound and tianeptine.

**30.** The method according to claim 28, comprising a competition assay on glutamine synthetase or a fragment thereof with a candidate compound and an hydrazine, preferably iproniazid or isoniazid.

**31.** The method according to claim 28, comprising a competition assay on glutamine synthetase or a fragment thereof with a candidate compound and a bisphosphonate, preferably pamidronate or risedronate.

**32.** The method according claim 28, comprising an assay on glutamine synthetase or a fragment thereof with a candidate

compound and an antibody raised against a fragment comprising or consisting of the amino acid sequence ITGT-NAEVMPAQWEFQIGPCEGIR.

**FIGURE 1A**

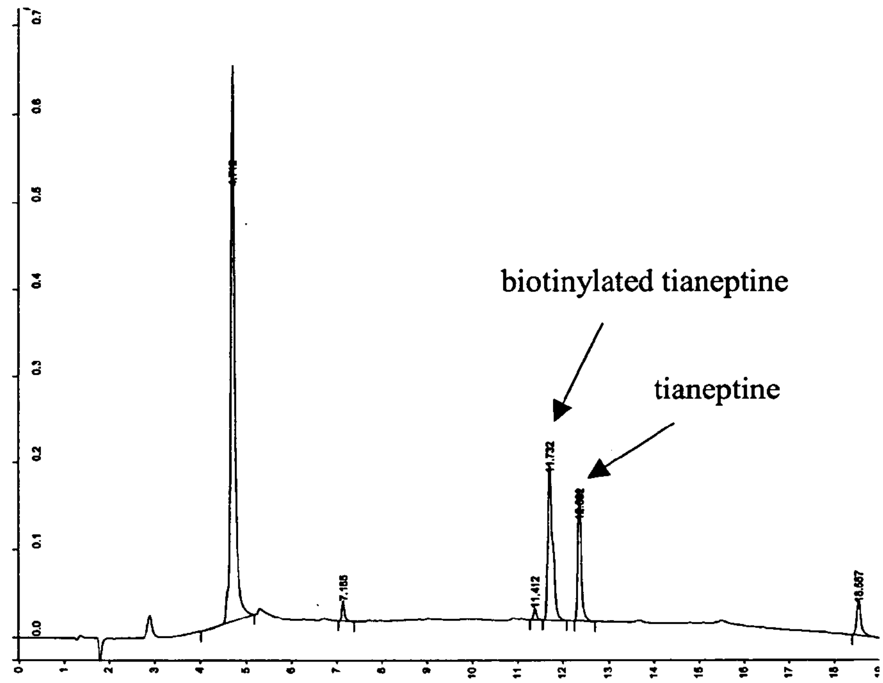


**FIGURE 1B**

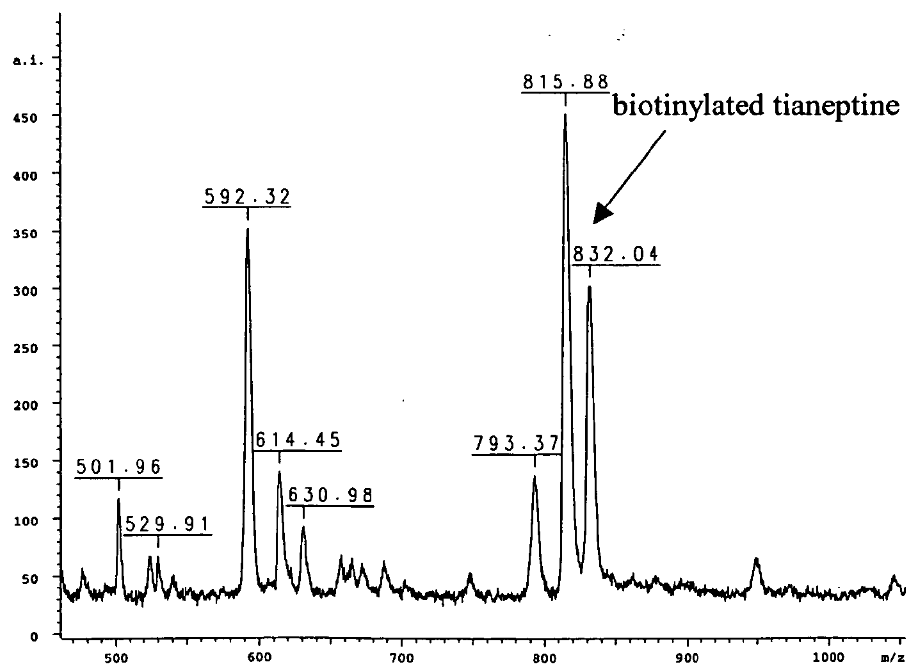

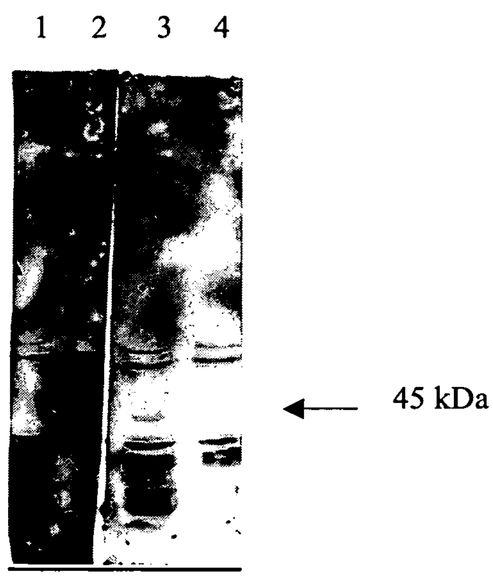


FIGURE 2

FIGURE 3A

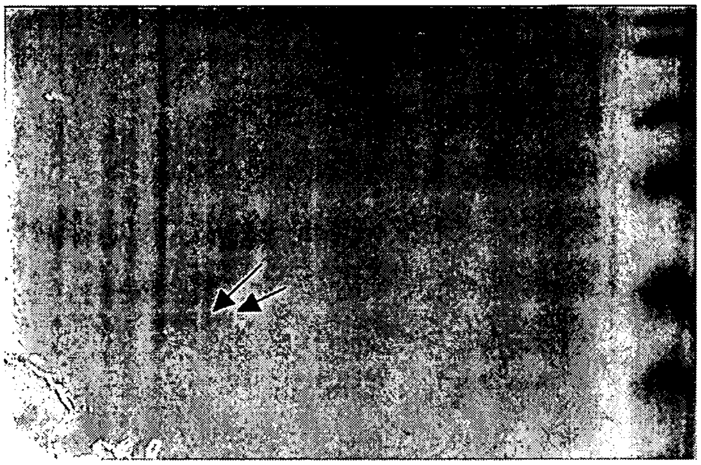

FIGURE 3B

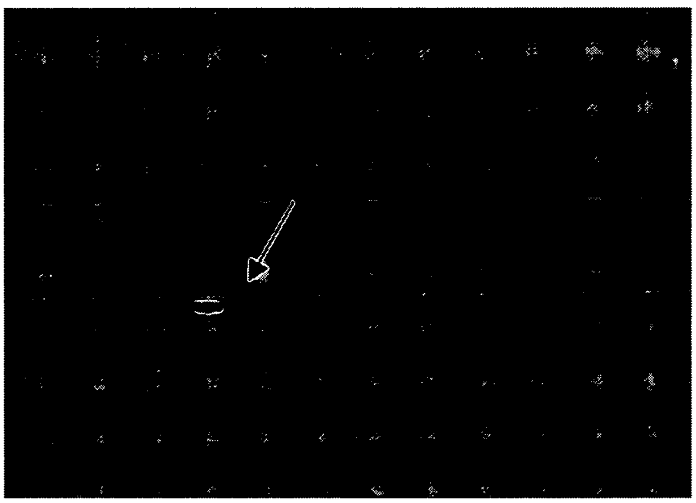

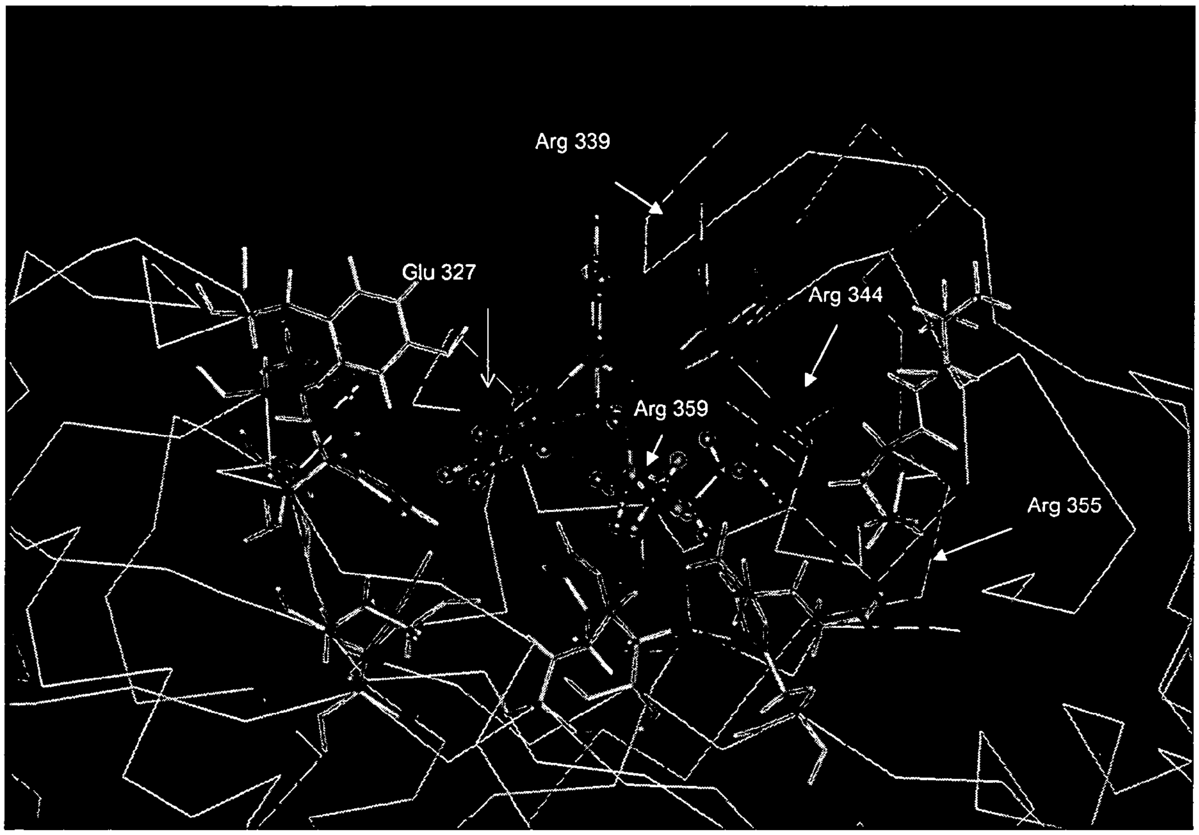


**FIGURE 4**

FIGURE 5A

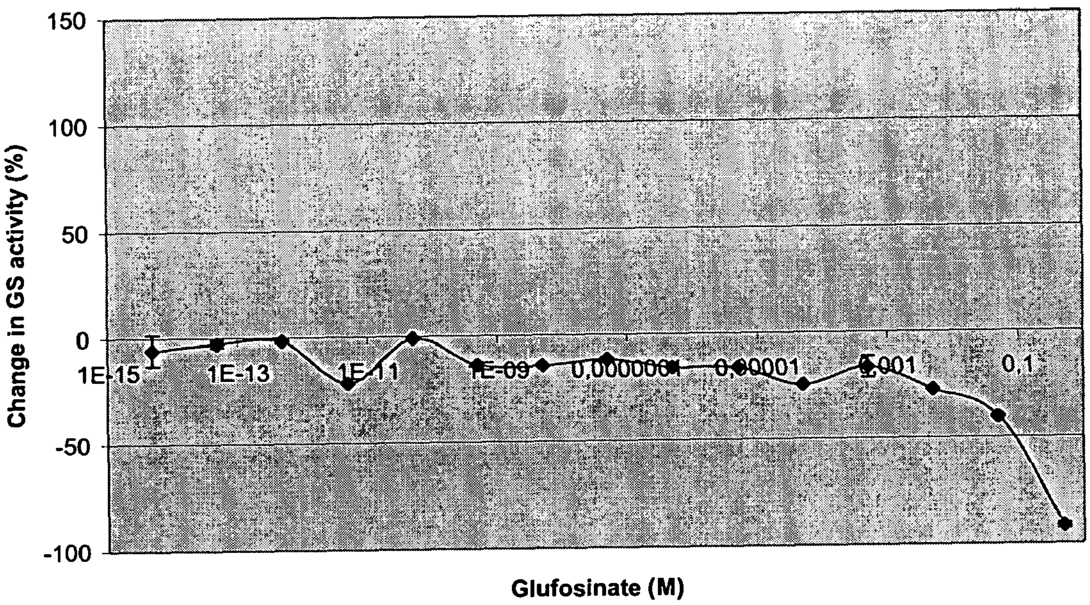


FIGURE 5B

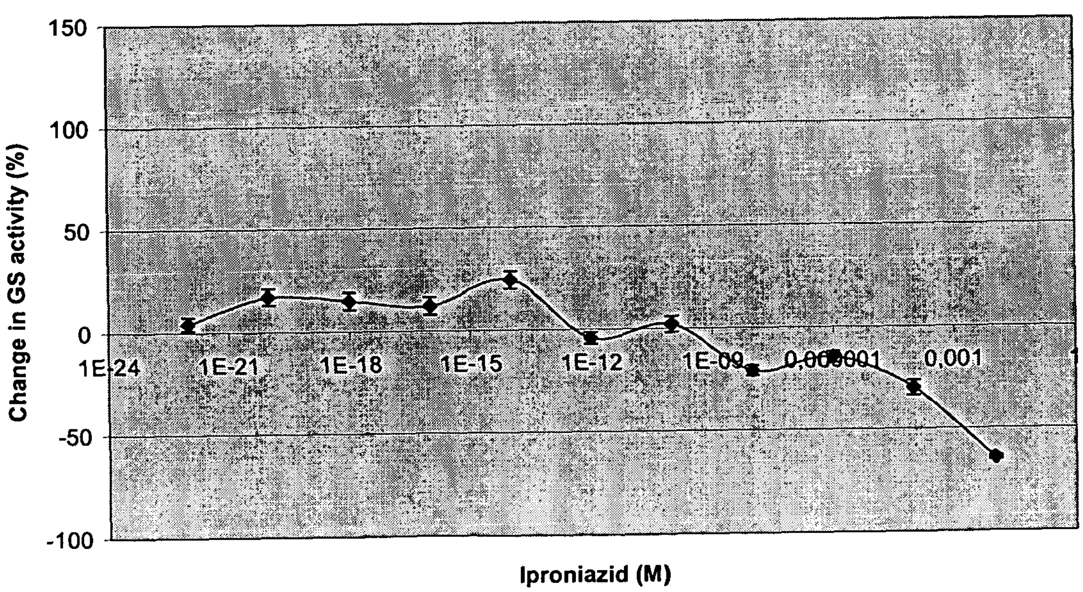

FIGURE 5C

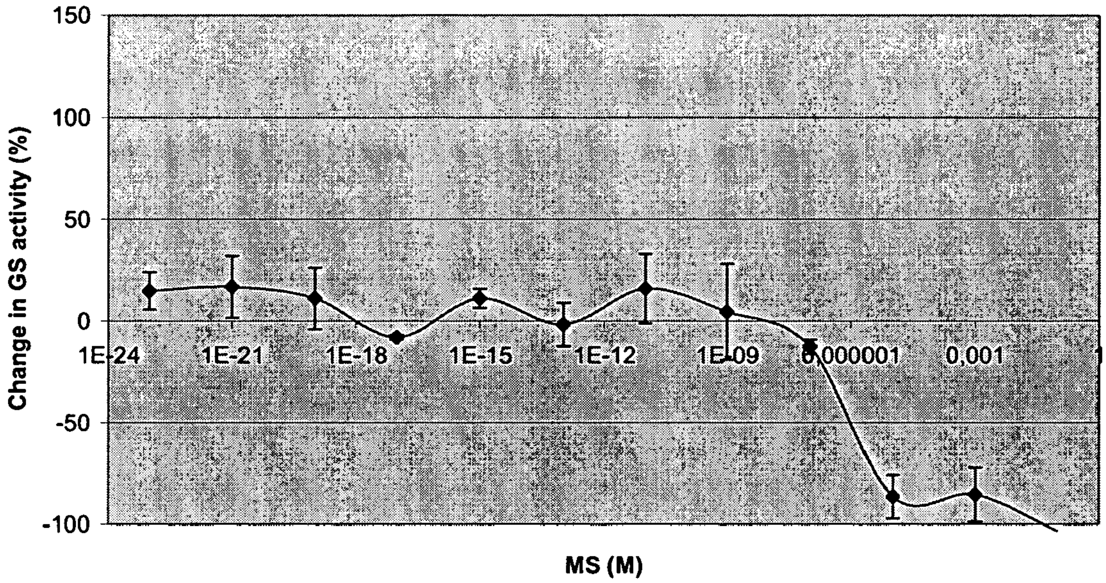


FIGURE 5D

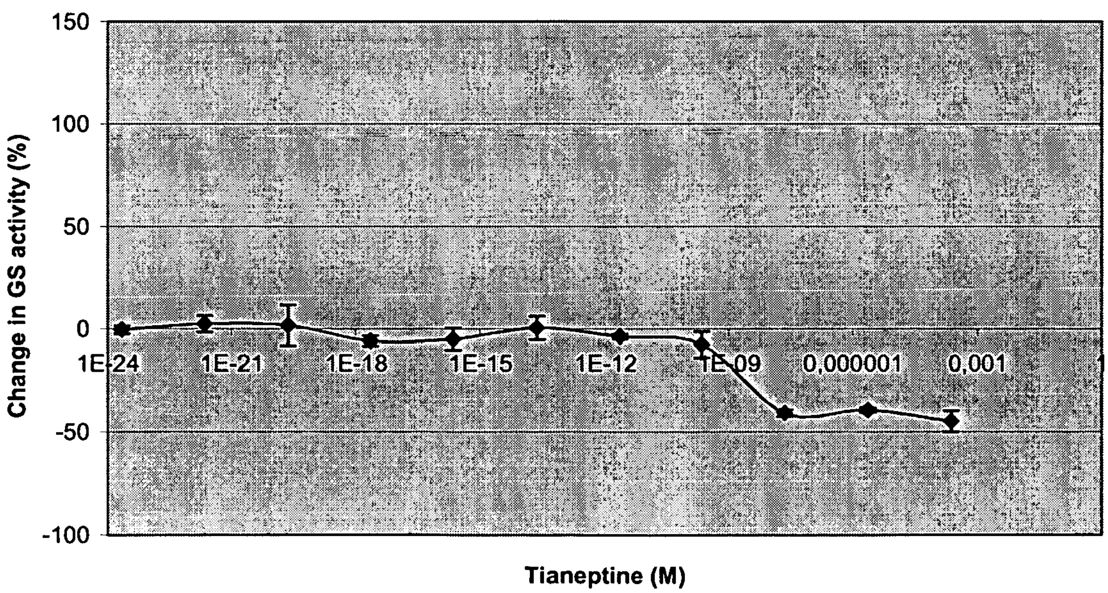

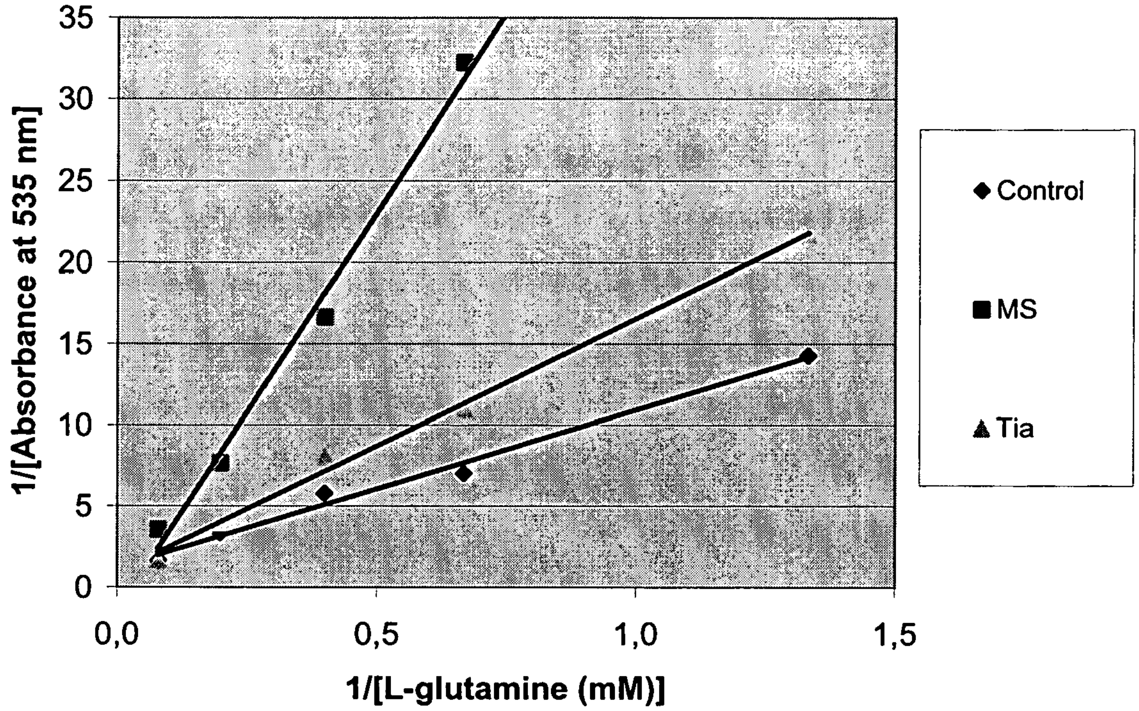
35
30
25
20
15
10
5
0
1/[Absorbance at 535 nm]
0,0
0,5
1,0
1,5
1/[L-glutamine (mM)]
Control
MS
Tia

FIGURE 6

FIGURE 7A

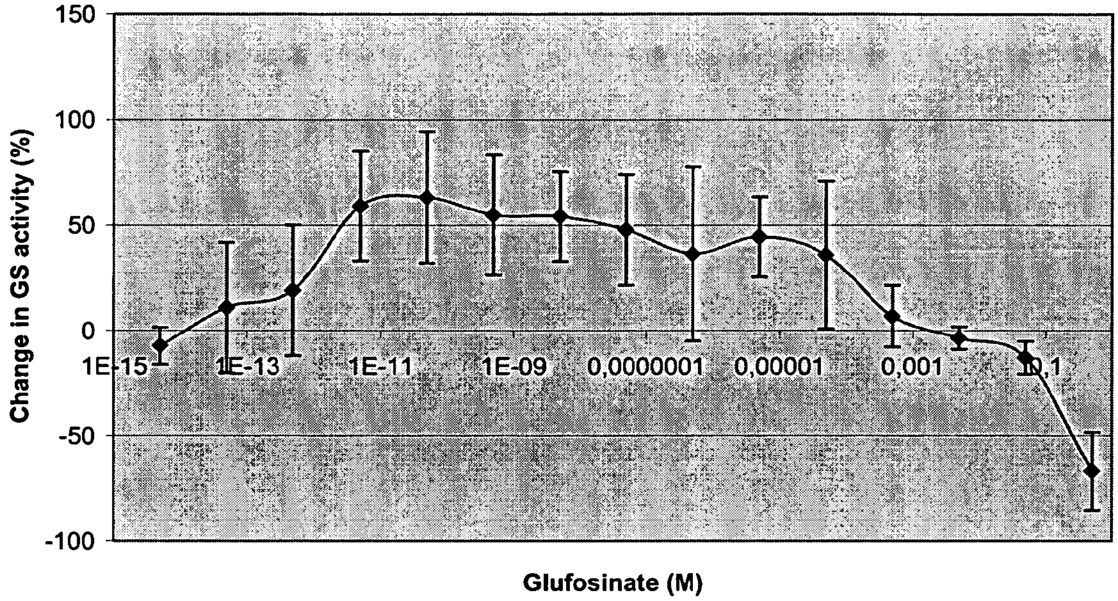


FIGURE 7B

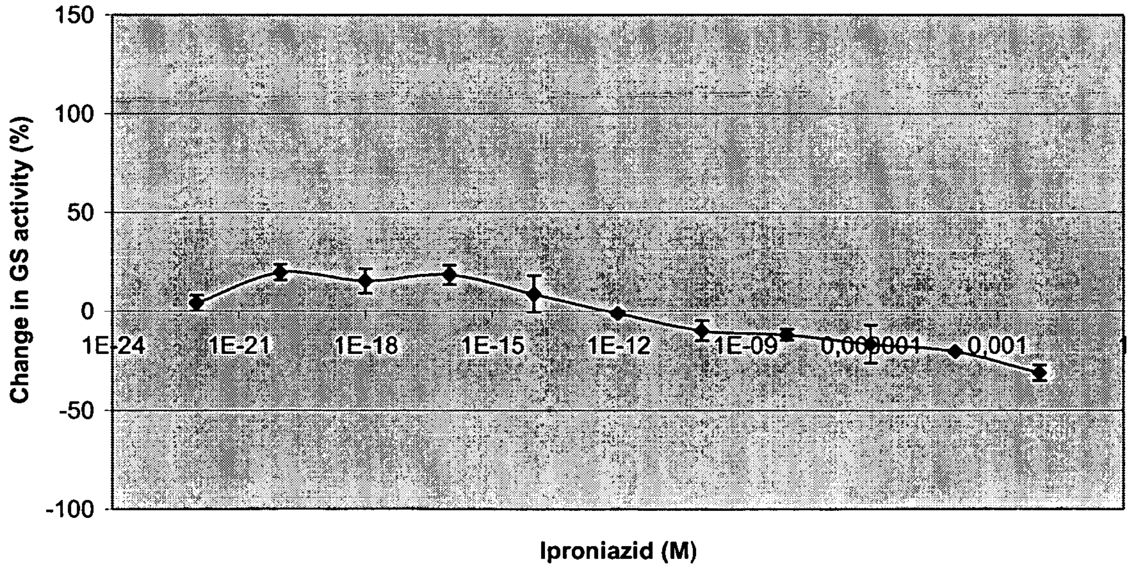

FIGURE 7C

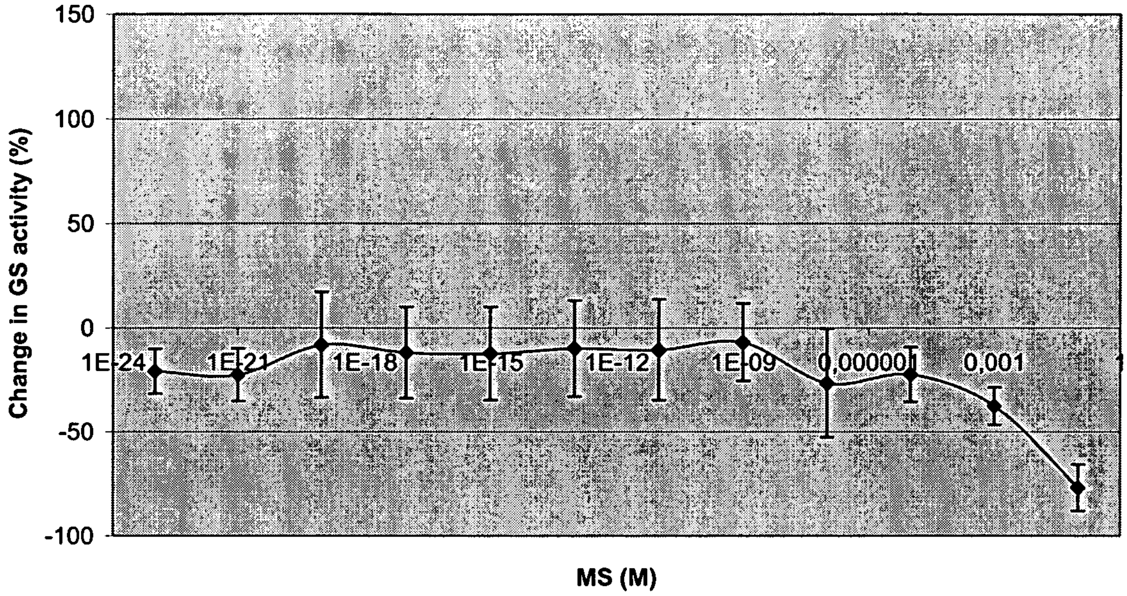


FIGURE 7D

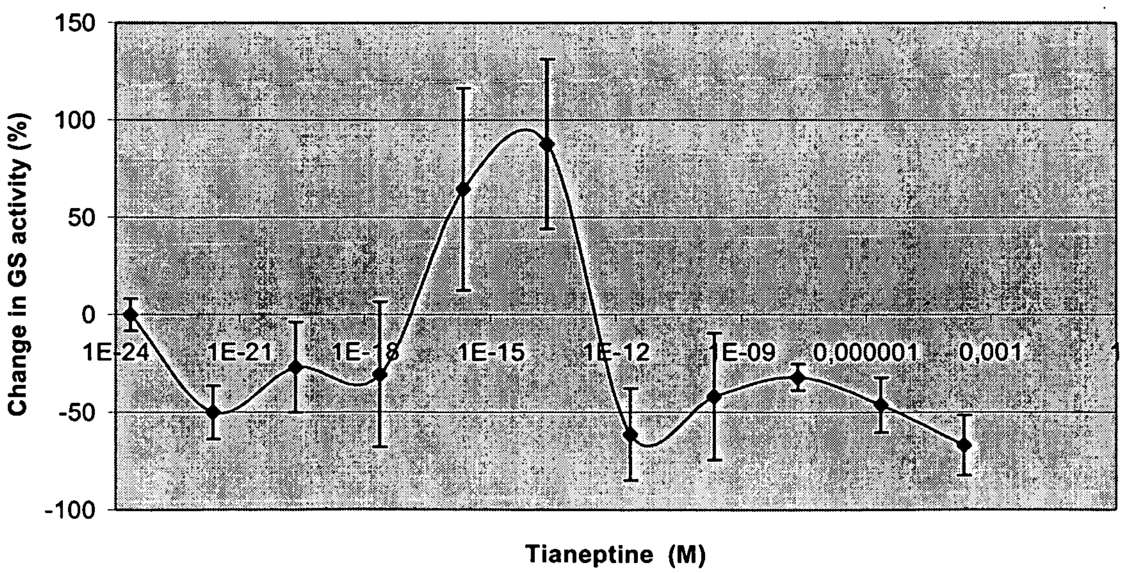

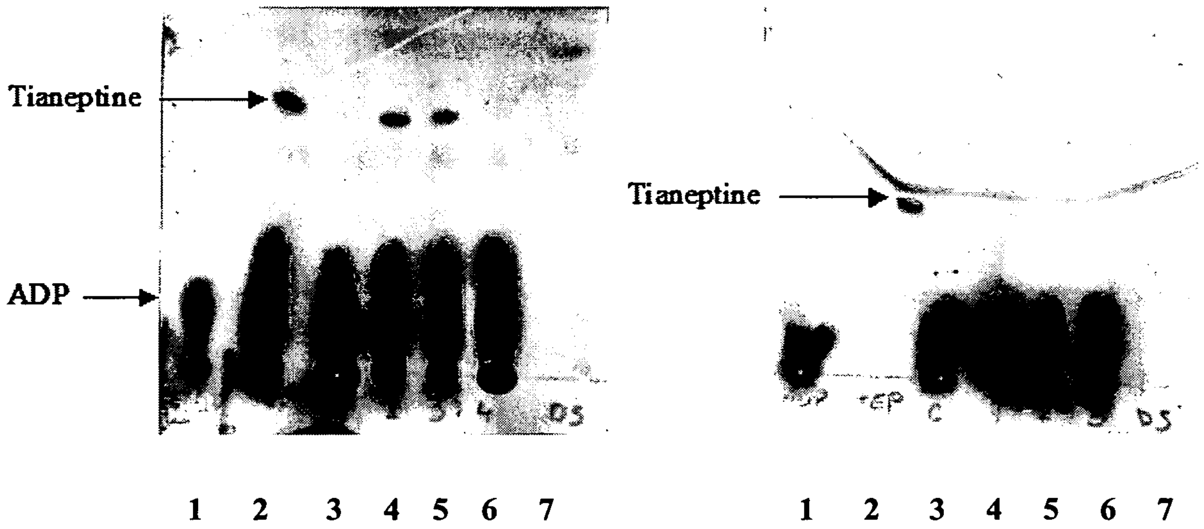


FIGURE 8

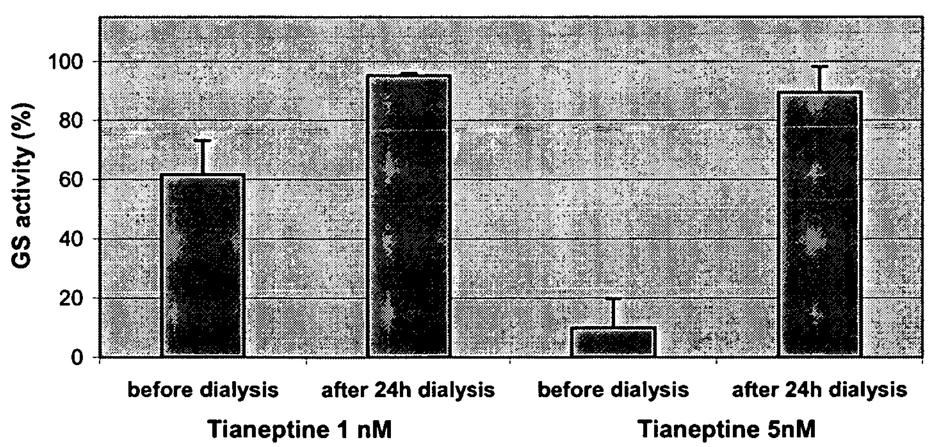


FIGURE 9

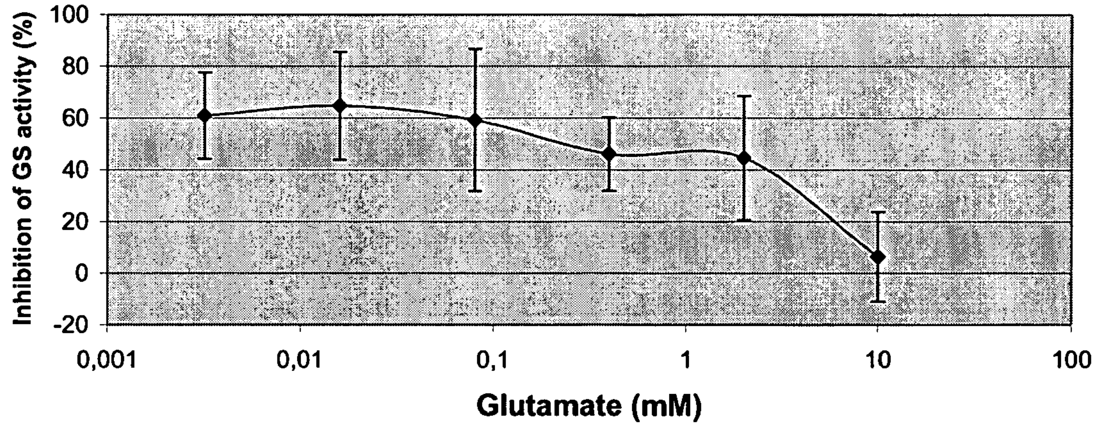


FIGURE 10

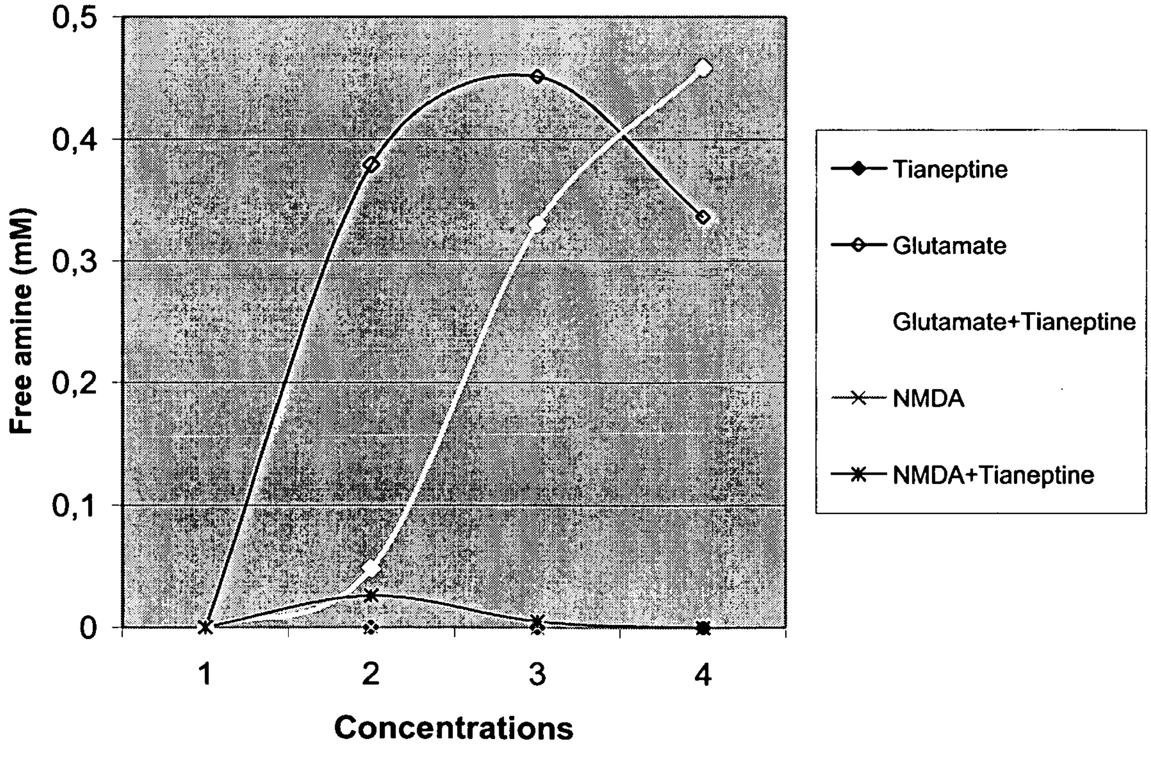


FIGURE 11

FIGURE 12A

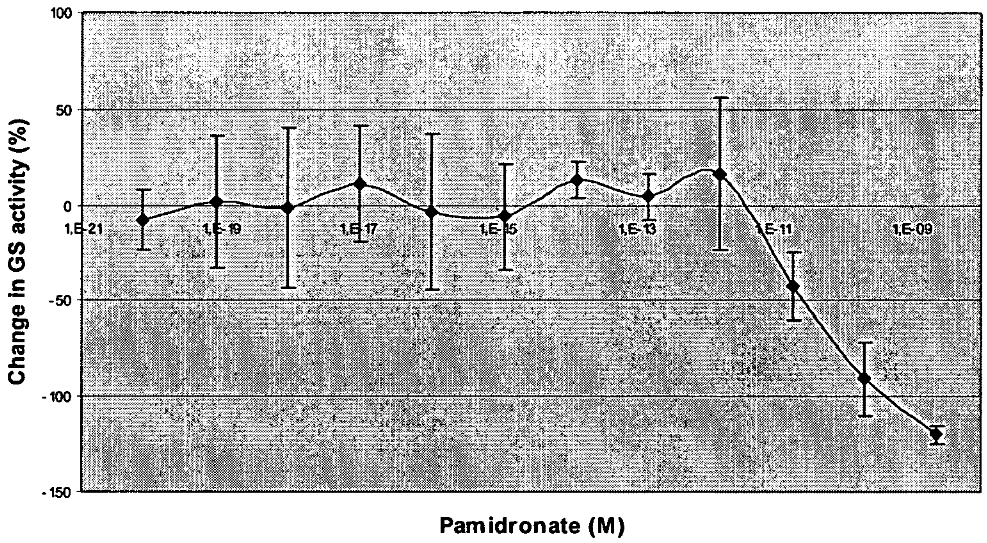


FIGURE 12B

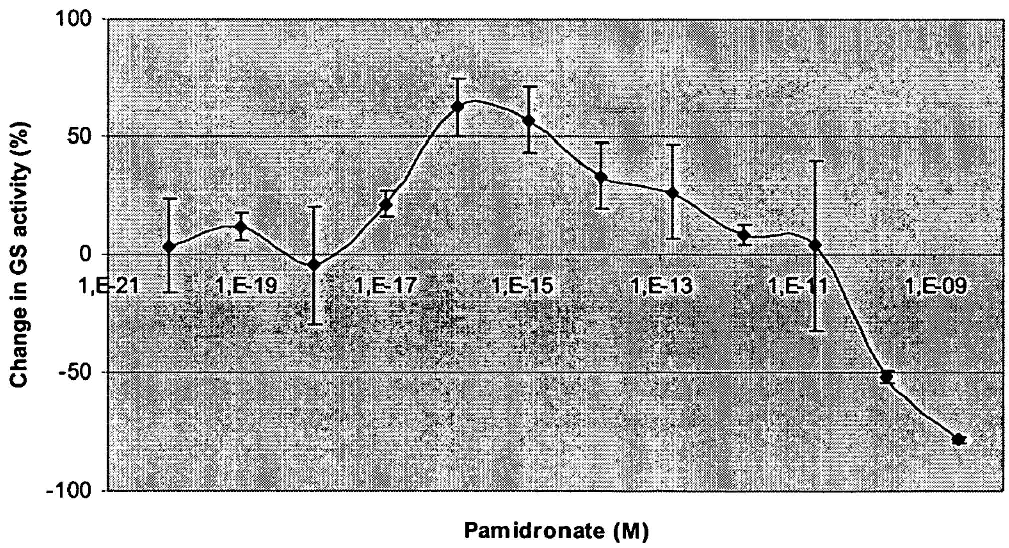

FIGURE 13A

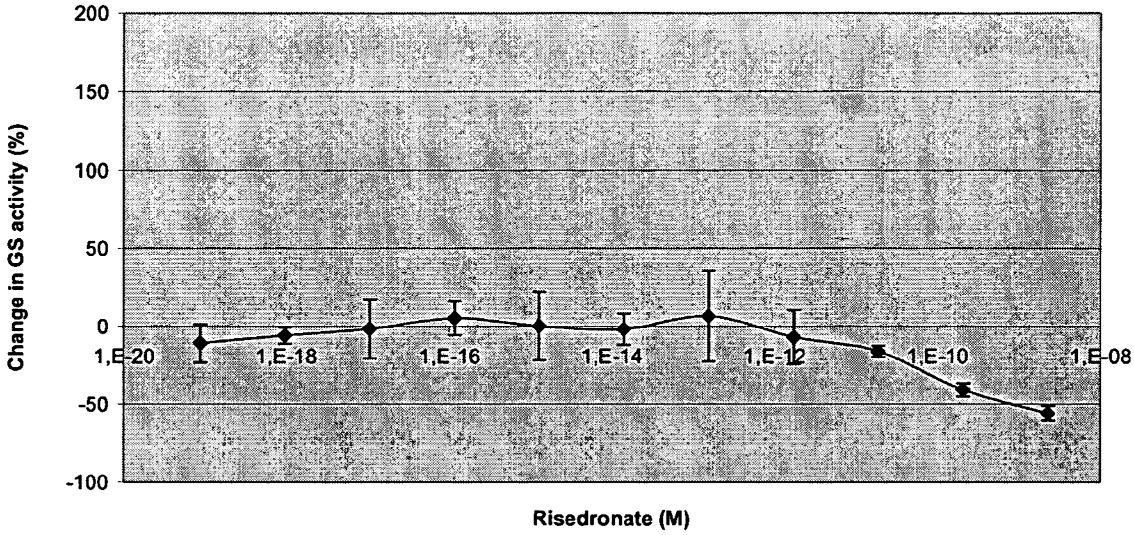


FIGURE 13B

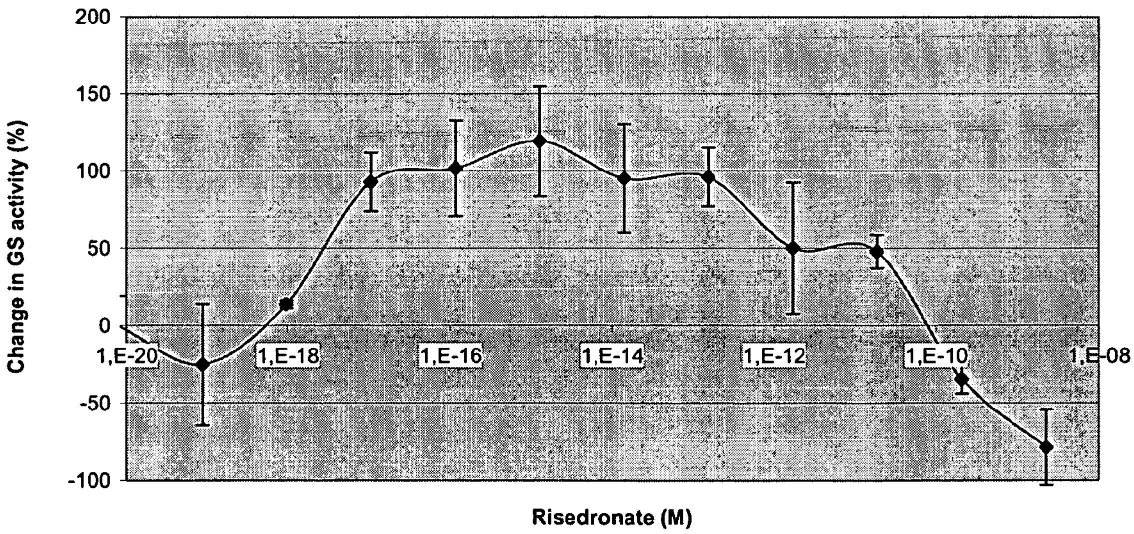

FIGURE 14A

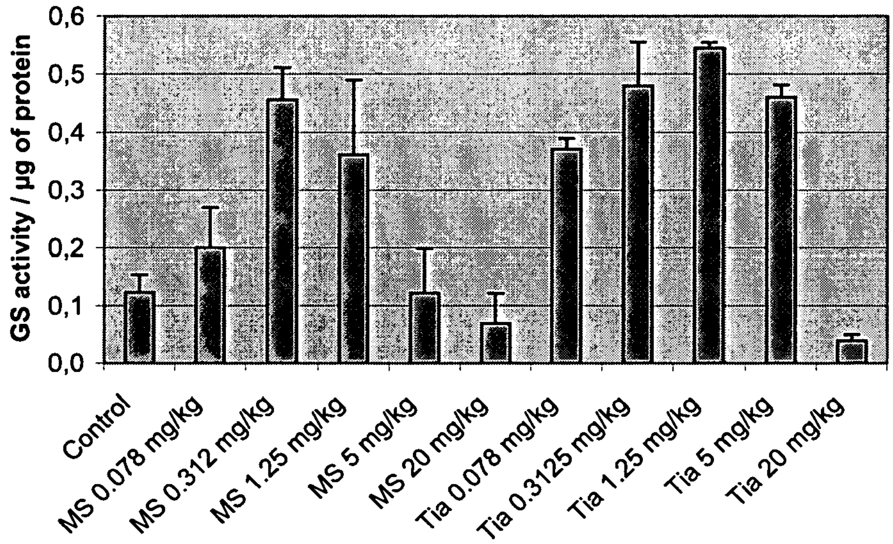


FIGURE 14B

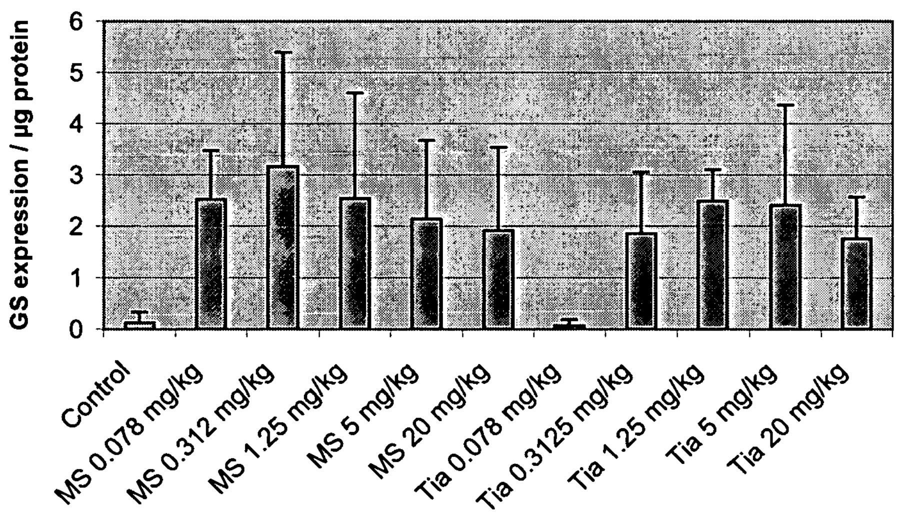

## FIGURE 15A

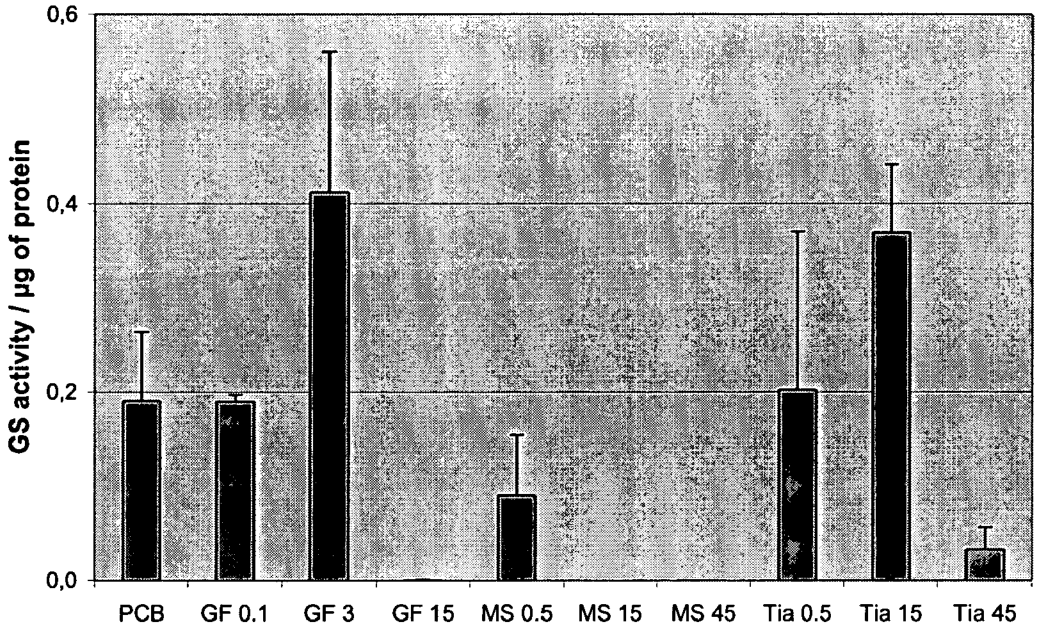


## FIGURE 15B

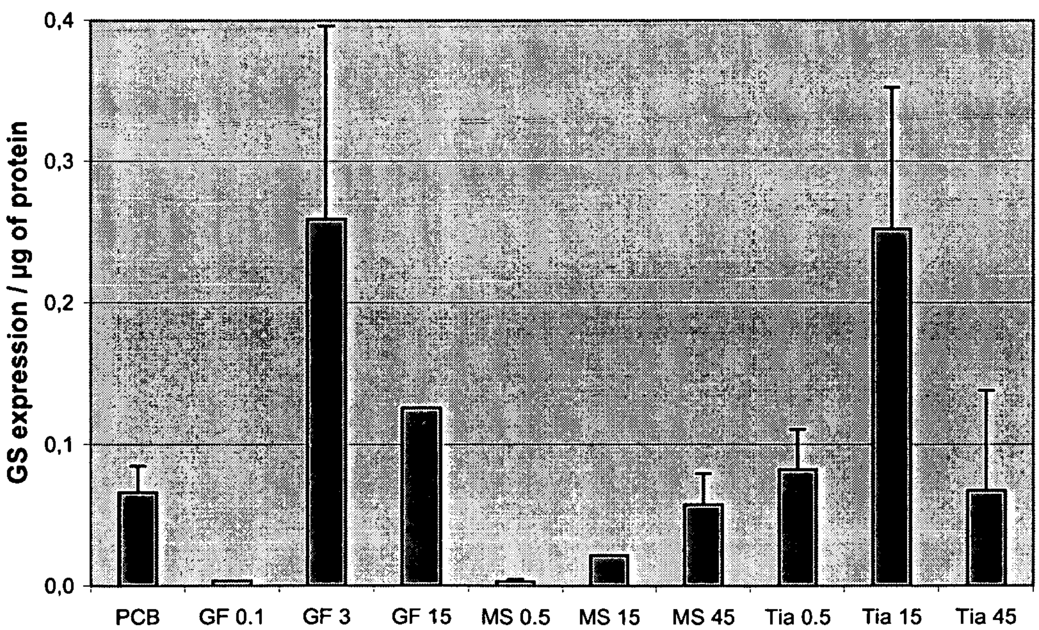

FIGURE 16A

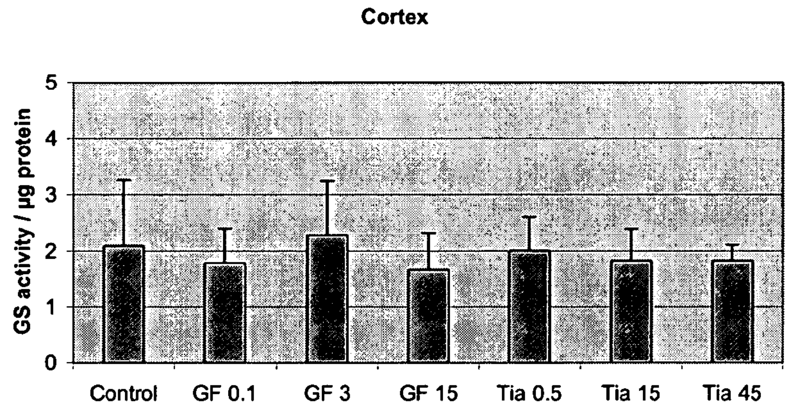


FIGURE 16B

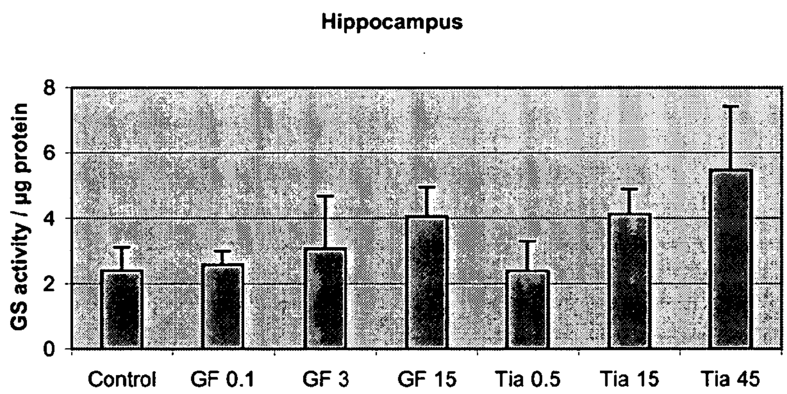


FIGURE 16C

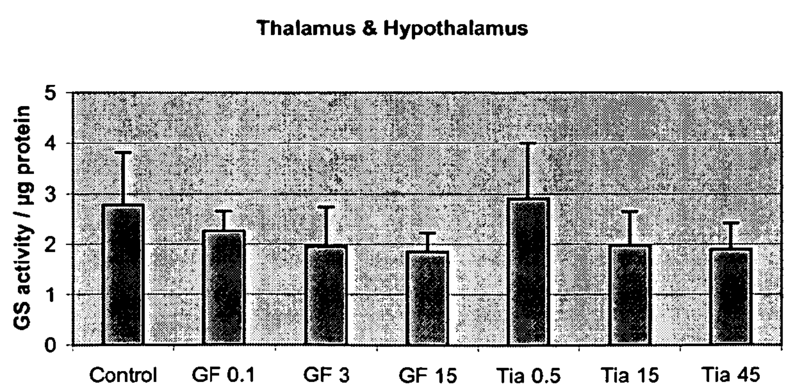

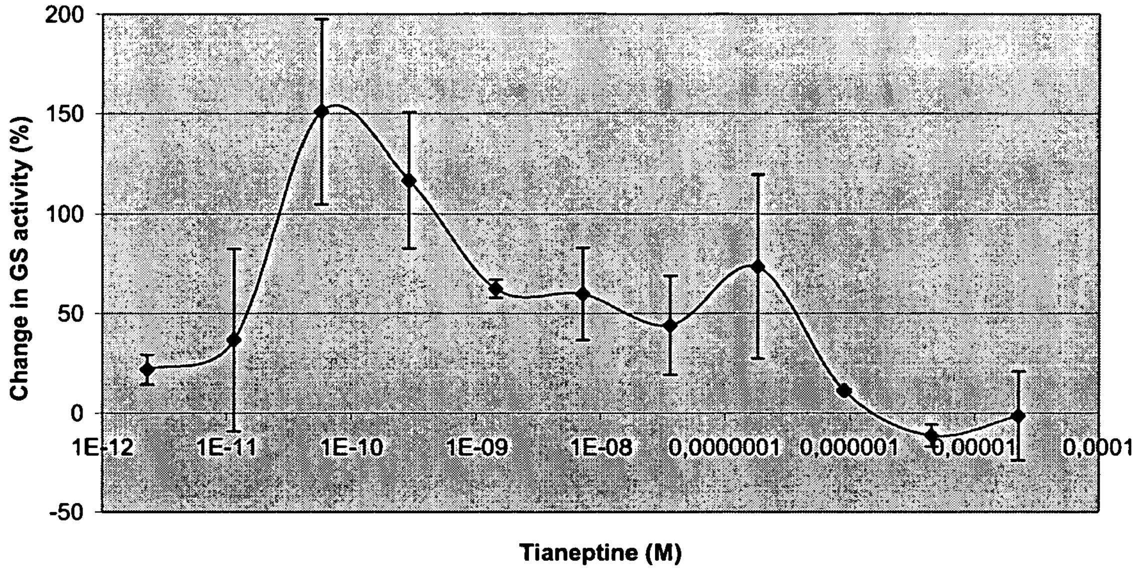
200
150
100
50
0
-50
Change in GS activity (%)
1E-12
1E-11
1E-10
1E-09
1E-08
0,0000001
0,000001
0,00001
0,0001
Tianeptine (M)

FIGURE 17

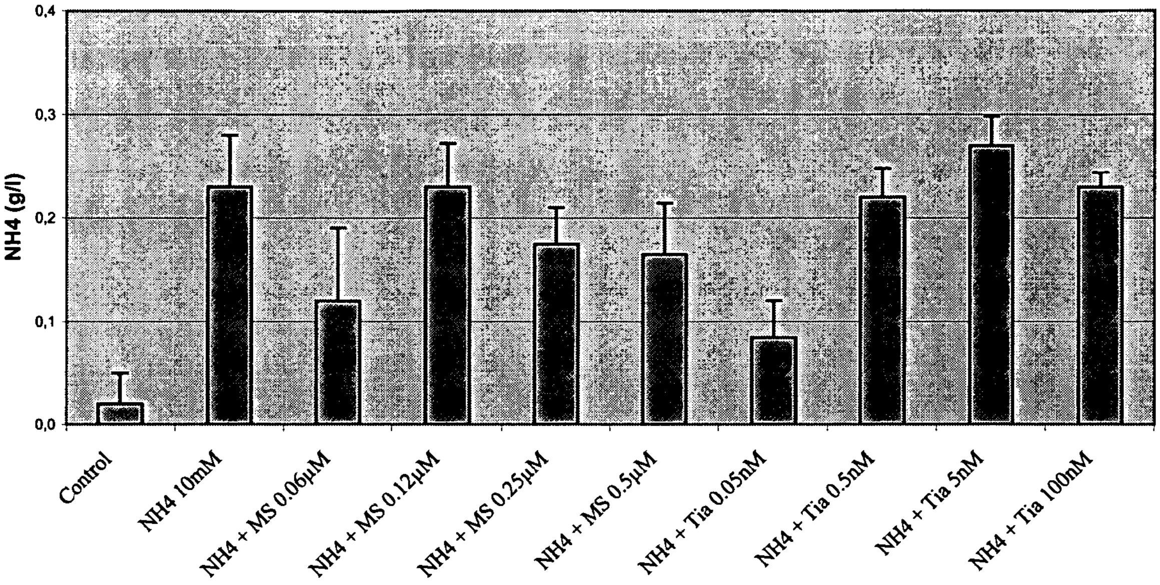


**FIGURE 18**

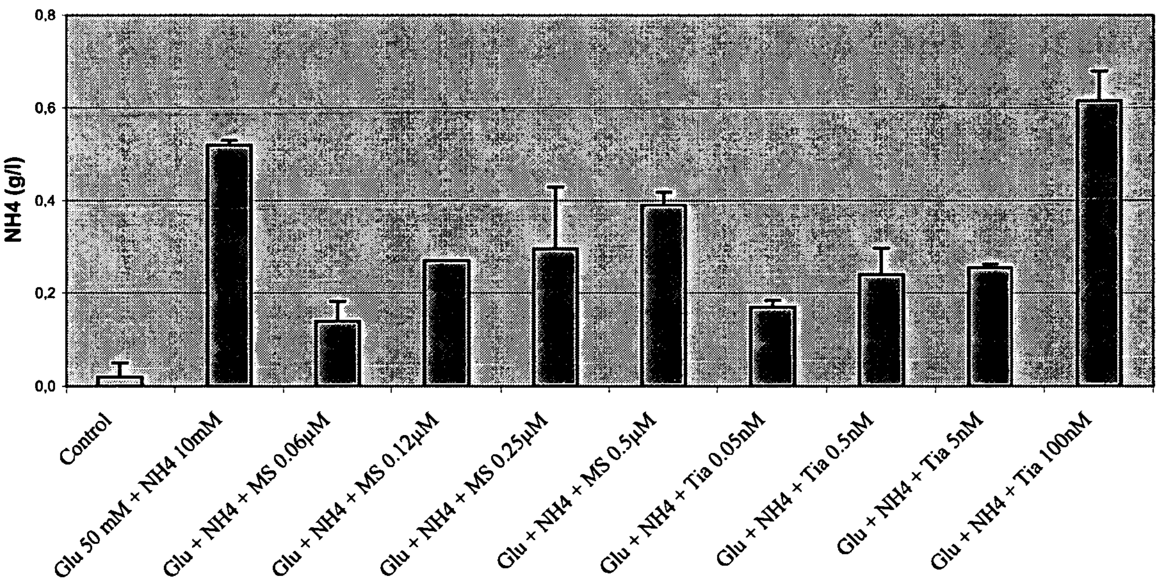


**FIGURE 19**

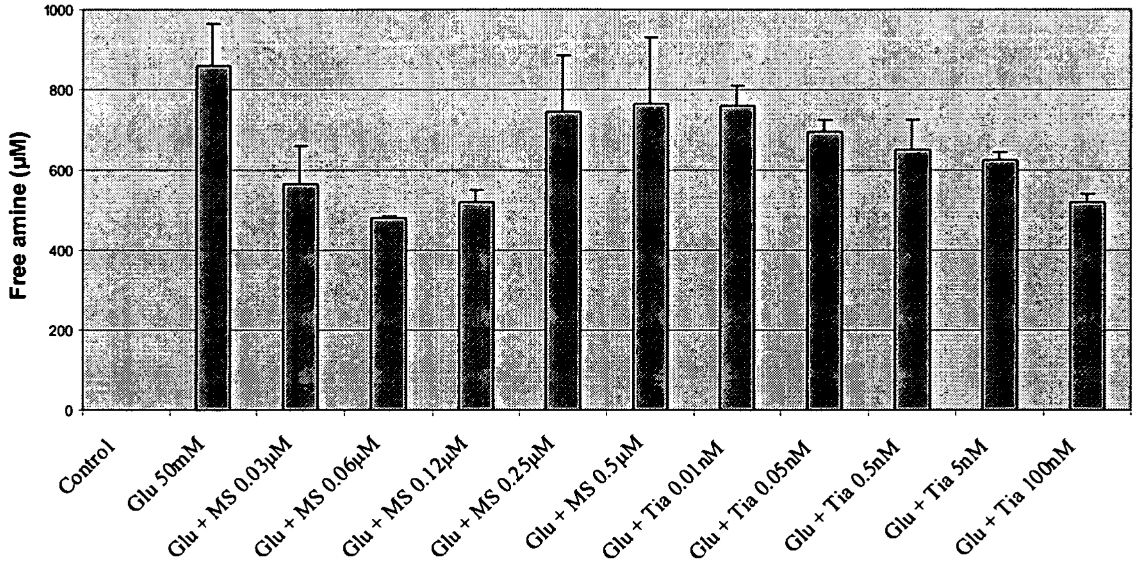


**FIGURE 20**

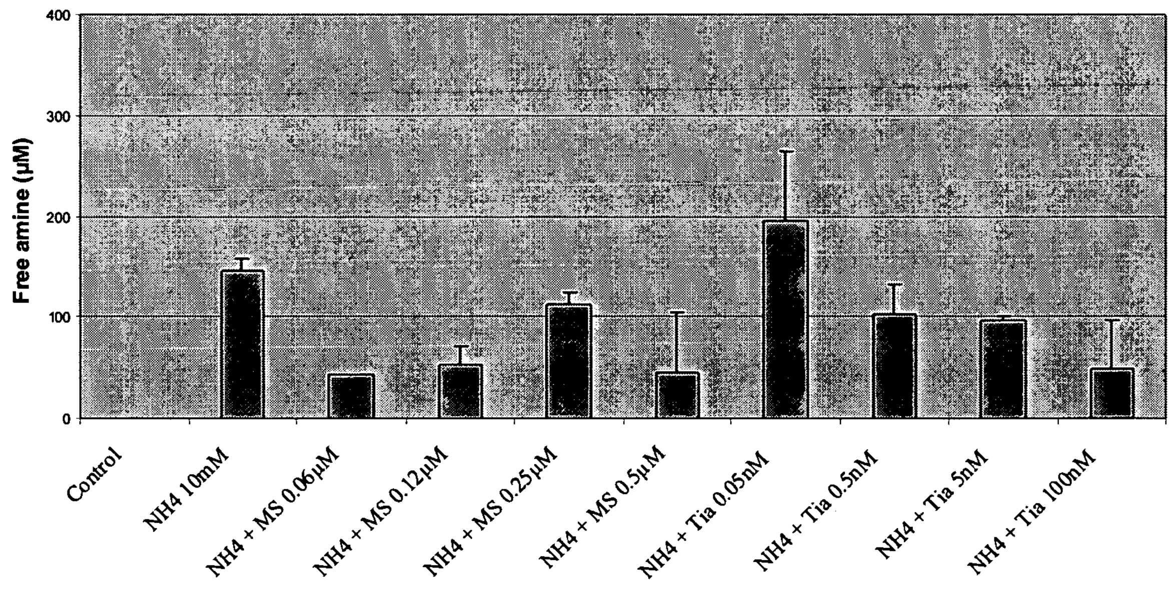


**FIGURE 21**

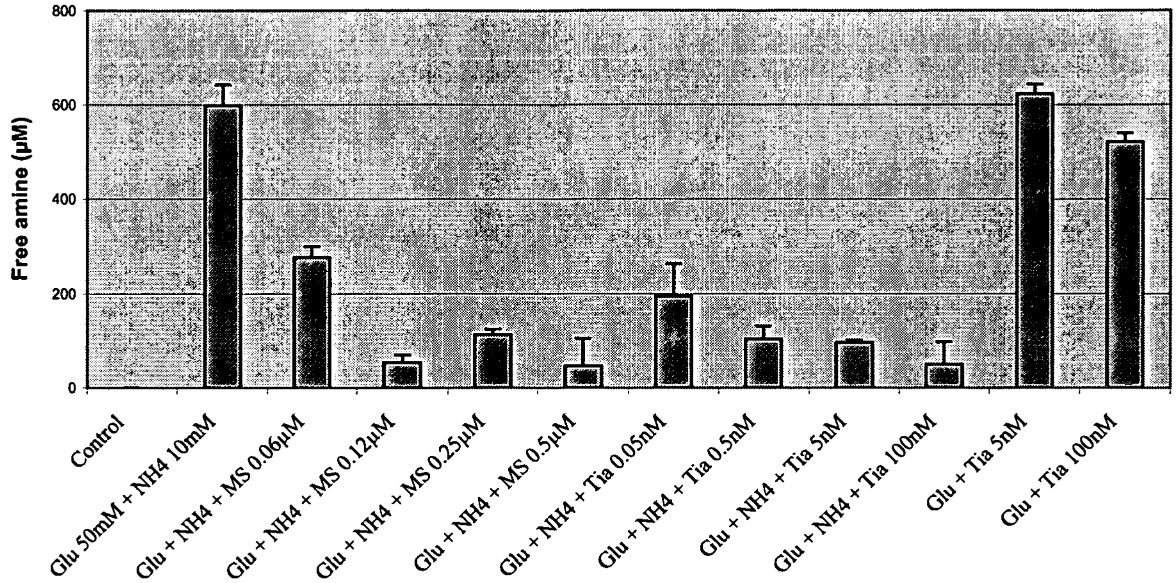


**FIGURE 22**

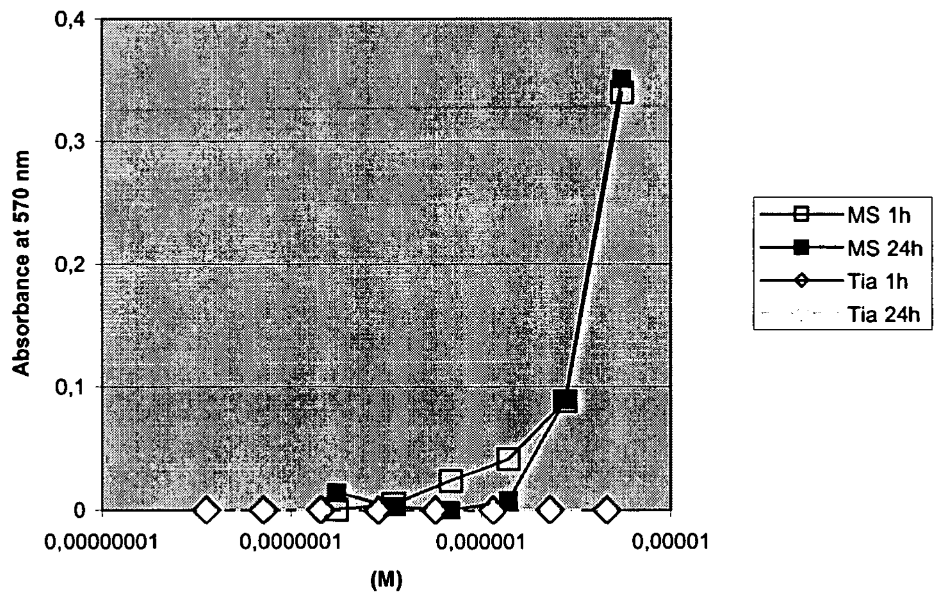


**FIGURE 23**

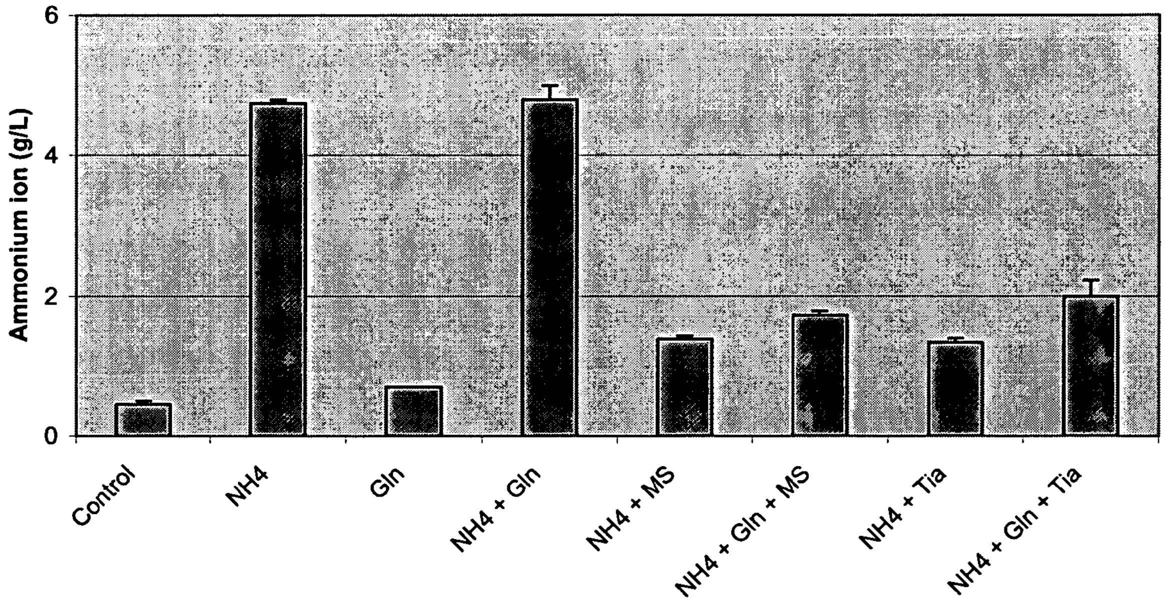


FIGURE 24A

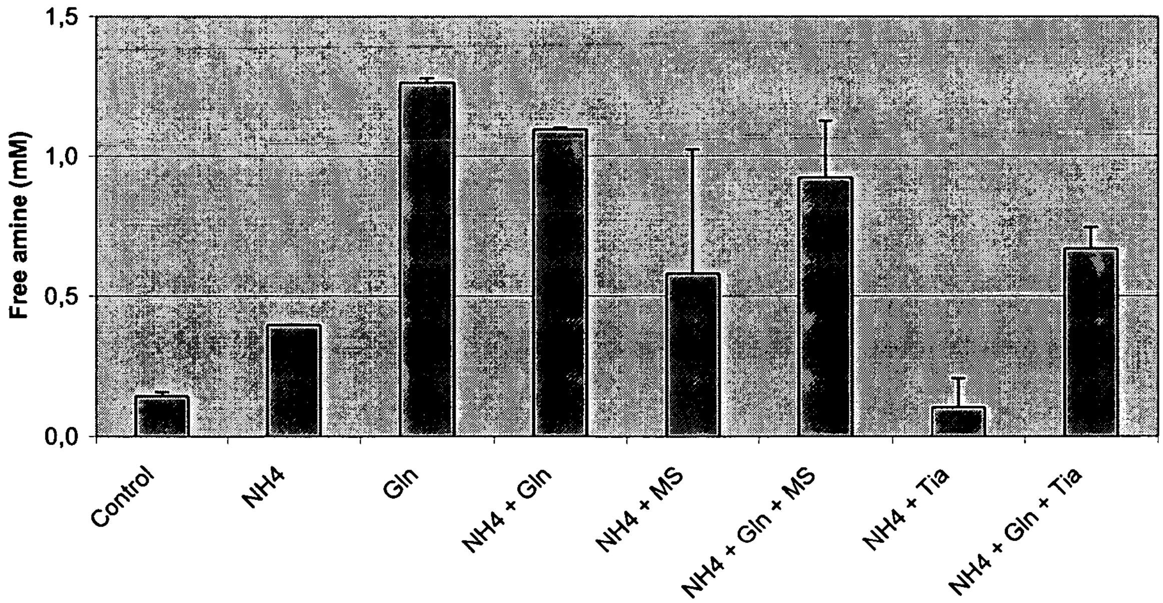


FIGURE 24B

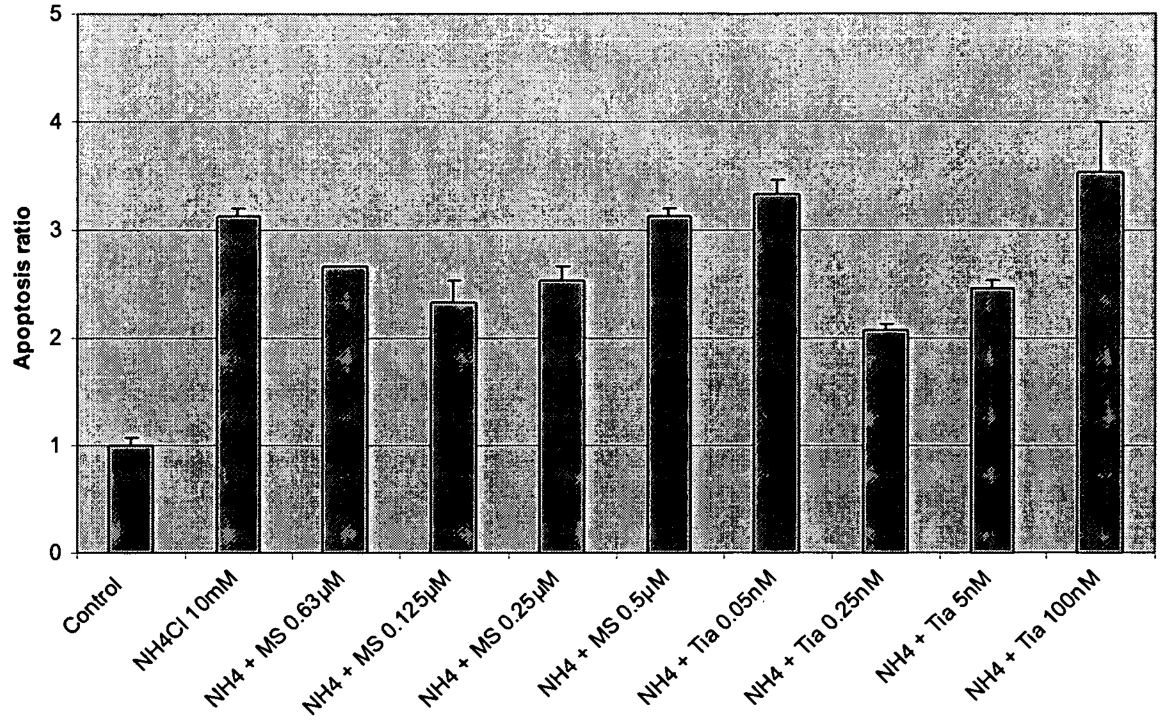


FIGURE 25

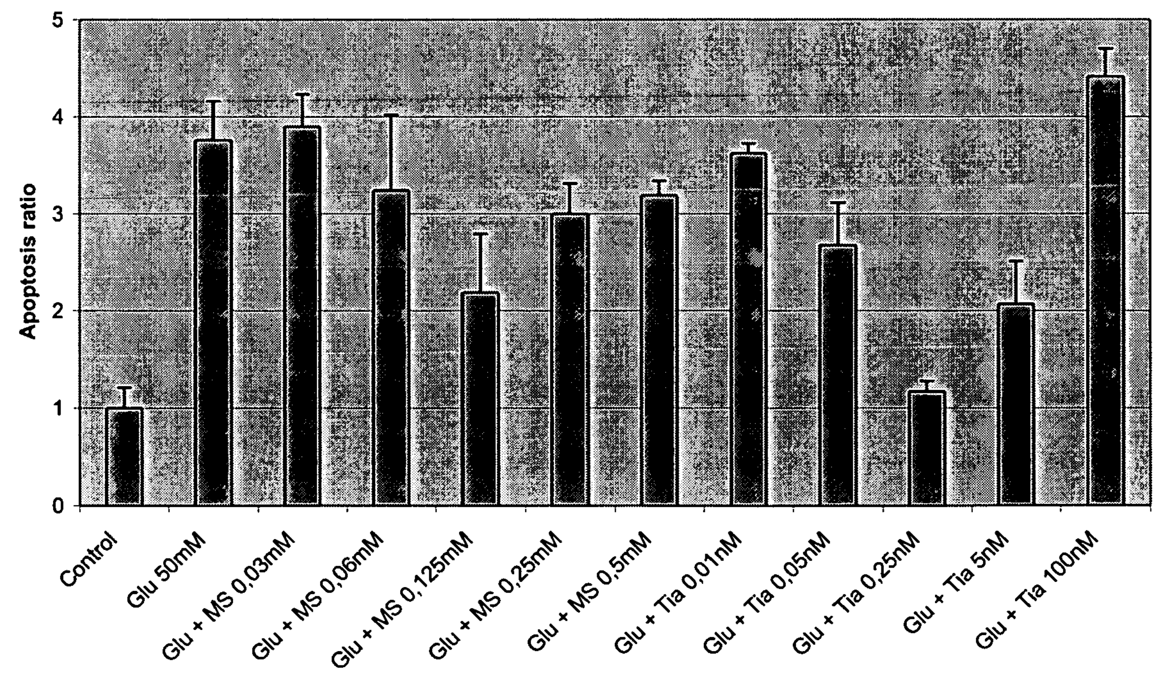


FIGURE 26

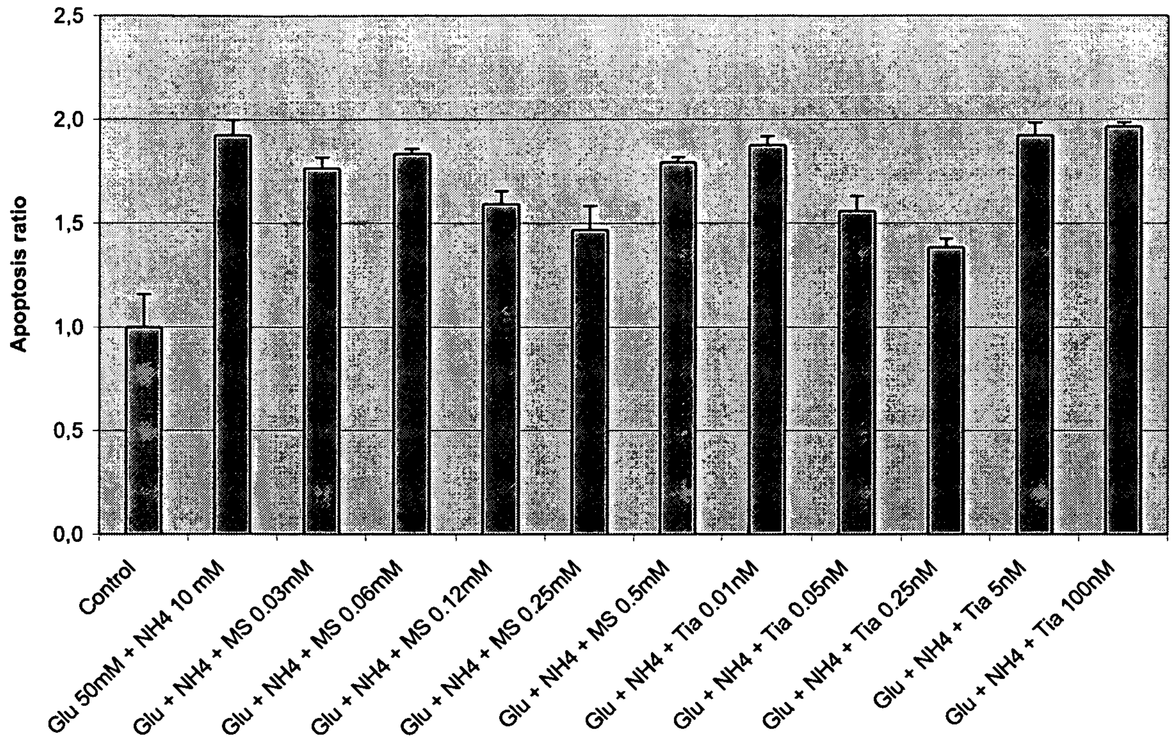
2,5
2,0
1,5
1,0
0,5
0,0
Apoptosis ratio
Control
Glu 50mM + NH4 10 mM
Glu + NH4 + MS 0.03mM
Glu + NH4 + MS 0.06mM
Glu + NH4 + MS 0.12mM
Glu + NH4 + MS 0.25mM
Glu + NH4 + MS 0.5mM
Glu + NH4 + Tia 0.01nM
Glu + NH4 + Tia 0.05nM
Glu + NH4 + Tia 0.25nM
Glu + NH4 + Tia 5nM
Glu + NH4 + Tia 100nM

FIGURE 27

FIGURE 28

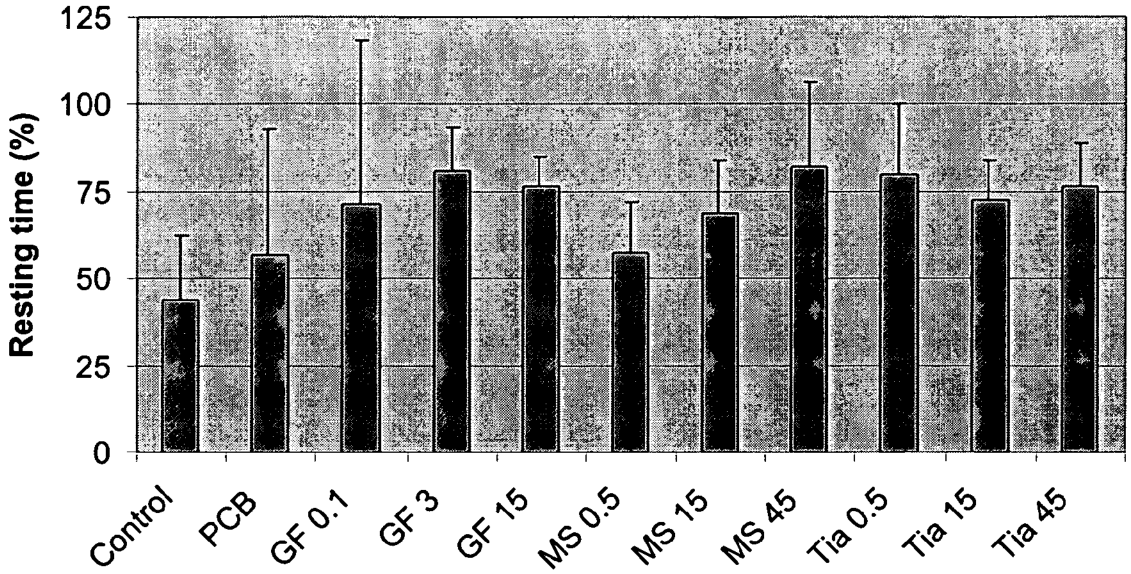


FIGURE 29

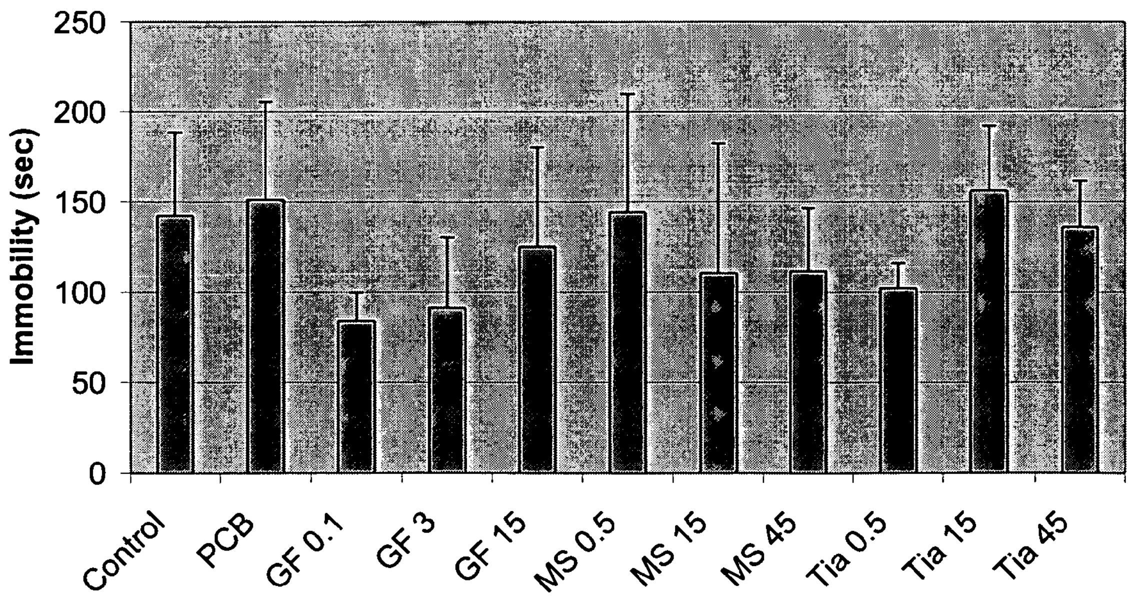

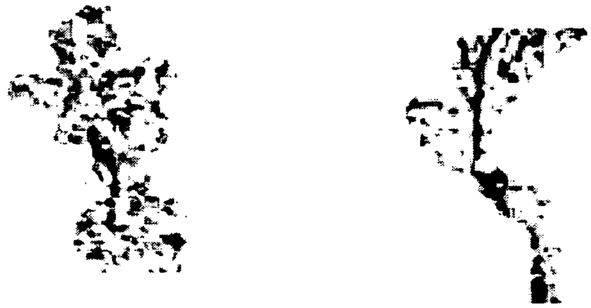

Control Placebo

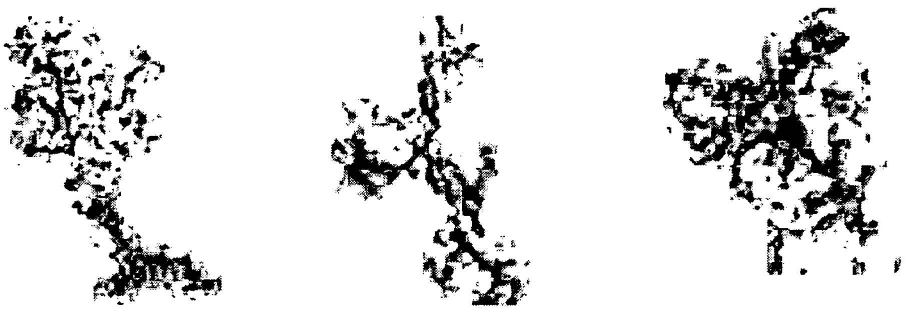

Glufosinate L-Methionine sulfoximine Tianeptine

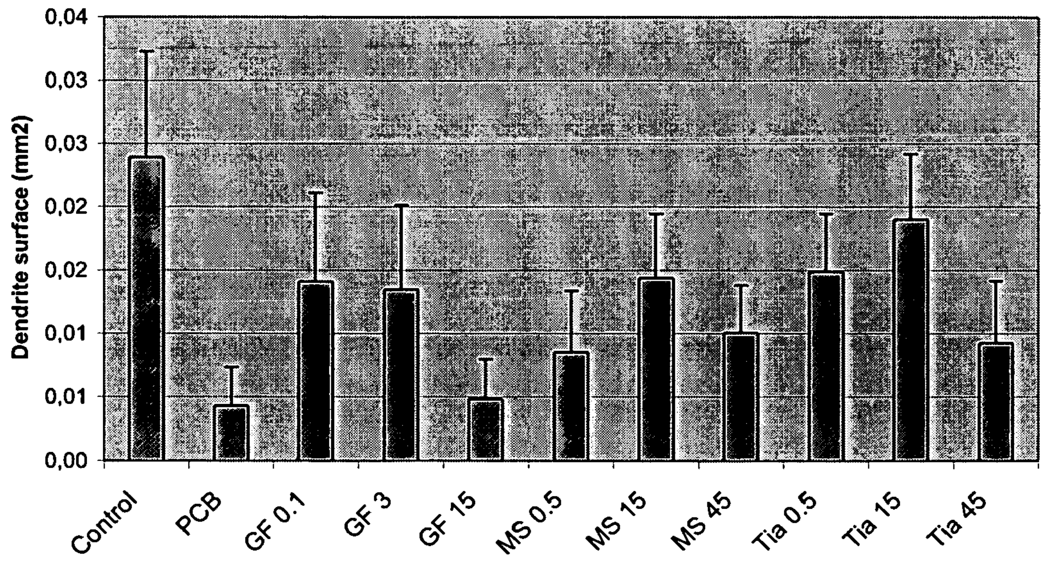


**FIGURE 30**

FIGURE 31A

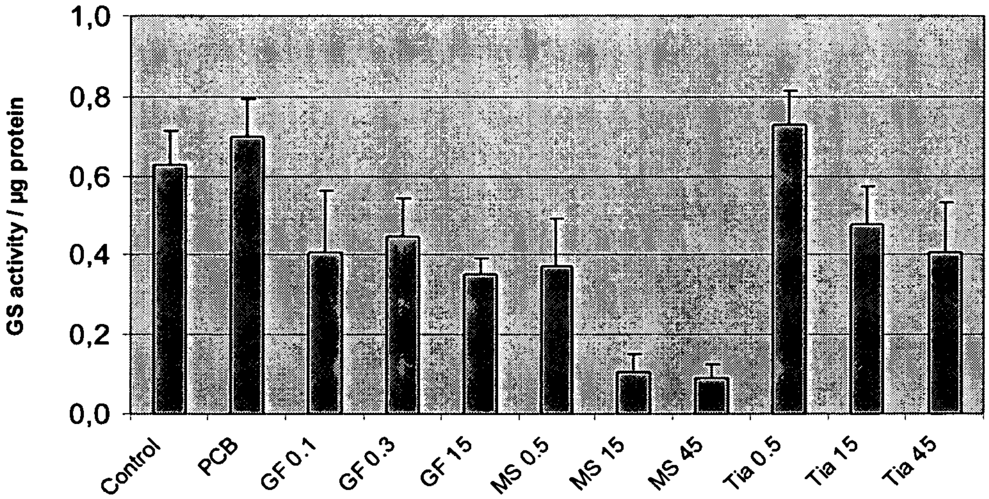


FIGURE 31B

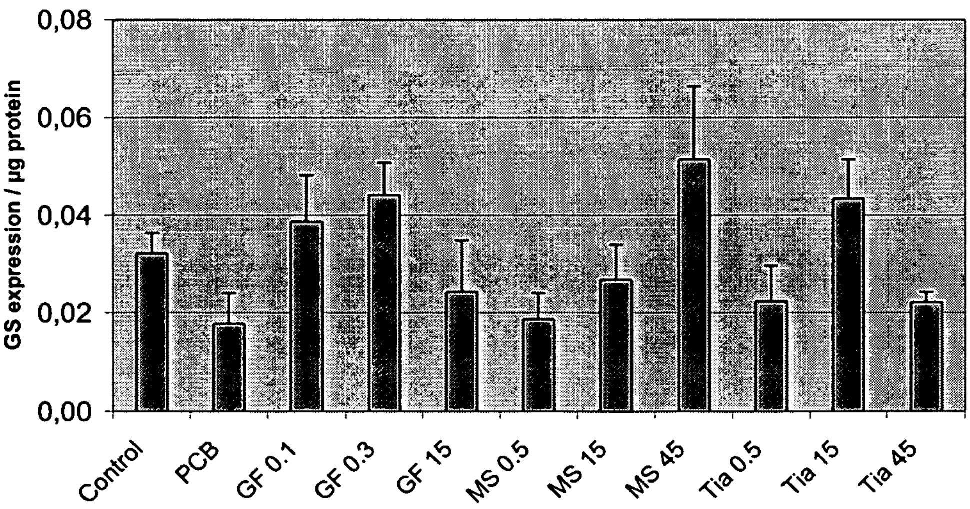

FIGURE 32A

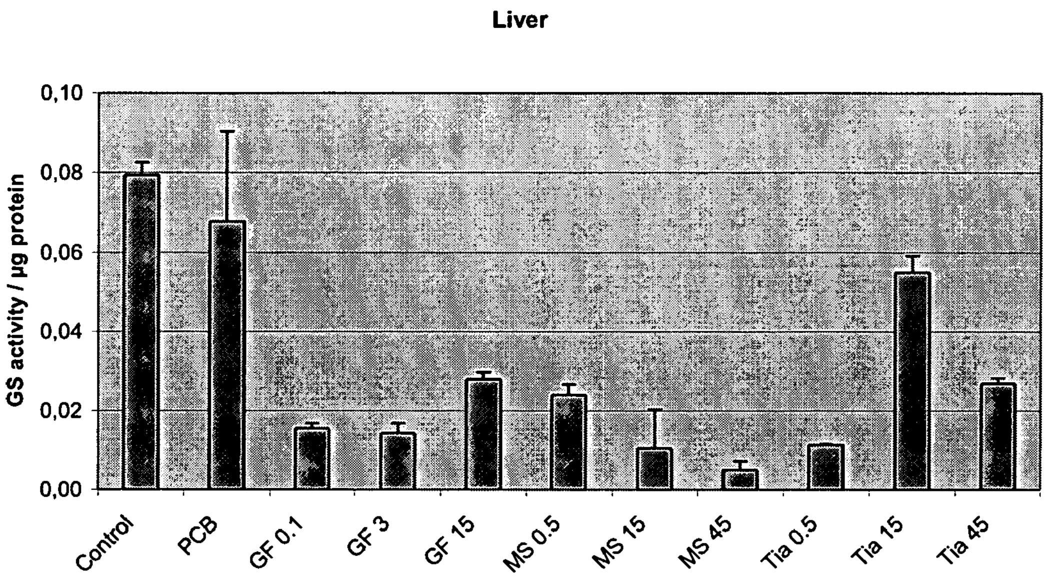


FIGURE 32B

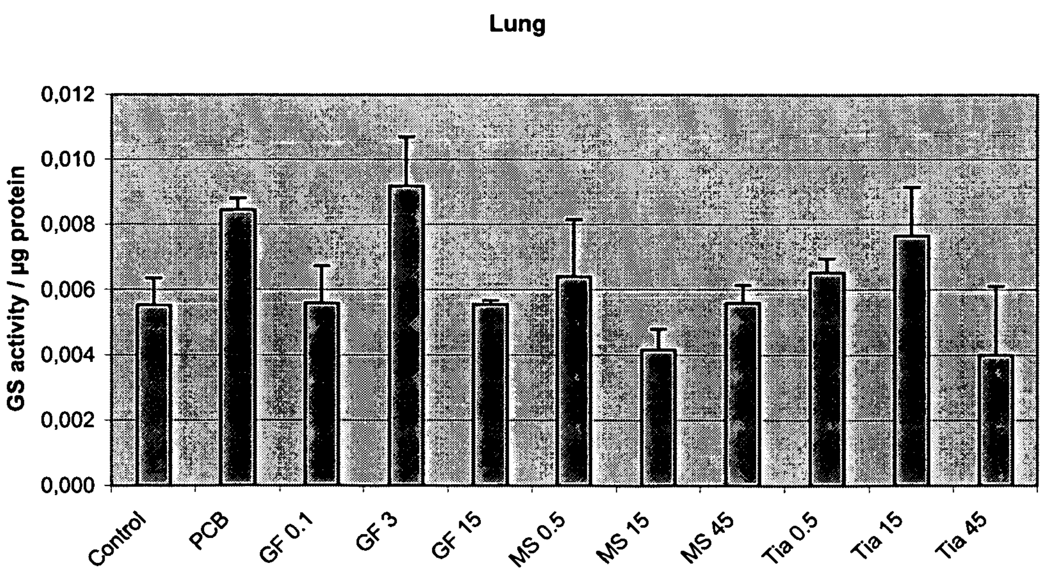

FIGURE 33A

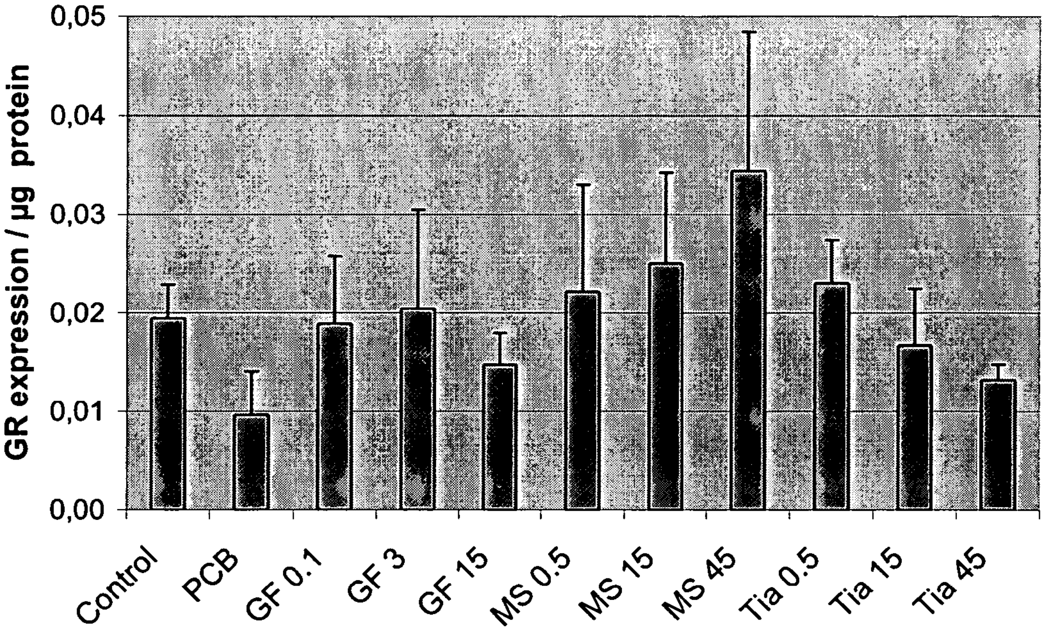


FIGURE 33 B

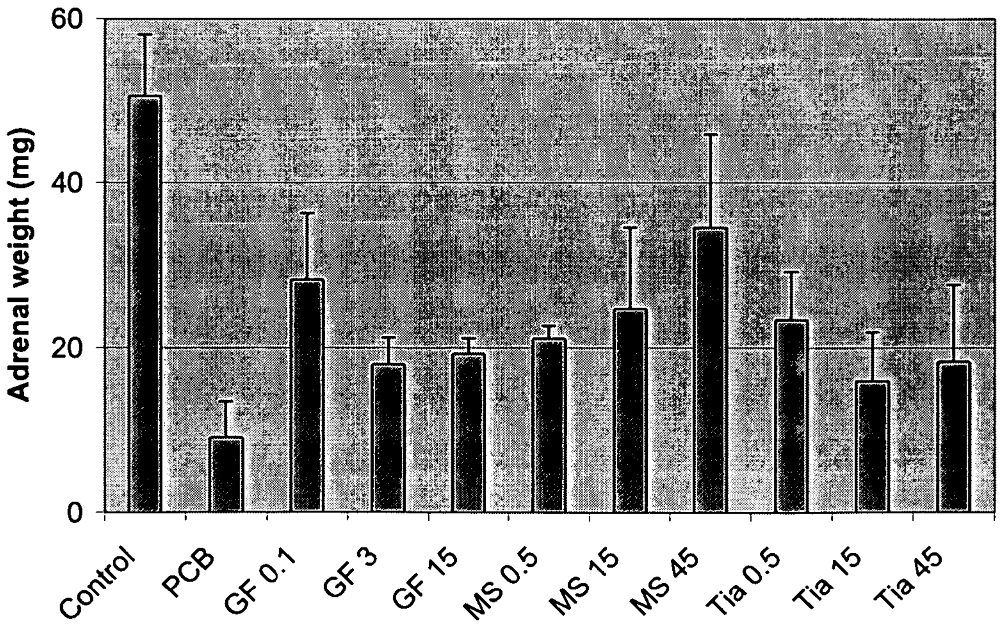

European Patent Office

# EUROPEAN SEARCH REPORT

Application Number
EP 05 29 1692

| Category | Citation of document with indication, where appropriate, of relevant passages | Relevant to claim | CLASSIFICATION OF THE APPLICATION (IPC) |
|---|---|---|---|
| X | US 2004/152778 A1 (BRUSILOW WILLIAM S) 5 August 2004 (2004-08-05) * paragraphs [0020], [0021]; claims 1,6,10,11 * | 1-12, 20-25 | INV. A61K31/00 A61K45/00 A61K31/198 G01N33/00 A61P7/00 A61K31/554 A61K31/663 |
| A | US 5 739 082 A (DONN ET AL) 14 April 1998 (1998-04-14) * the whole document * | 1-12 | |
| X | URI J V ET AL: "Phosphorus/phosphonic acid-containing antimicrobial antibiotics" ACTA MICROBIOLOGICA HUNGARICA 1988 HUNGARY, vol. 35, no. 3, 1988, pages 221-257, XP009056818 ISSN: 0231-4622 * page 231 - page 232 * | 1-12, 20-25 | |
| X | US 2003/144357 A1 (BRUSILOW SAUL ET AL) 31 July 2003 (2003-07-31) * the whole document * | 1-12, 20-25 | TECHNICAL FIELDS SEARCHED (IPC) |
| X | GOBEC S ET AL: "Aminophosphonic acids and phosphapeptides" FARMACEVTSKI VESTNIK 1996 SLOVENIA, vol. 47, no. 4, 1996, pages 391-404, XP009056811 ISSN: 0014-8229 * abstract * | 1-12, 20-25 | A61K A61P G01N |

-/--

~~The present search report has been drawn up for all claims~~

13

| Place of search | Date of completion of the search | Examiner |
|---|---|---|
| Munich | 26 April 2006 | Blott, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 05 29 1692

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | RUHLAND MONIKA ET AL: "A comparative investigation of the metabolism of the herbicide glufosinate in cell cultures of transgenic glufosinate-resistant and non-transgenic oilseed rape (Brassica napus) and corn (Zea mays)." ENVIRONMENTAL BIOSAFETY RESEARCH, vol. 1, October 2002 (2002-10), pages 29-37, XP001207600 ISSN: 1635-7922 * abstract * ----- | 7,8 | |
| D,X | EISENBERG DAVID ET AL: "Structure-function relationships of glutamine synthetases" BIOCHIMICA ET BIOPHYSICA ACTA, vol. 1477, no. 1-2, 7 March 2000 (2000-03-07), pages 122-145, XP000427890 ISSN: 0006-3002 * table 3 * ----- | 7,8 | TECHNICAL FIELDS SEARCHED (IPC) |
| X,D | WEDLER F C ET AL: "INTERACTION OF A NEW GAMMA GLUTAMYL PHOSPHATE ANALOG 4 PHOSPHONOACETYL-L-ALPHA AMINO BUTYRATE WITH GLUTAMINE SYNTHETASE EC-6.3.1.2 ENZYMES FROM ESCHERICHIA-COLI PLANT AND MAMMALIAN SOURCES" ARCHIVES OF BIOCHEMISTRY AND BIOPHYSICS, vol. 202, no. 2, 1980, pages 482-490, XP009056913 ISSN: 0003-9861 * abstract * ----- -/-- | 7,8 | |

~~The present search report has been drawn up for all claims~~

13

| Place of search | Date of completion of the search | Examiner |
|---|---|---|
| Munich | 26 April 2006 | Blott, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.................................................................
& : member of the same patent family, corresponding document

EPO FORM 1503 03.82 (P04C01)

European Patent Office

# EUROPEAN SEARCH REPORT

Application Number
EP 05 29 1692

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | FIRSOVA N A ET AL: "Inhibition of glutamine synthetase activity by biologically active derivatives of glutamic acid" BIOKHIMIYA 1986 RUSSIA, vol. 51, no. 5, 1986, pages 850-855, XP009056908 * abstract * ----- | 7,8 | |
| X,D | LEJCZAK B ET AL: "INHIBITION OF RAT LIVER GLUTAMINE SYNTHETASE EC-6.3.1.2 BY PHOSPHONIC ANALOGS OF GLUTAMIC-ACID" EXPERIENTIA (BASEL), vol. 37, no. 5, 1981, pages 461-462, XP009056930 ISSN: 0014-4754 * the whole document * ----- | 7,8 | |
| X | MCEWEN B S ET AL: "Molecular mechanisms of neuroplasticity and pharmacological implications: the example of tianeptine" EUROPEAN NEUROPSYCHOPHARMACOLOGY, ELSEVIER SCIENCE PUBLISHERS BV, AMSTERDAM, NL, vol. 14, December 2004 (2004-12), pages S497-S502, XP004646253 ISSN: 0924-977X * the whole document * ----- | 13,14, 16-19 | TECHNICAL FIELDS SEARCHED (IPC) |
| X | US 6 599 896 B1 (DESLANDES ANTOINE ET AL) 29 July 2003 (2003-07-29) * column 2, lines 49-52 * * column 5, lines 9-13 * ----- -/-- | 13,14, 16-19 | |

~~The present search report has been drawn up for all claims~~

13

| Place of search | Date of completion of the search | Examiner |
|---|---|---|
| Munich | 26 April 2006 | Blott, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 05 29 1692

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CEYHAN M ET AL: "Investigation of the effects of tianeptine and fluoxetine on pentylenetetrazole-induced seizures in rats" JOURNAL OF PSYCHIATRIC RESEARCH, OXFORD, GB, vol. 39, no. 2, March 2005 (2005-03), pages 191-196, XP004678011 ISSN: 0022-3956 * abstract * * page 194 * ----- | 13,14, 16-19 | |
| D,X | US 6 683 072 B1 (KUCHARIK ROBERT F ET AL) 27 January 2004 (2004-01-27) * the whole document * ----- | 13,14, 16-19 | |
| X | MCEWEN B S: "Antidepressant modulation of isolation and restraint stress effects on brain chemistry and morphology" EUROPEAN PSYCHIATRY, ELSEVIER, PARIS, FR, vol. 8, no. SUPPL 2, 1993, pages 41S-48S, XP002110223 ISSN: 0767-399X * page 44S, column 2 * ----- | 18,19 | TECHNICAL FIELDS SEARCHED (IPC) |
| X | TOON S ET AL: "Pharmacokinetic and pharmacodynamic interaction between the antidepressant tianeptine and oxazepam at steady-state." PSYCHOPHARMACOLOGY. 1990, vol. 101, no. 2, 1990, pages 226-232, XP009056704 ISSN: 0033-3158 * abstract * ----- | 18,19 | |
| X | US 2004/186111 A1 (SUN QUN ET AL) 23 September 2004 (2004-09-23) * paragraphs [0248], [3236], [3247] * ----- -/-- | 18,19 | |

~~The present search report has been drawn up for all claims~~

13

| Place of search | Date of completion of the search | Examiner |
|---|---|---|
| Munich | 26 April 2006 | Blott, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..............................................................
& : member of the same patent family, corresponding document

EPO FORM 1503 03.82 (P04C01)

European Patent Office

# EUROPEAN SEARCH REPORT

Application Number
EP 05 29 1692

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZINI R ET AL: "THE INFLUENCE OF VARIOUS DRUGS ON THE BINDING OF THIANEPTIN TO HUMAN PLASMA PROTEINS" INTERNATIONAL JOURNAL OF CLINICAL PHARMACOLOGY, THERAPY AND TOXICOLOGY, DUSTRI- VERLAG FEISTLE, DEISENHOFEN/MUENCHEN, DE, vol. 29, 1991, pages 64-66, XP000912070 ISSN: 0174-4879 * abstract * ----- | 18,19 | |
| X | TAKAHASHI H ET AL: "Glutamine synthetase inhibition prevents cerebral oedema during hyperammonemia." ACTA NEUROCHIRURGICA. SUPPLEMENTUM. 1990, vol. 51, 1990, pages 346-347, XP009056806 ISSN: 0065-1419 * the whole document * ----- | 20-25 | |
| X | EP 1 532 976 A (HOLT, JOHN ALFRED GORTON; HOLT, MARK ROWAN GORTON) 25 May 2005 (2005-05-25) * the whole document * * paragraph [0082] * ----- | 20-25 | TECHNICAL FIELDS SEARCHED (IPC) |
| X | WO 2004/028448 A (MILLER, KENNETH, E) 8 April 2004 (2004-04-08) * claims * ----- | 20-25 | |
| X | SWANSON R A ET AL: "METHIONINE SULFOXIMINE REDUCES CORTICAL INFARCT SIZE IN RATS AFTER MIDDLE CEREBRAL ARTERY OCCLUSION" STROKE, vol. 21, no. 2, 1990, pages 322-327, XP002378399 ISSN: 0039-2499 * abstract * ----- | 20-25 | |

13

~~The present search report has been drawn up for all claims~~

| Place of search | Date of completion of the search | Examiner |
|---|---|---|
| Munich | 26 April 2006 | Blott, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

Application Number

EP 05 29 1692

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☒ Only part of the further search fees have been paid within the fixed time limit. The present European search report has been drawn up for those parts of the European patent application which relate to the inventions in respect of which search fees have been paid, namely claims:

1-25

☐ None of the further search fees have been paid within the fixed time limit. The present European search report has been drawn up for those parts of the European patent application which relate to the invention first mentioned in the claims, namely claims:

European Patent Office

**LACK OF UNITY OF INVENTION SHEET B**

Application Number
EP 05 29 1692

The Search Division considers that the present European patent application does not comply with the requirements of unity of invention and relates to several inventions or groups of inventions, namely:

1. claims: 1-12,20-25

   Glufosinate, pharmaceutical compositions comprising glufosinate and their use for treating disorders which involve or can be influenced by glutamine synthetase activity

   ---

2. claims: 13-19

   Use of tianeptine for treating disorders which involve or can be influenced by glutamine synthetase activity and pharmaceutical compositions comprising tianeptine in combination with a drug selected from those cited in claim 18

   ---

3. claims: 20-25

   Use of a glutamine synthetase ligand, with the exception of tianeptine, preferably L-methionine sulfoximine, for treating disorders which involve or can be influenced by glutamine synthetase activity

   ---

4. claims: 20-25

   Use of a glutamine synthetase ligand, with the exception of tianeptine, preferably hydrazines, for treating disorders which involve or can be influenced by glutamine synthetase activity

   ---

5. claims: 20-25

   Use of a glutamine synthetase ligand, with the exception of tianeptine, preferably bisphosphonates, for treating disorders which involve or can be influenced by glutamine synthetase activity

   ---

6. claim: 26

   Use of tianeptine for the preparation of a composition aiming to facilitate the growth of non-vertebrate organisms with eukaryotic cells

   ---

7. claim: 27

European Patent Office

LACK OF UNITY OF INVENTION
SHEET B

Application Number
EP 05 29 1692

The Search Division considers that the present European patent application does not comply with the requirements of unity of invention and relates to several inventions or groups of inventions, namely:

Use of glufosinate for the preparation of a composition aiming to facilitate the growth of non-vertebrate organisms with eukaryotic cells

---

8. claim: 27

Use of MS for the preparation of a composition aiming to facilitate the growth of non-vertebrate organisms with eukaryotic cells

---

9. claim: 27

Use of a glutamine synthetase ligand selected from the group of hydrazines for the preparation of a composition aiming to facilitate the growth of non-vertebrate organisms with eukaryotic cells

---

10. claim: 27

Use of a glutamine synthetase ligand selected from the group of bisphosphonates for the preparation of a composition aiming to facilitate the growth of non-vertebrate organisms with eukaryotic cells

---

11. claims: 28-32

Method for screening, identifying and/or developing a new active compound, activating and/or modulating/regulating glutamine synthetase activity comprising an assay on glutamine synthetase or a fragment thereof with a candidate compound and a glutamine synthetase ligand selected from the group consisting of tianeptine, glufosinate, L-methionine sulfoximine, hydrazines and bisphosphonates

---

## ANNEX TO THE EUROPEAN SEARCH REPORT ON EUROPEAN PATENT APPLICATION NO. EP 05 29 1692

This annex lists the patent family members relating to the patent documents cited in the above-mentioned European search report.
The members are as contained in the European Patent Office EDP file on
The European Patent Office is in no way liable for these particulars which are merely given for the purpose of information.

26-04-2006

| Patent document cited in search report | Publication date | Patent family member(s) | Publication date |
|---|---|---|---|
| US 2004152778 A1 | 05-08-2004 | NONE | |
| US 5739082 A | 14-04-1998 | NONE | |
| US 2003144357 A1 | 31-07-2003 | AU 2003205362 A2 | 02-09-2003 |
| | | CA 2474813 A1 | 07-08-2003 |
| | | EP 1480632 A1 | 01-12-2004 |
| | | JP 2005516976 T | 09-06-2005 |
| | | WO 03063857 A1 | 07-08-2003 |
| US 6599896 B1 | 29-07-2003 | AU 3824800 A | 23-10-2000 |
| | | BR 0008703 A | 13-02-2002 |
| | | CA 2361988 A1 | 12-10-2000 |
| | | CN 1342082 A | 27-03-2002 |
| | | EP 1165089 A1 | 02-01-2002 |
| | | WO 0059511 A1 | 12-10-2000 |
| | | FR 2791891 A1 | 13-10-2000 |
| | | HU 0200145 A2 | 29-05-2002 |
| | | JP 2002541110 T | 03-12-2002 |
| | | NO 20014081 A | 22-08-2001 |
| | | NZ 513565 A | 30-04-2004 |
| | | PL 356739 A1 | 28-06-2004 |
| | | ZA 200106690 A | 14-11-2002 |
| US 6683072 B1 | 27-01-2004 | CA 2515048 A1 | 19-08-2004 |
| | | EP 1594509 A2 | 16-11-2005 |
| | | WO 2004069188 A2 | 19-08-2004 |
| US 2004186111 A1 | 23-09-2004 | NONE | |
| EP 1532976 A | 25-05-2005 | AU 2004231179 A1 | 02-06-2005 |
| WO 2004028448 A | 08-04-2004 | AU 2003294221 A1 | 19-04-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- FR 2104728
- FR 2635461
- FR 2716623
- US 6599896 B
- US 6683072 B
- EP 275957 A
- US 3758528 A
- US 6013660 A
- WO 2005040820 A

### Non-patent literature cited in the description


- **ABCOUWER, S.F. ; LUKASCEWICZ, G.C. ; RYAN, U.S. ; SOUBA, W.W.** Molecular regulation of lung endothelial glutamine synthetase expression. *Surgery,* 1995, vol. 118, 325-34
- **ABCOUWER, S.F. ; LUKASZEWICZ, G.C. ; SOUBA, W.W.** Glucocorticoids regulate glutamine synthetase expression in lung epithelial cells. *Am J Physiol,* 1996, vol. 270, L141-51
- **ABELL, L.M. ; SCHINELLER, J. ; KECK, P.J. ; VILLAFRANCA, J.J.** Effect of metal-ligand mutations on phosphoryl transfer reactions catalyzed by Escherichia coli glutamine synthetase. *Biochemistry,* 1995, vol. 34, 16695-702
- **ABELL, L.M. ; VILLAFRANCA, J.J.** Investigation of the mechanism of phosphinothricin inactivation of Escherichia coli glutamine synthetase using rapid quench kinetic technique. *Biochemistry,* 1991, vol. 30, 6135-41
- **ALBRECHT, J.** Glucose-derived osmolytes and energy impairment in brain edema accompanying liver failure: the role of glutamine reevaluated. *Gastroenterology,* 2003, vol. 125, 976-78
- **ANAND, A. ; CHARNEY, D.S. ; OREN, D.A. ; BERMAN, R.M. ; HU, X.S. ; CAPPIELLO, A. ; KRYSTAL, J.H.** Attenuation of the neuropsychiatric effects of ketamine with lamotrigine: support for hyperglutamatergic effects of N-methyl-D-aspartate receptor antagonists. *Arch Gen Psychiatry,* 2000, vol. 57, 270-6
- **ANANDARAJ, S.J. ; JAYARAM, H.N. ; COONEY, D.A. ; TYAGI, A.K. ; HAN, N ; THOMAS, J.H. ; CHITNIS, M. ; MONTGOMERY, J.A.** Interaction of L-alanosine (NSC 153, 353) with enzymes metabolizing L-aspartic acid, L-glutamic acid and their amides. *Biochem Pharmacol,* 1980, vol. 29, 227-45
- **ARAD, G. ; FREIKOPF, A. ; KULKA, R.G.** Glutamine-stimulated modification and degradation of glutamine synthetase in hepatoma tissue culture cells. *Cell,* 1976, vol. 8, 95-101
- **ARAD, G. ; KULKA, R.G.** Effects of glutamine, methionine sulfone and dexamethasone on rates of synthesis of glutamine synthetase in cultured hepatoma cells. *Biochim Biophys Acta,* 1978, vol. 544, 153-62
- **ARDAWI, M.S.** Glutamine-synthesizing activity in lungs of fed, starved, acidotic, diabetic, injured and septic rats. *Biochem J,* 1990, vol. 270, 829-32
- **ARDAWI, M.S. ; MAJZOUB, M.F.** Glutamine metabolism in skeletal muscle of septic rats. *Metabolism,* 1991, vol. 40, 155-64
- **ARDAWI, M.S. ; NEWSHOLME, E.A.** Glutamine metabolism in lymphocytes of the rat. *Biochem J,* 1983, vol. 212, 835-42
- **AULT, B. ; HILDEBRAND, L.M.** L-glutamate activates peripheral nociceptors. *Agents Actions,* 1993, vol. 39, C142-4
- **BABIJ, P. ; HUNDAL, H.S. ; RENNIE, M.J. ; WATT P.W.** Effects of corticosteroids on glutamine transport in rat skeletal muscle. *J Physiol Lond,* 1986, vol. 347, 35P
- **LE BACQUER, O. ; NAZIH, H. ; BLOTTIERE, H. ; MEYNIAL-DENIS, D. ; LABOISSE, C. ; DARMAUN, D.** Effects of glutamine deprivation on protein synthesis in a model of human enterocytes in culture. *Am J Physiol Gastrointest Liver Physiol,* 2001, vol. 281, G 1340-7
- **BAINS, J.S. ; SHAW, C.A.** Neurodegenerative disorders in humans: the role of glutathione in oxidative stress-mediated neuronal death. *Brain Res Brain Res Rev,* 1997, vol. 25, 335-58
- **BARNETT, N.L. ; POW, D.V. ; ROBINSON, S.R.** Inhibition of Muller cell glutamine synthetase rapidly impairs the retinal response to light. *Glia,* 2000, vol. 30, 64-73
- **BAYER, E. ; GUGEL, K.H. ; HAGELE, K. ; HAGENMAIER, H. ; JESSIPOW, S. ; KONIG, W.A. ; ZAHNER, H.** Metabolic products of microorganisms. 98. Phosphinothricin and phosphinothricyl-alanyl-analine. *Helv Chim Acta,* 1972, vol. 55, 224-39

- **BEAL, M.F.** Mechanisms of excitotoxicity in neurologic diseases. *FASEB J,* 1992, vol. 6, 3338-44
- **BERTRAND, G. ; GROSS, R. ; PUECH, R. ; LOUBATIERES-MARIANI, M.M. ; BOCKAERT, J.** Glutamate stimulates glucagon secretion via an excitatory amino acid receptor of the AMPA subtype in rat pancreas. *Eur J Pharmacol,* 1993, vol. 237, 45-50
- **BEUTLER, E.** Nutritional and metabolic aspects of glutathione. *Annu Rev Nutr,* 1989, vol. 9, 287-302
- **BHANGU, P.S. ; GENEVER, P.G. ; SPENCER, G.J. ; GREWAL, T.S. ; SKERRY, T.M.** Evidence for targeted vesicular glutamate exocytosis in osteoblasts. *Bone,* 2001, vol. 29, 16-23
- **BIRCH, M.A. ; GENEVER, P.G. ; LAKETIC-LJUBOJEVIC, I. ; PATTON, A.J. ; PEET, N.M. ; SKERRY, T.M.** Glutamate receptor activation is necessary for bone formation in vitro. *J Bone Miner Res,* 1997, vol. 12, 010
- **BJARNASON, I. ; MACPHERSON, A. ; HOLLANDER, D.** Intestinal permeability: an overview. *Gastroenterology,* 1995, vol. 108, 1566-81
- **BOELENS, P.G. ; HOUDIJK, A.P. ; DE THOUARS, H.N. ; TEERLINK, T. ; VAN ENGELAND, M.I. ; HAARMAN, H.J. ; VAN LEEUWEN, P.A.** Plasma taurine concentrations increase after enteral glutamine supplementation in trauma patients and stressed rats. *Am J Clin Nutr,* 2003, vol. 77, 250-6
- **BRECHLINA, P. ; UNTERHALT, A. ; TISCHNER, R. ; MACK, G.** Cytosolic and chloroplastic glutamine synthetase of sugarbeet (Beta vulgaris) respond differently to organ ontogeny and nitrogen source. *Physiologia Plantarum,* 2000, vol. 108, 263-69
- **BREMNER, J.D. ; KRYSTAL, J.H. ; SOUTHWICK, S.M. ; CHARNEY, D.S.** Functional neuroanatomical correlates of the effects of stress on memory. *J Trauma Stress,* 1995, vol. 8, 527-53
- **BRONSTEIN, J.M. ; FARBER, D.B. ; WASTERLAIN, C.G.** Regulation of type-II calmodulin kinase: functional implications. *Brain Res Brain Res Rev,* 1993, vol. 18, 135-47
- **BUSS, J.L. ; NEUZIL, J. ; PONKA, P.** Oxidative stress mediates toxicity of pyridoxal isonicotinoyl hydrazone analogs. *Arch Biochem Biophys,* 2004, vol. 421, 1-9
- **BUTTERWORTH, R.F.** Portal-systemic encephalopathy: a disorder of neuron-astrocytic metabolic trafficking. *Dev Neurosci,* 1993, vol. 15, 313-9
- **CAMPBELL, P.N. ; WORK, T.S. ; MELLANBY, E.** Isolation of a crystalline toxic factor from agenized wheat flour. *Nature (London),* 1950, vol. 165, 345-46
- **CAO, Y. ; FENG, Z. ; HOOS, A. ; KLIMBERG, V.S.** Glutamine enhances gut glutathione production. *JPENJParenter Enteral Nutr,* 1998, vol. 22, 224-7
- **CARLTON, S.M. ; HARGETT, G.L. ; COGGESHALL, R.E.** Localization and activation of glutamate receptors in unmyelinated axons of rat glabrous skin. *Neurosci Lett,* 1995, vol. 197, 25-8
- **CASTANON, N. ; BLUTHE, R.M. ; DANTZER, R.** Chronic treatment with the atypical antidepressant tianeptine attenuates sickness behavior induced by peripheral but not central lipopolysaccharide and interleukin-1beta in the rat. *Psychopharmacology (Berl),* 2001, vol. 154, 50-60
- **CASTANON, N. ; KONSMAN, J.P. ; MEDINA, C. ; CHAUVET, N. ; DANTZER, R.** Chronic treatment with the antidepressant tianeptine attenuates lipopolysaccharide-induced Fos expression in the rat paraventricular nucleus and HPA axis activation. *Psychoneuroendocrinology,* 2003, vol. 28, 19-34
- **CASTILHO, R.F. ; KOWALTOWSKI, A.J. ; MEINICKE, A.R. ; BECHARA, E.J. ; VERCESI, A.E.** Permeabilization of the inner mitochondrial membrane by Ca2+ ions is stimulated by t-butyl hydroperoxide and mediated by reactive oxygen species generated by mitochondria. *Free Radic Biol Med,* 1995, vol. 18, 479-86
- **CHANDRASEKHAR, S. ; SOUBA, W.W. ; ABCOUWER, S.F.** Identification of glucocorticoid-responsive elements that control transcription of rat glutamine synthetase. *Am J Physiol,* 1999, vol. 276, L319-31
- **CHAUDHARI, N. ; LANDIN, A.M. ; ROPER, S.D.** A metabotropic glutamate receptor variant functions as a taste receptor. *Nat Neurosci,* 2000, vol. 3, 113-9
- **CHENU, C.** Glutamatergic innervation in bone. *Microsc Res Tech,* 2002, vol. 58, 70-6
- **CHENU, C. ; SERRE, C.M. ; RAYNAL, C. ; BURT-PICHAT, B. ; DELMAS, P.D.** Glutamate receptors are expressed by bone cells and are involved in bone resorption. *Bone,* 1998, vol. 22, 295-9
- **CLARKE, V.R. ; BALLYK, B.A. ; HOO, K.H. ; MANDELZYS, A. ; PELLIZZARI, A. ; BATH, C.P. ; THOMAS, J. ; SHARPE, E.F. ; DAVIES, C.H. ; ORNSTEIN, P.L.** A hippocampal GluR5 kainate receptor regulating inhibitory synaptic transmission. *Nature,* 1997, vol. 389, 599-603
- **COCK, J.M. ; BROCK, I.W. ; WATSON, A.T. ; SWARUP, R. ; MORBY, A.P. ; CULLIMORE, J.V.** Regulation of glutamine synthetase genes in leaves of Phaseolus vulgaris. *Plant Mol Biol,* 1991, vol. 17, 761-71
- **CONRAD, C.D. ; GALEA, L.A. ; KURODA, Y. ; MCEWEN, B.S.** Chronic stress impairs rat spatial memory on the Y maze, and this effect is blocked by tianeptine pretreatment. *BehavNeurosci,* 1996, vol. 110, 1321-34

- **CONVIT, A. ; DE LEON, M.J. ; TARSHISH, C. ; DE SANTI, S. ; TSUI, W. ; RUSINEK, H. ; GEORGE, A.** Specific hippocampal volume reductions in individuals at risk for Alzheimer's disease. *Neurobiol Aging,* 1997, 131-8
- **COOPER, A.J. ; MORA, S.N. ; CRUZ, N.F. ; GELBARD, A.S.** Cerebral ammonia metabolism in hyperammonemic rats. *J Neurochem,* 1985, vol. 44, 1716-23
- **COOPER, A.J. ; PLUM, F.** Biochemistry and physiology of brain ammonia. *Physiol Rev,* 1987, vol. 67, 440-519
- **COSENTINO, M. ; DE PONTI, F. ; MARINO, F. ; GIARONI, C. ; LEONI, O. ; LECCHINI, S. ; FRIGO, G.** N-methyl-D-aspartate receptors modulate neurotransmitter release and peristalsis in the guinea pig isolated colon. *Neurosci Lett,* 1995, vol. 183, 139-42
- **CURI, R. ; NEWSHOLME, P. ; PITHON-CURI, T.C. ; PIRES-DE-MELO, M. ; GARCIA, C. ; HOMEM-DE-BITTENCOURT JUNIOR, P.I. ; GUIMARAES, A.R.** Metabolic fate of glutamine in lymphocytes, macrophages and neutrophils. *Braz J Med Biol Res,* 1999, vol. 32, 15-21
- **CUTHBERTSON, D.P.** Second annual Jonathan E. Rhoads Lecture. The metabolic response to injury and its nutritional implications: retrospect and prospect. *JPEN J Parenter Enteral Nutr,* 1979, vol. 3, 108-29
- **DANTZER, R. ; BLUTHE, R.M. ; LAYE, S. ; BRET-DIBAT, J.L. ; PARNET, P. ; KELLEY, K.W.** Cytokines and sickness behavior. *Ann N YAcad Sci,* 1998, vol. 840, 586-90
- **DANTZER, R. ; WOLLMAN, E. ; VITKOVIC, L. ; YIRMIYA, R.** Cytokines and depression: fortuitous or causative association?. *Mol Psychiatry,* 1999, vol. 4, 328-32
- **DARLING, H.F.** Isocarboxazid (marplan) in ambulatory psychiatric patients. *Am J Psychiatry,* 1959, vol. 116, 355-6
- **DAVID, P. ; LUSKY, M. ; TEICHBERG, V.I.** Involvement of excitatory neurotransmitters in the damage produced in chick embryo retinas by anoxia and extracellular high potassium. *Exp Eye Res,* 1988, vol. 46, 657-62
- **DAWSON, V.L. ; DAWSON, T.M. ; BARTLEY, D.A. ; UHL G.R. ; SNYDER SH.** Mechanisms of nitric oxide-mediated neurotoxicity in primary brain cultures. *J Neurosci,* 1993, vol. 13, 2651-61
- **DAWSON, V.L. ; DAWSON, T.M. ; LONDON, E.D. ; BREDT, D.S. ; SNYDER, S.H.** Nitric oxide mediates glutamate neurotoxicity in primary cortical cultures. *Proc Natl Acad Sci U S A,* 1991, vol. 88, 6368-71
- **DE GRAAF, A.A. ; DEUTZ, N.E. ; BOSMAN, D.K. ; CHAMULEAU, R.A. ; DE HAAN, J.G. ; BOVEE, W.M.** The use of in vivo proton NMR to study the effects of hyperammonemia in the rat cerebral cortex. *NMR Biomed,* 1991, vol. 4, 31-7
- **DEITCH, E.A.** Multiple organ failure. Pathophysiology and potential future therapy. *Ann Surg,* 1992, vol. 216, 117-34
- **DELBENDE, C. ; CONTESSE, V. ; MOCAER, E. ; KAMOUN, A. ; VAUDRY, H.** The novel antidepressant, tianeptine, reduces stress-evoked stimulation of the hypothalamo-pituitary-adrenal axis. *Eur J Pharmacol,* 1991, vol. 202, 391-6
- **DENG, A. ; VALDIVIELSO, J.M. ; MUNGER, K.A. ; BLANTZ, R.C. ; THOMSON, S.C.** Vasodilatory N-methyl-D-aspartate receptors are constitutively expressed in rat kidney. *JAm Soc Nephrol,* 2002, vol. 13, 1381-4
- **DICKMAN, K.G. ; YOUSSEF, J.G. ; MATHEW, S.M. ; SAID, S.I.** Ionotropic glutamate receptors in lungs and airways: molecular basis for glutamate toxicity. *Am J Respir Cell Mol Biol,* 2004, vol. 30, 139-44
- **DROGE, W. ; ECK, H.P. ; BETZLER, M. ; SCHLAG, P. ; DRINGS, P. ; EBERT, W.** Plasma glutamate concentration and lymphocyte activity. *J Cancer Res Clin Oncol,* 1988, vol. 114, 124-8
- **DUNFORD, J.E. ; THOMPSON, K. ; COXON, F.P. ; LUCKMAN, S.P. ; HAHN, F.M. ; POULTER, C.D. ; EBETINO, F.H. ; ROGERS, M.J.** Structure-activity relationships for inhibition of farnesyl diphosphate synthase in vitro and inhibition of bone resorption in vivo by nitrogen-containing bisphosphonates. *J Pharmacol Exp Ther,* 2001, vol. 296, 235-4
- **EATON, S.** Impaired energy metabolism during neonatal sepsis: the effects of glutamine. *Proc Nutr Soc,* 2003, vol. 62, 745-51
- **ECK, H.P. ; FREY, H. ; DROGE, W.** Elevated plasma glutamate concentrations in HIV-1-infected patients may contribute to loss of macrophage and lymphocyte functions. *Int Immunol,* 1989, vol. 1, 367-72
- **EDWARDS, J.W. ; WALKER, E.L. ; CORUZZI, G.M.** Cell-specific expression in transgenic plants reveals nonoverlapping roles for chloroplast and cytosolic glutamine synthetase. *Proc Natl Acad Sci U S A,* 1990, vol. 87, 3459-63
- **EID, T. ; THOMAS, M.J. ; SPENCER, D.D. ; RUNDEN-PRAN, E. ; LAI, J.C. ; MALTHANKAR, G.V. ; KIM, J.H. ; DANBOLT, N.C. ; OTTERSEN, O.P. ; DE LANEROLLE, N.C.** Loss of glutamine synthetase in the human epileptogenic hippocampus: possible mechanism for raised extracellular glutamate in mesial temporal lobe epilepsy. *Lancet,* 2004, vol. 363, 28-37

- **EISENBERG, D. ; GILL, H.S. ; PFLUEGL, G.M. ; ROTSTEIN, S.H.** Structure-function relationships of glutamine synthetases. *Biochim Biophys Acta,* 2000, vol. 1477, 122-45
- **EVANS, P.H.** Free radicals in brain metabolism and pathology. *Br Med Bull,* 1993, vol. 49, 577-87
- **EVSTIGNEEVA, Z.G. ; SOLOV'EVA, N.A. ; SIDEL'NIKOVA, L.I.** Methionine sulfoximine and phosphinothricin--glutamine synthetase inhibitors and activators and their herbicidal activity (A review. *Prikl Biokhim Mikrobiol,* 2003, vol. 39, 613-8
- **FALDUTO, M.T. ; YOUNG, A.P. ; HICKSON, R.C.** Exercise inhibits glucocorticoid-induced glutamine synthetase expression in red skeletal muscles. *Am J Physiol,* 1992, vol. 262, C214-20
- **FAROOQUI, A.A. ; WELLS, K. ; HORROCKS, L.A.** Breakdown of membrane phospholipids in Alzheimer disease. Involvement of excitatory amino acid receptors. *Mol Chem Neuropathol,* 1995, vol. 25, 155-73
- **FARRINGTON, G.K. ; KUMAR, A. ; WEDLER, F.C.** Design and synthesis of phosphonates inhibitors of glutamine synthetase. *J Med Chem,* 1987, vol. 30, 2062-7
- **FELIPO, V. ; BUTTERWORTH, R.F.** Mitochondrial dysfunction in acute hyperammonemia. *Neurochem Int,* 2002, vol. 40, 487-91
- **FELIPO, V. ; BUTTERWORTH, R.F.** Neurobiology of ammonia. *Prog Neurobiol,* 2002, vol. 67, 259-79
- **FENG, B. ; SHIBER, S.K. ; MAX, S.R.** Glutamine regulates glutamine synthetase expression in skeletal muscle cells in culture. *J Cell Physiol,* 1990, vol. 145, 376-80
- **FERGUS, A. ; LEE, K.S.** Regulation of cerebral microvessels by glutamatergic mechanisms. *Brain Res,* 1997, vol. 754, 35-45
- **FERRIER, B. ; CONJARD, A. ; MARTIN, M. ; BAVEREL, G.** Glutamine synthesis is heterogeneous and differentially regulated along the rabbit renal proximal tubule. *Biochem J,* 1999, vol. 337 (3), 543-50
- **FINCH, C.E. ; COHEN, D.M.** Aging, metabolism, and Alzheimer disease: review and hypotheses. *Exp Neurol,* 1997, vol. 143, 82-102
- **FIRSOVA, N.A. ; SELIVANOVA, K.M. ; ALEKSEEVA, L.V. ; EVSTIGNEEVA, Z.G.** Inhibition of glutamine synthetase activity by biologically active derivatives of glutamic acid. *Biokhimiia,* 1986, vol. 51, 850-5
- **FLARING, U.B. ; ROOYACKERS, O.E. ; WERNERMAN, J. ; HAMMARQVIST, F.** Glutamine attenuates post-traumatic glutathione depletion in human muscle. *Clin Sci (Lond),* 2003, vol. 104, 275-82
- **FOYER, C.H. ; FERRARIO, S.** Modulation of carbon and nitrogen metabolism in transgenic plants with a view to improved biomass production. *Biochem Soc Trans,* 1994, vol. 22, 909-15
- **FRANCONI, F. ; MICELI, M. ; DE MONTIS, M.G. ; CRISAFI, E.L. ; BENNARDINI, F. ; TAGLIAMONTE, A.** NMDA receptors play an anti-aggregating role in human platelets. *Thromb Haemost,* 1996, vol. 76, 84-7
- **FROST, H.M.** Emerging views about ''osteoporosis'', bone health, strength, fragility, and their determinants. *J Bone Miner Metab,* 2002, vol. 20, 319-25
- **FUENTES, S.I. ; ALLEN, D.J. ; ORTIZ-LOPEZ, A. ; HERNANDEZ, G.** Over-expression of cytosolic glutamine synthetase increases photosynthesis and growth at low nitrogen concentrations. *J Exp Bot,* 2001, vol. 52, 1071-81
- **FURUKAWA, S. ; SAITO, H. ; INOUE, T. ; MATSUDA, T. ; FUKATSU, K. ; HAN, I. ; IKEDA, S. ; HIDEMURA, A.** Supplemental glutamine augments phagocytosis and reactive oxygen intermediate production by neutrophils and monocytes from postoperative patients in vitro. *Nutrition,* 2000, vol. 16, 323-9
- **FUZIWARA, S. ; INOUE, K. ; DENDA, M.** NMDA-type glutamate receptor is associated with cutaneous barrier homeostasis. *J Invest Dermatol,* 2003, vol. 120, 1023-9
- **GABBOTT, P.L. ; SOMOGYI, J.** The 'single' section Golgi-impregnation procedure: methodological description. *J Neurosci Methods,* 1984, vol. 11, 221-30
- **GALLARDO, F. ; FU, J. ; CANTON, F.R. ; GARCIA-GUTIERREZ, A. ; CANOVAS, F.M. ; KIRBY, E.G.** Expression of a conifer glutamine synthetase gene in transgenic poplar. *Planta,* 1999, vol. 210, 19-26
- **GARCIA, C. ; PITHON-CURI, T.C. ; DE LOURDES FIRMANO, M. ; PIRES DE MELO, M. ; NEWSHOLME, P. ; CURI, R.** Effects of adrenaline on glucose and glutamine metabolism and superoxide production by rat neutrophils. *Clin Sci (Lond),* 1999, vol. 96, 549-55
- **GARTHWAITE, G. ; HAJOS, F. ; GARTHWAITE, J.** Ionic requirements for neurotoxic effects of excitatory amino acid analogues in rat cerebellar slices. *Neuroscience,* 1986, vol. 18, 437-47
- **GASIC, G. P. ; HOLLMANN, M.** Molecular neurobiology of glutamate receptors. *Ann Rev Physiol,* 1992, vol. 54, 507-36
- **GENEVER, P.G. ; MAXFIELD, S.J. ; KENNOVIN, G.D. ; MALTMAN, J. ; BOWGEN, C.J. ; RAXWORTHY, M.J. ; SKERRY, T.M.** Evidence for a novel glutamate-mediated signaling pathway in keratinocytes. *J Invest Dermatol,* 1999, vol. 112, 337-42

- **GENEVER, P.G. ; WILKINSON, D.J. ; PATTON, A.J. ; PEET, N.M. ; HONG,Y. ; MATHUR, A. ; ERUSALIMSKY, J.D. ; SKERRY, T.M.** Expression of a functional N-methyl-D-aspartate-type glutamate receptor by bone marrow megakaryocytes. *Blood,* 1999, vol. 93, 2876-83
- **GENEVER, P.G. ; SKERRY, T.M.** Regulation of spontaneous glutamate release activity in osteoblastic cells and its role in differentiation and survival: evidence for intrinsic glutamatergic signaling in bone. *Faseb J,* 2001, vol. 15, 1586-8
- **GIARONI, C. ; DE PONTI, F. ; COSENTINO, M. ; LECCHINI, S. ; FRIGO, G.** Plasticity in the enteric nervous system. *Gastroenterology,* 1999, vol. 117, 1438-58
- **GIARONI, C. ; ZANETTI, E. ; CHIARAVALLI, A.M. ; ALBARELLO, L. ; DOMINIONI, L. ; CAPELLA, C. ; LECCHINI, S. ; FRIGO, G.** Evidence for a glutamatergic modulation of the cholinergic function in the human enteric nervous system via NMDA receptors. *Eur J Pharmacol,* 2003, vol. 476, 63-9
- **GILL, S.S. ; PULIDO, O.M. ; MUELLER, R.W. ; MCGUIRE, P.F.** Molecular and immunochemical characterization of the ionotropic glutamate receptors in the rat heart. *Brain Res Bull,* 1998, vol. 46, 429-34
- **GILL, H.S. ; EISENBERG, D.** The crystal structure of phosphinothricin in the active site of glutamine synthetase illuminates the mechanism of enzymatic inhibition. *Biochemistry,* 2001, vol. 40, 1903-12
- **GLEVAREC, G. ; BOUTON, S. ; JASPARD, E. ; RIOU, M.T. ; CLIQUET, J.B. ; SUZUKI, A. ; LIMAMI, A.M.** Respective roles of the glutamine synthetase/glutamate synthase cycle and glutamate dehydrogenase in ammonium and amino acid metabolism during germination and post-germinative growth in the model legume Medicago truncatula. *Planta,* 2004, vol. 219, 286-97
- **GONOI, T. ; MIZUNO, N. ; INAGAKI, N. ; KUROMI, H. ; SEINO, Y. ; MIYAZAKI, J. ; SEINO, S.** Functional neuronal ionotropic glutamate receptors are expressed in the non-neuronal cell line MIN6. *J Biol Chem,* 1994, vol. 269, 16989-92
- **GONZALEZ-CADAVID, N.F. ; RYNDIN, I. ; VERNET, D. ; MAGEE, T.R. ; RAJFER, J.** Presence of NMDA receptor subunits in the male lower urogenital tract. *J Androl,* 2000, vol. 21, 566-78
- **GOROVITS, R. ; AVIDAN, N. ; AVISAR, N. ; SHAKED, I. ; VARDIMON, L.** Glutamine synthetase protects against neuronal degeneration in injured retinal tissue. *Proc Natl Acad Sci U S A,* 1997, vol. 94, 7024-9
- **GOVITRAPONG, P. ; EBADI, M. ; MURRIN, L.C.** Identification of a Cl-/Ca2+-dependent glutamate (quisqualate) binding site in bovine pineal organ. *J Pineal Res,* 1986, vol. 3, 223-34
- **GRIFFITH, O.W. ; ANDERSON, M.E. ; MEISTER, A.** Inhibition of glutathione biosynthesis by prothionine sulfoximine (S-n-propyl homocysteine sulfoximine), a selective inhibitor of gamma-glutamylcysteine synthetase. *J Biol Chem,* 1979, vol. 254, 1205-10
- **GURVITS, T.V. ; SHENTON, M.E. ; HOKAMA, H. ; OHTA, H. ; LASKO, N.B. ; GILBERTSON, M.W. ; ORR, S.P. ; KIKINIS, R. ; JOLESZ, F.A. ; MCCARLEY, R.W.** Magnetic resonance imaging study of hippocampal volume in chronic, combat-related posttraumatic stress disorder. *Biol Psychiatry,* 1996, vol. 40, 1091-9
- **HACK, R. ; EBERT, E. ; EHLING, G. ; LEIST, K.H.** Glufosinate ammonium--some aspects of its mode of action in mammals. *Food Chem Toxicol,* 1994, vol. 32, 461-70
- **HALESTRAP, A.P. ; WOODFIELD, K.Y. ; CONNERN, C.P.** Oxidative stress, thiol reagents, and membrane potential modulate the mitochondrial permeability transition by affecting nucleotide binding to the adenine nucleotide translocase. *J Biol Chem,* 1997, vol. 272, 3346-54
- **HARDER, D.R. ; ALKAYED, N.J. ; LANGE, A.R. ; GEBREMEDHIN, D. ; ROMAN, R.J.** Functional hyperemia in the brain: hypothesis for astrocyte-derived vasodilator metabolites. *Stroke,* 1998, vol. 29, 229-34
- **HAUSSINGER, D. ; SIES, H. ; GEROK, W.** Functional hepatocyte heterogeneity in ammonia metabolism. The intercellular glutamine cycle. *J Hepatol,* 1985, vol. 1, 3-14
- **HAWKES, N.D. ; THOMAS, G.A. ; JUREWICZ, A. ; WILLIAMS, O.M. ; HILLIER, C.E. ; MCQUEEN, I.N. ; SHORTLAND, G.** Non-hepatic hyperammonaemia:an important,potentially reversible cause of encephalopathy. *Postgard Med J,* 2001, vol. 77, 316-7
- **HAWKINS, R.A. ; JESSY, J. ; MANS, A.M. ; DE JOSEPH, M.R.** Effect of reducing brain glutamine synthesis on metabolic symptoms of hepatic encephalopathy. *J Neurochem,* 1993, vol. 60, 1000-6
- **HAYASHI, M. ; YAMADA, H. ; UEHARA, S. ; MORIMOTO, R. ; MUROYAMA, A. ; YATSUSHIRO, S. ; TAKEDA, J. ; YAMAMOTO, A. ; MORIYAMA, Y.** Secretory granule-mediated co-secretion of L-glutamate and glucagon triggers glutamatergic signal transmission in islets of Langerhans. *J Biol Chem,* 2003, vol. 278, 1966-74
- **HERTZ, L.** Functional interactions between neurons and astrocytes I. Turnover and metabolism of putative amino acid transmitters. *Prog Neurobiol,* 1979, vol. 13, 277-323
- **HERTZ, L. ; DRINGEN, R. ; SCHOUSBOE, A. ; ROBINSON, S.R.** Astrocytes: glutamate producers for neurons. *J Neurosci Res,* 1999, vol. 57, 417-28

- **HERTZ, L. ; ZIELKE, H.R.** Astrocytic control of glutamatergic activity: astrocytes as stars of the show. *Trends Neurosci,* 2004, vol. 27, 735-43
- **HICKSON, R.C. ; WEGRZYN, L.E. ; OSBORNE, D.F. ; KARL, I.E.** Glutamine interferes with glucocorticoid-induced expression of glutamine synthetase in skeletal muscle. *Am J Physiol,* 1996, vol. 270, E912-7
- **HIGUCHI, H. ; ADACHI, M. ; MIURA, S. ; GORES, G.J. ; ISHII, H.** The mitochondrial permeability transition contributes to acute ethanol-induced apoptosis in rat hepatocytes. *Hepatology,* 2001, vol. 34, 320-8
- **HINOI, E. ; FUJIMORI, S. ; NAKAMURA, Y. ; BALCAR, V.J. ; KUBO, K. ; OGITA, K. ; YONEDA, Y.** Constitutive expression of heterologous N-methyl-D-aspartate receptor subunits in rat adrenal medulla. *J Neurosci Res,* 2002, vol. 68, 36-45
- **HINOI E ; FUJIMORI S ; TAKARADA T ; TANIURA H ; YONEDA Y.** Facilitation of glutamate release by ionotropic glutamate receptors in osteoblasts. *Biochem Biophys Res Commun,* 2002, vol. 297, 452-8
- **HINOI, E. ; FUJIMORI, S. ; YONEDA, Y.** Modulation of cellular differentiation by N-methyl-D-aspartate receptors in osteoblasts. *FASEB J,* 2003, vol. 17, 1532-4
- **HINOI, E. ; FUJIMORI, S. ; YONEYAMA, M. ; YONEDA, Y.** Blockade by N-methyl-D-aspartate of elevation of activator protein-1 binding after stress in rat adrenal gland. *J Neurosci Res,* 2002, vol. 70, 161-71
- **HINOI, E. ; TAKARADA, T. ; UESHIMA, T. ; TSUCHIHASHI, Y. ; YONEDA, Y.** Glutamate signaling in peripheral tissues. *Eur J Biochem,* 2004, vol. 271, 1-13
- **HINOI, E. ; YONEDA, Y.** Expression of GluR6/7 subunits of kainate receptors in rat adenohypophysis. *Neurochem Int,* 2001, vol. 38, 539-47
- **HITCHCOCK, I.S. ; SKERRY, T.M. ; HOWARD, M.R. ; GENEVER, P.G.** NMDA receptor-mediated regulation of human megakaryocytopoiesis. *Blood,* 2003, vol. 102, 1254-9
- **HOLLMANN, M. ; HEINEMANN, S.** Cloned glutamate receptors. *Annu Rev Neurosci,* 1994, vol. 17, 31-108
- **HONG, R.W. ; ROUNDS, J.D. ; HELTON, W.S. ; ROBINSON, M.K. ; WILMORE, D.W.** Glutamine preserves liver glutathione after lethal hepatic injury. *Ann Surg,* 1992, vol. 215, 114-9
- **HOUDIJK, A.P. ; NIJVELDT, R.J. ; VAN LEEUWEN, P.A.** Glutamine-enriched enteral feeding in trauma patients: reduced infectious morbidity is not related to changes in endocrine and metabolic responses. *JPEN J Parenter Enteral Nutr,* 1999, vol. 23, 52-8
- **HOUDIJK, A.P. ; RIJNSBURGER, E.R. ; JANSEN, J. ; WESDORP, R.I. ; WEISS, J.K. ; MCCAMISH, M.A. ; TEERLINK, T. ; MEUWISSEN, S.G. ; HAARMAN, H.J. ; THIJS, L.G.** Randomised trial of glutamine-enriched enteral nutrition on infectious morbidity in patients with multiple trauma. *Lancet,* 1998, vol. 352, 772-6
- **HOURANI, B.T. ; HAMLIN, E.M. ; REYNOLDS, T.B.** Cerebrospinal fluid glutamine as a measure of hepatic encephalopathy. *Arch Intern Med,* 1971, vol. 127, 1033-6
- **HOYER, S.** Possible role of ammonia in the brain in dementia of Alzheimer type. *Adv Exp Med Biol,* 1994, vol. 368, 197-205
- **HREBICEK, J. ; KOLOUSEK, J.** Preparoxysmal changes of spontaneous and evoked electrical activity of the cat brain after administration of methionine sulphoximine. *Epilepsia,* 1968, vol. 9, 145-62
- **HUNDAL, H.S. ; BABIJ, P. ; WATT, P.W. ; WARD, M.R. ; RENNIE, M.J.** Glutamine transport and metabolism in denervated rat skeletal muscle. *Am J Physiol,* 1990, vol. 259, E148-54
- **INAGAKI, N. ; KUROMI, H. ; GONOI, T. ; OKAMOTO, Y. ; ISHIDA, H. ; SEINO, Y. ; KANEKO, T. ; IWANAGA, T. ; SEINO, S.** Expression and role of ionotropic glutamate receptors in pancreatic islet cells. *Faseb J,* 1995, vol. 9, 686-91
- **IRVING, A.J. ; SCHOFIELD, J.G. ; WATKINS, J.C. ; SUNTER, D.C. ; COLLINGRIDGE, G.L.** 1S,3R-ACPD stimulates and L-AP3 blocks Ca2+ mobilization in rat cerebellar neurons. *Eur J Pharmacol,* 1990, vol. 186, 363-5
- **ISAACKS, R.E. ; BENDER, A.S. ; KIM, C.Y. ; SHI, Y.F. ; NORENBERG, M.D.** Effect of ammonia and methionine sulfoximine on myo-inositol transport in cultured astrocytes. *Neurochem Res,* 1999, vol. 24, 51-9
- **ISHIYAMA, K. ; INOUE, E. ; TABUCHI, M. ; YAMAYA, T. ; TAKAHASHI, H.** Biochemical background and compartmentalized functions of cytosolic glutamine synthetase for active ammonium assimilation in rice roots. *Plant Cell Physiol,* 2004, vol. 45, 1640-7
- **ISRAELI, E. ; BERENSHTEIN, E. ; WENGROWER, D. ; APTEKAR, L. ; KOHEN, R. ; ZAJICEK, G. ; GOLDIN, E.** Prophylactic administration of topical glutamine enhances the capability of the rat colon to resist inflammatory damage. *Dig Dis Sci,* 2004, vol. 49, 1705-12
- **ITO, K. ; YAMAMOTO, K. ; KAWANISHI, S.** Manganese-mediated oxidative damage of cellular and isolated DNA by isoniazid and related hydrazines: non-Fenton-type hydroxyl radical formation. *Biochemistry,* 1992, vol. 31, 11606-13
- **IZUMI, Y. ; BENZ, A.M. ; CLIFFORD, D.B. ; ZORUMSKI, C.F.** Nitric oxide inhibitors attenuate N-methyl-D-aspartate excitotoxicity in rat hippocampal slices. *Neurosci Lett,* 1992, vol. 135, 227-30

- **JACKSON, B. ; GEE, A.N. ; GUYON-GELLIN, Y. ; NIESOR, E. ; BENTZEN, C.L. ; KERNS, W.D. ; SUCKLING, K.E.** Hypocholesterolaemic and antiatherosclerotic effects of tetra-iso-propyl 2-(3,5-di-tert-butyl-4-hydroxyphenyl)ethyl-1,1-diphosphonate (SR-9223i). *Arzneimittelforschung,* 2000, vol. 50, 380-6
- **JANE, D.E. ; JONES, P.L. ; POOK, P.C. ; TSE, H.W. ; WATKINS, J.C.** Actions of two new antagonists showing selectivity for different sub-types of metabotropic glutamate receptor in the neonatal rat spinal cord. *Br J Pharmacol,* 1994, vol. 112, 809-16
- **JAYAKUMAR, A.R. ; RAMA RAO, K.V. ; SCHOUSBOE, A. ; NORENBERG, M.D.** Glutamine-induced free radical production in cultured astrocytes. *Glia,* 2004, vol. 46, 296-301
- **JOHNSON, C.R. ; BOETTCHER, B.R. ; CHERPECK, R.E. ; DOLSON, M.G.** Design and synthesis of potent inhibitors of glutamine synthetase: 1. Cyclic analogs of phosphonothricin. *Bioorg Chem,* 1990, vol. 18, 154-9
- **JOHNSTON, G.A.** The intraspinal distribution of some depressant amino acids. *J Neurochem,* 1968, vol. 15, 1013-7
- **JONES, E.A. ; WEISSENBORN, K.** Neurology and the liver. *J Neurol Neurosurg Psychiatry,* 1997, vol. 63, 279-93
- **JOU, M.J. ; JOU, S.B. ; CHEN, H.M. ; LIN, C.H. ; PENG, T.I.** Critical role of mitochondrial reactive oxygen species formation in visible laser irradiation-induced apoptosis in rat brain astrocytes (RBA-1. *J Biomed Sci,* 2002, vol. 9, 507-16
- **KAFARSKI, P. ; LEJCZAK, B. ; FORLANI, G.** Herbicidally active aminomethylenebisphophonic acids. *Heteroatom Chemistry,* 2000, vol. 11, 449-53
- **KAFARSKI, P. ; LEJCZAK, B.** Aminophosphonic acids of potential medical importance. *Curr Med Chem Anti-Canc Agents,* 2001, vol. 1, 301-12
- **KANAMORI, K. ; BLUML, S. ; ROSS, B.** Magnetic resonance spectroscopy in the study of hyperammonemia and hepatic encephalopathy. *Adv Exp Med Biol,* 1997, vol. 420, 185-94
- **KARINCH, A.M. ; PAN, M. ; LIN, C.M. ; STRANGE, R. ; SOUBA, W.W.** Glutamine metabolism in sepsis and infection. *J Nutr,* 2001, vol. 131, 2535S-8S
- **KING, D.S.** Tuberculosis. *N Engl J Med,* 1952, vol. 247, 718-25
- **KIRCHGESSNER, A.L.** Glutamate in the enteric nervous system. *Curr Opin Pharmacol,* 2001
- **KIRCHGESSNER, A.L. ; LIU, M.T. ; ALCANTARA, F.** Excitotoxicity in the enteric nervous system. *J Neurosci,* 1997, vol. 17, 8804-16
- **KIYAMA, H. ; SATO, K. ; TOHYAMA, M.** Characteristic localization of non-NMDA type glutamate receptor subunits in the rat pituitary gland. *Brain Res Mol Brain Res,* 1993, vol. 19, 262-8
- **KOLLEGGER, H. ; MCBEAN, G.J. ; TIPTON, K.F.** The inhibition of glutamine synthetase in rat corpus striatum in vitro by methionine sulfoximine increases the neurotoxic effects of kainate and N-methyl-D-aspartate. *Neurosci Lett,* 1991, vol. 130, 95-8
- **KOSENKO, E. ; LLANSOLA, M. ; MONTOLIU, C. ; MONFORT, P. ; RODRIGO, R. ; HERNANDEZ-VIADEL, M. ; ERCEG, S. ; SANCHEZ-PEREZ, A.M. ; FELIPO, V.** Glutamine synthetase activity and glutamine content in brain: modulation by NMDA receptors and nitric oxide. *Neurochem Int,* 2003, vol. 43, 493-9
- **KOSOWER, N.S. ; KOSOWER, E.M.** The glutathione status of cells. *Int Rev Cytol,* 1978, vol. 54, 109-60
- **KOSTANYAN, I.A. ; MERKULOVA, M.I. ; NAVOLOTSKAYA, E.V. ; NURIEVA, R.I.** Study of interaction between L-glutamate and human blood lymphocytes. *Immunol Lett,* 1997, vol. 58, 177-80
- **KOYAMA, I. ; NAKAMURA, T. ; OGASAWARA, M. ; NEMOTO, M. ; YOSHIDA, T.** The protective effect of taurine on the biomembrane against damage produced by the oxygen radical. *Adv Exp Med Biol,* 1992, vol. 315, 355-9
- **KOZIKOWSKI, A.P. ; TUCKMANTEL, W. ; LIAO, Y. ; MANEV, H. ; IKONOMOVIC, S. ; WROBLEWSKI, J.T.** Synthesis and metabotropic receptor activity of the novel rigidified glutamate analogues (+)- and (-)-trans-azetidine-2,4-dicarboxylic acid and their N-methyl derivatives. *J Med Chem,* 1993, vol. 36, 2706-8
- **KRISTENSEN, P.** Differential expression of AMPA glutamate receptor mRNAs in the rat adrenal gland. *FEBS Lett,* 1993, vol. 332, 14-8
- **KRIZBAI, I.A. ; DELI, M.A. ; PESTENACZ, A. ; SIKLOS, L. ; SZABO, C.A. ; ANDRAS, I. ; JOO, F.** Expression of glutamate receptors on cultured cerebral endothelial cells. *J Neurosci Res,* 1998, vol. 54, 814-9
- **KVAMME, E.** Synthesis of glutamate and its regulation. *Prog Brain Res,* 1998, vol. 116, 73-85
- **LABOW, B.I. ; ABCOUWER, S.F. ; LIN, C.M. ; SOUBA, W.W.** Glutamine synthetase expression in rat lung is regulated by protein stability. *Am J Physiol,* 1998, vol. 275, L877-86
- **LABOW, B. I. ; SOUBA, W.W. ; ABCOUWER, S.F.** Glutamine synthetase expression in muscle is regulated by transcriptional and posttranscriptional mechanisms. *Am J Physiol,* 1999, vol. 276, E1136-45
- **LABOW, B.I. ; SOUBA, W.W. ; ABCOUWER, S.F.** Mechanisms governing the expression of the enzymes of glutamine metabolism--glutaminase and glutamine synthetase. *J Nutr,* 2001, vol. 131, 2467S-74S

- **LACOSTE, L. ; CHAUDHARY, K.D. ; LAPOINTE, J.** Derepression of the glutamine synthetase in neuroblastoma cells at low concentrations of glutamine. *J Neurochem,* 1982, vol. 39, 78-85
- **LAM, H.M. ; COSCHIGANO, K. ; SCHULTZ, C. ; MELO-OLIVEIRA, R. ; TJADEN, G. ; OLIVEIRA, I. ; NGAI, N. ; HSIEH, M.H. ; CORUZZI, G.** Use of Arabidopsis mutants and genes to study amide amino acid biosynthesis. *Plant Cell,* 1995, vol. 7, 887-98
- **LAM, H.M. ; COSCHIGANO, K.T. ; OLIVEIRA, I.C. ; MELO-OLIVEIRA, R. ; CORUZZI, G.M.** The Molecular-Genetics of Nitrogen Assimilation into Amino Acids in Higher Plants. *Annu Rev Plant Physiol Plant Mol Biol,* 1996, vol. 47, 569-93
- **LANGSTON-UNKEFER, P.J. ; ROBINSON, A.C. ; KNIGHT, T.J. ; DURBIN, R.D.** Inactivation of pea seed glutamine synthetase by the toxin, tabtoxinine-beta-lactam. *J Biol Chem,* 1987, vol. 262, 1608-13
- **LANYON, L. ; SKERRY, T.** Postmenopausal osteoporosis as a failure of bone's adaptation to functional loading: a hypothesis. *J Bone Miner Res,* 2001, vol. 16, 1937-47
- **LAPOUBLE, E. ; MONTECOT, C. ; SEVESTRE, A. ; PICHON, J.** Phosphinothricin induces epileptic activity via nitric oxide production through NMDA receptor activation in adult mice. *Brain Res,* 2002
- **LARA, M. ; CULLIMORE, J.V. ; LEA, P.J. ; MIFLIN, B.J. ; JOHNSTON, A.W.B. ; LAMB J.W.** Appearance of a novel form of plant glutamine synthetase during nodule development in Phaseolus vulgaris. *L. Planta,* 1983, vol. 157, 254-258
- Glutamine synthetase and its inhibition. **LEA, P.J. ; RIDDLEY S.M.** Herbicides and plant metabolism (Seminar series / Society for Experimental Biology; 38). Cambridge University Press, 1989, 137-70
- Primary nitrogen metabolism. **LEA, P.J.** Plant biochemistry. Academic Press, 1997, 273-306
- **LECHIN, F. ; VAN DER DIJS, B. ; OROZCO, B. ; JARA, H. ; RADA, I. ; LECHIN, M.E. ; LECHIN, A.E.** Neuropharmacologic treatment of bronchial asthma with the antidepressant tianeptine: a double-blind, crossover placebo-controlled study. *Clin Pharmacol Ther,* 1998, vol. 64, 223-32
- **LEE, S.K. ; MBWAMBO, Z.H. ; CHUNG, H. ; LUYENGI, L. ; GAMEZ, E.J. ; MEHTA, R.G. ; KINGHORN, A.D. ; PEZZUTO, J.M.** Evaluation of the antioxidant potential of natural products. *Comb Chem High Throughput Screen,* 1998, vol. 1, 35-46
- **LEJCZAK, B. ; STARZEMSKA, H. ; MASTALERZ, P.** Inhibition of rat liver glutamine synthetase by phosphonic analogues of glutamic acid. *Experientia,* 1981, vol. 37, 461-2
- **LEONARD, J.V. ; MORRIS, A.A.** Urea cycle disorders. *Semin Neonatol,* 2002, vol. 7, 27-35
- **LEUNG, J.C. ; TRAVIS, B.R. ; VERLANDER, J.W. ; SANDHU, S.K. ; YANG, S.G. ; ZEA, A.H. ; WEINER, I.D. ; SILVERSTEIN, D.M.** Expression and developmental regulation of the NMDA receptor subunits in the kidney and cardiovascular system. *Am J Physiol Regul Integr Comp Physiol,* 2002, vol. 283, R964-71
- **LI, N. ; LEWIS, P. ; SAMUELSON, D. ; LIBONI, K. ; NEU, J.** Glutamine regulates Caco-2 cell tight junction proteins. *Am J Physiol Gastrointest Liver Physiol,* 2004, vol. 287, G726-33
- **LIMAMI, A.M. ; PHILLIPSON, B. ; AMEZIANE, R. ; PERNOLLET, N. ; JIANG, Q. ; ROY, R. ; DELEENS, E. ; CHAUMONT-BONNET, M. ; GRESSHOFF, P.M. ; HIREL, B.** Does root glutamine synthetase control plant biomass production in Lotus japonicus L.?. *Planta,* 1999, vol. 209, 495-502
- **LIMAMI, A.M. ; ROUILLON, C. ; GLEVAREC, G. ; GALLAIS, A. ; HIREL, B.** Genetic and physiological analysis of germination efficiency in maize in relation to nitrogen metabolism reveals the importance of cytosolic glutamine synthetase. *Plant Physiology,* 2002, vol. 130, 1860-70
- **LIPTON, S.A. ; GENDELMAN, H.E.** Seminars in medicine of the Beth Israel Hospital, Boston. Dementia associated with the acquired immunodeficiency syndrome. *N Engl J Med,* 1995, vol. 332, 934-40
- **LIPTON, S.A. ; ROSENBERG, P.A.** Excitatory amino acids as a final common pathway for neurologic disorders. *N Engl J Med,* 1994, vol. 330, 613-22
- **LIU, H.P. ; TAY, S.S.W. ; LEONG S.K.** Localization of glutamate receptor subunits of the α-amino-3-hydroxy-5-methyl-D-aspartate (AMPA) type in the pancreas of newborn guinea pigs. *Pancreas,* 1997, vol. 14, 360-68
- **LIU, M.T. ; ROTHSTEIN, J.D. ; GERSHON, M.D. ; KIRCHGESSNER, A.L.** Glutamatergic enteric neurons. *J Neurosci,* 1997, vol. 17, 4764-84
- **LODIN, Z.** Analysis of changes of the central nervous system induced by MSI. *J. Cesk Fysiol,* 1958, vol. 7, 122-8
- **LODIN, Z. ; KOLOUSEK, J.** Epileptic symptoms induced by methionine sulfoximine. *J. Cesk Neurol,* 1956, vol. 19, 83-9
- **LOGUSCH, E.W.** A simple preparation of S-alkyl homocysteine derivatives: S-phosphonomethyl homocysteines as inhibitors of glutamine synthetase. *Tetrahedron Lett,* 1988, vol. 29, 6055-58
- **LOGUSCH, E.W. ; WALKER, D.M. ; MCDONALD, J.F. ; FRANZ, J.E.** Synthesis of alpha and gamma alkyl-substituted phosphinothricin: potent new inhibitors of glutamine synthetase. *J Org Chem.,* 1988, vol. 53, 4069-4074

- **LOGUSCH, E.W. ; WALKER, D.M. ; MCDONALD, J.F. ; FRANZ, J.E.** Substrate variability as a factor in enzyme inhibitor design: inhibition of ovine brain glutamine synthetase by alpha- and gamma-substituted phosphinothricins. *Biochemistry,* 1989, vol. 28, 3043-51
- **LOGUSCH, E.W. ; WALKER, D.M. ; MCDONALD, J.F. ; FRANZ, J.E. ; VILLAFRANCA, J.J. ; DIIANNI, C.L. ; COLANDUONI, J.A. ; LI, B. ; SCHINELLER, J.B.** Inhibition of Escherichia coli glutamine synthetase by alpha- and gamma-substituted phosphinothricins. *Biochemistry,* 1990, vol. 29, 366-72
- **LOMBARDI, G. ; DIANZANI, C. ; MIGLIO, G. ; CANONICO, P.L. ; FANTOZZI, R.** Characterization of ionotropic glutamate receptors in human lymphocytes. *Br J Pharmacol,* 2001, vol. 133, 936-44
- **LOUZADA-JUNIOR, P., JR. ; DIAS, J. J. ; SANTOS, W. F. ; LACHAT, J. J. ; BRADFORD, H. F. ; COUTINHO NETTO, J.** Glutamate release in experimental ischaemia of the retina: an approach using microdialysis. *J. Neurochem.,* 1992, vol. 59, 358-363
- **LUINE.** Stress, Serotonin and Tianeptine. Symposium at the 8th World Congress of Psychiatry. Athens. *Br J Psychiatry,* 1992, 6-75
- **LUKASZEWICZ, G.C. ; SOUBA, W.W. ; ABCOUWER, S.F.** Induction of muscle glutamine synthetase gene expression during endotoxemia is adrenal gland dependent. *Shock,* 1997, vol. 7, 332-8
- **MAINOUS, M.R. ; BLOCK, E.F. ; DEITCH, E.A.** Nutritional support of the gut: how and why. *New Horiz,* 1994, vol. 2, 193-201
- **MAGARINOS, A.M. ; MCEWEN, B.S.** Stress-induced atrophy of apical dendrites of hippocampal CA3c neurons: involvement of glucocorticoid secretion and excitatory amino acid receptors. *Neuroscience,* 1995, vol. 69, 89-98
- **MANHART, N. ; VIERLINGER, K. ; SPITTLER, A. ; BERGMEISTER, H. ; SAUTNER, T. ; ROTH, E.** Oral feeding with glutamine prevents lymphocyte and glutathione depletion of Peyer's patches in endotoxemic mice. *Ann Surg,* 2001, vol. 234, 92-7
- **MARCINKIEWICZ, J. ; GRABOWSKA, A. ; BERETA, J. ; STELMASZYNSKA, T.** Taurine chloramine, a product of activated neutrophils, inhibits in vitro the generation of nitric oxide and other macrophage inflammatory mediators. *JLeukoc Biol,* 1995, vol. 58, 667-74
- **MASON, D.J. ; SUVA, L.J. ; GENEVER, P.G. ; PATTON, A.J. ; STEUCKLE, S. ; HILLAM, R.A. ; SKERRY, T.M.** Mechanically regulated expression of a neural glutamate transporter in bone: a role for excitatory amino acids as osteotropic agents?. *Bone,* 1997, vol. 20, 199-205
- **MASSEY, S.C. ; MILLER, R.F.** N-methyl-D-aspartate receptors of ganglion cells in rabbit retina. *J Neurophysiol,* 1990, vol. 63, 16-30
- **MATSUMURA, N. ; TAKEUCHI, C. ; HISHIKAWA, K. ; FUJII, T. ; NAKAKI, T.** Glufosinate ammonium induces convulsion through N-methyl-D-aspartate receptors in mice. *Neurosci Lett,* 2001, vol. 304, 123-5
- **MCCAULEY, R. ; KONG, S.E. ; HALL, J.** Glutamine and nucleotide metabolism within enterocytes. *JPEN J Parenter Enteral Nutr,* 1998, vol. 22, 105-11
- **MCGRATH-MORROW, S.A. ; STAHL, J.** Inhibition of glutamine synthetase in a549 cells during hyperoxia. *Am JRespir Cell Mol Biol,* 2002, vol. 27, 99-106
- **MCNEARNEY, T. ; SPEEGLE, D. ; LAWAND, N. ; LISSE, J. ; WESTLUND, K.N.** Excitatory amino acid profiles of synovial fluid from patients with arthritis. *J Rheumatol,* 2000, vol. 27, 739-45
- **MCROBERTS, J.A. ; COUTINHO, S.V. ; MARVIZON, J.C. ; GRADY, E.F. ; TOGNETTO, M. ; SENGUPTA, J.N. ; ENNES, H.S. ; CHABAN, V.V. ; AMADESI, S. ; CREMINON, C.** Role of peripheral N-methyl-D-aspartate (NMDA) receptors in visceral nociception in rats. *Gastroenterology,* 2001, vol. 120, 1737-48
- **MEISTER, A.** Glutamine synthetase from mammalian tissues. *Methods Enzymol,* 1985, vol. 113, 185-99
- **MEISTER, A.** Glutathione deficiency produced by inhibition of its synthesis, and its reversal; applications in research and therapy. *Pharmacol Ther,* 1991, vol. 51, 155-94
- **MEISTER, A. ; TATE, S.S.** Glutathione and related gamma-glutamyl compounds: biosynthesis and utilization. *Annu Rev Biochem,* 1976, vol. 45, 559-604
- **MELIS, G.C. ; TER WENGEL, N. ; BOELENS, P.G. ; VAN LEEUWEN, P.A.** Glutamine: recent developments in research on the clinical significance of glutamine. *Curr Opin Clin Nutr Metab Care,* 2004, vol. 7, 59-70
- **MENG, W. ; TOBIN, J.R. ; BUSIJA, D.W.** Glutamate-induced cerebral vasodilation is mediated by nitric oxide through N-methyl-D-aspartate receptors. *Stroke,* 1995, vol. 26, 857-62
- **MERLE, B. ; ITZSTEIN, C. ; DELMAS, P.D. ; CHENU, C.** NMDA glutamate receptors are expressed by osteoclast precursors and involved in the regulation of osteoclastogenesis. *J Cell Biochem,* 2003, vol. 90, 424-36
- **MEWETT, K.N. ; OAKES, D.J. ; OLVERMAN, H.J. ; SMITH, D.A. ; WATKINS, J.C.** Pharmacology of the excitatory actions of sulphonic and sulphinic amino acids. *Adv Biochem Psychopharmacol,* 1983, vol. 37, 163-74

- **MIFLIN, B.J. ; HABASH, D.Z.** The role of glutamine synthetase and glutamate dehydrogenase in nitrogen assimilation and possibilities for improvement in the nitrogen utilization of crops. *J Exp Bot,* 2002, vol. 53, 979-87
- Ammonia assimilation. **MIFLIN B.J. ; LEA P.J.** The biochemistry of plants. Vol. 5. Amino acids and their derivatives. Academic Press, 1980, vol. 5, 169-202
- **MIGGE, A. ; CARRAYOL, E. ; HIREL, B. ; BECKER, T.W.** Leaf-specific overexpression of plastidic glutamine synthetase stimulates the growth of transgenic tobacco seedlings. *Planta,* 2000, vol. 210, 252-60
- **MILLER, A.L.** Therapeutic considerations of L-glutamine: a review of the literature. *Altern Med Rev,* 1999, vol. 4, 239-48
- **MILMAN, G. ; PORTNOFF, L.S. ; TIEMEIER, D.C.** Immunochemical evidence for glutamine-mediated degradation of glutamine synthetase in cultured Chinese hamster cells. *J Biol Chem,* 1975, vol. 250, 1393-9
- **MONAGHAN, D.T. ; BRIDGES, R.J. ; COTMAN, C.W.** The excitatory amino acid receptors: their classes, pharmacology, and distinct properties in the function of the central nervous system. *Annu Rev Pharmacol Toxicol,* 1989, vol. 29, 365-402
- **MORALES-PIGA, A.** Tiludronate. A new treatment for an old ailment: Paget's disease of bone. *Expert Opin Pharmacother,* 1999, vol. 1, 157-70
- **MORRIS, D. ; STRAUGHAN, D.W.** The interaction of alpha- and beta -L-aspartyl-L-glutamic acid and of their N-acetyl derivatives on central neurones and certain isolated peripheral tissues. *Biochem Pharmacol,* 1965, vol. 14, 1679-81
- **MOSINGER, J.L. ; PRICE, M.T. ; BAI H.Y. ; XIAO, H. ; WOZNIAK, D.F. ; OLNEY, J.W.** Blockade of both NMDA and non-NMDA receptors is required for optimal protection against ischemic neuronal degeneration in the in vivo adult mammalian retina. *Exp Neurol,* 1991, vol. 113, 10-7
- **MUHLBAUER, R.C. ; BAUSS, F. ; SCHENK, R. ; JANNER, M. ; BOSIES, E. ; STREIN, K. ; FLEISCH, H.** BM 21.0955, a potent new bisphosphonate to inhibit bone resorption. *J Bone Miner Res,* 1991, vol. 6, 1003-11
- **MURTHY, C.R. ; RAMA RAO, K.V. ; BAI, G. ; NORENBERG, M.D.** Ammonia-induced production of free radicals in primary cultures of rat astrocytes. *J Neurosci Res,* 2001, vol. 66, 282-8
- **NAGATA, Y. ; HOMMA, H. ; LEE, J.A. ; IMAI, K.** D-Aspartate stimulation of testosterone synthesis in rat Leydig cells. *FEBS Lett,* 1999, vol. 444, 160-4
- **NAKAKI, T. ; MISHIMA, A. ; SUZUKI, E. ; SHINTANI, F. ; FUJII, T.** Glufosinate ammonium stimulates nitric oxide production through N-methyl D-aspartate receptors in rat cerebellum. *Neurosci Lett,* 2000, vol. 290, 209-12
- **NEAL, M.J. ; CUNNINGHAM, J.R. ; HUTSON, P.H. ; HOGG, J.** Effects of ischaemia on neurotransmitter release from the isolated retina. *J Neurochem,* 1994, vol. 62, 1025-33
- **NEU, J. ; DEMARCO, V. ; LI, N.** Glutamine: clinical applications and mechanisms of action. *Curr Opin Clin Nutr Metab Care,* 2002, vol. 5, 69-75
- **NEVILLE-WEBBE, H.L. ; HOLEN, I. ; COLEMAN, R.E.** The anti-tumour activity of bisphosphonates. *Cancer Treat Rev,* 2002, vol. 28, 305-19
- **NEWSHOLME, P.** Why is L-glutamine metabolism important to cells of the immune system in health, postinjury, surgery or infection?. *J Nutr,* 2001, vol. 131, 2515S-22S
- **NORENBERG, M.D.** The astrocyte in liver disease. *Adv Cell Neurobiol,* 1981, vol. 2, 303-51
- Hepatic encephalopathy: a disorder of astrocytes. **NORENBERG, M.D.** Astrocytes. Academic Press, 1986, 425-460
- **NORENBERG, M.D.** The role of astrocytes in hepatic encephalopathy. *Neurochem Pathol,* 1987, vol. 6, 13-33
- **NORENBERG, M.D. ; BENDER, A.S.** Astrocyte swelling in liver failure: role of glutamine and benzodiazepines. *Acta Neurochir Suppl (Wien),* 1994, vol. 60, 24-7
- **NORENBERG, M.D. ; MARTINEZ-HERNANDEZ, A.** Fine structural localization of glutamine synthetase in astrocytes of rat brain. *Brain Res,* 1979, vol. 161, 303-10
- **NORENBERG, M.D. ; NEARY, J.T. ; BENDER, A.S. ; DOMBRO, R.S.** Hepatic encephalopathy: a disorder in glial-neuronal communication. *Prog Brain Res,* 1992, vol. 94, 261-9
- **NORENBERG, M.D. ; RAMA RAO, K.V. ; JAYAKUMAR, A.R.** Ammonia neurotoxicity and the mitochondrial permeability transition. *J Bioenerg Biomembr,* 2004, vol. 36, 303-7
- **OBOJSKA, A. ; BERLICKI, L. ; KAFARSKI, P. ; LEJCZAK, B. ; CHICCA, M. ; FORLANI, G.** Herbicidal pyridyl derivatives of aminomethylene-bisphosphonic acid inhibit plant glutamine synthetase. *J Agric Food Chem,* 2004, vol. 52, 3337-44
- **OBRENOVITCH, T.P. ; URENJAK, J.** Altered glutamatergic transmission in neurological disorders: from high extracellular glutamate to excessive synaptic efficacy. *Prog Neurobiol,* 1997, vol. 51, 39-87
- **OBRENOVITCH, T.P. ; URENJAK, J. ; ZILKHA, E. ; JAY, T.M.** Excitotoxicity in neurological disorders--the glutamate paradox. *Int J Dev Neurosci,* 2000, vol. 18, 281-7

- **OHTAKE, T. ; YASUDA, H. ; TAKAHASHI, H. ; GOTO, T. ; SUZUKI, K. ; YONEMURA, K. ; HISHIDA, A.** Decreased plasma and cerebrospinal fluid glutamine concentrations in a patient with bialaphos poisoning. *Hum Exp Toxicol,* 2001, vol. 20, 429-34
- **OLIVEIRA, I.C. ; BREARS, T. ; KNIGHT T.J. ; CLARK, A. ; CORUZZI, G.M.** Overexpression of cytosolic glutamine synthetase. Relation to nitrogen, light, and photorespiration. *Plant Physiology,* 2002, vol. 129, 1170-80
- **OLKKU, A. ; BODINE, P.V. ; LINNALA-KANKKUNEN, A. ; MAHONEN, A.** Glucocorticoids induce glutamine synthetase expression in human osteoblastic cells: a novel observation in bone. *Bone,* 2004, vol. 34, 320-9
- **OLNEY, J.W.** Excitotoxin-mediated neuron death in youth and old age. *Prog Brain Res,* 1990, vol. 86, 37-51
- **OLVERMAN, H.J. ; SHARMAN, D.F.** Dopamine in the blood platelets of the sheep. *Comp Biochem Physiol C,* 1983, vol. 75, 223-5
- **OMURA, S. ; MURATA, M. ; IMAMURA, N. ; IWAI, Y. ; TANAKA, H. ; FURUSAKI, A. ; MATSUMOTO, H.** Oxetin, a new antimetabolite from an actinomycete. Fermentation, isolation, structure and biological activity. *J Antibiot,* 1984, vol. 37, 1324-32
- **ORNSTEIN, P.L. ; BLEISCH, T.J. ; ARNOLD, M.B. ; KENNEDY, J.H. ; WRIGHT, R.A. ; JOHNSON, B.G. ; TIZZANO, J.P. ; HELTON, D.R. ; KALLMAN, M.J. ; SCHOEPP, D.D.** 2-substituted (2SR)-2-amino-2-((1SR,2SR)-2-carboxycycloprop-1-yl)glycines as potent and selective antagonists of group II metabotropic glutamate receptors. 2. Effects of aromatic substitution, pharmacological characterization, and bioavailability. *J Med Chem,* 1998, vol. 41, 358-78
- **ORREGO, F. ; VILLANUEVA, S.** The chemical nature of the main central excitatory transmitter: a critical appraisal based upon release studies and synaptic vesicle localization. *Neuroscience,* 1993, vol. 56, 539-55
- **ORZECHOWSKI , O.P. ; BRODNICKA A ; WILCZAK J ; JANK M ; BALASINSKA B ; GRZELKOWSKA K ; PLOSZAJ T ; OLCZAK J ; MROWCZYNSKA A.** Excess of glucocorticoids impairs whole-body antioxidant status in young rats. relation to the effect of dexamethasone in soleus muscle and spleen. *Horm Metab Res,* 2000, vol. 32, 174-80
- **PATEL, A.J. ; HUNT, A. ; FARAJI-SHADAN, F.** Effect of removal of glutamine and addition of dexamethasone on the activities of glutamine synthetase, ornithine decarboxylase and lactate dehydrogenase in primary cultures of forebrain and cerebellar astrocytes. *Brain Res,* 1986, vol. 391, 229-38
- **PATTON, A.J. ; GENEVER, P.G. ; BIRCH, M.A. ; SUVA, L.J. ; SKERRY, T.M.** Expression of an N-methyl-D-aspartate-type receptor by human and rat osteoblasts and osteoclasts suggests a novel glutamate signaling pathway in bone. *Bone,* 1998, vol. 22, 645-9
- **PAULSEN, R.E. ; ODDEN, E. ; FONNUM, F.** Importance of glutamine for gamma-aminobutyric acid synthesis in rat neostriatum in vivo. *J Neurochem,* 1988, vol. 51, 1294-9
- **PEET, N.M. ; GRABOWSKI, P.S. ; LAKETIC-LJUBOJEVIC, I. ; SKERRY, T.M.** The glutamate receptor antagonist MK801 modulates bone resorption in vitro by a mechanism predominantly involving osteoclast differentiation. *Faseb J,* 1999, vol. 13, 2179-85
- **PELLICCIARI, R. ; LUNEIA, R. ; COSTANTINO, G. ; MARINOZZI, M. ; NATALINI, B. ; JAKOBSEN, P. ; KANSTRUP, A. ; LOMBARDI, G. ; MORONI, F. ; THOMSEN, C.** 1-Aminoindan-1,5-dicarboxylic acid: a novel antagonist at phospholipase C-linked metabotropic glutamate receptors. *J Med Chem,* 1995, vol. 38, 3717-9
- **PITHON-CURI, T.C. ; SCHUMACHER, R.I. ; FREITAS, J.J. ; LAGRANHA, C. ; NEWSHOLME, P. ; PALANCH, A.C. ; DOI, S.Q. ; CURI, R.** Glutamine delays spontaneous apoptosis in neutrophils. *Am J Physiol Cell Physiol,* 2003, vol. 284, C 1355-61
- **PLAISANT, F. ; DOMMERGUES, M.A. ; SPEDDING, M. ; CECCHELLI, R. ; BRILLAULT, J. ; KATO, G. ; MUNOZ, C. ; GRESSENS, P.** Neuroprotective properties of tianeptine: interactions with cytokines. *Neuropharmacology,* 2003, vol. 44, 801-9
- **PLANAS, M. ; SCHWARTZ, S. ; ARBOS, M.A. ; FARRIOL, M.** Plasma glutamine levels in septic patients. *JPEN J Parenter Enteral Nutr,* 1993, vol. 17, 299-300
- **POTSIC, B. ; HOLLIDAY, N. ; LEWIS, P. ; SAMUELSON, D. ; DEMARCO, V. ; NEU, J.** Glutamine supplementation and deprivation: effect on artificially reared rat small intestinal morphology. *Pediatr Res,* 2002, vol. 52, 430-6
- **PUSHKIN, A.V. ; EVSTIGNEEVA, Z.G. ; KRETOVICH, V.L.** *Fiziol. Rast.,* 1974, vol. 21, 512-7
- **PUSHKIN, A.V. ; EVSTIGNEEVA, Z.G. ; KRETOVICH, V.L.** *Biokhimiya,* 1975, vol. 40, 53-6
- **RABINOVITZ, M. ; OLSON, M.E. ; GREENBERG, D.M.** 8-Hydroxylysine: an inhibitor of glutamine and protein synthesis by the Ehrlich ascites carcinoma cell. *Cancer Res,* 1957, vol. 17, 885-9
- **RAO, K.V. ; NORENBERG, M.D.** Manganese induces the mitochondrial permeability transition in cultured astrocytes. *J Biol Chem,* 2004, vol. 279, 32333-8

- **RAO, K.V. ; JAYAKUMAR, A.R. ; NORENBERG, M.D.** Induction of the mitochondrial permeability transition in cultured astrocytes by glutamine. *Neurochemistry International,* 2003, vol. 43, 517-23
- **RAO, S.L. ; MEISTER, A.** In vivo formation of methionine sulfoximine phosphate, a protein-bound metabolite of methionine sulfoximine. *Biochemistry,* 1972, vol. 11, 1123-7
- **RASTOGI, R. ; CHOUREY, P.S. ; MUHITCH, M.J.** The maize glutamine synthetase GS1-2 gene is preferentially expressed in kernel pedicels and is developmentally-regulated. *Plant Cell Physiol,* 1998, vol. 39, 443-6
- **REAGAN, L.P. ; ROSELL, D.R. ; WOOD, G.E. ; SPEDDING, M. ; MUNOZ, C. ; ROTHSTEIN, J. ; MCEWEN, B.S.** Chronic restraint stress up-regulates GLT-1 mRNA and protein expression in the rat hippocampus: reversal by tianeptine. *Proc Natl Acad Sci U S A,* 2004, vol. 101, 2179-84
- **REIS, H.J. ; MASSENSINI, A.R. ; PRADO, M.A. ; GOMEZ, R.S. ; GOMEZ, M.V. ; ROMANO-SILVA, M.A.** Calcium channels coupled to depolarization-evoked glutamate release in the myenteric plexus of guinea-pig ileum. *Neuroscience,* 2000, vol. 101, 237-42
- **REITER, R.J.** Oxidative processes and antioxidative defense mechanisms in the aging brain. *Faseb J,* 1995, vol. 9, 526-33
- **REITSMA, P.H. ; FRIJLINK, W.B. ; BIJVOET, O.L.** Dichloromethylene diphosphonate and calcium and phosphate metabolism. *N Engl J Med,* 1980, vol. 303, 162-3
- **ROH, J.H. ; SUZUKI, H. ; AZAKAMI, H. ; YAMASHITA, M. ; MUROOKA, Y. ; KUMAGAI H.** Purification, characterization, and crystallization of monoamine oxidase from Escherichia coli K-12. *Biosci Biotechnol Biochem,* 1994, vol. 58, 1652-6
- **RHOADS, J.M. ; ARGENZIO, R.A. ; CHEN, W. ; GOMEZ, G.G.** Asparagine stimulates piglet intestinal Cl-secretion by a mechanism requiring a submucosal glutamate receptor and nitric oxide. *J Pharmacol Exp Ther,* 1995, vol. 274, 404-12
- **RICHIE, J.P., JR. ; MILLS, B.J. ; LANG, C.A.** Correction of a glutathione deficiency in the aging mosquito increases its longevity. *Proc Soc Exp Biol Med,* 1987, vol. 184, 113-7
- **ROBINSON, M.K. ; AHN, M.S. ; ROUNDS, J.D. ; COOK, J.A. ; JACOBS, D.O. ; WILMORE, D.W.** Parenteral glutathione monoester enhances tissue antioxidant stores. *JPEN J Parenter Enteral Nutr,* 1992, vol. 16, 413-8
- **ROSS, B.D. ; DANIELSEN, E.R. ; BLUML, S.** Proton magnetic resonance spectroscopy: the new gold standard for diagnosis of clinical and subclinical hepatic encephalopathy?. *Dig Dis,* 1996, vol. 14 (1), 30-9
- **ROTH, E. ; OEHLER, R. ; MANHART, N. ; EXNER, R. ; WESSNER, B. ; STRASSER, E. ; SPITTLER, A.** Regulative potential of glutamine--relation to glutathione metabolism. *Nutrition,* 2002, vol. 18, 217-21
- **ROTHWELL, N. ; ALLAN, S. ; TOULMOND, S.** The role of interleukin 1 in acute neurodegeneration and stroke: pathophysiological and therapeutic implications. *J Clin Invest,* 1997, vol. 100, 2648-52
- **ROWE, W.B. ; RONZIO, R.A. ; MEISTER, A.** Inhibition of glutamine synthetase by methionine sulfoximine. Studies on methionine sulfoximine phosphate. *Biochemistry,* 1969, vol. 8, 2674-80
- **RUEPPEL, M.L. ; LUNDT, S.L. ; GASS, J.D. ; MEISTER, A.** Specific synthesis of 1-(5-glutamyl)-2-methylhydrazine by glutamine synthetase. *Biochemistry,* 1972, vol. 11, 2839-44
- **RUHLAND, M. ; ENGELHARDT, G. ; PAWLIZKI, K.** A comparative investigation of the metabolism of the herbicide glufosinate in cell cultures of transgenic glufosinate-resistant and non-transgenic oilseed rape (Brassica napus) and corn (Zea mays). *Environ Biosafety Res,* 2002, vol. 1, 29-37
- **SADUNISHVILI, T. ; GVARLIANI, N. ; NUTSUBIDZE, N. ; KVESITADZE, G.** Effect of methionine sulfoximine on nitrogen metabolism and externally supplied ammonium assimilation in kidney bean. *Ecotoxicol Environ Saf,* 1996, vol. 34, 70-5
- **SAID, S.I. ; BERISHA, H.I. ; PAKBAZ, H.** Excitotoxicity in the lung: N-methyl-D-aspartate-induced, nitric oxide-dependent, pulmonary edema is attenuated by vasoactive intestinal peptide and by inhibitors of poly(ADP-ribose) polymerase. *Proc Natl Acad Sci USA,* 1996, vol. 93, 4688-92
- **SAID, S. I. ; PAKBAZ, H. ; BERISHA, H. I. ; RAZA, S. ; FODA, H. D.** NMDA receptor activation: critical role in oxidant tissue injury. *Free Radic Biol Med,* 2000, vol. 28, 1300-2
- **SANCHEZ-MATEO, C.C. ; DARIAS, V. ; ALBERTOS, L.M. ; EXPOSITO-ORTA, M.A.** Psychopharmacological effects of tianeptine analogous hetero[2,1] benzothiazepine derivatives. *Arzneimittelforschung,* 2003, vol. 53, 12-20
- **SARIN, V.K. ; KENT, S.B. ; TAM, J.P. ; MERRIFIELD, R.B.** Quantitative monitoring of solid-phase peptide synthesis by the ninhydrin reaction. *Anal Biochem,* 1981, vol. 117, 147-57
- **SATO, K. ; KIYAMA, H. ; SHIMADA, S. ; TOHYAMA, M.** Gene expression of KA type and NMDA receptors and of a glycine transporter in the rat pineal gland. *Neuroendocrinology,* 1993, vol. 58, 77-9

- **SCHELTINGA, M.R. ; YOUNG, L.S. ; BENFELL, K. ; BYE, R.L. ; ZIEGLER, T.R. ; SANTOS, A.A. ; ANTIN, J.H. ; SCHLOERB, P.R. ; WILMORE, D.W.** Glutamine-enriched intravenous feedings attenuate extracellular fluid expansion after a standard stress. *Ann Surg,* 1991, vol. 214, 385-93
- **SCHEPPACH, W. ; BINGHAM, S. ; BOUTRON-RUAULT, M.C. ; GERHARDSSON DE VERDIER, M. ; MORENO, V. ; NAGENGAST, F.M. ; REIFEN, R. ; RIBOLI, E. ; SEITZ, H.K. ; WAHRENDORF, J.** WHO consensus statement on the role of nutrition in colorectal cancer. *Eur J Cancer Prev,* 1999, vol. 8, 57-62
- **SCHEPPACH, W. ; LOGES, C. ; BARTRAM, P. ; CHRISTL, S.U. ; RICHTER, F. ; DUSEL, G. ; STEHLE, P. ; FUERST, P. ; KASPER, H.** Effect of free glutamine and alanyl-glutamine dipeptide on mucosal proliferation of the human ileum and colon. *Gastroenterology,* 1994, vol. 107, 429-34
- **SCHOEPP, D.D. ; GOLDSWORTHY, J. ; JOHNSON, B.G. ; SALHOFF, C.R. ; BAKER, S.R.** 3,5-dihydroxyphenylglycine is a highly selective agonist for phosphoinositide-linked metabotropic glutamate receptors in the rat hippocampus. *J Neurochem,* 1994, vol. 63, 769-72
- **SEILER, N.** Is ammonia a pathogenetic factor in Alzheimer's disease?. *Neurochem Res,* 1993, vol. 18, 235-45
- **SEKIYAMA, N. ; HAYASHI, Y. ; NAKANISHI, S. ; JANE, D.E. ; TSE, H.W. ; BIRSE, E.F. ; WATKINS, J.C.** Structure-activity relationships of new agonists and antagonists of different metabotropic glutamate receptor subtypes. *Br J Pharmacol,* 1996, vol. 117, 1493-503
- **SHAKED, I. ; BEN-DROR, I. ; VARDIMON, L.** Glutamine synthetase enhances the clearance of extracellular glutamate by the neural retina. *J Neurochem,* 2002, vol. 83, 574-80
- **SHANNON, H.E. ; SAWYER, B.D.** Glutamate receptors of the N-methyl-D-aspartate subtype in the myenteric plexus of the guinea pig ileum. *J Pharmacol Exp Ther,* 1989, vol. 251, 518-23
- **SHAW, C.A. ; BAINS, J.S. ; PASQUALOTTO, B.A. ; CURRY, K.** Methionine sulfoximine shows excitotoxic actions in rat cortical slices. *Can J Physiol Pharmacol,* 1999, vol. 77, 871-7
- **SHELDRICK, W.F.** World Nitrogen Survey. 1987
- **SHELINE, Y.I. ; WANG, P.W. ; GADO, M.H. ; CSERNANSKY, J.G. ; VANNIER, M.W.** Hippocampal atrophy in recurrent major depression. *Proc Natl Acad Sci U S A,* 1996, vol. 93, 3908-13
- **SHIGEMOTO, R. ; OHISHI, H. ; NAKANISHI, S. ; MIZUNO, N.** Expression of the mRNA for the rat NMDA receptor (NMDAR1) in the sensory and autonomic ganglion neurons. *Neurosci Lett,* 1992, vol. 144, 229-32
- **SHINOZAKI, Y. ; TOBITA, T. ; MIZUTANI, M. ; MATSUZAKI, T.** Isolation and identification of two new diterpene glycosides from Nicotiana tabacum. *Biosci Biotechnol Biochem,* 1996, vol. 60, 903-5
- **SHOHAMI, E. ; GATI, I. ; BEIT-YANNAI, E. ; TREMBOVLER, V. ; KOHEN, R.** Closed head injury in the rat induces whole body oxidative stress: overall reducing antioxidant profile. *J Neurotrauma,* 1999, vol. 16, 365-76
- **SIBSON, N.R. ; DHANKHAR, A. ; MASON, G.F. ; BEHAR, K.L. ; ROTHMAN, D.L. ; SHULMAN, R.G.** In vivo 13C NMR measurements of cerebral glutamine synthesis as evidence for glutamate-glutamine cycling. *Proc Natl Acad Sci U S A,* 1997, vol. 94, 2699-704
- **SIGGINS, G.R. ; BERGER, T. ; FRENCH, E.D. ; SHIER, T. ; BLOOM, F.E.** Ethanol, salsolinol and tetrahydropapaveroline alter the discharge of neurons in several brain regions: comparison to opioid effects. *Prog Clin Biol Res,* 1982, vol. 90, 275-87
- **SINSKY, M. ; DONNERER, J.** Evidence for a neurotransmitter role of glutamate in guinea pig myenteric plexus neurons. *Neurosci Lett,* 1998, vol. 258, 109-12
- **SKERRY, T.M. ; GENEVER, P.G.** Glutamate signalling in non-neuronal tissues. *Trends Pharmacol Sci,* 2001, vol. 22, 174-81
- **SLUKA, K.A. ; JORDAN, H.H. ; WILLIS, W.D. ; WESTLUND, K.N.** Differential effects of N-methyl-D-aspartate (NMDA) and non-NMDA receptor antagonists on spinal release of amino acids after development of acute arthritis in rats. *Brain Res,* 1994, vol. 664, 77-84
- **SNINSKY, C. A. ; BROODERSON, R. J. ; BROOME, T. A. ; BERGERON, R.J.** *Gastroenterology,* 1994, vol. 106, A569
- **SPENCER, G.J. ; GENEVER, P.G.** Long-term potentiation in bone: a role for glutamate in strain-induced cellular memory?. *BMC Cell Biol,* 2003, vol. 4, 9
- **SPENCER, G.J. ; HITCHCOCK, I.S. ; GENEVER, P.G.** Emerging neuroskeletal signalling pathways: a review. *FEBS Lett,* 2004, vol. 559, 6-12
- **SPITTLER, A. ; SAUTNER, T. ; GORNIKIEWICZ, A. ; MANHART, N. ; OEHLER, R. ; BERGMANN, M. ; FUGGER, R. ; ROTH, E.** Postoperative glycyl-glutamine infusion reduces immunosuppression: partial prevention of the surgery induced decrease in HLA-DR expression on monocytes. *Clin Nutr,* 2001, vol. 20, 37-42
- **STITT, M. ; MULLER, C. ; MATT, P. ; GIBON, Y. ; CARILLO, P. ; MORCUENDE, R. ; SCHEIBLE, W.R. ; KRAPP, A.** Steps towards an integrated view of nitrogen metabolism. *J Exp Bot,* 2002, vol. 53, 959-70

- **STORTO, M. ; DE GRAZIA, U. ; BATTAGLIA, G. ; FELLI, M.P. ; MARODER, M. ; GULINO, A. ; RAGONA, G. ; NICOLETTI, F. ; SCREPANTI, I. ; FRATI, L.** Expression of metabotropic glutamate receptors in murine thymocytes and thymic stromal cells. *J Neuroimmunol,* 2000, vol. 109, 112-20
- **STORTO, M. ; DE GRAZIA, U. ; KNOPFEL, T. ; CANONICO, P.L. ; COPANI, A. ; RICHELMI, P. ; NICOLETTI, F. ; VAIRETTI, M.** Selective blockade of mGlu5 metabotropic glutamate receptors protects rat hepatocytes against hypoxic damage. *Hepatology,* 2000, vol. 31, 649-55
- **STORTO, M. ; SALLESE, M. ; SALVATORE, L. ; POULET, R. ; CONDORELLI, D.F. ; DELL'ALBANI, P. ; MARCELLO, M.F. ; ROMEO, R. ; PIOMBONI, P. ; BARONE, N.** Expression of metabotropic glutamate receptors in the rat and human testis. *FEBS Letters,* 1999, vol. 444, 160-4
- **STORTO, M. ; SALLESE, M. ; SALVATORE, L. ; POULET, R. ; CONDORELLI, D.F. ; DELL'ALBANI, P. ; MARCELLO, M.F. ; ROMEO, R. ; PIOMBONI, P. ; BARONE, N.** Expression of metabotropic glutamate receptors in the rat and human testis. *J Endocrinol,* 2001, vol. 170, 71-8
- **SUGIYAMA, T. ; SADZUKA, Y.** Enhancing effects of green tea components on the antitumor activity of adriamycin against M5076 ovarian sarcoma. *Cancer Lett,* 1998, vol. 133, 19-26
- **SWANSON, R.A. ; SHIRAISHI, K. ; MORTON, M.T. ; SHARP, F.R.** Methionine sulfoximine reduces cortical infarct size in rats after middle cerebral artery occlusion. *Stroke,* 1990, vol. 21, 322-7
- **TAKEUCHI, M. ; SAKAMOTO, S. ; KAWAMUKI, K. ; KURIHARA, H. ; NAKAHARA, H. ; ISOMURA, Y.** Studies on novel bone resorption inhibitors. II. Synthesis and pharmacological activities of fused aza-heteroarylbisphosphonate derivatives. *Chem Pharm Bull,* 1998, vol. 46, 1703-9
- **TAKAHASHI, H. ; KOEHLER, R.C. ; BRUSILOW, S.W. ; TRAYSTMAN, R.J.** Inhibition of brain glutamine accumulation prevents cerebral edema in hyperammonemic rats. *Am J Physiol,* 1991, vol. 261, H825-9
- **TAKAHASHI, H. ; KOEHLER, R.C. ; HIRATA, T. ; BRUSILOW, S.W. ; TRAYSTMAN, R.J.** Restoration of cerebrovascular CO2 responsivity by glutamine synthesis inhibition in hyperammonemic rats. *Circ Res,* 1992, vol. 71, 1220-30
- **TEITELBAUM, J.S. ; ZATORRE, R.J. ; CARPENTER, S. ; GENDRON, D. ; EVANS, A.C. ; GJEDDE, A. ; CASHMAN, N.R.** Neurologic sequelae of domoic acid intoxication due to the ingestion of contaminated mussels. *N Engl J Med,* 1990, vol. 322, 1781-7
- **THOMSEN, C. ; SUZDAK, P.D.** 4-Carboxy-3-hydroxyphenylglycine, an antagonist at type I metabotropic glutamate receptors. *Eur J Pharmacol,* 1993, vol. 245, 299-301
- **TOBIN, A.K. ; YAMAYA, T.** Cellular compartmentation of ammonium assimilation in rice and barley. *J Exp Bot,* 2001, vol. 52, 591-604
- **TONG, Q. ; MA, J. ; KIRCHGESSNER, A.L.** Vesicular glutamate transporter 2 in the brain-gut axis. *Neuroreport,* 2001, vol. 12, 3929-34
- **TONG, Q. ; OUEDRAOGO, R. ; KIRCHGESSNER, A.L.** Localization and function of group III metabotropic glutamate receptors in rat pancreatic islets. *Am J Physiol Endocrinol Metab,* 2002, vol. 282, E1324-33
- **TSACOPOULOS, M. ; POITRY-YAMATE, C.L. ; POITRY, S. ; PERROTTET, P. ; VEUTHEY, A.L.** The nutritive function of glia is regulated by signals released by neurons. *Glia,* 1997, vol. 21, 84-91
- **TUMANI, H. ; SMIRNOV, A. ; BARCHFELD, S. ; OLGEMOLLER, U. ; MAIER, K. ; LANGE, P. ; BRUCK, W. ; NAU, R.** Inhibition of glutamine synthetase in rabbit pneumococcal meningitis is associated with neuronal apoptosis in the dentate gyrus. *Glia,* 2000, vol. 30, 11-8
- **VARNEY, M.A. ; COSFORD, N.D. ; JACHEC, C. ; RAO, S.P. ; SACAAN, A. ; LIN, F.F. ; BLEICHER, L. ; SANTORI, E.M. ; FLOR, P.J. ; ALLGEIER, H.** SIB-1757 and SIB-1893: selective, noncompetitive antagonists of metabotropic glutamate receptor type 5. *J Pharmacol Exp Ther,* 1999, vol. 290, 170-81
- **VITOUSEK, P.W. ; ABER, J.D. ; HOWARTH, R.W. ; LIKENS, G.E. ; MATSON, P.A. ; SCHINDLER, D.W. ; SCHLESINGER, W.H. ; TILMAN, D.G.** Human alteration of the global nitrogen cycle: sources and consequences. *Ecol Appl,* 1997, vol. 7, 737-50
- **VOGELS, B.A. ; VAN STEYNEN, B. ; MAAS, M.A. ; JORNING, G.G. ; CHAMULEAU, R.A.** The effects of ammonia and portal-systemic shunting on brain metabolism, neurotransmission and intracranial hypertension in hyperammonaemia-induced encephalopathy. *J Hepatol,* 1997, vol. 26, 387-95
- **WADA, J.A. ; IKEDA, H. ; BERRY, K.** Reversible behavioral and electrographic manifestations induced by methionine sulfoximine. *Neurology,* 1967, vol. 17, 854-68
- **WALKER, D.M. ; MCDONALD, J.F. ; LOGUSCH E.W.** *J Chem Soc Chem Commun,* 1987, 1710-11
- **WATANABE, M. ; MISHINA, M. ; INOUE, Y.** Distinct gene expression of the N-methyl-D-aspartate receptor channel subunit in peripheral neurons of the mouse sensory ganglia and adrenal gland. *Neurosci Lett,* 1994, vol. 165, 183-6
- **WATANABE, Y. ; GOULD, E. ; DANIELS, D.C. ; CAMERON, H. ; MCEWEN, B.S.** Tianeptine attenuates stress-induced morphological changes in the hippocampus. *Eur J Pharmacol,* 1992, vol. 222, 157-62

- **WEAVER, C.D. ; YAO, T.L. ; POWERS, A.C. ; VERDOORN, T.A.** Differential expression of glutamate receptor subtypes in rat pancreatic islets. *J Biol Chem,* 1996, vol. 271, 12977-84
- **WEAVER, C.D. ; PARTRIDGE, J.G. ; YAO, T.L. ; MOATES, M. ; MAGNUSON, M.A. ; VERDOORN, T.A.** Activation of glycine and glutamate receptors increases intracellular calcium in cells derived from the endocrine pancreas. *Mol Pharmacol,* 1998, vol. 54, 639-46
- **WEDLER, F.C. ; HORN, B.R. ; ROBY, W.G.** Interaction of a new gamma-glutamyl-phosphate analog, 4-(phosphonoacetyl)-L-alpha-aminobutyrate, with glutamine synthetase enzymes from Escherichia coli, plant, and mammalian sources. *Arch Biochem Biophys,* 1980, vol. 202, 482-90
- **WEISS, M.D. ; DEMARCO, V. ; STRAUSS, D.M. ; SAMUELSON, D.A. ; LANE, M.E. ; NEU, J.** Glutamine synthetase: a key enzyme for intestinal epithelial differentiation?. *JPEN J Parenter Enteral Nutr,* 1999, vol. 23, 140-6
- **WELBOURNE, T.C. ; DASS, P.D.** Function of renal gamma-glutamyltransferase: significance of glutathione and glutamine interactions. *Life Sci,* 1982, vol. 30, 793-801
- **WERMUTH, C.G. ; MANN, A. ; SCHOENFELDER, A. ; WRIGHT, R.A. ; JOHNSON, B.G. ; BURNETT, J.P. ; MAYNE, N.G. ; SCHOEPP, D.D.** 2S,4S)-2-amino-4-(4,4-diphenylbut-1-yl)- pentane-1,5-dioic acid: a potent and selective antagonist for metabotropic glutamate receptors negatively linked to adenylate cyclase. *J Med Chem,* 1996, vol. 39, 814-6
- **WHITE, F.P. ; DUTTON, G.R. ; NORENBERG, M.D.** Microvessels isolated from rat brain: localization of astrocyte processes by immunohistochemical techniques. *J Neurochem,* 1981, vol. 36, 328-32
- **WILEY, J.W. ; LU, Y.X. ; OWYANG, C.** Evidence for a glutamatergic neural pathway in the myenteric plexus. *Am JPhysiol,* 1991, vol. 261, G693-700
- **WILLARD-MACK, C.L. ; KOEHLER, R.C. ; HIRATA, T. ; CORK, L.C. ; TAKAHASHI, H. ; TRAYSTMAN, R.J. ; BRUSILOW, S.W.** Inhibition of glutamine synthetase reduces ammonia-induced astrocyte swelling in rat. *Neuroscience,* 1996, vol. 71, 589-99
- **WILMORE, D.W.** The effect of glutamine supplementation in patients following elective surgery and accidental injury. *J Nutr,* 2001, vol. 131, 2543S-9S
- **WIREN, M. ; MAGNUSSON, K.E. ; LARSSON, J.** The role of glutamine, serum and energy factors in growth of enterocyte-like cell lines. *Int J Biochem Cell Biol,* 1998, vol. 30, 1331-6
- **WRONSKI, T.J. ; YEN, C.F. ; SCOTT, K.S.** Estrogen and diphosphonate treatment provide long-term protection against osteopenia in ovariectomized rats. *J Bone Miner Res,* 1991, vol. 6, 387-94
- **YAMADA, H. ; YATSUSHIRO, S. ; ISHIO, S. ; HAYASHI, M. ; NISHI, T. ; YAMAMOTO, A. ; FUTAI, M. ; YAMAGUCHI, A. ; MORIYAMA, Y.** Metabotropic glutamate receptors negatively regulate melatonin synthesis in rat pinealocytes. *J Neurosci,* 1998, vol. 18, 2056-62
- **YATSUSHIRO, S. ; YAMADA, H. ; HAYASHI, M. ; YAMAMOTO, A. ; MORIYAMA, Y.** Ionotropic glutamate receptors trigger microvesicle-mediated exocytosis of L-glutamate in rat pinealocytes. *J Neurochem,* 2000, vol. 75, 288-97
- **YONEDA, Y. ; OGITA, K.** Localization of [3H]glutamate binding sites in rat adrenal medulla. *Brain Res,* 1986, vol. 383, 387-91
- **YOSHIDA, Y. ; HIGASHI, T. ; NOUSO, K. ; NAKATSUKASA, H. ; NAKAMURA, S.I. ; WATANABE, A. ; TSUJI, T.** Effects of zinc deficiency/zinc supplementation on ammonia metabolism in patients with decompensated liver cirrhosis. *Acta Med Okayama,* 2001, vol. 55, 349-55
- **YOUNG, V.R. ; AJAMI, A.M.** Glutamine: the emperor or his clothes?. *J Nutr,* 2001, vol. 131, 2449S-59S
- **ZALKIN, H. ; SMITH, J.L.** Enzymes utilizing glutamine as an amide donor. *Adv Enzymol Relat Areas Mol Biol,* 1998, vol. 72, 87-144
- **ZEEVALK, G.D. ; HYNDMAN, A.G. ; NICKLAS, W.J.** Excitatory amino acid-induced toxicity in chick retina: amino acid release, histology, and effects of chloride channel blockers. *JNeurochem,* 1989, vol. 53, 1610-9
- **ZEEVALK, G.D. ; NICKLAS, W.J.** Chemically induced hypoglycemia and anoxia: relationship to glutamate receptor-mediated toxicity in retina. *J Pharmacol Exp Ther,* 1990, vol. 253, 1285-92
- **ZELLER, E.A. ; BARSKY, J.** In vivo inhibition of liver and brain monoamine oxidase by 1-Isonicotinyl-2-isopropyl hydrazine. *Proc Soc Exp Biol Med,* 1952, vol. 81, 459-61
- **ZHLOBA, A.F. ; RABOTNOVA, V.F. ; KIRILLOVA, M.S.** On the quantitative determination of ammonia in biological fluids under clinical conditions]. *Lab Delo,* 1968, vol. 10, 621-2
- **ZIEGLER, T.R. ; BAZARGAN, N. ; LEADER, L.M. ; MARTINDALE, R.G.** Glutamine and the gastrointestinal tract. *Curr Opin Clin Nutr Metab Care,* 2000, vol. 3, 355-62
- **ZIEGLER, T.R. ; EVANS, M.E. ; FERNANDEZ-ESTIVARIZ, C. ; JONES, D.P.** Trophic and cytoprotective nutrition for intestinal adaptation, mucosal repair, and barrier function. *Annu Rev Nutr,* 2003, vol. 23, 229-61
- **ZWINGMANN, C. ; BRAND, A. ; RICHTER-LANDSBERG, C. ; LEIBFRITZ, D.** Multinuclear NMR spectroscopy studies on NH4Cl-induced metabolic alterations and detoxification processes in primary astrocytes and glioma cells. *Dev Neurosci,* 1998, vol. 20, 417-26

- Use of NMR spectroscopy for the study of ammonia metabolism in astrocytes and neurons: Role of glutamine synthesis in astrocytes. **ZWINGMANN, C. ; SHOKATI, T. ; BUTTERWORTH, R.F. ; LEIBFRITZ D.** Encephalopathy and Nitrogen Metabolism in Liver Failure. Kluwer, 2003, 299-311
- **ZWINGMANN, C. ; CHATAURET., N. ; LEIBFRITZ, D. ; BUTTERWORTH, R.F.** Selective increase of brain lactate synthesis in experimental acute liver failure: results of a [1H-13C] nuclear magnetic resonance study. *Hepatology,* 2003, vol. 37, 420-28

SEQUENCE LISTING

```
<110>  NewThera

<120>  NOVEL USES FOR DRUGS TARGETING GLUTAMINE SYNTHETASE

<130>  B0265EP

<160>  1

<170>  PatentIn version 3.1

<210>  1
<211>  24
<212>  PRT
<213>  artificial sequence

<220>
<223>  Fragment from GS
<400>  1

Ile Thr Gly Thr Asn Ala Glu Val Met Pro Ala Gln Trp Glu Phe Gln
1               5                   10                  15

Ile Gly Pro Cys Glu Gly Ile Arg
            20
```